(19) Europäisches Patentamt / European Patent Office / Office européen des brevets

(11) EP 4 644 373 A1

(12) **EUROPEAN PATENT APPLICATION**
published in accordance with Art. 153(4) EPC

(43) Date of publication:
**05.11.2025 Bulletin 2025/45**

(21) Application number: **23912115.5**

(22) Date of filing: **26.12.2023**

(51) International Patent Classification (IPC):
***C07D 213/65*** (2006.01) ***A01N 43/40*** (2006.01)
***A01N 43/54*** (2006.01) ***A01N 43/58*** (2006.01)
***A01N 47/24*** (2006.01) ***A01P 3/00*** (2006.01)
***C07D 213/81*** (2006.01) ***C07D 213/84*** (2006.01)
***C07D 213/86*** (2006.01) ***C07D 231/18*** (2006.01)
***C07D 231/20*** (2006.01) ***C07D 237/24*** (2006.01)
***C07D 237/26*** (2006.01) ***C07D 237/28*** (2006.01)
***C07D 239/34*** (2006.01) ***C07D 249/04*** (2006.01)
***C07D 253/07*** (2006.01) ***C07D 401/12*** (2006.01)
***C07D 403/12*** (2006.01) ***C07D 405/12*** (2006.01)
***C07D 409/12*** (2006.01)

(52) Cooperative Patent Classification (CPC):
**A01N 43/40; A01N 43/54; A01N 43/58; A01N 47/24; A01P 3/00; C07D 213/65; C07D 213/81; C07D 213/84; C07D 213/86; C07D 231/18; C07D 231/20; C07D 237/24; C07D 237/26; C07D 237/28; C07D 239/34;** (Cont.)

(86) International application number:
**PCT/JP2023/046585**

(87) International publication number:
**WO 2024/143336 (04.07.2024 Gazette 2024/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.12.2022 JP 2022212728**
**20.04.2023 JP 2023069619**
**10.08.2023 JP 2023131664**

(71) Applicant: **Nippon Soda Co., Ltd.**
**Tokyo 100-7010 (JP)**

(72) Inventors:
- **SUZUKI Hiroto**
  **Odawara-shi, Kanagawa 250-0280 (JP)**
- **NAKAMURA Yuka**
  **Odawara-shi, Kanagawa 250-0280 (JP)**
- **NISHIKI Haruka**
  **Odawara-shi, Kanagawa 250-0280 (JP)**
- **KOBAYASHI Hiroyuki**
  **Odawara-shi, Kanagawa 250-0280 (JP)**
- **SAIGA Tomoyuki**
  **Odawara-shi, Kanagawa 250-0280 (JP)**
- **KIM JaeHyun**
  **Odawara-shi, Kanagawa 250-0280 (JP)**
- **SHIBAYAMA Kotaro**
  **Odawara-shi, Kanagawa 250-0280 (JP)**

(74) Representative: **Sonnenhauser, Thomas Martin**
**Wuesthoff & Wuesthoff**
**Patentanwälte und Rechtsanwalt PartG mbB**
**Schweigerstraße 2**
**81541 München (DE)**

# (54) HYDRAZIDE COMPOUND AND AGRICULTURAL/HORTICULTURAL BACTERICIDAL AGENT

(57) In the present invention, a compound represent by formula Z-I has excellent bactericidal activity and has excellent stability. (In the formula: $Q^1$ represents a phenyl group, or the like; $Q^2$ represents a phenyl group, or the like; partial structure Het represents a six-membered heteroaryl ring, or the like; $Z^a$ represents an oxygen atom, or the like; T represents a partial structure (T-1) or (T-2); Z represents an oxygen atom, or the like; $R^6$ represent a hydrogen atom, a C1-6 alkyl group, or the like; $Z^b$ represents a C1-6 alkoxy group, or the like; $R^4$ represents a hydrogen atom, or the like; $R^5$ represents a hydrogen atom, or the like; n represents any integer from 1 to 3; and

$R^7$ represents a hydrogen atom, a C1-6 alkyl group, or the like.)

[Chemical formula 1]

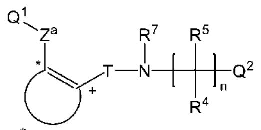

(Z-1)

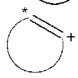

(Het)

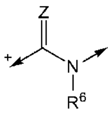

(T-1)

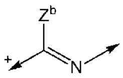

(T-2)

(52) Cooperative Patent Classification (CPC): (Cont.)
**C07D 249/04; C07D 253/07; C07D 401/12; C07D 403/12; C07D 405/12; C07D 409/12; C07D 487/04**

## Description

TECHNICAL FIELD

The present invention relates to a hydrazide compound and an agricultural and horticultural fungicide. The present invention more specifically relates to a hydrazide compound that exhibits excellent fungicidal activity against pathogens that affect agricultural and horticultural plants, has excellent safety, and can be synthesized in an industrially advantageous manner, as well as an agricultural and horticultural fungicide containing the same as an active ingredient.

Priority is claimed on Japanese Patent Application No. 2022-212728, filed December 28, 2022, Japanese Patent Application No. 2023-069619, filed April 20, 2023, and Japanese Patent Application No. 2023-131664, filed August 10, 2023, the content of which is incorporated herein by reference.

BACKGROUND ART

Various compounds that exhibit fungicidal activity against pathogens that affect agricultural and horticultural plants have been proposed. Not only sufficiently high efficacy, but also difficulty in causing drug resistance, no chemical damage to plants or soil pollution, and low toxicity to livestock and fish are required, so as to make such compounds usable practically as agricultural and horticultural fungicides.

Incidentally, Patent Document 1 discloses compounds of formula (A) and the like.

[Chemical Formula 1]

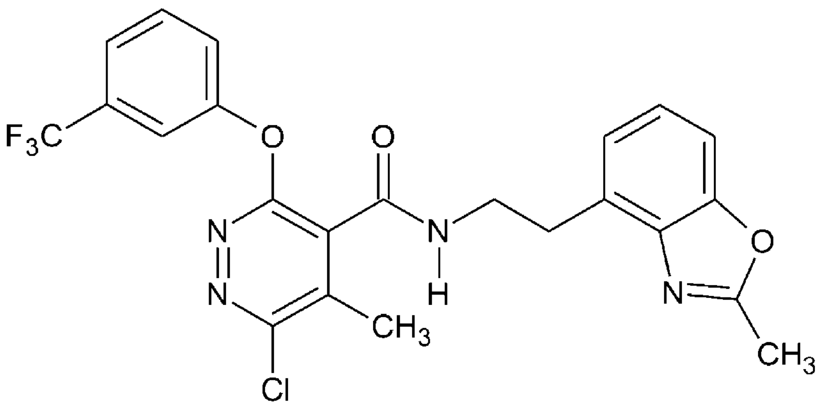

(A)

Citation List

Patent Document

Patent Document 1: Japanese Unexamined Patent Application Publication (Translation of PCT Application) No. 2022-508277

SUMMARY OF INVENTION

Technical Problem

An object of the present invention is to provide a hydrazide compound that exhibits excellent fungicidal activity against pathogens that affect agricultural and horticultural plants, has excellent safety, and can be synthesized in an industrially advantageous manner, as well as an agricultural and horticultural fungicide containing the same as an active ingredient.

Solution to Problem

As a result of intensive research to solve the above-mentioned problems, the present invention encompassing the following aspects has been completed.

(1) A compound of formula (Z-1) or a salt thereof.

[Chemical Formula 2]

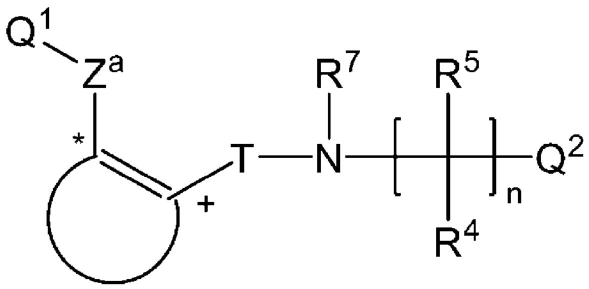

(Z − I)

(In the formula (Z-1),

$Q^1$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group,
$Q^2$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group,
a carbon atom that constitutes a ring in $Q^2$ may bond, instead of $R^7$, to a nitrogen atom having $R^7$,
a partial structure Het:

[Chemical Formula 3]

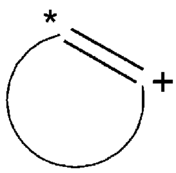

(Het)

is a substituted or unsubstituted six-membered heteroaryl ring, a substituted or unsubstituted six-membered nitrogen-containing hetero ring having an oxo group, or a substituted or unsubstituted nine- or ten-membered heteroaryl ring,
carbon atoms marked with * and + in the partial structure correspond to carbon atoms marked with * and + in the formula (Z-1),
$Z^a$ is an oxygen atom, a sulfur atom, or a group of formula: $NR^a$,
$R^a$ is a hydrogen atom, or a substituted or unsubstituted C1-6 alkyl group,
T is a partial structure (T-1) or a partial structure (T-2),

[Chemical Formula 4]

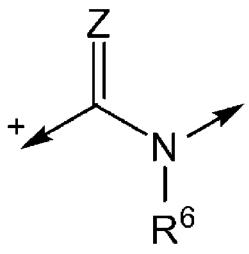

(T－1)

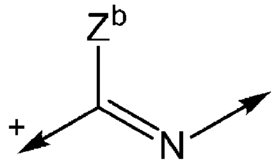

(T－2)

in the partial structure (T-1), Z is an oxygen atom, a sulfur atom, or a group of formula: $NR^b$, an arrow marked with + is bonded to a carbon atom marked with + in the partial structure, the other arrow bonded to a nitrogen atom having $R^7$,

$R^b$ is a hydrogen atom, a substituted or unsubstituted C1-6 alkyl group, a hydroxy group, or a substituted or unsubstituted C1-6 alkoxy group,

$R^6$ is a hydrogen atom, a substituted or unsubstituted C1-6 alkyl group, a substituted or unsubstituted C2-6 alkenyl group, a substituted or unsubstituted C2-6 alkynyl group, a formyl group, a substituted or unsubstituted C1-6 alkylcarbonyl group, a substituted or unsubstituted C1-6 alkoxycarbonyl group, a substituted or unsubstituted C3-6 cycloalkyl group, a substituted or unsubstituted phenyl group, a substituted or unsubstituted naphthyl group, or a substituted or unsubstituted five- or six-membered heterocyclyl group,

in the partial structure (T-2), $Z^b$ is a C1-6 alkoxy group, or C1-6 alkylthio group,

$R^4$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a halogeno group,

$R^5$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a halogeno group,

$R^4$ and $R^5$ on an identical carbon atom may form together a divalent organic group,

n is an integer of 1 to 3,

R7 is a hydrogen atom, a substituted or unsubstituted C1-6 alkyl group, a substituted or unsubstituted C2-6 alkenyl group, a substituted or unsubstituted C2-6 alkynyl group, a formyl group, a group of formula: $R^c$-CO-, a group of formula: $R^c$-CS-, a group of formula: $R^c$-CO-CO-, a group of formula: $R^c$-$SO_2$-, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a cyano group,

$R^c$ is each independently a substituted or unsubstituted C1-6 alkyl group, a substituted or unsubstituted C1-6 alkoxy group, a substituted or unsubstituted C3-6 cycloalkoxy group, a substituted or unsubstituted C1-6 alkylthio group, a substituted or unsubstituted amino group, a substituted or unsubstituted C3-6 cycloalkyl group, a substituted or unsubstituted phenyl group, a substituted or unsubstituted naphthyl group, a substituted or unsubstituted three- to six-membered heterocyclyl group, or a substituted or unsubstituted three- to six-membered heterocyclyloxy group.)

(2) The compound or the salt thereof according to (1) mentioned above, wherein the formula (Z-1) is formula (I).

[Chemical Formula 5]

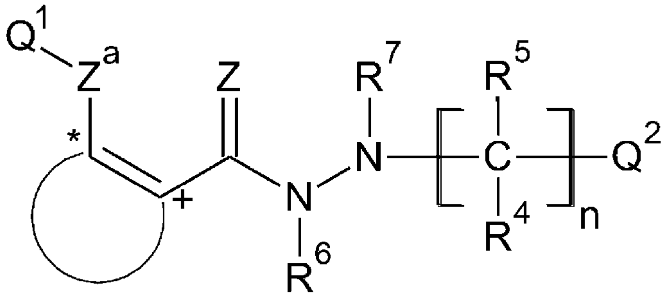

(I)

(In the formula (I),

$Q^1$, $Q^2$, $Z^a$, $R^4$, $R^5$, $R^7$ and n are identical to $Q^1$, $Q^2$, $Z^a$, $R^4$, $R^5$, $R^7$ and n in the formula (Z-1),
Z and $R^6$ are identical to Z and $R^6$ in the partial structure (T-1).)

(3) The compound or the salt thereof according to (1) or (2) mentioned above, wherein the partial structure Het:

[Chemical Formula 6]

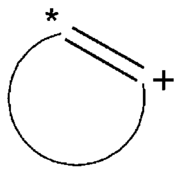

(Het)

is a six-membered heteroaryl ring of formula (II),

[Chemical Formula 7]

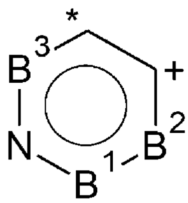

(II)

(in the formula (II),

$B^1$ is a nitrogen atom, or a group of formula: $CR^1$,
$R^1$ is a hydrogen atom, a substituted or unsubstituted C1-6 alkyl group, a substituted or unsubstituted C2-6 alkenyl group, a substituted or unsubstituted C2-6 alkynyl group, a hydroxy group, a substituted or unsubstituted C1-6 alkoxy group, a substituted or unsubstituted C1-6 alkylcarbonyl group, a substituted or unsubstituted C1-6 alkoxycarbonyl group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, a substituted or unsubstituted phenyloxy group, a substituted or unsubstituted naphthyloxy group, a substituted or unsubstituted five- or six-membered heterocyclyloxy group, a substituted or unsubstituted amino group, a substituted or unsubstituted aminocarbonyl group, an N-(C1-6 alkoxy)imino group, a nitro group, a cyano group, or a halogeno group,
$B^2$ is a nitrogen atom, or a group of formula: $CR^2$,
$R^2$ is a hydrogen atom, a substituted or unsubstituted C1-6 alkyl group, a substituted or unsubstituted C2-6 alkenyl group, a substituted or unsubstituted C2-6 alkynyl group, a hydroxy group, a substituted or unsubstituted

C1-6 alkoxy group, a substituted or unsubstituted C1-6 alkylcarbonyl group, a substituted or unsubstituted C1-6 alkoxycarbonyl group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, a substituted or unsubstituted phenyloxy group, a substituted or unsubstituted naphthyloxy group, a substituted or unsubstituted five- or six-membered heterocyclyloxy group, a substituted or unsubstituted amino group, a substituted or unsubstituted aminocarbonyl group, an N-(C1-6 alkoxy)imino group, a nitro group, a cyano group, or a halogeno group,

$R^1$ and $R^2$ may form together a divalent organic group,

$B^3$ is a nitrogen atom, or a group of formula: $CR^3$,

$R^3$ is a hydrogen atom, a substituted or unsubstituted C1-6 alkyl group, a substituted or unsubstituted C2-6 alkenyl group, a substituted or unsubstituted C2-6 alkynyl group, a hydroxy group, a substituted or unsubstituted C1-6 alkoxy group, a substituted or unsubstituted C1-6 alkylcarbonyl group, a substituted or unsubstituted C1-6 alkoxycarbonyl group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, a substituted or unsubstituted phenyloxy group, a substituted or unsubstituted naphthyloxy group, a substituted or unsubstituted five- or six-membered heterocyclyloxy group, a substituted or unsubstituted amino group, a substituted or unsubstituted aminocarbonyl group, a nitro group, a cyano group, or a halogeno group, and

carbon atoms marked with * and + in the partial structure correspond to carbon atoms marked with * and + in the formula (Z-1).)

(4) The compound or the salt thereof according to (3) mentioned above, wherein

$Z^a$ is an oxygen atom,

Z is an oxygen atom,

$R^6$ is a hydrogen atom, and

$R^7$ is a formyl group, a group of formula: $R^c$-CO-, a group of formula: $R^c$-CS-, a group of formula: $R^c$-CO-CO-, or a group of formula: $R^c$-$SO_2$-.

(5) An agricultural and horticultural fungicide containing at least one selected from the group consisting of the compound and the salt thereof of any one of (1) to (4), as an active ingredient.

(6) A method for producing a compound of formula (I-1), the method including a step in which a compound of formula (im-1) and a compound of formula (im-2) are reacted in the presence of an acid scavenger.

[Chemical Formula 8]

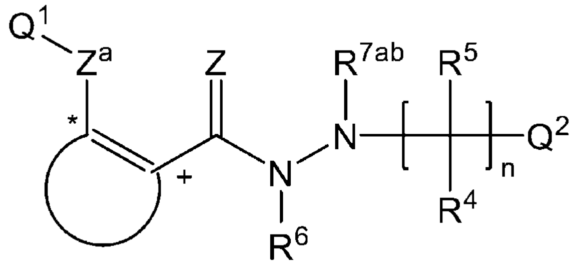

(I-1)

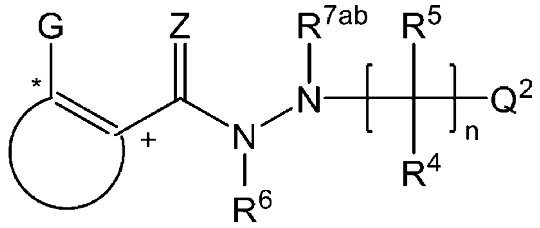

(im-1)

$Q^1-Z^a-H$ (im-2)

(in each formula shown above,

$Q^1$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group,

$Q^2$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group,

a carbon atom that constitutes a ring in $Q^2$ may bond, instead of $R^{7ab}$, to a nitrogen atom having $R^{7ab}$,

the partial structure Het:

[Chemical Formula 9]

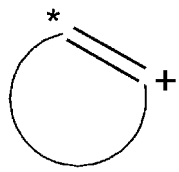

(Het)

is a substituted or unsubstituted six-membered heteroaryl ring, a substituted or unsubstituted six-membered nitrogen-containing hetero ring having an oxo group, or a substituted or unsubstituted nine- or ten-membered heteroaryl ring,

carbon atoms marked with * and + in the partial structure correspond to carbon atoms marked with * and + in the formula (Z-1),

$Z^a$ is an oxygen atom, a sulfur atom, or a group of formula: $NR^a$,

$R^a$ is a hydrogen atom, or a substituted or unsubstituted C1-6 alkyl group,

Z is an oxygen atom, a sulfur atom, or a group of formula: $NR^b$,

$R^b$ is a hydrogen atom, a substituted or unsubstituted C1-6 alkyl group, a hydroxy group, or a substituted or unsubstituted C1-6 alkoxy group,

$R^4$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a halogeno group,

$R^5$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a halogeno group,
$R^4$ and $R^5$ on an identical carbon atom may form together a divalent organic group,
n is an integer of 1 to 3,
$R^6$ is a hydrogen atom, a substituted or unsubstituted C1-6 alkyl group, a substituted or unsubstituted C2-6 alkenyl group, a substituted or unsubstituted C2-6 alkynyl group, a formyl group, a substituted or unsubstituted C1-6 alkylcarbonyl group, a substituted or unsubstituted C1-6 alkoxycarbonyl group, a substituted or unsubstituted C3-6 cycloalkyl group, a substituted or unsubstituted phenyl group, a substituted or unsubstituted naphthyl group, or a substituted or unsubstituted five- or six-membered heterocyclyl group,
$R^{7ab}$ is a substituted or unsubstituted C1-6 alkyl group, a substituted or unsubstituted C2-6 alkenyl group, a substituted or unsubstituted C2-6 alkynyl group, a formyl group, a group of formula: $R^c$-CO-, a group of formula: $R^c$-CS-, a group of formula: $R^c$-CO-CO-, a group of formula: $R^c$-$SO_2$-, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a cyano group,
$R^c$ is each independently a substituted or unsubstituted C1-6 alkyl group, a substituted or unsubstituted C1-6 alkoxy group, a substituted or unsubstituted C3-6 cycloalkoxy group, a substituted or unsubstituted C1-6 alkylthio group, a substituted or unsubstituted amino group, a substituted or unsubstituted C3-6 cycloalkyl group, a substituted or unsubstituted phenyl group, a substituted or unsubstituted naphthyl group, a substituted or unsubstituted three- to six-membered heterocyclyl group, or a substituted or unsubstituted three- to six-membered heterocyclyloxy group. and
G is a halogeno group.)

(7) A method for producing a compound of formula (I-3), the method containing a step in which a compound of formula (im-5) and a compound of formula (im-6) are reacted in the presence of a condensing agent when $U^1$ in the formula (im-5) is a hydroxy group, or in the presence of an acid scavenger when $U^1$ is a halogeno group.

[Chemical Formula 10]

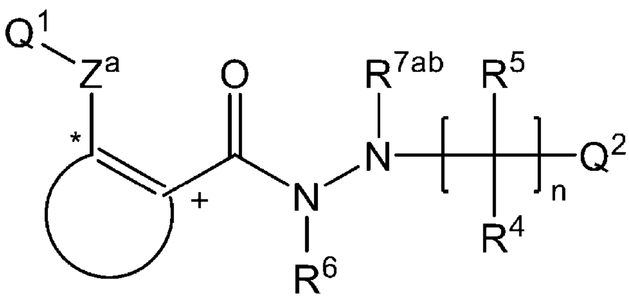

(I－3)

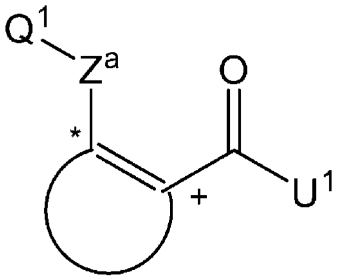

(ｉｍ－５)

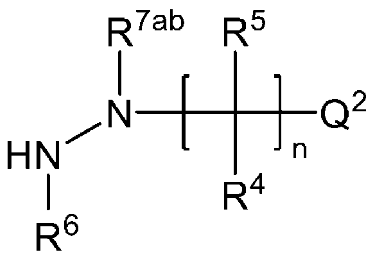

(ｉｍ－６)

(In each formula shown above,

$Q^1$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group,
$Q^2$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group,
a carbon atom that constitutes a ring in $Q^2$ may bond, instead of $R^{7ab}$, to a nitrogen atom having $R^{7ab}$,
the partial structure Het:

[Chemical Formula 11]

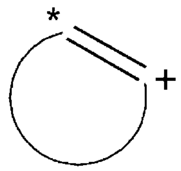

(Het)

is a substituted or unsubstituted six-membered heteroaryl ring, a substituted or unsubstituted six-membered nitrogen-containing hetero ring having an oxo group, or a substituted or unsubstituted nine- or ten-membered heteroaryl ring,
carbon atoms marked with * and + in the partial structure correspond to carbon atoms marked with * and + in the formula (Z-1),
$Z^a$ is an oxygen atom, a sulfur atom, or a group of formula: $NR^a$,
$R^a$ is a hydrogen atom, or a substituted or unsubstituted C1-6 alkyl group,
$R^b$ is a hydrogen atom, a substituted or unsubstituted C1-6 alkyl group, a hydroxy group, or a substituted or unsubstituted C1-6 alkoxy group,
$R^4$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a halogeno group,
$R^5$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a halogeno group,
$R^4$ and $R^5$ on an identical carbon atom may form together a divalent organic group,
n is an integer of 1 to 3,
$R^6$ is a hydrogen atom, a substituted or unsubstituted C1-6 alkyl group, a substituted or unsubstituted C2-6 alkenyl group, a substituted or unsubstituted C2-6 alkynyl group, a formyl group, a substituted or unsubstituted C1-6 alkylcarbonyl group, a substituted or unsubstituted C1-6 alkoxycarbonyl group, a substituted or unsubstituted C3-6 cycloalkyl group, a substituted or unsubstituted phenyl group, a substituted or unsubstituted naphthyl group, or a substituted or unsubstituted five- or six-membered heterocyclyl group,
$R^{7ab}$ is a substituted or unsubstituted C1-6 alkyl group, a substituted or unsubstituted C2-6 alkenyl group, a substituted or unsubstituted C2-6 alkynyl group, a formyl group, a group of formula: $R^c$-CO-, a group of formula: $R^c$-CS-, a group of formula: $R^c$-CO-CO-, a group of formula: $R^c$-$SO_2$-, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a cyano group,
$R^c$ is each independently a substituted or unsubstituted C1-6 alkyl group, a substituted or unsubstituted C1-6 alkoxy group, a substituted or unsubstituted C3-6 cycloalkoxy group, a substituted or unsubstituted C1-6 alkylthio group, a substituted or unsubstituted amino group, a substituted or unsubstituted C3-6 cycloalkyl group, a substituted or unsubstituted phenyl group, a substituted or unsubstituted naphthyl group, a substituted or unsubstituted three- to six-membered heterocyclyl group, or a substituted or unsubstituted three- to six-membered heterocyclyloxy group, and
$U^1$ is a hydroxy group or a halogeno group.)

(8) A method for producing a compound of formula (I-A1), the method including a step in which a compound of formula (im-A1) and a compound of formula (im-A2) are reacted in the presence of an acid scavenger.

[Chemical Formula 12]

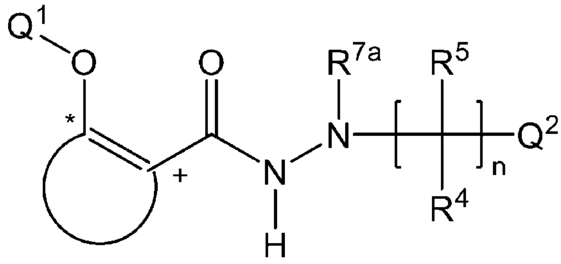

(I−A1)

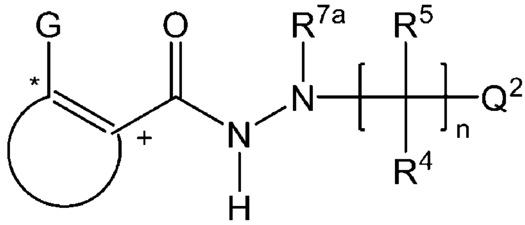

(im−A1)

$Q^1$—O—H (im−A2)

(in each formula shown above,

$Q^1$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group,

$Q^2$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group,

a carbon atom that constitutes a ring in $Q^2$ may bond, instead of $R^{7a}$, to a nitrogen atom having $R^{7a}$,

the partial structure Het:

[Chemical Formula 13]

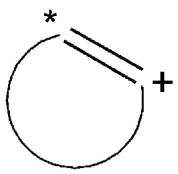

(Het)

is a substituted or unsubstituted six-membered heteroaryl ring, a substituted or unsubstituted six-membered nitrogen-containing hetero ring having an oxo group, or a substituted or unsubstituted nine- or ten-membered heteroaryl ring,

carbon atoms marked with * and + in the partial structure correspond to carbon atoms marked with * and + in the formula (Z-1),

$R^4$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a halogeno group,

$R^5$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a halogeno group,

$R^4$ and $R^5$ on an identical carbon atom may form together a divalent organic group,

n is an integer of 1 to 3,

$R^{7a}$ is a formyl group, a group of formula: $R^c$-CO-, a group of formula: $R^c$-CS-, a group of formula: $R^c$-CO-CO-, or a group of formula: $R^c$-$SO_2$-,
$R^c$ is each independently a substituted or unsubstituted C1-6 alkyl group, a substituted or unsubstituted C1-6 alkoxy group, a substituted or unsubstituted C3-6 cycloalkoxy group, a substituted or unsubstituted C1-6 alkylthio group, a substituted or unsubstituted amino group, a substituted or unsubstituted C3-6 cycloalkyl group, a substituted or unsubstituted phenyl group, a substituted or unsubstituted naphthyl group, a substituted or unsubstituted three- to six-membered heterocyclyl group, or a substituted or unsubstituted three- to six-membered heterocyclyloxy group,
G is a halogeno group.)

(9) A method for producing a compound of formula (I-A1), the method including: a step in which a compound of formula (im-A5) and a compound of formula (im-6a) are reacted in the presence of a condensing agent when $U^1$ in the formula (im-A5) is a hydroxy group, or in the presence of an acid scavenger when $U^1$ is a halogeno group.

[Chemical Formula 14]

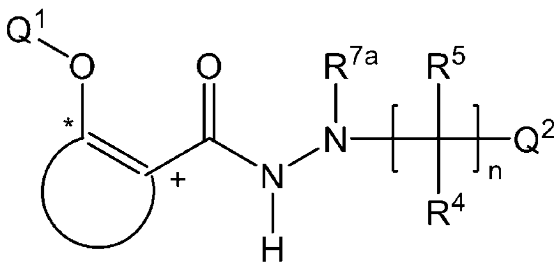

(I-A1)

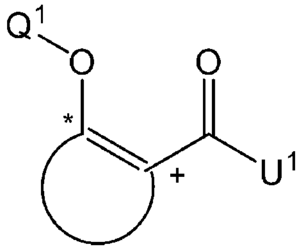

(im-A5)

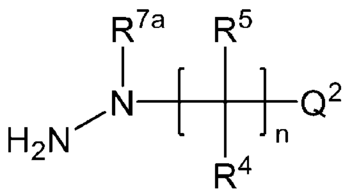

(im-6a)

(In each formula shown above,

$Q^1$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group,
$Q^2$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group,
a carbon atom that constitutes a ring in $Q^2$ may bond, instead of $R^{7a}$, to a nitrogen atom having $R^{7a}$,
the partial structure Het:

[Chemical Formula 15]

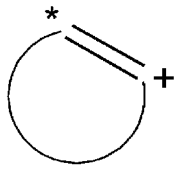

(Het)

is a substituted or unsubstituted six-membered heteroaryl ring, a substituted or unsubstituted six-membered nitrogen-containing hetero ring having an oxo group, or a substituted or unsubstituted nine- or ten-membered heteroaryl ring,

carbon atoms marked with * and + in the partial structure correspond to carbon atoms marked with * and + in the formula (Z-1),

$R^4$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a halogeno group,

$R^5$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a halogeno group,

$R^4$ and $R^5$ on an identical carbon atom may form together a divalent organic group,

n is an integer of 1 to 3,

$R^{7a}$ is a formyl group, a group of formula: $R^c$-CO-, a group of formula: $R^c$-CS-, a group of formula: $R^c$-CO-CO-, or a group of formula: $R^c$-$SO_2$-,

$R^c$ is each independently a substituted or unsubstituted C1-6 alkyl group, a substituted or unsubstituted C1-6 alkoxy group, a substituted or unsubstituted C3-6 cycloalkoxy group, a substituted or unsubstituted C1-6 alkylthio group, a substituted or unsubstituted amino group, a substituted or unsubstituted C3-6 cycloalkyl group, a substituted or unsubstituted phenyl group, a substituted or unsubstituted naphthyl group, a substituted or unsubstituted three- to six-membered heterocyclyl group, or a substituted or unsubstituted three- to six-membered heterocyclyloxy group, and

$U^1$ is a hydroxy group or a halogeno group.)

Advantageous Effects of Invention

The hydrazide compound according to the present invention exhibits excellent fungicidal activity against pathogens that affect agricultural and horticultural plants, has excellent safety, and can be synthesized in an industrially advantageous manner. The agricultural and horticultural fungicide according to the present invention can effectively prevent diseases of agricultural and horticultural plants.

DESCRIPTION OF EMBODIMENTS

A hydrazide compound according to the present invention is a compound of formula (Z-1) (hereinafter, may be indicated as compound (Z-I)) or a salt of the compound (Z-I).

[Chemical Formula 16]

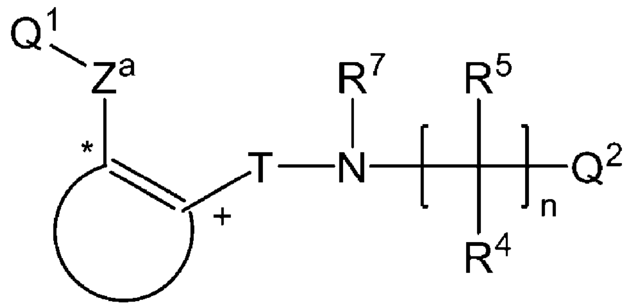

(Z − I)

In the present invention, the term "unsubstituted" refers to a group consisting of a mother nucleus. In the case where only the name of a group serving as a mother nucleus is provided without accompanying the term "substituted", this refers to "unsubstituted" unless specifically indicated otherwise.

On the other hand, the term "substituted" means that any hydrogen atom of a group serving as a mother nucleus is substituted with a group (substituent) having a structure that is identical to or different from the mother nucleus. Thus, a "substituent" is another group bound to a group serving as the mother nucleus. The number of substituents may be one or two or more. Two or more substituents may be identical to or different from each other.

The term "C1-6", for example, indicates that the number of carbon atoms of the group serving as the mother nucleus is 1 to 6. The number of carbon atoms does not include the number of carbon atoms present in substituents. For example, a butyl group having an ethoxy group as a substituent thereof is classified as a C2 alkoxy C4 alkyl group.

There are no particular limitations on "substituents" provided that they are chemically available and achieve the effects of the present invention.

Specific examples of groups that can be "substituents" include the following groups:

C1-6 alkyl groups such as a methyl group, an ethyl group, a n-propyl group, an i-propyl group, a n-butyl group, a s-butyl group, an i-butyl group, a t-butyl group, a n-pentyl group, and a n-hexyl group;
C2-6 alkenyl groups such as a vinyl group, a 1-propenyl group, a 2-propenyl group (an allyl group), a 1-butenyl group, a 2-butenyl group, a 3-butenyl group, a 1-methyl-2-propenyl group, and a 2-methyl-2-propenyl group;
C2-6 alkynyl groups such as an ethynyl group, a 1-propynyl group, a 2-propynyl group, a 1-butynyl group, a 2-butynyl group, a 3-butynyl group, and a 1-methyl-2-propynyl group;
C3-6 cycloalkyl groups such as a cyclopropyl group, a cyclobutyl group, a cyclopentyl group, and a cyclohexyl group;
aryl groups such as a phenyl group, a tolyl group, a xylyl group, a trimethylphenyl group, and a naphthyl group;
phenyl C1-6 alkyl groups such as a benzyl group and a phenethyl group;
three- to six-membered heterocyclyl groups;
three- to six-membered heterocyclyl C1-6 alkyl groups;
a hydroxy group;
C1-6 alkoxy groups such as a methoxy group, an ethoxy group, a n-propoxy group, an i-propoxy group, a n-butoxy group, a s-butoxy group, an i-butoxy group, and a t-butoxy group;
C2-6 alkenyloxy groups such as a vinyloxy group, an allyloxy group, a propenyloxy group, and a butenyloxy group;
C2-6 alkynyloxy groups such as an ethynyloxy group, and a propargyloxy group;
a phenoxy group, a naphthoxy group;
phenyl C1-6 alkoxy groups such as a benzyloxy group, and a phenethyloxy group;
five- or six-membered heteroaryloxy groups such as a thiazolyloxy group, and a pyridyloxy group;
five- or six-membered heteroaryl C1-6 alkyloxy groups such as a thiazolylmethyloxy group, and a pyridylmethyloxy group;
a formyl group;
C1-6 alkylcarbonyl groups such as an acetyl group, and a propionyl group;
a formyloxy group;
C1-6 alkylcarbonyloxy groups such as an acetyloxy group, and a propionyloxy group;
a benzoyl group;
C1-6 alkoxycarbonyl groups such as a methoxycarbonyl group, an ethoxycarbonyl group, a n-propoxycarbonyl group, an i-propoxycarbonyl group, a n-butoxycarbonyl group, and a t-butoxycarbonyl group;
C1-6 alkoxycarbonyloxy groups such as a methoxycarbonyloxy group, an ethoxycarbonyloxy group, a n-propoxycarbonyloxy group, an i-propoxycarbonyloxy group, a n-butoxycarbonyloxy group, and a t-butoxycarbonyloxy

group;
a carboxyl group;
halogeno groups such as a fluoro group, a chloro group, a bromo group, and an iodo group;
C1-6 haloalkyl groups such as a chloromethyl group, a chloroethyl group, a trifluoromethyl group, a 1,2-dichloro-n-propyl group, a 1-fluoro-n-butyl group, and a perfluoro-n-pentyl group;
C2-6 haloalkenyl groups such as a 2-chloro-1-propenyl group, and a 2-fluoro-1-butenyl group;
C2-6 haloalkynyl groups such as a 4,4-dichloro-1-butynyl group, a 4-fluoro-1-pentynyl group, and a 5-bromo-2-pentynyl group;
C1-6 haloalkoxy groups such as a trifluoromethoxy group, a 2-chloro-n-propoxy group, and a 2,3-dichlorobutoxy group;
C2-6 haloalkenyloxy groups such as a 2-chloropropenyloxy group, and a 3-bromobutenyloxy group;
C1-6 haloalkylcarbonyl groups such as a chloroacetyl group, a trifluoroacetyl group, and a trichloroacetyl group;
an amino group;
C1-6 alkyl-substituted amino groups such as a methylamino group, a dimethylamino group, and a diethylamino group;
an anilino group, a naphthylamino group;
phenyl C1-6 alkylamino groups such as a benzylamino group, and a phenethylamino group;
a formylamino group;
C1-6 alkylcarbonylamino groups such as an acetylamino group, a propanoylamino group, a butyrylamino group, and an i-propylcarbonylamino group;
C1-6 alkoxycarbonylamino groups such as a methoxycarbonylamino group, an ethoxycarbonylamino group, a n-propoxycarbonylamino group, and an i-propoxycarbonylamino group;
unsubstituted or substituted aminocarbonyl groups such as an aminocarbonyl group, a dimethylaminocarbonyl group, a phenylaminocarbonyl group, and a N-phenyl-N-methylaminocarbonyl group;
imino C1-6 alkyl groups such as an iminomethyl group, a (1-imino)ethyl group, and a (1-imino)-n-propyl group;
substituted or unsubstituted N-hydroxyimino C1-6 alkyl groups such as a N-hydroxy-iminomethyl group, a (1-(N-hydroxy)-imino)ethyl group, a (1-(N-hydroxy)-imino)propyl group, a N-methoxy-iminomethyl group, and a (1-(N-methoxy)-imino)ethyl group;
an aminocarbonyloxy group;
C1-6 alkyl-substituted aminocarbonyloxy groups such as an ethylaminocarbonyloxy group, and a dimethylaminocarbonyloxy group;
a mercapto group;
C1-6 alkylthio groups such as a methylthio group, an ethylthio group, a n-propylthio group, an i-propylthio group, a n-butylthio group, an i-butylthio group, a s-butylthio group, and a t-butylthio group;
C1-6 haloalkylthio groups such as a trifluoromethylthio group, and a 2,2,2-trifluoroethylthio group;
a phenylthio group, a naphthylthio group;
five- or six-membered heteroarylthio groups such as a thiazolylthio group, and a pyridylthio group;
C1-6 alkylsulfinyl groups such as a methylsulfinyl group, an ethylsulfinyl group, and a t-butylsulfinyl group;
C1-6 haloalkylsulfinyl groups such as a trifluoromethylsulfinyl group, and a 2,2,2-trifluoroethylsulfinyl group;
a phenylsulfinyl group;
five- or six-membered heteroarylsulfinyl groups such as a thiazolylsulfinyl group, and a pyridylsulfinyl group;
C1-6 alkylsulfonyl groups such as a methylsulfonyl group, an ethylsulfonyl group, and a t-butylsulfonyl group;
C1-6 haloalkylsulfonyl groups such as a trifluoromethylsulfonyl group, and a 2,2,2-trifluoroethylsulfonyl group;
a phenylsulfonyl group;
five- or six-membered heteroarylsulfonyl groups such as a thiazolylsulfonyl group, and a pyridylsulfonyl group;
C1-6 alkylsulfonyloxy groups such as a methylsulfonyloxy group, an ethylsulfonyloxy group, and a t-butylsulfonyloxy group;
C1-6 haloalkylsulfonyloxy groups such as a trifluoromethylsulfonyloxy group, and a 2,2,2-trifluoroethylsulfonyloxy group;
tri C1-6 alkyl-substituted silyl groups such as a trimethylsilyl group, a triethylsilyl group, and a t-butyldimethylsilyl group;
a triphenylsilyl group;
a cyano group; and a nitro group.

Any hydrogen atom of the "substituent" may be substituted with a group having a different structure. Examples of such a "substituent" include C1-6 alkyl groups, C1-6 haloalkyl groups, C1-6 alkoxy groups, C1-6 haloalkoxy groups, halogeno groups, a cyano group, and a nitro group.

The "three- to six-membered heterocyclyl group" contains, as a ring member atom, one to four hetero atoms selected from the group consisting of a nitrogen atom, an oxygen atom and a sulfur atom. The heterocyclyl group may be

monocyclic or polycyclic. If at least one ring of a polycyclic heterocyclyl group is a hetero ring, the remaining rings thereof may be any of saturated alicyclic rings, unsaturated alicyclic rings and aromatic rings. Examples of the "three- to six-membered heterocyclyl group" include three- to six-membered saturated heterocyclyl groups, five- or six-membered heteroaryl groups, and five- or six-membered partially-unsaturated heterocyclyl groups.

Examples of the "three- to six-membered saturated heterocyclyl groups" include an aziridinyl group, an epoxy group, an azetidinyl group, an oxetanyl group, a pyrrolidinyl group, a tetrahydrofuranyl group, a thiazolidinyl group, a piperidyl group, a piperazinyl group, a morpholinyl group, a dioxolanyl group, and a dioxanyl group.

Examples of the "five-membered heteroaryl groups" include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, and a tetrazolyl group.

Examples of the "six-membered heteroaryl groups" include a pyridyl group, a pyrazinyl group, a pyrimidinyl group, a pyridazinyl group, and a triazinyl group.

Examples of the "five- or six-membered partially-unsaturated heterocyclyl groups" include: five-membered partially-unsaturated heterocyclyl groups such as a pyrrolinyl group, a dihydrofuranyl group, a dihydrothiophenyl group, an imidazolynyl group, a pyrazolynyl group, an oxazolynyl group, an isoxazolynyl group, a thiazolynyl group, and an isothiazolynyl group; and six-membered partially-unsaturated heterocyclyl groups such as a dihydropyranyl group.

$Q^1$ in the formula (Z-1) is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group.

$Q^2$ in the formula (Z-1) is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group.

The heterocyclyl group contains, as a ring member atom, at least one hetero atom selected from the group consisting of a nitrogen atom, an oxygen atom and a sulfur atom. The heterocyclyl group may be monocyclic or polycyclic. If at least one ring of a polycyclic heterocyclyl group is a hetero ring, the remaining rings thereof may be any of saturated alicyclic rings, unsaturated alicyclic rings and aromatic rings.

Examples of the "five- or six-membered heterocyclyl group" as $Q^1$ or $Q^2$ include five- or six-membered saturated heterocyclyl groups, five- or six-membered heteroaryl groups, and five- or six-membered partially-unsaturated heterocyclyl groups.

Examples of the "five- or six-membered saturated heterocyclyl group" as $Q^1$ or $Q^2$ include a pyrrolidinyl group, a tetrahydrofuranyl group, a tetrahydropyranyl group, a thiazolidinyl group, a piperidyl group, a piperazinyl group, a morpholinyl group, a dioxolanyl group, and a dioxanyl group, and a tetrahydropyranyl group or a piperidyl group is preferable.

Examples of the "five-membered heteroaryl group" as $Q^1$ or $Q^2$ include a pyrrolyl group, a furyl group, a thienyl group, an imidazolyl group, a pyrazolyl group, an oxazolyl group, an isooxazolyl group, a thiazolyl group, an isothiazolyl group, a triazolyl group, an oxadiazolyl group, a thiadiazolyl group, and a tetrazolyl group, and a thienyl group, a pyrazolyl group, or a thiazolyl group is preferable.

Examples of the "six-membered heteroaryl group" as $Q^1$ or $Q^2$ include a pyridyl group, a pyrazinyl group, a pyrimidinyl group, a pyridazinyl group, and a triazinyl group, and a pyridyl group is preferable.

Examples of the "five- or six-membered partially-unsaturated heterocyclyl group" as $Q^1$ or $Q^2$ include: five-membered partially-unsaturated heterocyclyl groups such as a pyrrolynyl group, a dihydrofuranyl group, a dihydrothiophenyl group, an imidazolynyl group, a pyrazolynyl group, an oxazolynyl group, an isoxazolynyl group, a thiazolynyl group, and an isothiazolynyl group; and six-membered partially-unsaturated heterocyclyl groups such as a dihydropyranyl group.

Examples of the "nine-membered heterocyclyl group" as $Q^1$ or $Q^2$ include an indolyl group, an isoindolyl group, a benzofuranyl group, a benzothienyl group, an indazolyl group, a benzoimidazolyl group, a benzoxazolyl group, a benzoisoxazolyl group, a benzothiazolyl group, a benzoisothiazolyl group, and a benzodioxolanyl group, and a benzothiazolyl group or a benzodioxolanyl group is preferable.

Examples of the "ten-membered heterocyclyl group" as $Q^1$ or $Q^2$ include a quinolynyl group, an isoquinolynyl group, a cinnolinyl group, a phthalazinyl group, a quinazolinyl group, and a quinoxalinyl group, and a quinolynyl group is preferable.

Preferable examples of a substituent on the "five- or six-membered heterocyclyl group", "phenyl group", "naphthyl group", or "nine- or ten-membered heterocyclyl group" as $Q^1$ or $Q^2$ include halogeno groups, C1-6 alkyl groups, C1-6 haloalkyl groups, a hydroxy group, C1-6 alkoxy groups, C1-6 haloalkoxy groups, C1-6 alkoxy groups substituted with C1-6 alkoxy groups, C1-6 alkylcarbonyl groups, C1-6 alkylthio groups, C1-6 alkylsulfonyl groups, C3-6 cycloalkyl groups, a phenyl group, a phenyloxy group, five-membered heteroaryl groups, six-membered heteroaryl groups, a cyano group, a nitro group, tri C1-6 alkylsilyl groups; amino groups substituted with C1-6 alkyl groups or C1-6 alkylcarbonyl groups; carboxamide groups substituted with C1-6 alkyl groups; phenyl groups substituted with at least one substituent selected from C1-6 alkyl groups, C1-6 alkoxy groups, halogeno groups, C1-6 haloalkyl groups, and C1-6 haloalkoxy groups; five-membered heteroaryl groups substituted with at least one substituent selected from C1-6 alkyl groups, C1-6 alkoxy groups, halogeno groups, C1-6 haloalkyl groups, and C1-6 haloalkoxy groups; and six-membered heteroaryl groups substituted with at least one substituent selected from C1-6 alkyl groups, C1-6 alkoxy groups, halogeno groups, C1-6 haloalkyl groups, and C1-6 haloalkoxy groups.

Two substituents on the "five- or six-membered heterocyclyl group", "phenyl group", "naphthyl group" or "nine- or ten-membered heterocyclyl group" of $Q^1$ or $Q^2$ may form together a divalent organic group, or two substituents on a phenyl group substituted with two substituents, a five-membered heteroaryl group substituted with two substituents, or a six-membered heteroaryl group substituted with two substituents may form together a divalent organic group. Examples of the divalent organic group include C3-6 alkylene groups such as a trimethylene group and a tetramethylene group; and C1-3 alkylene dioxy groups such as a methylenedioxy group, and a dimethylenedioxy group.

For example, it is preferable that two substituents on a phenyl group form together a trimethylene group to form an indanyl group as $Q^1$ or $Q^2$.

A carbon atom that constitutes a ring in $Q^2$ may bond, instead of $R^7$, to a nitrogen atom having $R^7$ in the formula (Z-1). For example, it is preferable that a carbon atom that constitutes a ring in $Q^2$ bond, instead of $R^7$, to a nitrogen atom having $R^7$ in the formula (Z-1), thereby forming a dihydroquinone ring.

Among them, $Q^1$ in the formula (Z-1) is more preferably a substituted or unsubstituted phenyl group, a substituted or unsubstituted naphthyl group, a substituted or unsubstituted tetrahydropyranyl group, a substituted or unsubstituted piperidyl group, a substituted or unsubstituted thienyl group, a substituted or unsubstituted pyridyl group, a substituted or unsubstituted benzodioxolanyl group, or a substituted or unsubstituted quinolynyl group, even more preferably a substituted or unsubstituted phenyl group, a substituted or unsubstituted naphthyl group, a substituted or unsubstituted thienyl group, a substituted or unsubstituted pyridyl group, a substituted or unsubstituted benzodioxolanyl group, or a substituted or unsubstituted quinolynyl group.

A substituent on the "five- or six-membered heterocyclyl group", "phenyl group", "naphthyl group" or "nine- or ten-membered heterocyclyl group" as $Q^1$ is more preferably a halogeno group, a C1-6 alkyl group, a C1-6 haloalkyl group, a C1-6 alkoxy group, a C1-6 haloalkoxy group, a C1-6 alkylcarbonyl group, a C1-6 alkylthio group, a C1-6 alkylsulfonyl group, a C3-6 cycloalkyl group, a phenyl group, a phenyloxy group, a cyano group, a nitro group, a tri C1-6 alkylsilyl group; an amino group substituted with a C1-6 alkyl group or a C1-6 alkylcarbonyl group; and/or, a carboxamide group substituted with a C1-6 alkyl, and even more preferably a halogeno group, a C1-6 alkyl group, a C1-6 haloalkyl group, a C1-6 alkoxy group, a C1-6 haloalkoxy group, a C1-6 alkylcarbonyl group, a C1-6 alkylthio group, a C3-6 cycloalkyl group, a cyano group, a nitro group; and/or an amino group substituted with a C1-6 alkyl group.

When the "five- or six-membered heterocyclyl group", "phenyl group", "naphthyl group" or "nine- or ten-membered heterocyclyl group" as $Q^1$ has a substituent, the number of substituents is preferably one or two.

It is preferable that $Q^1$ be an indanyl group in which two substituents on a phenyl group form together a trimethylene group.

$Q^2$ in the formula (Z-1) is more preferably a substituted or unsubstituted phenyl group, a substituted or unsubstituted naphthyl group, a substituted or unsubstituted thienyl group, a substituted or unsubstituted thiazolyl group, a substituted or unsubstituted pyridyl group, a substituted or unsubstituted benzodioxolanyl group, a substituted or unsubstituted benzothiazolyl group, or a substituted or unsubstituted quinolynyl group.

A substituent on the "phenyl group", "naphthyl group", "five- or six-membered heterocyclyl group", or "nine- or ten-membered heterocyclyl group" as $Q^2$ is more preferably a halogeno group, a C1-6 alkyl group, a hydroxy group, a C1-6 haloalkyl group, a C1-6 alkoxy group, a C1-6 alkoxy group substituted with a C1-6 alkoxy group, a C1-6 haloalkoxy group, a C3-6 cycloalkyl group, a phenyloxy group, a pyrazolyl group, a cyano group, a nitro group; an amino group substituted with a C1-6 alkyl group; and/or a phenyl group substituted with a halogeno group.

When the "phenyl group", "naphthyl group", "five- or six-membered heterocyclyl group", or "nine- or ten-membered heterocyclyl group" as $Q^2$ has a substituent, the number of substituents is preferably one or two.

It is preferable that $Q^2$ be an indanyl group in which two substituents on a phenyl group form together a trimethylene group.

In addition, it is preferable that a carbon atom that constitutes a ring in $Q^2$ bond, instead of $R^7$, to a nitrogen atom having $R^7$ in the formula (Z-1), thereby forming a dihydroquinoline ring.

In the formula (Z-1), the partial structure Het is a substituted or unsubstituted six-membered heteroaryl ring, a substituted or unsubstituted six-membered nitrogen-containing hetero ring having an oxo group, or a substituted or unsubstituted nine- or ten-membered heteroaryl ring.

[Chemical Formula 17]

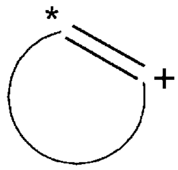

(Het)

Carbon atoms marked with * and + in the partial structure Het correspond to carbon atoms marked with * and + in the formula (I).

The heteroaryl ring is an aromatic ring containing, as a ring member atom, at least one hetero atom selected from the group consisting of a nitrogen atom, an oxygen atom and a sulfur atom. The heteroaryl ring may be monocyclic or polycyclic. If at least one ring of a polycyclic heteroaryl ring is a heteroaryl ring, the remaining rings thereof may be any of saturated alicyclic rings, unsaturated alicyclic rings and aromatic rings.

Examples of the "six-membered heteroaryl ring" as the partial structure Het include a pyridine ring, a pyrazine ring, a pyrimidine ring, a pyridazine ring, and a triazine ring. Among them, a pyridine ring, a pyrimidine ring, a pyridazine ring, and a triazine ring are preferable.

Examples of the "nine-membered heteroaryl ring" as the partial structure Het include an indole ring, an isoindole ring, an indazole ring, a benzoimidazole ring, an imidazopyridine ring, an imidazopyridazine ring, and an imidazopyrimidine ring. Among them, an imidazopyridazine ring and an imidazopyrimidine ring are preferable.

Examples of the "ten-membered heteroaryl ring" as the partial structure Het include a quinoline ring, an isoquinoline ring, a quinazoline ring, a quinoxaline ring, a dihydropyranopyridine ring, and a pyranopyridine ring. Among them, a dihydropyranopyridine ring and a pyranopyridine ring are preferable.

As a substituent on the "six-membered heteroaryl ring" or "nine- or ten-membered heteroaryl ring" of the partial structure Het, at least one selected from substituted or unsubstituted C1-6 alkyl groups, substituted or unsubstituted C2-6 alkenyl groups, substituted or unsubstituted C2-6 alkynyl groups, a hydroxy group, substituted or unsubstituted C1-6 alkoxy groups, substituted or unsubstituted C1-6 alkylcarbonyl groups, substituted or unsubstituted C1-6 alkoxycarbonyl groups, substituted or unsubstituted C1-6 alkylcarbonyloxy groups, substituted or unsubstituted C1-6 alkoxycarbonyloxy groups, substituted or unsubstituted C1-6 alkylthio groups, substituted or unsubstituted C1-6 alkylsulfinyl groups, substituted or unsubstituted C1-6 alkylsulfonyl groups, substituted or unsubstituted C3-6 cycloalkyl groups, substituted or unsubstituted phenyl groups, substituted or unsubstituted naphthyl groups, substituted or unsubstituted five- or six-membered heterocyclyl groups, substituted or unsubstituted phenyloxy groups, substituted or unsubstituted naphthyloxy groups, substituted or unsubstituted five- or six-membered heterocyclyloxy groups, substituted or unsubstituted amino groups, substituted or unsubstituted aminocarbonyl groups, N-(C1-6 alkoxy)imino groups, a nitro group, a cyano group, and halogeno groups is mentioned, at least one selected from substituted or unsubstituted C1-6 alkyl groups, substituted or unsubstituted C2-6 alkenyl groups, a hydroxy group, substituted or unsubstituted C1-6 alkoxy groups, substituted or unsubstituted C1-6 alkylcarbonyl groups, substituted or unsubstituted C1-6 alkoxycarbonyl groups, substituted or unsubstituted C1-6 alkylthio groups, substituted or unsubstituted C1-6 alkylsulfonyl groups, substituted or unsubstituted C3-6 cycloalkyl groups, substituted or unsubstituted phenyl groups, substituted or unsubstituted five-membered heterocyclyl groups, substituted or unsubstituted phenyloxy groups, substituted or unsubstituted amino groups, N-(C1-6 alkoxy) imino groups, a cyano group, and halogeno groups is preferably mentioned, and at least one selected from halogeno groups, C1-6 alkyl groups, C1-6 haloalkyl groups, C2-6 alkenyl groups, C2-6 alkenyl groups substituted with C1-6 alkoxy groups, a hydroxy group, C1-6 alkoxy groups, C1-6 haloalkoxy groups, C1-6 alkylcarbonyl groups, C1-6 alkoxycarbonyl groups, C3-6 cycloalkyl groups, C1-6 alkylthio groups, C1-6 alkylsulfinyl groups, C1-6 alkylsulfonyl groups, a phenyl group, a phenyloxy group, five-membered heteroaryl groups, six-membered heteroaryl groups, a cyano group, N-(C1-6 alkoxy)imino groups; phenyl groups substituted with at least one substituent selected from C1-6 alkyl groups, C1-6 alkoxy groups, halogeno groups, C1-6 haloalkyl groups, C1-6 haloalkoxy groups, and five-membered heteroaryl groups; phenyloxy groups substituted with halogeno groups; five-membered heteroaryl groups substituted with at least one substituent selected from C1-6 alkyl groups, C1-6 alkoxy groups, halogeno groups, C1-6 haloalkyl groups, and C1-6 haloalkoxy groups; six-membered heteroaryl groups substituted with at least one substituent selected from C1-6 alkyl groups, C1-6 alkoxy groups, halogeno groups, C1-6 haloalkyl groups, and C1-6 haloalkoxy groups; and amino groups substituted with at least one substituent selected from the group consisting of C1-6 alkyl groups and C1-6 alkoxycarbonyl groups is more preferably mentioned.

Among them, a substituent on the "six-membered heteroaryl ring" as the partial structure Het is preferably at least one selected from halogeno groups, C1-6 alkyl groups, C1-6 haloalkyl groups, C2-6 alkenyl groups, C2-6 alkenyl groups substituted with C1-6 alkoxy groups, C1-6 alkoxy groups, C1-6 alkylcarbonyl groups, C1-6 alkoxycarbonyl groups, C1-6 alkylthio groups, C1-6 alkylsulfonyl groups, C3-6 cycloalkyl groups, a phenyl group, phenyl groups substituted with

halogeno groups, phenyloxy group substituted with halogeno groups, a pyrazolyl group, N-(C1-6 alkoxy)imino groups, amino groups substituted with C1-6 alkyl groups, amino groups substituted with C1-6 alkoxycarbonyl groups, and a cyano group.

As a substituent on the "nine- or ten-membered heteroaryl ring" as the partial structure Het, a C1-6 alkyl group is preferable.

When the "six-membered heteroaryl ring" or "nine- or ten-membered heteroaryl ring" as the partial structure Het has a substituent, the number of substituents is preferably one or two.

Two substituents on the "six-membered heteroaryl ring" or "nine- or ten-membered heteroaryl ring" as the partial structure may form together a divalent organic group.

For example, two substituents on the "six-membered heteroaryl ring" may form together a C3-6 alkylene group, a C3-4 alkenylene group, a C1-3 alkylenedioxy group, or a propenyleneoxy group, more preferably a trimethylene group, a divinylene group, or a propenyleneoxy group, and more preferably a trimethylene group or a divinylene group.

The six-membered nitrogen-containing hetero ring having an oxo group is a ring having at least one nitrogen atom as a ring member atom, in which at least one carbon atom that constitutes a ring is substituted with an oxo group.

Examples of the "the six-membered nitrogen-containing hetero group having an oxo group" include a 2-pyridone ring, a 3-pyridone ring, a 4-pyridone ring, a 3-pyridazinone ring, a 4-pyridazinone ring, a 2-pyrimidinone ring, a 4-pyrimidinone ring, and a 2-pyrazinone ring, and a pyridazinone ring is preferable.

Examples of a substituent on the "six-membered nitrogen-containing hetero group having an oxo group" as the partial structure Het include halogeno groups, C1-6 alkyl groups, C1-6 haloalkyl groups, a hydroxy group, C1-6 alkoxy groups, C1-6 haloalkoxy groups, C3-6 cycloalkyl groups, a phenyl group, five-membered heteroaryl groups, six-membered heteroaryl groups, a cyano group; phenyl groups substituted with at least one substituent selected from C1-6 alkyl groups, C1-6 alkoxy groups, halogeno groups, C1-6 haloalkyl groups, and C1-6 haloalkoxy groups; five-membered heteroaryl groups substituted with at least one substituent selected from C1-6 alkyl groups, C1-6 alkoxy groups, halogeno groups, C1-6 haloalkyl groups, and C1-6 haloalkoxy groups; and six-membered heteroaryl groups substituted with at least one substituent selected from C1-6 alkyl groups, C1-6 alkoxy groups, halogeno groups, C1-6 haloalkyl groups, and C1-6 haloalkoxy groups. Among these, the substituent on the "six-membered nitrogen-containing hetero group having an oxo group" as the partial structure Het is preferably a C1-6 alkyl group.

As one example of the "six-membered heteroaryl ring" as the partial structure Het, a ring of formula (II) may be mentioned.

Carbon atoms marked with * and + in the formula (II) correspond to carbon atoms marked with * and + in the formula (I).

[Chemical Formula 18]

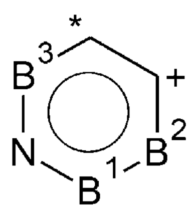

(II)

In the formula (II), $B^1$ is a nitrogen atom, or a group of formula: $CR^1$, and preferably a group of formula: $CR^1$.

In the formula (II), $B^2$ is a nitrogen atom, or a group of formula: $CR^2$, and preferably a group of formula: $CR^2$.

In the formula (II), $B^3$ is a nitrogen atom, or a group of formula: $CR^3$.

Specific examples of the "six-membered heteroaryl ring" of formula (II) include the following ones.

[Chemical Formula 19]

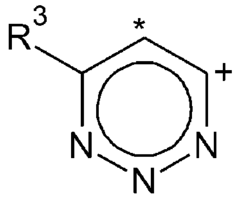
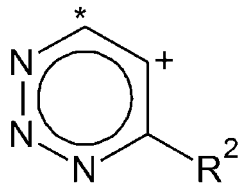
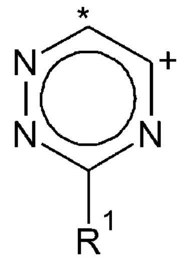

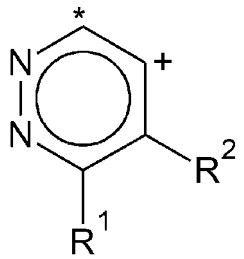
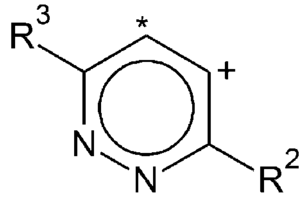
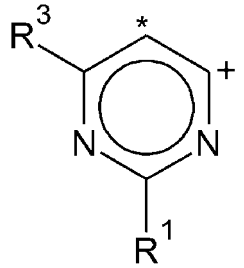

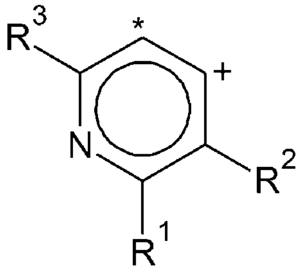
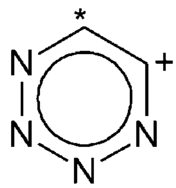

$R^1$ is a hydrogen atom, a substituted or unsubstituted C1-6 alkyl group, a substituted or unsubstituted C2-6 alkenyl group, a substituted or unsubstituted C2-6 alkynyl group, a hydroxy group, a substituted or unsubstituted C1-6 alkoxy group, a substituted or unsubstituted C1-6 alkylcarbonyl group, a substituted or unsubstituted C1-6 alkoxycarbonyl group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, a substituted or unsubstituted phenyloxy group, a substituted or unsubstituted naphthyloxy group, a substituted or unsubstituted five- or six-membered heterocyclyloxy group, a substituted or unsubstituted amino group, a substituted or unsubstituted aminocarbonyl group, an N-(C1-6 alkoxy)imino group, a nitro group, a cyano group, or a halogeno group.

The "C1-6 alkyl group" as $R^1$ may be a linear chain or a branched chain. Examples of the "C1-6 alkyl group" as $R^1$ include a methyl group, an ethyl group, a n-propyl group, a n-butyl group, a n-pentyl group, a n-hexyl group, an i-propyl group, an i-butyl group, a s-butyl group, a t-butyl group, an i-pentyl group, a neopentyl group, a 2-methylbutyl group, and an i-hexyl group.

Examples of the "C2-6 alkenyl group" as $R^1$ include a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group, and a 5-hexenyl group.

Examples of the "C2-6 alkynyl group" as $R^1$ include an ethynyl group, a 1-propynyl group, a 2-propynyl group, a 1-butynyl group, a 2-butynyl group, a 3-butynyl group, a 1-methyl-2-propynyl group, a 2-methyl-3- butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group, and a 1,1-dimethyl-2-butynyl group.

Examples of the "C1-6 alkoxy group" as $R^1$ include a methoxy group, an ethoxy group, a n-propoxy group, an i-propoxy group, a n-butoxy group, a s-butoxy group, an i-butoxy group, and a t-butoxy group.

Examples of the "C1-6 alkylcarbonyl group" as $R^1$ include an acetyl group, a propionyl group, a n-propylcarbonyl group, an i-propylcarbonyl group, a n-butylcarbonyl group, and a t-butylcarbonyl group.

Examples of the "C1-6 alkoxycarbonyl group" as $R^1$ include a methoxycarbonyl group, an ethoxycarbonyl group,

a n-propoxycarbonyl group, an i-propoxycarbonyl group, a n-butoxycarbonyl group, and a t-butoxycarbonyl group.

Examples of the "C1-6 alkylcarbonyloxy group" as $R^1$ include an acetyloxy group, a propionyloxy group, a n-propylcarbonyloxy group, an i-propylcarbonyloxy group, a n-butylcarbonyloxy group, and a t-butylcarbonyloxy group.

Examples of the "C1-6 alkoxycarbonyloxy group" as $R^1$ include a methoxycarbonyloxy group, an ethoxycarbonyloxy group, a n-propoxycarbonyloxy group, an i-propoxycarbonyloxy group, a n-butoxycarbonyloxy group, and a t-butoxycarbonyloxy group.

Examples of the "C1-6 alkylthio group" as $R^1$ include a methylthio group, an ethylthio group, a n-propylthio group, a n-butylthio group, a n-pentylthio group, a n-hexylthio group, an i-propylthio group, and an i-butylthio group.

Examples of the "C1-6 alkylsulfinyl group" as $R^1$ include a methylsulfinyl group, an ethylsulfinyl group, and a t-butylsulfinyl group.

Examples of the "C1-6 alkylsulfonyl group" as $R^1$ include a methylsulfonyl group, an ethylsulfonyl group, and a t-butylsulfonyl group.

As a substituent on the "C1-6 alkyl group", "C2-6 alkenyl group", "C2-6 alkynyl group", "C1-6 alkoxy group", "C1-6 alkylcarbonyl group", "C1-6 alkoxycarbonyl group", "C1-6 alkylcarbonyloxy group", "C1-6 alkoxycarbonyloxy group", "C1-6 alkylthio group", "C1-6 alkylsulfinyl group", or "C1-6 alkylsulfonyl group" as $R^1$, a halogeno group such as a fluoro group, a chloro group, a bromo group, or an iodo group, a hydroxy group, a C1-6 alkoxy group such as a methoxy group, an ethoxy group, a n-propoxy group, an i-propoxy group, a n-butoxy group, a s-butoxy group, an i-butoxy group, or a t-butoxy group, a C1-6 haloalkoxy group such as a 2-chloro-n-propoxy group, a 2,3-dichlorobutoxy group, or a trifluoromethoxy group, a C3-6 cycloalkyl group, a phenyl group, a five-membered heteroaryl group, a six-membered heteroaryl group, a cyano group; a phenyl group substituted with at least one substituent selected from C1-6 alkyl groups, C1-6 alkoxy groups, halogeno groups, C1-6 haloalkyl groups, and C1-6 haloalkoxy groups; a five-membered heteroaryl group substituted with at least one substituent selected from C1-6 alkyl groups, C1-6 alkoxy groups, halogeno groups, C1-6 haloalkyl groups, and C1-6 haloalkoxy groups; or a six-membered heteroaryl group substituted with at least one substituent selected from C1-6 alkyl groups, C1-6 alkoxy groups, halogeno groups, C1-6 haloalkyl groups, and C1-6 haloalkoxy groups is preferable, and a halogeno group is more preferable.

Examples of the "C3-6 cycloalkyl group" as $R^1$ include a cyclopropyl group, a cyclobutyl group, a cyclopentyl group, and a cyclohexyl group. Among these, a cyclopropyl group or a cyclohexyl group is preferable, and a cyclopropyl group is more preferable.

The "five- or six-membered heterocyclyl group" as $R^1$ is a group that contains, as a ring member atom, one, two, three or four hetero atoms selected from the group consisting of a nitrogen atom, an oxygen atom and a sulfur atom. When at least two hetero atoms are contained, the hetero atoms may be identical to or different from each other.

Examples of the "five- or six-membered heterocyclyl group" include a pyrrolidinyl group, a tetrahydrofuranyl group, a thiazolidinyl group, a tetrahydro-2H-pyranyl group, a piperidyl group, a piperazinyl group, a morpholinyl group, a dioxolanyl group, a dioxanyl group; a pyrrolynyl group, a dihydrofuranyl group, an imidazolynyl group, a pyrazolynyl group, an oxazolynyl group, an isoxazolynyl group, a dihydropyranyl group, and a pyrazolyl group, and the "five- or six-membered heterocyclyl group" is preferably a pyrazolyl group.

Examples of the "five- or six-membered heterocyclyloxy group" as $R^1$ include a pyrrolidinyloxy group, a tetrahydrofuranyloxy group, a thiazolidinyloxy group, a tetrahydro-2H-pyranyloxy group, a piperidyloxy group, a piperazinyloxy group, a morpholinyloxy group, a dioxolanyloxy group, a dioxanyloxy group; a pyrrolynyloxy group, a dihydrofuranyloxy group, an imidazolynyloxy group, a pyrazolynyloxy group, an oxazolynyloxy group, an isooxazolynyloxy group; and a dihydropyranyloxy group.

As a substituent on the "C3-6 cycloalkyl group", "phenyl group", "naphthyl group", "five- or six-membered heterocyclyl group", "phenyloxy group", "naphthyloxy group", or "five- or six-membered heterocyclyloxy group" as $R^1$, a halogeno group, a C1-6 alkyl group, a C1-6 haloalkyl group, a hydroxy group, a C1-6 alkoxy group, a C1-6 haloalkoxy group, a C3-6 cycloalkyl group, a phenyl group, a five-membered heteroaryl group, a six-membered heteroaryl group, a cyano group; a phenyl group substituted with at least one substituent selected from C1-6 alkyl groups, C1-6 alkoxy groups, halogeno groups, C1-6 haloalkyl groups, and C1-6 haloalkoxy groups; a five-membered heteroaryl group substituted with at least one substituent selected from C1-6 alkyl groups, C1-6 alkoxy groups, halogeno groups, C1-6 haloalkyl groups, and C1-6 haloalkoxy groups; or a six-membered heteroaryl group substituted with at least one substituent selected from C1-6 alkyl groups, C1-6 alkoxy groups, halogeno groups, C1-6 haloalkyl groups, and C1-6 haloalkoxy groups is preferable, and a halogeno group is more preferable.

Examples of the "phenyl group substituted with (a) C1-6 alkyl group(s)" as $R^1$ include a tolyl group, a xylyl group, and a trimethylphenyl group.

Examples of the "phenyloxy group substituted with (a) C1-6 alkyl group(s)" as $R^1$ include a tolyloxy group, a xylyloxy group, and a trimethylphenoxy group.

The "amino group" as $R^1$ is a group of $NH_2$. The "substituted amino group" is a group formed by substituting a hydrogen atom in a group of $NH_2$ with another group.

The "aminocarbonyl group" as $R^1$ is a group of $NH_2CO$. The "substituted aminocarbonyl group" is a group formed

by substituting a hydrogen atom in a group of $NH_2CO$ with another group.

As a substituent on the "amino group", or "aminocarbonyl group" as $R^1$, a C1-6 alkyl group, a C1-6 haloalkyl group, a C1-6 alkoxycarbonyl group, a C3-6 cycloalkyl group, a phenyl group, a five-membered heteroaryl group, a six-membered heteroaryl group; a phenyl group substituted with at least one substituent selected from C1-6 alkyl groups, C1-6 alkoxy groups, halogeno groups, C1-6 haloalkyl groups, and C1-6 haloalkoxy groups; a five-membered heteroaryl groups substituted with at least one substituent selected from C1-6 alkyl groups, C1-6 alkoxy groups, halogeno groups, C1-6 haloalkyl groups, and C1-6 haloalkoxy groups; or a six-membered heteroaryl groups substituted with at least one substituent selected from C1-6 alkyl groups, C1-6 alkoxy groups, halogeno groups, C1-6 haloalkyl groups, and C1-6 haloalkoxy groups is preferable, and a C1-6 alkyl group or a C1-6 alkoxycarbonyl group is more preferable.

Examples of the "N-(C1-6 alkoxy)imino group" as $R^1$ include an N-(methoxy)imino group, and an N-(ethoxy) imino group.

Examples of the "halogeno group" as $R^1$ include a fluoro group, a chloro group, a bromo group, and an iodo group.

Among these, $R^1$ is preferably a hydrogen atom, a substituted or unsubstituted C1-6 alkyl group, a substituted or unsubstituted C1-6 alkoxy group, a substituted or unsubstituted C1-6 alkylthio group, a substituted or unsubstituted C1-6 alkylsulfonyl group, a substituted or unsubstituted C3-6 cycloalkyl group, a substituted or unsubstituted phenyl group, a substituted or unsubstituted five-membered heterocyclyl group, a substituted or unsubstituted amino group, a cyano group, or a halogeno group, and more preferably a hydrogen atom, a C1-6 alkyl group, a C1-6 haloalkyl group, a C1-6 alkoxy group, a C1-6 alkylthio group, a C1-6 alkylsulfonyl group, a C3-6 cycloalkyl group, a phenyl group substituted with a halogeno group, a pyrazolyl group, an amino group substituted with a C1-6 alkyl group, an amino group substituted with a C1-6 alkoxycarbonyl group, a cyano group, or a halogeno group.

$R^2$ is a hydrogen atom, a substituted or unsubstituted C1-6 alkyl group, a substituted or unsubstituted C2-6 alkenyl group, a substituted or unsubstituted C2-6 alkynyl group, a hydroxy group, a substituted or unsubstituted C1-6 alkoxy group, a substituted or unsubstituted C1-6 alkylcarbonyl group, a substituted or unsubstituted C1-6 alkoxycarbonyl group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, a substituted or unsubstituted phenyloxy group, a substituted or unsubstituted naphthyloxy group, a substituted or unsubstituted five- or six-membered heterocyclyloxy group, a substituted or unsubstituted amino group, a substituted or unsubstituted aminocarbonyl group, an N-(C1-6 alkoxy)imino group, a nitro group, a cyano group, or a halogeno group.

$R^1$ and $R^2$ may form together a divalent organic group. Examples of the divalent organic group include: C3-6 alkylene groups such as a trimethylene group, and a tetramethylene group; C3-4 alkenylene groups such as a propenylene group (prop-1-en-1,3-diyl group, $-CH_2-CH=CH-$), and a divinylene group (buta-1,3-dien-1,4-diyl group, $-CH=CH-CH=CH-$); C1-3 alkylenedioxy groups such as a methylenedioxy group and a dimethylenedioxy group; and propenyleneoxy group. Among these, the divalent organic group formed by $R^1$ and $R^2$ together is preferably a C3-6 alkylene group or a C3-4 alkenylene group, and more preferably a trimethylene group or a divinylene group.

$R^3$ is a hydrogen atom, a substituted or unsubstituted C1-6 alkyl group, a substituted or unsubstituted C2-6 alkenyl group, a substituted or unsubstituted C2-6 alkynyl group, a hydroxy group, a substituted or unsubstituted C1-6 alkoxy group, a substituted or unsubstituted C1-6 alkylcarbonyl group, a substituted or unsubstituted C1-6 alkoxycarbonyl group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, a substituted or unsubstituted phenyloxy group, a substituted or unsubstituted naphthyloxy group, a substituted or unsubstituted five- or six-membered heterocyclyloxy group, a substituted or unsubstituted amino group, a substituted or unsubstituted aminocarbonyl group, a nitro group, a cyano group, or a halogeno group.

Examples of the "substituted or unsubstituted C1-6 alkyl group", "substituted or unsubstituted C2-6 alkenyl group", "substituted or unsubstituted C2-6 alkynyl group", "substituted or unsubstituted C1-6 alkoxy group", "substituted or unsubstituted C1-6 alkylcarbonyl group", "substituted or unsubstituted C1-6 alkoxycarbonyl group", "substituted or unsubstituted C1-6 alkylcarbonyloxy group", "substituted or unsubstituted C1-6 alkoxycarbonyloxy group", "substituted or unsubstituted C1-6 alkylthio group", "substituted or unsubstituted C1-6 alkylsulfinyl group", "substituted or unsubstituted C1-6 alkylsulfonyl group", "substituted or unsubstituted C3-6 cycloalkyl group", "substituted or unsubstituted phenyl group", "substituted or unsubstituted naphthyl group", "substituted or unsubstituted five- or six-membered heterocyclyl group", "substituted or unsubstituted phenyloxy group", "substituted or unsubstituted naphthyloxy group", "substituted or unsubstituted five- or six-membered heterocyclyloxy group", "substituted or unsubstituted amino group", "substituted or unsubstituted aminocarbonyl group", "N-(C1-6 alkoxy)imino group", "halogeno group' as $R^2$ or $R^3$ include the same groups as those provided as exemplary examples of $R^1$.

Among these, R2 is preferably a hydrogen atom, a substituted or unsubstituted C1-6 alkyl group, a substituted or unsubstituted C2-6 alkenyl group, a substituted or unsubstituted C1-6 alkoxy group, a substituted or unsubstituted C1-6 alkylcarbonyl group, a substituted or unsubstituted C1-6 alkoxycarbonyl group, a substituted or unsubstituted phenyloxy group, an N-(C1-6 alkoxy)imino group, a cyano group, or a halogeno group, and more preferably a hydrogen atom, a C1-6 alkyl group, a C2-6 alkenyl group, a C2-6 alkenyl group substituted with a C1-6 alkoxy group, a C1-6 alkoxy group, a C1-6 alkylcarbonyl group, a C1-6 alkoxycarbonyl group, a phenyloxy group substituted with a halogeno group, an N-(C1-6 alkoxy)imino group, a cyano group, or a halogeno group.

$R^3$ is preferably a hydrogen atom.

$Z^a$ in the formula (Z-1) is an oxygen atom, a sulfur atom, or a group of formula: $NR^a$.

$R^a$ is a hydrogen atom, or a substituted or unsubstituted C1-6 alkyl group.

The "C1-6 alkyl group" as $R^a$ may be a linear chain or a branched chain. Examples of the "C1-6 alkyl group" as $R^1$ include a methyl group, an ethyl group, a n-propyl group, a n-butyl group, a n-pentyl group, a n-hexyl group, an i-propyl group, an i-butyl group, a s-butyl group, a t-butyl group, an i-pentyl group, a neopentyl group, a 2-methylbutyl group, and an i-hexyl group.

Examples of the "substituted or unsubstituted C1-6 alkyl group" include the same groups as those mentioned as $R^1$.

As a substituent on the "C1-6 alkyl group" as $R^a$, a halogeno group such as a fluoro group, a chloro group, a bromo group, or an iodo group, a hydroxy group, a C1-6 alkoxy group such as a methoxy group, an ethoxy group, a n-propoxy group, an i-propoxy group, a n-butoxy group, a s-butoxy group, an i-butoxy group, or a t-butoxy group, a C1-6 haloalkoxy group such as a 2-chloro-n-propoxy group, a 2,3-dichlorobutoxy group, a trifluoromethoxy group, a C3-6 cycloalkyl group, a phenyl group, a five-membered heteroaryl group, a six-membered heteroaryl group, a cyano group; a phenyl group substituted with at least one substituent selected from C1-6 alkyl groups, C1-6 alkoxy groups, halogeno groups, C1-6 haloalkyl groups, and C1-6 haloalkoxy groups; five-membered heteroaryl groups substituted with at least one substituent selected from C1-6 alkyl groups, C1-6 alkoxy groups, halogeno groups, C1-6 haloalkyl groups, and C1-6 haloalkoxy groups; or a six-membered heteroaryl groups substituted with at least one substituent selected from C1-6 alkyl groups, C1-6 alkoxy groups, halogeno groups, C1-6 haloalkyl groups, and C1-6 haloalkoxy groups is preferable.

Among these, $R^a$ is preferably a hydrogen atom.

$Z^a$ in the formula (Z-1) is preferably an oxygen atom.

T in the formula (Z-1) is a partial structure (T-1) or a partial structure (T-2).

[Chemical Formula 20]

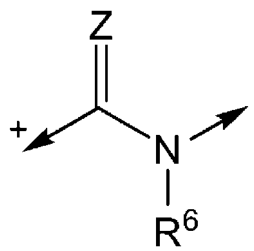

(T−1)

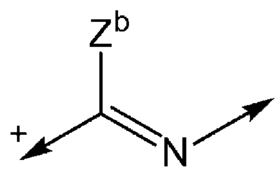

(T−2)

Z in the partial structure (T-1) is an oxygen atom, a sulfur atom, or a group of formula: $NR^b$.

An arrow marked with + is bonded to a carbon atom marked with + in the partial structure, and the other arrow bonded to a nitrogen atom having $R^7$.

$R^b$ is a hydrogen atom, a substituted or unsubstituted C1-6 alkyl group, a hydroxy group, or a substituted or unsubstituted C1-6 alkoxy group. Examples of the "substituted or unsubstituted C1-6 alkyl group" or "substituted or unsubstituted C1-6 alkoxy group" as $R^b$ include the same groups as those provided as exemplary examples of $R^1$. A hydroxy group is preferable.

Among these, Z in the partial structure (T-1) is preferably an oxygen atom.

$R^6$ in the partial structure (T-1) is a hydrogen atom, a substituted or unsubstituted C1-6 alkyl group, a substituted or unsubstituted C2-6 alkenyl group, a substituted or unsubstituted C2-6 alkynyl group, a formyl group, a substituted or unsubstituted C1-6 alkylcarbonyl group, a substituted or unsubstituted C1-6 alkoxycarbonyl group, a substituted or

unsubstituted C3-6 cycloalkyl group, a substituted or unsubstituted phenyl group, a substituted or unsubstituted naphthyl group, or a substituted or unsubstituted five- or six-membered heterocyclyl group.

Examples of the "substituted or unsubstituted C1-6 alkyl group", "substituted or unsubstituted C2-6 alkenyl group", "substituted or unsubstituted C2-6 alkynyl group", "substituted or unsubstituted C1-6 alkylcarbonyl group", "substituted or unsubstituted C1-6 alkoxycarbonyl group", "substituted or unsubstituted C3-6 cycloalkyl group", "substituted or unsubstituted phenyl group", "substituted or unsubstituted naphthyl group", or "substituted or unsubstituted five- or six-membered heterocyclyl group" as $R^6$ include the same groups as those provided as exemplary examples of $R^1$.

Among these, $R^6$ in the partial structure (T-1) is preferably a hydrogen atom or an unsubstituted C1-6 alkyl group, and more preferably a hydrogen atom.

In the partial structure (T-2), $Z^b$ is a C1-6 alkoxy group or a C1-6 alkylthio group.

An arrow marked with + is bonded to a carbon atom marked with + in the partial structure, and the other arrow bonded to a nitrogen atom having $R^7$.

Examples of the "C1-6 alkyl group" or "C1-6 alkylthio group" as $Z^b$ include the same groups as those provided as exemplary examples of $R^1$.

Among these, $Z^b$ in the partial structure (T-2) is preferably a methoxy group or a methylthio group, and more preferably a methoxy group.

$R^4$ in the formula (Z-1) is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a halogeno group.

$R^5$ in the formula (Z-1) is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a halogeno group.

Examples of the "substituted or unsubstituted C1-6 alkyl group", "substituted or unsubstituted C2-6 alkenyl group", "substituted or unsubstituted C2-6 alkynyl group", "substituted or unsubstituted C3-6 cycloalkyl group", "substituted or unsubstituted phenyl group", "substituted or unsubstituted naphthyl group", "substituted or unsubstituted five- or six-membered heterocyclyl group", and "halogeno group" as $R^4$ or $R^5$ include the same groups as those provided as exemplary examples of $R^1$.

$R^4$ and $R^5$ on an identical carbon atom may form together a divalent organic group. Examples of the divalent organic group include C2-5 alkylene groups such as a dimethylene group, a trimethylene group, and a tetramethylene group.

Among these, $R^4$ and $R^5$ in the formula (Z-1) are preferably hydrogen atoms. Alternatively, it is preferable that $R^4$ and $R^5$ on an identical carbon atom form together a trimethylene group.

$R^7$ in the formula (Z-1) is a hydrogen atom, a substituted or unsubstituted C1-6 alkyl group, a substituted or unsubstituted C2-6 alkenyl group, a substituted or unsubstituted C2-6 alkynyl group, a formyl group, a group of formula: $R^c$-CO-, a group of formula: $R^c$-CS-, a group of formula: $R^c$-CO-CO-, a group of formula: $R^c$-$SO_2$-, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a cyano group.

Examples of the "substituted or unsubstituted C1-6 alkyl group", "substituted or unsubstituted C2-6 alkenyl group", "substituted or unsubstituted C2-6 alkynyl group", "substituted or unsubstituted C3-6 cycloalkyl group", "substituted or unsubstituted phenyl group", "substituted or unsubstituted naphthyl group", and "substituted or unsubstituted five- or six-membered heterocyclyl group" as $R^7$ include the same groups as those provided as exemplary examples of $R^1$.

$R^c$ is each independently a substituted or unsubstituted C1-6 alkyl group, a substituted or unsubstituted C1-6 alkoxy group, a substituted or unsubstituted C3-6 cycloalkoxy group, a substituted or unsubstituted C1-6 alkylthio group, a substituted or unsubstituted amino group, a substituted or unsubstituted C3-6 cycloalkyl group, a substituted or unsubstituted phenyl group, a substituted or unsubstituted naphthyl group, a substituted or unsubstituted three- to six-membered heterocyclyl group, or a substituted or unsubstituted three- to six-membered heterocyclyloxy group.

Examples of the "substituted or unsubstituted C1-6 alkyl group", "substituted or unsubstituted C1-6 alkoxy group", "substituted or unsubstituted amino group", "substituted or unsubstituted C3-6 cycloalkyl group", "substituted or unsubstituted C1-6 alkylthio group", "substituted or unsubstituted phenyl group", "substituted or unsubstituted naphthyl group", "substituted or unsubstituted five- or six-membered heterocyclyl group", and "substituted or unsubstituted five- or six-membered heterocyclyloxy group" as $R^c$ include the same groups as those provided as exemplary examples of $R^1$.

Examples of the "C3-6 cycloalkoxy group" as $R^c$ include a cyclopropyloxy group, a cyclobutyloxy group, and a cyclopentyloxy group.

Examples of the "three- or four-membered heterocyclyl group" as $R^c$ include an aziridinyl group, an epoxy group, an azetidinyl group, and an oxetanyl group.

Examples of the "three- or four-membered heterocyclyloxy group" as $R^c$ include an aziridinyloxy group, an

epoxyoxy group, a 3-azetidinyloxy group, and a 3-oxetanyloxy group.

As a substituent on the "C3-6 cycloalkoxy group", "three- or four-membered heterocyclyl group", or "three- or four-membered heterocyclyloxy group" as $R^c$, a halogeno group, a C1-6 alkyl group, a C1-6 haloalkyl group, a hydroxy group, a C1-6 alkoxy group, a C1-6 haloalkoxy group, a C3-6 cycloalkyl group, a phenyl group, five-membered heteroaryl group, six-membered heteroaryl group, a cyano group; a phenyl group substituted with at least one substituent selected from C1-6 alkyl groups, C1-6 alkoxy groups, halogeno groups, C1-6 haloalkyl groups, and C1-6 haloalkoxy groups; a five-membered heteroaryl group substituted with at least one substituent selected from C1-6 alkyl groups, C1-6 alkoxy groups, halogeno groups, C1-6 haloalkyl groups, and C1-6 haloalkoxy groups; or a six-membered heteroaryl group substituted with at least one substituent selected from C1-6 alkyl groups, C1-6 alkoxy groups, halogeno groups, C1-6 haloalkyl groups, and C1-6 haloalkoxy groups is preferable.

$R^7$ in the formula (Z-1) is preferably a hydrogen atom; C1-6 alkyl group; a C1-6 haloalkyl group; a formyl group; a group of formula: $R^c$-CO-; a group of formula: $R^c$-CS-; a group of formula: $R^c$-CO-CO-; a group of formula: $R^c$-$SO_2$-; or a phenyl group substituted with a C1-6 alkyl group, and more preferably a formyl group; a group of formula: $R^c$-CO-; a group of formula: $R^c$-CS-; a group of formula: $R^c$-CO-CO-, or a group of formula: $R^c$-$SO_2$-.

$R^c$ is preferably each independently a C1-6 alkyl group; a C1-6 haloalkyl group; a C1-6 alkoxy group; a C1-6 haloalkoxy group; a C1-6 alkoxy group substituted with a C3-6 cycloalkyl group; a C1-6 alkoxy group substituted with a phenyl group; a C3-6 cycloalkoxy group; a C1-6 alkylthio group; an amino group substituted with a C1-6 alkyl group; a C3-6 cycloalkyl group; a phenyl group; a pyrazolyl group substituted with a C1-6 alkyl group; an oxetanyl group; or an oxetanyloxy group, and more preferably a C1-6 alkyl group; a C1-6 haloalkyl group; a C1-6 alkoxy group; a C1-6 haloalkoxy group; a C1-6 alkoxy group substituted with a C3-6 cycloalkyl group; a C1-6 alkoxy group substituted with a phenyl group; a C3-6 cycloalkoxy group; a C1-6 alkylthio group; an amino group substituted with a C1-6 alkyl group; a C3-6 cycloalkyl group; a phenyl group; a pyrazolyl group substituted with a C1-6 alkyl group; an oxetanyl group; or an oxetanyloxy group.

n indicates the repeating number of structural unit in square brackets and is an integer of 1 to 3.

n is preferably 1 or 2, and more preferably 1.

The hydrazide compound according to the present invention is preferably a compound of formula (I) or a salt thereof. Each symbol in formula (I) means the same as that in the formula (Z-1).

[Chemical Formula 21]

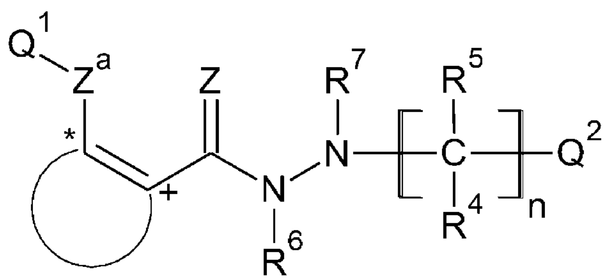

(I)

The hydrazide compound according to the present invention, in which n is 1, is a compound of formula (Ia) or a salt thereof. Each symbol in formula (Ia) means the same symbol as that in the formula (Z-1).

[Chemical Formula 22]

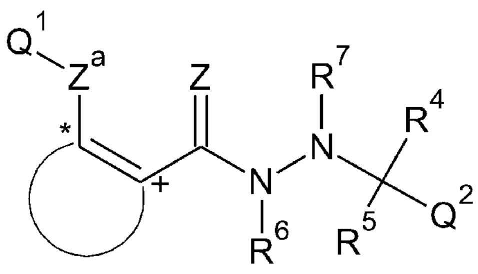

(Ia)

As one example of the hydrazide compound according to the present invention, the following compound of formula (I-A1) or a salt thereof can be mentioned.

Each symbol in formula (1-A1) means the same as that in the formula (Z-1).

Herein, $R^{7a}$ is a formyl group, a group of formula: $R^c$-CO-, a group of formula: $R^c$-CS-, a group of formula: $R^c$-CO-CO-, or a group of formula: $R^c$-$SO_2$-.

$R^c$ is the same as that defined as $R^7$ above.

[Chemical Formula 23]

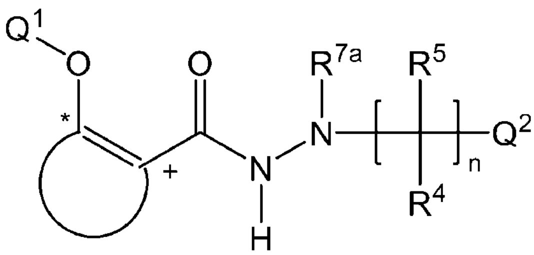

( I -A1 )

Specific examples of the hydrazide compound according to the present invention include a compound of formula (IIa) or a salt thereof, and a compound of formula (IIb) or a salt thereof. Each symbol in formula (IIa) or formula (IIb) means the same as that in formula (Z-I) and formula (II).

[Chemical Formula 24]

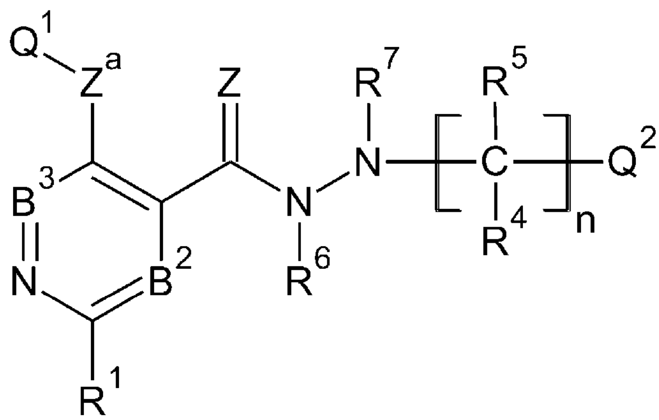

(IIa)

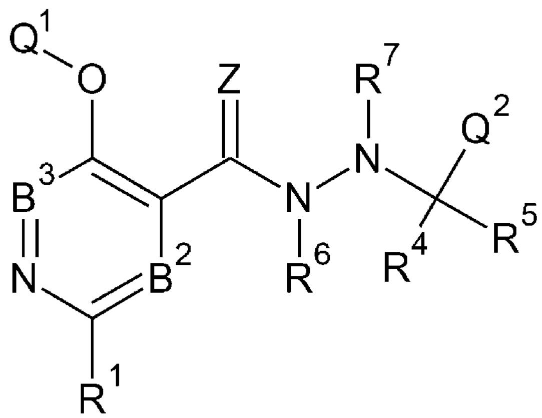

(IIb)

The salt of the compound (Z-I) is not particularly limited as long as the salt is an agriculturally and horticulturally acceptable salt. Examples of the salt of the compound (I) include salts of inorganic acids such as hydrochloric acid and sulfuric acid; salts of organic acids such as acetic acid and lactic acid; salts of alkali metals such as lithium, sodium, and potassium; salts of alkaline earth metals such as calcium and magnesium; salts of transition metals such as iron and

copper; ammonia; and salts of organic bases such as triethylamine, tributylamine, pyridine, and hydrazine.

The method for producing the compound (Z-I) or the salt of the compound (Z-1) is not particularly limited. For example, the compound (Z-I) or the salt of the compound (Z-1) can be obtained by a conventionally-known production method as described in the Examples, or the like. In addition, the salt of the compound (Z-I) can be obtained from the compound (Z-I) by a conventionally-known method.

Method for producing the compound (I)

Production method 1

A compound of formula (I-1) can be produced by a method including a reaction step of a compound of formula (im-1) and a compound of formula (im-2), as shown in Scheme 1.

[Chemical Formula 25]

Scheme 1

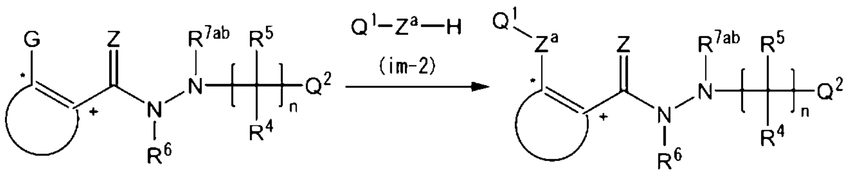


( im-1)

( I -1 )

Among the compounds (I), the compound of formula (I-1) is a compound in which $R^7$ is a group other than a hydrogen atom in the definition of formula (Z-I) (hereinafter, may be indicated as $R^{7ab}$).

The compound of formula (im-1) is a compound in which G is a halogeno group, and the other symbols are the same as those defined in the compound of formula (1-1).

The compound of formula (im-2) is a compound in which symbols are the same as those defined in the compound of formula (Z-I).

The compound of formula (I-1) can be produced by reacting the compound of formula (im-1) with the compound of formula (im-2) by a conventionally-known method. The reaction may be carried out in the presence of an acid scavenger.

Examples of the acid scavenger include common inorganic bases and organic bases. Alkali metal carbonates, alkaline earth metal acetates, tertiary amines or aromatic amines are preferable.

The compound of formula (im-2) may be commercially available, or may be produced by a conventionally-known method.

The compound of formula (im-1) may be produced by at least one method described in the present specification (see Production method 4).

Production method 2

A compound of formula (I-3) can be produced by a method including a reaction step of a compound of formula (im-5) and a compound of formula (im-6), as shown in Scheme 2.

[Chemical Formula 26]

Scheme 2

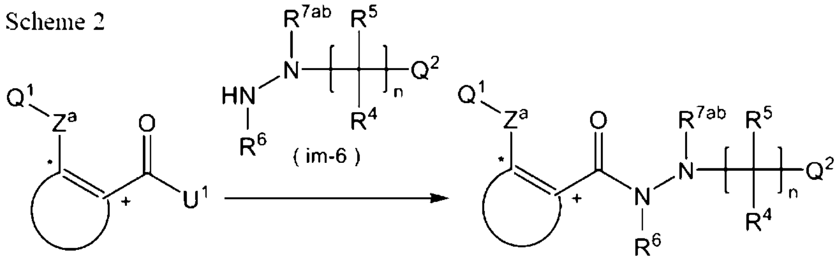


( im-5 )

( I -3 )

Among the compound (I), the compound of formula (I-3) is a compound in which Z is an oxygen atom, and $R^7$ is a group other than a hydrogen atom in the definition of formula (Z-1) ($R^{7ab}$).

The compound of formula (im-5) is a compound in which $U^1$ is a hydroxy group or a halogeno group, and the other symbols are the same as those defined in the compound of formula (I).

The compound of formula (im-6) is a compound in which $R7^{ab}$ is the same as that defined in the compound of formula (I-1), and the other symbols are the same as those defined in the compound of formula (Z-I).

The compound of formula (I-3) can be produced by reacting the compound of formula (im-5) in which $U^1$ is a hydroxy group with the compound of formula (im-6) in the presence of a condensing agent.

Although examples of the condensing agent include halogenating agents (such as phosgene, phosphorus tribromide, phosphorus trichloride, phosphorus pentachloride, phosphorus trichloride oxide, oxalyl chloride, and thionyl chloride), dehydrating agents (such as ethyl chloroformate, methyl chloroformate, isopropyl chloroformate, isobutyl chloroformate, and methanesulfonyl chloride), and carbonyldiimidazole, the condensing agent is not limited to these.

Alternatively, the compound of formula (I-3) can be produced by reacting the compound of formula (im-5) in which $U^1$ is a hydroxy group with an organic base in the presence of a condensing agent such as carbodiimide (such as N,N'-dicyclohexylcarbodiimide (DCC)), 1-hydroxy-7-azobenzotriazole (HOAt), O-(7-azabenzotriazol-1-yl)-N,N,N',N'-tetramethyluronium hexafluorophosphate (HATU), or propylphosphonic acid anhydride. 1-Hydroxybenzotriazole may also be used in combination.

The compound of formula (I-3) can also be produced by reacting the compound of formula (im-5) in which $U^1$ is a halogen atom with the compound of formula (im-6) by a conventionally-known method in the presence of an acid scavenger.

Examples of the acid scavenger include common inorganic bases and organic bases. Alkali metal carbonates, alkaline earth metal acetates, tertiary amines or aromatic amines are preferable.

The compound of formula (im-5) may be commercially available, or may be produced by a method described in documents (such as such as Japanese Unexamined Patent Application Publication (Translation of PCT Application) No. 2022-508277, or Japanese Unexamined Patent Application Publication (Translation of PCT Application) No. 2022-514651). Alternatively, the compound of formula (im-5) may be produced by at least one method described in the present specification (see Production method 4).

The compound of formula (im-6) may be commercially available, or may be produced by at least one of methods described in the present specification (see Production methods 5, 6, and 7).

Herein, the compound of formula (I-3) can be converted to a compound in which Z is a sulfur atom by using a thionating agent.

Although examples of the thionating agent include sulfur, hydrogen sulfide acid, sodium sulfide, sodium hydrosulfide, boron trisulfide, bis(diethylaluminum) sulfide, ammonium sulfide, phosphorus pentasulfide, and Lawesson's reagent, the thionating agent is not limited to these.

The reaction may be carried out in the presence of an inorganic base or an organic base, as needed. Alkali metal carbonates, alkaline earth metal acetates, tertiary amines or aromatic amines are preferable.

Production method 3

A compound of (1m-1a) can be produced by a method including a reaction step of a compound of formula (im-7) and the compound of formula (im-6), as shown in Scheme 3.

[Chemical Formula 27]

Scheme 3

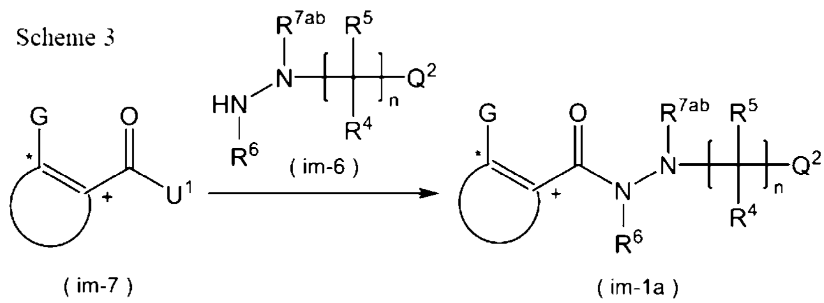

( im-7 ) ( im-6 ) ( im-1a )

Among the compounds of formula (im-1), the compound of (1m-1a) is a compound in which Z is an oxygen atom.

The compound of formula (im-7) is a compound in which G is a halogeno group, $U^1$ is a hydroxy group or a halogeno group, and the other symbols are the same as those defined in the compound of formula (Z-I).

Agents used in Production method 3 and reaction conditions may be the same as those described in Production method 2.

The compound of formula (im-7) may be commercially available, or may be produced by a method described in documents (such as Japanese Unexamined Patent Application Publication (Translation of PCT Application) No. 2022-508277, or Japanese Unexamined Patent Application Publication (Translation of PCT Application) No. 2022-514651).

The compound of formula (1m-1a) can be converted to a compound in which Z is a sulfur atom by using a thionating agent. As the method which allows conversion to proceed, the method described in Production method 2 may be used.

Production method 4

The compound of formula (im-5) can be produced by a method including: a reaction step of a compound of formula (im-9) and the compound of formula (im-2); and a reaction step in which a compound of formula (im-10) obtained by the reaction is converted to the compound of formula (im-5), as shown in Scheme 4.

[Chemical Formula 28]

Scheme 4

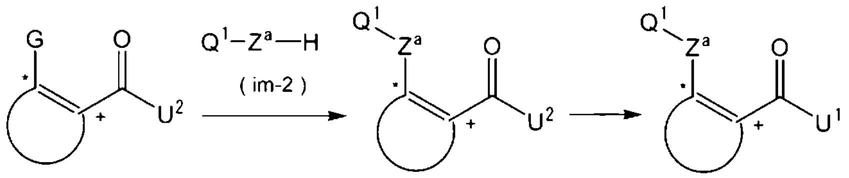


( im-9 ) ( im-10 ) ( im-5 )

The compound of formula (im-9) is a compound in which $U^2$ is a C1-6 alkoxy group, G is a halogeno group, and the other symbols are the same as those defined in the compound of formula (Z-I).

The compound of formula (im-10) is a compound in which $U^2$ is a C1-6 alkoxy group, and the other symbols are the same as those defined in the compound of formula (Z-I).

The compound of formula (im-10) can be produced by reacting the compound of formula (im-9) with the compound of formula (im-2) by a conventionally-known method. The reaction may be carried out in the presence of an acid scavenger.

Examples of the acid scavenger include common inorganic bases and organic bases. Alkali metal carbonates, alkaline earth metal acetates, tertiary amines or aromatic amines are preferable.

The compound of formula (im-5) in which $U^1$ is a hydroxy group can be produced by hydrolyzing the compound of formula (im-10) under acidic conditions or basic conditions.

For example, a method in which trifluoroacetic acid is used to realize acidic conditions, and a method in which lithium hydroxide is used in a mixed solvent system of tetrahydrofuran (THF) and water to realize basic conditions can be mentioned.

The compound of formula (im-5) in which $U^1$ is a halogeno group can be produced by reacting the compound of formula (im-5) in which $U^1$ is a hydroxy group with a halogenating agent.

The compound of formula (im-9) may be commercially available, or may be produced by a conventionally-known method.

Production method 5

A compound of formula (im-6a) can be produced by a method including: a reaction step of a compound of formula (im-19) and a compound of formula (im-20); and a reaction step of a compound of formula (im-21) obtained by the reaction and hydrazines, as shown in Scheme 5.

[Chemical Formula 29]

Scheme 5

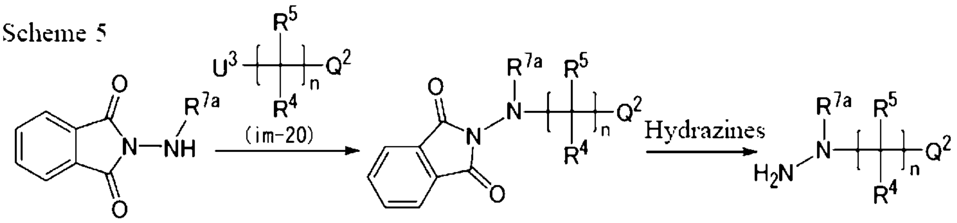


(im-19) (im-21) (im-6a)

Among the compounds of formula (im-6), the compound of formula (im-6a) is a compound in which R7[ab] is "a formyl group, a group of formula: $R^c$-CO-, a group of formula: $R^c$-CS-, a group of formula: $R^c$-CO-CO-, or a group of formula: $R^c$-$SO_2$-", in the definition of formula (Z-I) (hereinafter, may be indicated as $R^{7a}$), and $R^6$ is a hydrogen atom.

The compound of formula (im-19) is a compound in which $R^{7a}$ is the same as that defined in the compound of formula (im-6a).

The compound of formula (im-20) is a compound in which $U^3$ is a hydroxy group or a halogeno group, and the other symbols are the same as those defined in the compound of formula (Z-I).

The compound of formula (im-21) is a compound in which $R^{7a}$ is the same as that defined in the compound of formula (im-6a), and the other symbols are the same as those defined in the compound of formula (Z-I).

The compound of formula (im-21) can be produced by reacting the compound of formula (im-20) in which $U^3$ is a hydroxy group with the compound of formula (im-19) in the presence of dialkyl azodicarboxylate and triphenylphosphine, the so-called Mitunobu reaction.

Although examples of the dialkyl azodicarboxylate include diethyl azodicarboxylate, and diisopropyl azodicarboxylate, the dialkyl azodicarboxylate is not limited to these.

The compound of formula (im-21) can be produced by reacting the compound of formula (im-20) in which $U^3$ is a halogen atom with the compound of formula (im-19) in the presence of an acid scavenger by a conventionally-known method.

Examples of the acid scavenger include common inorganic bases and organic bases. Alkali metal carbonates, alkaline earth metal acetates, tertiary amines or aromatic amines are preferable.

The compound of formula (im-6a) can be produced by reacting the compound of formula (im-21) with a hydrazine.

Examples of the hydrazine include hydrazine monohydrate and methylhydrazine.

The compound of formula (im-19) may be commercially available, or may be produced by reacting a compound of formula: $NH_2$-NH-$R^{7a}$ (wherein $R^{7a}$ is the same as that defined in the compound of formula (im-6a)) and phthalic anhydride.

The compound of formula (im-20) may be commercially available, or may be produced by a conventionally-known method.

Production method 6

The compound of formula (im-6b) can be produced by a method including a reaction step in which a compound of formula (im-22) is N-aminated, as shown in Scheme 6.

[Chemical Formula 30]

Scheme 6

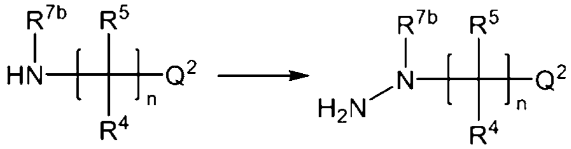

( im-22 ) ( im-6b )

Among the compounds of formula (im-6), the compound of formula (im-6b) is a compound in which R7[ab] is "a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted

phenyl group, a substituted or unsubstituted naphthyl group, or a substituted or unsubstituted five- or six-membered heterocyclyl group" in the definition of formula (Z-1) (hereinafter, may be indicated as $R7^b$) and $R^6$ is a hydrogen atom.

The compound of formula (im-22) is a compound in which $R^{7b}$ is the same as that defined in the compound of formula (im-6b), and the other symbols are the same as those defined in the compound of formula (Z-I).

The compound of formula (im-6b) can be produced by a method in which the compound of formula (im-22) is reacted with an aminating agent such as O-mesitylenesulfonylhydroxylamine or O- (2,4-dinitrophenyl)hydroxyamino, or a method in which the compound of formula (im-22) is reacted with sodium nitrite under acidic conditions to convert it to an N-nitrosamine, followed by reacting the resultant with a reducing agent. Although examples of the reducing agent include lithium aluminum hydride and diisobutylaluminum hydride, the reducing agent is not limited to these.

The compound of formula (im-22) may be commercially available, or may be produced by a conventionally-known method.

Production method 7

A compound of formula (im-6c) can be produced by a method including a reaction step of the compound of formula (im-6a) or the compound of formula (im-6b) with the compound of formula (im-23), as shown in Scheme 7.

[Chemical Formula 31]

Scheme 7

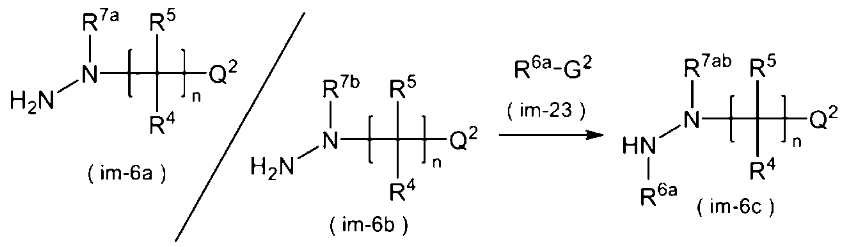

( im-6a ) ( im-6b ) ( im-6c )

Among the compounds of formula (im-6), the compound of formula (im-6c) is a compound in which $R^6$ is a group other than a hydrogen atom in the definition of formula (Z-I) (hereinafter, may be indicated as $R^{6a}$).

The compound of formula (im-23) is a compound in which $G^2$ is a halogeno group, and $R^{6a}$ is the same as that defined in the formula (im-6c).

The compound of formula (im-6c) can be produced by reacting the compound of formula (im-6a) or the compound of formula (im-6b) with the compound of formula (im-23). The reaction is preferably carried out in the presence of an inorganic base.

The compound of formula (im-23) may be commercially available, or may be produced by a conventionally-known method.

Production method of compound (I-A1)

Production method 8

The compound of formula (I-A1) can be produced by a method including a reaction step of a compound of formula (im-A1) and a compound of formula (im-A2), as shown in Scheme 8.

[Chemical Formula 32]

Scheme 8

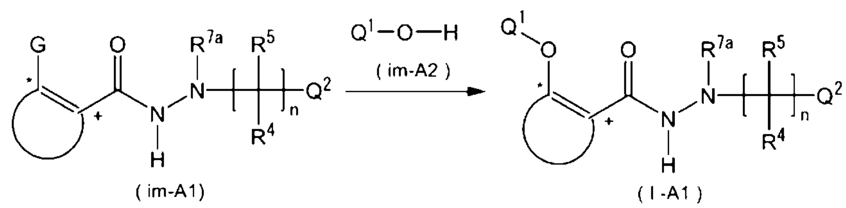

( im-A1) ( I -A1 )

The compound of formula (im-A1) is a compound in which G is a halogeno group, and the other symbols are the same as those defined in the compound of formula (I-A1).
The compound of formula (im-A2) is a compound in which a symbol is the same as that defined in the compound of formula (Z-1).
The compound of formula (I-A1) can be produced by reacting the compound of formula (im-A1) with the compound of formula (im-A2) by a conventionally-known method. The reaction may be carried out in the presence of an acid scavenger.
Examples of the acid scavenger include common inorganic bases and organic bases. Alkali metal carbonates, alkaline earth metal acetates, tertiary amines or aromatic amines are preferable.
The compound of formula (im-A2) may be commercially available, or may be produced by a conventionally-known method.
The compound of formula (im-A1) may be produced by at least one method described in the present specification (see Production method 10).

Production method 9

The compound of formula (I-A1) can be produced by a method including a reaction step of a compound of formula (im-A5) and the compound of formula (im-6a), as shown in Scheme 9.

[Chemical Formula 33]

Scheme 9

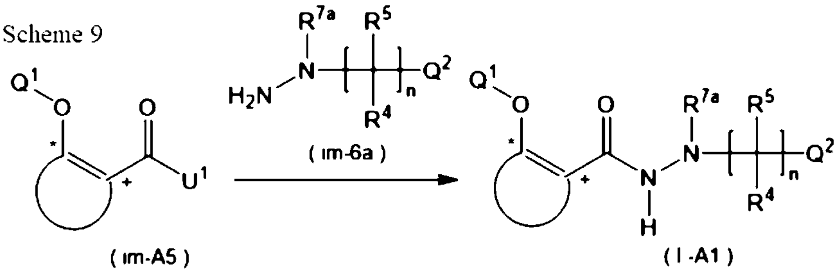

The compound of formula (im-A5) is a compound in which $U^1$ is a hydroxy group or a halogeno group, the other symbols are the same as that defined in the compound of formula (Z-I).
The compound of formula (im-6a) is a compound in which $R^{7a}$ is the same as that defined in the compound of formula (I-A1), and the other symbols are the same as those defined in the compound of formula (Z-I).
The compound of formula (I-A1) can be produced by reacting the compound of formula (im-A5) in which $U^1$ is a hydroxy group with the compound of formula (im-6a) in the presence of a condensing agent.
Although examples of the condensing agent include halogenating agents (such as phosgene, phosphorus tribromide, phosphorus trichloride, phosphorus pentachloride, phosphorus trichloride oxide, oxalyl chloride, and thionyl chloride), dehydrating agents (such as ethyl chloroformate, methyl chloroformate, isopropyl chloroformate, isobutyl chloroformate, and methanesulfonyl chloride), and carbonyldiimidazole, the condensing agent is not limited to these.
Alternatively, the compound of formula (I-A1) can be produced by reacting the compound of formula (im-A5) in which $U^1$ is a hydroxy group with an organic base in the presence of a condensing agent such as carbodiimide (such as N,N'-dicyclohexylcarbodiimide (DCC)), 1-hydroxy-7-azobenzotriazole (HOAt), O-(7-azabenzotriazol-1-yl)-N,N,N',N'-tetramethyluronium hexafluorophosphate (HATU), or propylphosphonic acid anhydride. 1-Hydroxybenzotriazole may also be used in combination.
The compound of formula (I-A1) can be produced by reacting the compound of formula (im-A5) in which $U^1$ is a halogen atom with the compound of formula (im-6a) by a conventionally-known method in the presence of an acid scavenger.
Examples of the acid scavenger include common inorganic bases and organic bases. Alkali metal carbonates, alkaline earth metal acetates, tertiary amines or aromatic amines are preferable.
The compound of formula (im-A5) may be commercially available, or may be produced by a method described in documents (such as Japanese Unexamined Patent Application Publication (Translation of PCT Application) No. 2022-508277, or Japanese Unexamined Patent Application Publication (Translation of PCT Application) No. 2022-514651). Alternatively, the compound of formula (im-A5) may be produced by at least one of methods described in the present specification (see Production methods 4 and 11).
The compound of formula (im-6a) may be commercially available. Alternatively, the compound of formula (im-6a) may be produced by at least one method described in the present specification (see Production method 5).

Production method 10

The compound of formula (im-A1) can be produced by a method including a reaction step of the compound of formula (im-7) and the compound of formula (im-6a), as shown in Scheme 10.

[Chemical Formula 34]

Scheme 10

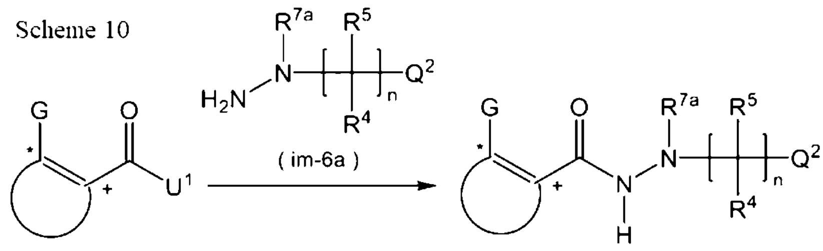


( im-7 ) ( im-A1 )

The compound of formula (im-7) is a compound in which G is a halogeno group, $U^1$ is a hydroxy group or a halogeno group, and the other symbols are the same as those defined in the compound of formula (Z-I).
Agents used in Production method 10 and reaction conditions may be the same as those described in Production method 9.
The compound of formula (im-7) may be commercially available, or may be produced by a method described in documents (such as Japanese Unexamined Patent Application Publication (Translation of PCT Application) No. 2022-508277, or Japanese Unexamined Patent Application Publication (Translation of PCT Application) No. 2022-514651).

Production method 11

The compound of formula (im-A5) can be produced by a method including: a reaction step of the compound of formula (im-9) and the compound of formula (im-A2); and a reaction step in which a compound of formula (im-A10) obtained by the reaction is converted to the compound of formula (im-A5), as shown in Scheme 11.

[Chemical Formula 35]

Scheme 11

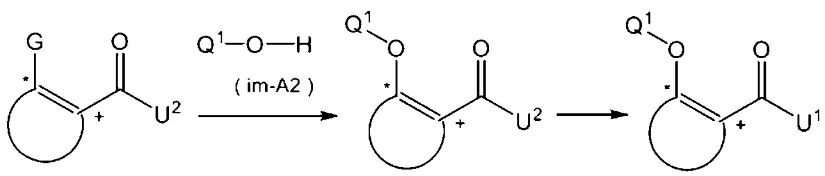


( im-9 ) ( im-A10 ) ( im-A5 )

The compound of formula (im-9) is a compound in which $U^2$ is a C1-6 alkoxy group, G is a halogeno group, and the other symbols are the same as those defined in the compound of formula (Z-I).
The compound of formula (im-A10) is a compound in which $U^2$ is a C1-6 alkoxy group, and the other symbols are the same as those defined in the compound of formula (Z-I).
The compound of formula (im-A10) can be produced by reacting the compound of formula (im-9) with the compound of formula (im-A2) by a conventionally-known method. The reaction may be carried out in the presence of an acid scavenger.
Examples of the acid scavenger include common inorganic bases and organic bases. Alkali metal carbonates, alkaline earth metal acetates, tertiary amines or aromatic amines are preferable.
The compound of formula (im-A5) in which $U^1$ is a hydroxy group can be produced by hydrolyzing the compound of formula (im-A10) under acidic conditions or basic conditions.
For example, a method in which trifluoroacetic acid is used to realize acidic conditions, and a method in which lithium hydroxide is used in a mixed solvent system composed of THF and water to realize basic conditions can be mentioned.

The compound of formula (im-A5) in which $U^1$ is a halogeno group can be produced by reacting the compound of formula (im-A5) in which $U^1$ is a hydroxy group with a halogenating agent.
The compound of formula (im-9) may be commercially available, or may be produced by a conventionally-known method.

Production method of compound (I')

Among the compounds (Z-I), a compound in which T in the formula (Z-1) is the partial structure (T-2) is a compound of the following formula (I'). Each symbol in formula (I') means the same as that in the formula (Z-1).

[Chemical Formula 36]

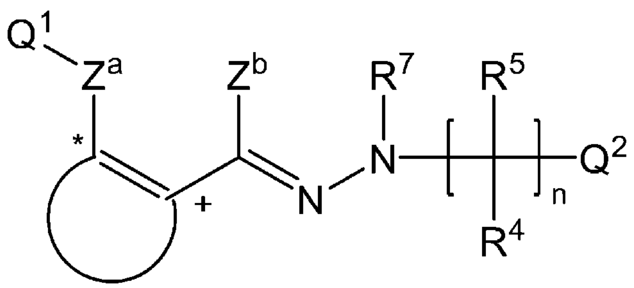

( I ' )

The compound of formula (I') (hereinafter, may be indicated as compound (I')) can be produced by thioamidating the compound (I) using a thionating agent such as Lawesson's reagent, followed by reacting the resultant with a corresponding alkyl halide, when $Z^b$ is a C1-6 alkylthio group.
The compound (I') can be produced by reacting the compound (I) with an oxonium salt such as trimethyloxonium tetrafluoroborate or triethyloxonium tetrafluoroborate, when $Z^b$ is a C1-6 alkoxy group.
An agricultural and horticultural fungicide according to the present invention contains at least one selected from the group consisting of the compound (I) and the salt of the compound (I), as an active ingredient (hereinafter, may be indicated as an active ingredient (I)). The amount of the active ingredient (I) contained in the agricultural and horticultural fungicide according to the present invention is not particularly limited, as long as fungicidal effects are exhibited.
The agricultural and horticultural fungicide according to the present invention can be used to control plant diseases caused by a wide variety of filamentous fungi, such as fungi belonging to algae fungi (Oomycetes), sac fungi (Ascomycetes), imperfect fungi (Deuteromycetes), Basidiomycete fungi (Basidiomycetes) or zygomycete fungi (Zygomycetes).
Examples of plant diseases (pathogens) to be controlled include the following.
Sugar beet: brown spot disease (Cercospora beticola), black root disease (Aphanomyces cochlioides), root rot disease (Thanatephorus cucumeris), leaf rot disease (Thanatephorus cucumeris), rust disease (Uromyces betae), powdery mildew disease (Oidium sp.), spot blotch disease (Ramularia beticola), damping-off disease (Aphanomyces cochlioides, or Pythium ultimum), and the like.
Peanut: brown spot disease (Mycosphaerella arachidis), leaf mold (Ascochyta sp.), rust disease (Puccinia arachidis), damping-off disease (Pythium debaryanum), rust spot disease (Alternaria alternata), stem rot disease (Sclerotium rolfsii), black rust disease (Mycosphaerella berkeleyi), red crown rot disease (Calonectria ilicicola), and the like.
Cucumber: powdery mildew disease (Sphaerotheca fuliginea), downy mildew disease (Pseudoperonospora cubensis), gummy stem blight disease (Mycosphaerella melonis), wilt disease (Fusarium oxysporum), sclerotinia rot disease (Sclerotinia sclerotiorum), gray mold disease (Botrytis cinerea), anthracnose (Colletotrichum orbiculare), scab (Cladosporium cucumerinum), brown spot disease (Corynespora cassiicola), damping-off disease (Pythium debaryanum, Rhizoctonia solani Kuhn), Phomopsis root rot disease (Phomopsis sp.), Bacterial spot (Pseudomonas syringae pv. Lachrymans), and the like.
Tomato: gray mold disease (Botrytis cinerea), leaf mold disease (Cladosporium fulvum), late blight disease (Phytophthora infestans), verticillium wilt disease (Verticillium albo-atrum, Verticillium dahliae), powdery mildew disease (Oidium neolycopersici), early blight disease (Alternaria solani), leaf mold disease (Pseudocercospora fuligena), bacterial wilt disease (Ralstonia solanacearum), sclerotinia rot disease (Sclerotinia sclerotiorum), and the like.
Eggplant: gray mold disease (Botrytis cinerea), black rot disease (Corynespora melongenae), powdery mildew disease (Erysiphe cichoracearum), leaf mold disease (Mycovellosiella nattrassii), sclerotinia rot disease (Sclerotinia sclerotiorum), verticillium wilt disease (Verticillium dahliae), phomopsis blight disease (Phomopsis vexans), and the like.

Pepper: Phytophthora rot disease (Phytophthora capsici), gray mold disease (Botrytis cinerea), sclerotinia rot disease (Sclerotinia sclerotiorum), anthracnose (Colletotrichum aenigma, Colletotrichum capsici, Colletotrichum fructicola, Colletotrichum jiangxiense), powdery mildew disease (Leveillula taurica), and the like.

Strawberry: gray mold disease (Botrytis cinerea), powdery mildew disease (Sphaerotheca humuli), anthracnose (Colletotrichum acutatum, Colletotrichum fragariae), phytophthora rot disease (Phytophthora cactorum), soft rot disease (Rhizopus stolonifer), fusarium wilt disease (Fusarium oxysporum), verticillium wilt disease (Verticillium dahliae), sclerotinia rot disease (Sclerotinia sclerotiorum), and the like.

Onion: neck rot disease (Botrytis allii), gray mold disease (Botrytis cinerea), leaf blight disease (Botrytis squamosa), downy mildew disease (Peronospora destructor), phytophthora porri disease (Phytophthora porri), leaf blight disease (Ciobrinia allii), small sclerotial neck rot disease (Botrytis squamosa), fusarium basal rot disease (Fusarium oxysporum), pink root rot disease (Pyrenochaeta terrestris), white rot disease (Sclerotium cepivorum), rust disease (Puccinia allii), southern blight disease (Sclerotium rolfsii) and the like.

Green onion: soft rot disease (Pectobacterium carotovorum), downy mildew disease (Peronospora destructor), leaf scorch disease (Pleospora allii), bulb black rot disease (Sclerotium cepivorum), rust disease (Puccinia allii), leaf blight disease (Botrytis squamosa), southern blight disease (Sclerotium rolfsii), pink root rot disease (Pyrenochaeta terrestris), and the like.

Cabbage: clubroot disease (Plasmodiophora brassicae), soft rot disease (Erwinia carotovora), black rot disease (Xanthomonas campesrtis pv. campestris), bacterial black spot disease (Pseudomonas syringae pv. maculicala, Pseudomonas syringae pv. alisalensis), downy mildew disease (Peronospora parasitica), sclerotinia rot disease (Sclerotinia sclerotiorum), black spot disease (Alternaria brassicicola), gray mold disease (Botrytis cinerea), black leg disease (Phoma lingam), Pythium rot disease (Pythium aphanidermatum, Pythium ultimum), white rust disease (Albugo macrospora),and the like.

Lettuce: bacterial rot disease (Pseudomonas cichorii, Pseudomonas marginalis), bacterial soft rot disease (Pectobacterium carotovorum), downy mildew disease (Bremia lactucae), gray mold disease (Botrytis cinerea), stem rot disease (Sclerotinia sclerotiorum), big vein disease (Mirafiori lettuce big-vein ophiovirus), root rot disease (Fusarium oxysporum), bottom rot disease (Rhizoctonia solani), powdery mildew disease (Golovinomyces orontii), and the like.

Common bean: sclerotinia rot disease (Sclerotinia sclerotiorum), gray mold disease (Botrytis cinerea), anthracnose (Colletotrichum lindemuthianum), angular spot disease (Phaeoisariopsis griseola), and the like.

Pea: Mycosphaerella blight disease (Mycosphaerella blight), gray mold disease (Botrytis cinerea), stem rot disease (Sclerotinia sclerotiorum), powdery mildew disease (Erysiphe pisi), and the like.

Apple: powdery mildew disease (Podosphaera leucotricha), scab disease (Venturia inaequalis), Monilinia disease (Monilinia mali), black spot disease (Mycosphaerella pomi), valsa canker disease (Valsa mali), alternaria blotch disease (Alternaria mali), rust disease (Gymnosporangium yamadae), ring rot disease (Botryosphaeria berengeriana), anthracnose (Glomerella cingulata, Colletotrichum acutatum), leaf srot disease (Diplocarpon mali), fly speck disease (Zygophiala jamaicensis), Sooty blotch disease (Gloeodes pomigena), violet root rot disease (Helicobasidium mompa), white root rot disease (Rosellinia necatrix), gray mold disease (Botrytis cinerea), fire blight disease (Erwinia amylovora), silver leaf disease (Chondrostereum purpureum), crown gall disease (Rhizobium radiobacter, Rhizobium rhizogenes) and the like.

Japanese apricot: scab disease (Cladosporium carpophilum), gray mold disease (Botrytis cinerea), brown rot disease (Monilinia mumecola), sooty blotch disease (Peltaster sp.), plum pockets disease (Taphrina pruni), cylindrosporium leaf spot disease (Phloeosporella padi), and the like.

Persimmon: powdery mildew disease (Phyllactinia kakicola), anthracnose (Gloeosporium kaki), angular leaf spot (Cercospora kaki), circular leaf spot (Mycosphaerella nawae), gray mold disease (Botrytis cinerea), fly speck disease (Zygophiala jamaicensis) and the like.

Peach: brown rot disease (Monilinia fructicola, Monilia fructigena), scab disease (Cladosporium carpophilum), phomopsis rot disease (Phomopsis sp.), bacterial shot hole disease (Xanthomonas campestris pv. pruni), leaf curl disease (Taphrina deformans), anthracnose (Colletotrichum gloeosporioides), cylindrosporium leaf spot disease (Phloeosporella padi), Coriolus versicolor disease (Coriolus versicolor) and the like.

Almond: brown rot disease (Monilinia laxa), spot blotch disease (Stigmina carpophila), scab disease (Cladosporium carpophilum), red leaf spot disease (Polystigma rubrum), alternaria blotch disease (Alternaria alternata), anthracnose (Colletotrichum gloeospoides), and the like.

Yellow peach: brown rot disease (Monilinia fructicola), anthracnose (Colletotrichum acutatum), black spot disease (Alternaria sp.), Monilinia Kusanoi disease (Monilinia kusanoi), Mycosphaerella leaf spot disease (Mycosphaerella cerasella), powdery mildew disease (Podosphaera tridactyla), and the like.

Grape: gray mold disease (Botrytis cinerea), powdery mildew disease (Uncinula necator), ripe rot disease (Glomerella cingulata, Colletotrichum acutatum), downy mildew disease (Plasmopara viticola), anthracnose (Elsinoe ampelina), brown spot disease (Pseudocercospora vitis), black rot disease (Guignardia bidwellii), white rot disease (Coniella castaneicola), rust disease (Phakopsora ampelopsidis), Cottony bunch disease (the pathogen thereof has not

been identified), crown gall disease (Rhizobium radiobacter, Rhizobium vitis), and the like.

Pear: scab disease (Venturia nashicola), rust disease (Gymnosporangium asiaticum), black spot disease (Alternaria kikuchiana), ring rot disease (Botryosphaeria berengeriana), powdery mildew disease (Phyllactinia mali), cytospora canker disease (Phomopsis fukushii), brown spot blotch disease (Stemphylium vesicarium), anthracnose (Glomerella cingulata), and the like.

Tea: ring spot disease (Pestalotiopsis longiseta, P. theae), anthracnose (Colletotrichum theae-sinensis), net blister blight disease (Exobasidium reticulatum), bacterial shoot blight disease (Pseudomonas syringae), blister blight disease (Exobasidium vexans), and the like.

Citrus fruits: scab disease (Elsinoe fawcetti), blue mold disease (Penicillium italicum), common green mold disease (Penicillium digitatum), gray mold disease (Botrytis cinerea), melanose disease (Diaporthe citri), canker disease (Xanthomonas campestris pv.Citri), powdery mildew disease (Oidium sp.), brown rot disease (Phytophthora citrophthora), anthracnose (Colletotrichum fioriniae), and the like.

Kiwi fruit: bacterial bud rot disease (Pseudomonas marginalis, Pseudomonas syringae, Pseudomonas viridiflava), bacterial canker disease (Pseudomonas syringae), gray mold disease (Botrytis cinerea), soft rot disease (Botryosphaeria dothidea, Diaporthe sp., Lasiodiplodia theobromae), sooty spot disease (Pseudocercospora actinidiae), and the like.

Olive: anthracnose (Colletotrichum acutatum, Colletotrichum gloeosporioides), Peacock spot (Spilocaea oleaginea), and the like.

Chestnut: anthracnose (Colletotrichum gloeosporioides), and the like.

Wheat: powdery mildew disease (Blumeria graminis f.sp. tritici), red mold disease (Gibberella zeae, Fusarium avenaceum, Fusarium culmorum, Fusarium crookwellense, Microdochium nivale), red rust disease (Puccinia recondita), yellow rust disease (Puccinia striiformis), brown snow mold disease (Pythium iwayamai), pink snow mold disease (Monographella nivalis), eye spot disease (Pseudocercosporella herpotrichoides), leaf scorch disease (Septoria tritici), glume blotch disease (Leptosphaeria nodorum), typhulasnow blight disease (Typhula incarnata), sclerotinia snow blight disease (Myriosclerotinia borealis), damping-off disease (Gaeumannomyces graminis), ergot disease (Claviceps purpurea), stinking smut disease (Tilletia caries), loose smut disease (Ustilago nuda), blast disease (Pyricularia grisea), damping-off disease (Pythium spp., Fusarium spp., Rhizoctonia spp.), seedling blight disease (Pythium spp., Fusarium spp., Rhizoctonia spp.) and the like.

Barley: leaf spot disease (Pyrenophora graminea), net blotch disease (Pyrenophora teres), leaf blotch disease (Rhynchosporium secalis), loose smut disease (Ustilago tritici, U.nuda), damping-off disease (Pythium spp., Fusarium spp., Rhizoctonia spp.), seedling blight disease (Pythium spp., Fusarium spp., Rhizoctonia spp.) and the like.

Rice: blast disease (Pyricularia oryzae), sheath blight disease (Rhizoctonia solani), bakanae disease (Gibberella fujikuroi), brown spot disease (Cochliobolus miyabeanus), damping-off disease (Pythium graminicolum), bacterial leaf blight disease (Xanthomonas oryzae), bacterial seedling blight disease (Burkholderia plantarii), brown stripe disease (Acidovorax avenae), bacterial grain rot disease (Burkholderia glumae), Cercospora leaf spot disease (Cercospora oryzae), false smut disease (Ustilaginoidea virens), rice brown spot disease (Alternaria alternata, Curvularia intermedia), kernel discoloration of rice (Alternaria padwickii), pink coloring of rice grains (Epicoccam purpurascenns), and the like.

Tobacco: sclerotinia rot disease (Sclerotinia sclerotiorum), powdery mildew disease (Erysiphe cichoracearum), phytophthora rot disease (Phytophthora nicotianae), and the like.

Tulip: gray mold disease (Botrytis cinerea), Botrytis blight disease (Botrytis tulipae), leaf rot disease (Rhizoctonia solani), bulb rot disease (Rhizoctonia solani), bulb-coat rot disease (Rhizoctonia solani), and the like.

Rose: black spot disease (Diplocarpon rosae), powdery mildew disease (Erysiphe simulans, Podosphaera pannosa), gray mold disease (Botrytis cinerea), and the like.

Chrysanthemum: gray mold disease (Botrytis cinerea), rust disease (Puccinia horiana), downy mildew disease (Paraperonospora minor, Peronospora danica), Pythium root and stem rot disease (Pythium aphanidermatum, Pythium dissotocum, Pythium helicoides, Pythium oedochilum, Pythium sylvaticum), wilt disease (Rhizoctonia solani), Fusarium blight disease (Fusarium solani), and the like.

Gerbera: gray mold disease (Botrytis cinerea), powdery mildew disease (Podosphaera xanthii), and the like.

Lily: Botrytis blight disease (Botrytis elliptica, Pestalotiopsis sp.), gray mold disease (Botrytis cinerea), and the like.

Sunflower: downy mildew disease (Plasmopara halstedii), sclerotinia rot disease (Sclerotinia sclerotiorum), gray mold disease (Botrytis cinerea), and the like.

Bent grass: Sclerotinia snow blight disease (Sclerotinia borealis), large patch (Rhizoctonia solani), brown patch (Rhizoctonia solani), Dollar spot (Sclerotinia homoeocarpa), blast disease (Pyricularia sp.), Pythium red blight disease (Pythium aphanidermatum), anthracnose (Colletotrichum graminicola), and the like.

Orchard grass: powdery mildew disease (Erysiphe graminis), and the like.

Soybean: purple stain disease (Cercospora kikuchii), downy mildew disease (Peronospora manshurica), phytophthora rot disease (Phytophthora sojae), rust disease (Phakopsora pachyrhizi), sclerotinia rot disease (Sclerotinia

sclerotiorum), anthracnose (Colletotrichum truncatum), gray mold disease (Botrytis cinerea), Sphaceloma scab (Elsinoe glycines), black spot disease (Diaporthe phaseolorum var. sojae), damping-off disease (Pythium spp., Fusarium spp., Rhizoctonia spp.), seedling blight disease (Pythium spp.,Fusarium spp.,Rhizoctonia spp.), and the like.

Potato: Phytophthora rot disease (Phytophthora infestans), early blight disease (Aleternaria solani), scurf disease (Thanatephorus cucumeris), verticillium wilt disease (Verticillium albo-atrum, V. dahliae, V. nigrescens), blackleg disease (Pectobacterium atrosepticum), soft rot disease (Pectobacterium carotovorum), gray mold disease (Botrytis cinerea), scab (Streptomyces spp.), stem rot disease (Sclerotinia sclerotiorum), and the like.

Yam: leaf mold disease (hasibu-byo) (Cylindrosporium dioscoreae), anthracnose (Colletotrichum gloeosporioides), blue mold disease (Penicillium sclerotigenum), and the like.

Sweet potato: violet root rot disease (Helicobasidium mompa), wilt disease (Fusarium oxysporum), and the like.

Taro: Phytophthora rot disease (Phytophthora colocasiae), stem rot disease (Rhizoctonia solani), and the like.

Ginger: root rot disease (Pythium ultimum, Pythium myriotylum), leaf spot disease (Phyllosticta zingiberis), and the like.

Banana: Panama disease (Fusarium oxysporum), Sigatoka disease (Mycosphaerella fijiensis, M. musicola), and the like.

Mango: anthracnose (Colletotrichum aenigma), canker disease (Xanthomonas campestris), stem-end rot disease (Diaporthe pseudophoenicicola, Lasiodiplodia theobromae, Lasiodiplodia spp., Neofusicoccum parvum, Neofusicoccum sp.), gray mold disease (Botrytis cinerea), and the like.

Rape seed: sclerotinia rot disease (Sclerotinia sclerotiorum), root rot disease (Phoma lingam), black leaf spot disease (Alternaria brassicae), powdery mildew disease (Erysiphe cruciferarum, Erysiphe cichoracearum, Oidium matthiolae), downy mildew disease (Peronospora parasitica), and the like.

Coffee: rust disease (Hemileia vastatrix), anthracnose (Colletotrichum coffeanum), brown eye spot disease (Cercospora coffeicola), and the like.

Sugarcane: brown rust disease (Puccinia melanocephala), and the like.

Corn: zonate spot disease (Gloecercospora sorghi), rust disease (Puccinia sorghi), southern rust disease (Puccinia polysora), smut disease (Ustilago maydis), brown spot disease (Cochliobolus heterostrophus), northern leaf blight disease (Setophaeria turcica), damping-off disease (Pythium spp., Fusarium spp., Rhizoctonia spp.), seedling blight disease (Pythium spp., Fusarium spp., Rhizoctonia spp.), and the like.

Cotton: seedling blight disease (Pythium sp.), rust disease (Phakopsora gossypii), sour rot disease (Mycosphaerella areola), anthracnose (Glomerella gossypii), and the like.

Hop: downy mildew disease (Pseudoperonospora humuli), powdery mildew disease (Oidium sp., Podosphaera macularis), gray mold disease (Botrytis cinerea), and the like.

The agricultural and horticultural fungicide according to the present invention is preferably applied to cereals; vegetables; root vegetables; potatoes; trees, such as fruit-bearing trees, tea, coffee, or cacao; feed crops; lawn grasses; or plants such as cotton.

The agricultural and horticultural fungicide according to the present invention may be applied to any part of plants, such as leaves, stems, stalks, flowers, buds, fruits, seeds, sprouts, roots, tubers, tuberous roots, shoots, or slips. The agricultural and horticultural fungicide according to the present invention may also be applied to varieties, improved varieties, cultivars, mutants, hybrid bodies, or gene recombinants (GMO) of the plants.

The agricultural and horticultural fungicide according to the present invention may be used to carry out seed treatment, foliage application, soil application, or submerged application so as to control various diseases generated in agricultural and horticultural crop plants encompassing flowers, lawn grasses, and herbages.

The agricultural and horticultural fungicide according to the present invention may further contain additional components in addition to the active ingredient (I). Examples of the additional components include conventional carriers used to make formulations. Examples of other additional components include conventional fungicides, insecticides, acaricides, nematicides, soil pest control agents, plant regulatory agents, synergists, fertilizers, soil improvement agents, and animal feeds (hereinafter, may be indicated as active ingredient (II)). The agricultural and horticultural fungicide according to the present invention may exhibit synergistic effects by containing the active ingredient (I) and the active ingredient (II).

Specific examples of the active ingredient (II) which may be mixed or used with the agricultural and horticultural fungicide according to the present invention are shown below.

(1) Nucleic acid biosynthesis inhibitors:

(a) RNA polymerase I inhibitors: benalaxyl, benalaxyl-M, furalaxyl, metalaxyl, metalaxyl-M, oxadixyl, clozylacon, ofurace;
(b) Adenosine deaminase inhibitors: bupirimate, dimethirimol, ethirimol;
(c) DNA/RNA synthesis inhibitors: hymexazol, octhilinone;

(d) DNA topoisomerase II inhibitors: oxolinic acid.

(2) Mitotic inhibitors and cell division inhibitors:

(a) β-tubulin polymerization inhibitors: benomyl, carbendazim, chlorfenazole, fuberidazole, thiabendazole, thiophanate, thiophanate-methyl, diethofencarb, zoxamide, ethaboxam;
(b) Cell division inhibitors: pencycuron;
(c) Spectrin-like protein delocalization inhibitors: fluopicolide, fluopimomide.

(3) Respiration inhibitors:

(a) Complex I - NADH oxidation-reduction enzyme inhibitors: diflumetorimu, tolfenpyrad;
(b) Complex II - succinate dehydrogenase inhibitors: benodanil, flutolanil, mepronil, isofetamide, fluopyram, fenfuram, furmecyclox, carboxin, oxycarboxin, thifluzamide, benzovindiflupyr, bixafen, fluxapyroxad, furametpyr, isopyrazam, penflufen, penthiopyrad, sedaxane, boscalid, pydiflumetofen, isoflucypram, pyraziflumid, inpyrfluxam;
(c) Complex III - ubiquinol oxidase Qo inhibitors: azoxystrobin, coumoxystrobin, coumethoxystrobin, enoxastrobin, flufenoxystrobin, picoxystrobin, pyraoxystrobin, pyraclostrobin, pyrametostrobin, triclopyricarb, kresoxim-methyl, trifloxystrobin, dimoxystrobin, fenaminstrobin, metominostrobin, orysastrobin, famoxadone, fluoxastrobin, fenamidone, pyribencarb, metyltetraprole, mandestrobin;
(d) Complex III - ubiquinol reductase Qi inhibitors: cyazofamid, amisulbrom, fenpicoxamid;
(e) Oxidative phosphorylation uncoupling agents: binapacryl, meptyldinocap, dinocap, fluazinam, ferimzone;
(f) Oxidative phosphorylation inhibitors (ATP synthase inhibitors): fentin acetate, fentin chloride, fentin hydroxide;
(g) ATP production inhibitors: silthiofam;
(h) Complex III: Qx (unknown) inhibitor of cytochrome bc1 (ubiquinone reductase): ametoctradin.

(4) Amino acid and protein synthesis inhibitors

(a) Methionine biosynthesis inhibitors: andoprim, cyprodinil, mepanipyrim, pyrimethanil;
(b) Protein synthesis inhibitors: blasticidin-S, kasugamycin, kasugamycin hydrochloride, streptomycin, oxytetracycline.

(5) Signal transduction inhibitors:

(a) Signal transduction inhibitors: quinoxyfen, proquinazid;
(b) Inhibitors of MAP / histidine kinase in osmotic signal transduction: fenpiclonil, fludioxonil, chlozolinate, iprodione, procymidone, vinclozolin.

(6) Lipids and cell membrane synthesis inhibitors:

(a) Inhibitors of phospholipid biosynthesis and methyltransferase: edifenphos, iprobenfos, pyrazophos, isoprothiolane;
(b) Lipid peroxidation agent: biphenyl, chloroneb, dicloran, quintozene, tecnazene, tolclofos-methyl, etridiazole;
(c) Agents that act on the cell membrane: iodocarb, propamocarb, propamocarb-hydrochloride, propamocarb-fosetylate, prothiocarb;
(d) Microorganisms that disrupt the cell membrane of pathogens: bacillus subtilis bacteria, bacillus subtilis strain QST713, bacillus subtilis strain FZB24, bacillus subtilis strain MBI600, bacillus subtilis strain D747, bacillus amyloliquefaciens;
(e) Agents that disrupt the cell membrane: melaleuca alternifolia (tea tree) extract.

(7) Inhibitors of sterol biosynthesis in the cell membrane:

(a) Inhibitors of demethylation at the C14 position in sterol biosynthesis: triforine, pyrifenox, pyrisoxazole, fenarimol, flurprimidol, nuarimol, imazalil, imazalil-sulphate, oxpoconazole, oxpoconazole fumarate, pefurazoate, prochloraz, triflumizole, viniconazole, azaconazole, bitertanol, bromuconazole, cyproconazole, diclobutrazol, difenoconazole, diniconazole, diniconazole-M, epoxiconazole, etaconazole, fenbuconazole, fluquinconazole, flusilazole, flutriafol, furconazole, furconazole-cis, hexaconazole, imibenconazole, ipconazole, metconazole, myclobutanil, penconazole, propiconazole, fluquinconazole, simeconazole, tebuconazole, tetraconazole,

triadimefon, triadimenol, triticonazole, prothioconazole, voriconazole, mefentrifluconazole;

(b) Inhibitors of Δ14 reductase and Δ8→Δ7-isomerase in sterol biosynthesis: aldimorph, dodemorph, dodemorph acetate, fenpropimorph, tridemorph, fenpropidin, piperalin, spiroxamine;

(c) Inhibitors of 3-keto reductase in C4 demethylation in sterol biosynthesis: fenhexamid, fenpyrazamine;

(d) Inhibitors of squalene epoxidase in sterol biosynthesis: pyributicarb, naftifine, terbinafine.

(8) Cell wall synthesis inhibitors

(a) Trehalase inhibitor: validamycin;

(b) Chitin synthesis inhibitors: polyoxin, polyoxorim;

(c) Cellulose synthase inhibitors: dimethomorph, flumorph, pyrimorph, benthiavalicarb, benthiavalicarb-isopropyl, iprovalicarb, valifenalate, mandipropamid.

(9) Melanin biosynthesis inhibitors

(a) Melanin biosynthesis reductase inhibitors: phthalide, pyroquilon, tricyclazole;

(b) Melanin biosynthesis anhydrase inhibitors: carpropamid, diclocymet, fenoxanil.

(c) Melanin biosynthesis polyketide synthase inhibitors: tolprocarb.

(10) Host plant resistance inducer:

(a) Agents that act on the salicylic acid synthesis pathway: acibenzolar-S-methyl;

(b) Others: probenazole, tiadinil, isotianil, dichlobentiazox, ipfentrifluconazole, laminarin, reynoutria sachalinensis extract, phosphorous acid, phosphite.

(11) Agents, action of which is unclear: cymoxanil, fosetyl-aluminum, phosphoric acid, phosphate, tecloftalam, triazoxide, flusulfamide, diclomezine, methasulfocarb, cyflufenamid, metrafenone, pyriofenone, dodine, dodine free base, flutianil.

(12) Agents having multi-functionality: copper (copper salt), Bordeaux mixture, copper hydroxide, copper naphthalate, copper oxide, copper oxychloride, copper sulfate, sulfur, sulfur products, calcium polysulfide, ferbam, mancozeb, maneb, mancopper, metiram, polycarbamate, propineb, thiram, zineb, ziram, captan, captafol, folpet, chlorothalonil, dichlofluanid, tolylfluanid, guazatine, guazatine triacetate (another name: iminoctadine triacetate), iminoctadine trialbesilate, anilazine, dithianon, quinomethionate, fluoroimide.

(13) Other agents: DBEDC, fluorofolpet, guazatine acetate, bis (8-quinolinolato) copper (II), propamidine, chloropicrin, cyprofuram, agrobacterium, bethoxazin, diphenylamine, methyl isothiocyanate (MITC), mildiomycin, capsaicin, cufraneb, cyprosulfamide, dazomet, debacarb, dichlorophen, difenzoquat, difenzoquat·methyl sulfonate, flumetover, fosetyl-calcium, fosetyl-sodium, irumamycin, natamycin, nitrothal isopropyl, oxamocarb, pyrrolnitrin, tebufloquin, tolnifanide, zarilamid, algophase, amicarthiazol, oxathiapiprolin, fluoxapiprolin, metiram zinc, benthiazole, trichlamide, uniconazole, oxyfenthiin, picarbutrazox, dichlobentiazox, quinofumelin, thiuram, ambam, agrobacterium radiobacter, coniothyrium minitans, pseudomonas fluorescens, pseudomonas rhodesiae, talaromyces flavus, trichoderma atroviride, non-pathogenic erwinia carotovora, bacillus simplex, variovorax paradoxus, lactobacillus plantarum, florylpicoxamid, pyrapropoyne, fluindapyr, aminopyrifen, pyridachlometyl, ipflufenoquin, dipymetitrone.

Specific examples of the insecticides, acaricides, nematicides, soil insect pest control agents, and anthelmintic agents as the active ingredient (II) which may be mixed or used with the agricultural and horticultural fungicide according to the present invention are shown below.

(1) Acetylcholinesterase (AChE) inhibitors:

(a) Carbamate-based acetylcholinesterase (AChE) inhibitors: alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, triazamate, trimethacarb, XMC, xylylcarb, fenothiocarb, MIPC, MPMC, MTMC, aldoxycarb, allyxycarb, aminocarb, bufencarb, chloethocarb, metam-sodium, promecarb;

(b) Organophosphate-based acetylcholinesterase (AChE) inhibitors: acephate, azamethiphos, azinphos-ethyl, azinphos-methyl, cadusafos, chloroethoxyfos, chlorfenvinphos, chlormephos, chlorpyrifos, chlorpyrifos-methyl, coumaphos, cyanophos, demeton-S-methyl, diazinon, dichlorvos, dicrotophos, dimethoate, dimethylvinphos,

disulfoton, EPN, ethion, ethoprophos, famphur, fenamiphos, fenitrothion, fenthion, fosthiazate, heptenophos, imicyafos, isofenphos, isocarbophos, Isopropyl=O-(methoxyaminothiophosphoryl) salicylate, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion, bromophos-ethyl, BRP, carbophenothion, cyanofenphos, CYAP, demeton-S methyl sulfone, dialifos, dichlofenthion, dioxabenzofos, etrimfos, fensulfothion, flupyrazofos, fonofos, formothion, fosmethilan, isazofos, iodofenphos, methacrifos, pirimiphos-ethyl, phosphocarb, propaphos, prothoate, sulprofos;

(2) GABAergic chloride ion channel blockers:
acetoprole, chlordane, endosulfan, ethiprole, fipronil, pyrafluoprole, pyriprole, camphechlor, heptachlor, dienochlor, flufiprole;
(3) Sodium channel modulators

(a) Pyrethroid-based sodium channel modulators:
acrinathrin, allethrin, d-cis-trans allethrin, d-trans allethrin, bifenthrin, bioallethrin, bioallethrin s-cyclopentenyl isomer, bioresmethrin, cycloprothrin, cyfluthrin, β-cyfluthrin, cyhalothrin, λ-cyhalothrin, γ-cyhalothrin, cypermethrin, α-cypermethrin, β-cypermethrin, θ-cypermethrin, ζ-cypermethrin, cyphenothrin [(1R)-trans isomer], deltamethrin, empenthrin [(EZ)-(1R)-isomer], esfenvalerate, etofenprox, fenpropathrin, fenvalerate, flucythrinate, flumethrin, τ-fluvalinate, halfenprox, imiprothrin, kadethrin, permethrin, phenothrin [(1R)-trans isomer], prallethrin, pyrethrum, pyrethrin, resmethrin, silafluofen, tefluthrin, tetramethrin, tetramethrin [(1R)-isomer], tralomethrin, transfluthrin, allethrin, pyrethrin I, pyrethrin II, profluthrin, dimefluthrin, bioethanomethrin, biopermethrin, transpermethrin, fenfluthrin, fenpirithrin, flubrocythrinate, flufenprox, metofluthrin, protrifenbute, pyresmethrin, terallethrin, κ-bifenthrin, chloroprallethrin, heptafluthrin, meperfluthrin, ε-metofluthrin, momfluorothrin, ε- momfluorothrin, κ-tefluthrin, tetra methylfluthrin, bioethanomethrin;
(b) Sodium channel modulators (DDTs):
DDT, methoxychlor;

(4) Nicotinic acetylcholine receptor (nAChR) competitive modulators:
acetamiprid, clothianidin, dinotefuran, imidacloprid, nitenpyram, nithiazine, thiacloprid, thiamethoxam, nicotine, sulfoxaflor, flupyradifurone, triflumezopyrim, dichloromezotiaz, flupyrimin;
(5) Nicotinic acetylcholine receptor (nAChR) allosteric modulators:
spinetoram, spinosad;
(6) Glutamate-gated chloride ion channel (GluCl) allosteric modulators:
abamectin, emamectin, emamectin-benzoate, lepimectin, milbemectin, ivermectin, selamectin, doramectin, eprinomectin, ivermectin, moxidectin, selamectin, milbemycin, milbemycin oxime, nemadectin;
(7) Juvenile hormone-like substances:
hydroprene, kinoprene, methoprene, fenoxycarb, pyriproxyfen, diofenolan, epofenonane, triprene;
(8) Other non-specific (multi-site) inhibitors:
methyl bromide, alkyl halides, chloropicrin, sodium aluminum fluoride, sulfuryl fluoride, borax, boric acid, disodium octaborate, sodium borate, sodium metaborate, tartar emetic, dazomet, metam, metam potassium salt, metam sodium salt;
(9) Chordotonal organ TRPV channel modulators:
flonicamid, pymetrozine, pyrifluquinazon, afidopiropen;
(10) Acarian growth inhibitors:
clofentezine, diflovidazin, hexythiazox, etoxazole.
(11) Insect midgut inner membrane disrupting agent derived from microorganisms:
bacillus thuringiensis subsp. Israelensis, bacillus sphaericus, bacillus thuringiensis subsp. aizawai, bacillus thuringiensis subsp. kurstaki, bacillus thuringiensis subsp. tenebrionis, Bt crop protein: Cry1Ab, Cry1Ac, Cry1Fa, Cry1A.105, Cry2Ab, Vip3A, mCry3A, Cry3Ab, Cry3Bb, Cry34Ab1 / Cry35Ab1; bacillus sphaericus.
(12) Mitochondrial ATP biosynthetic enzyme inhibitors:
diafenthiuron, azocyclotin, cyhexatin, fenbutatin oxide, propargite, tetradifon;
(13) Oxidative phosphorylation uncouplers that disrupt the proton gradient:
chlorfenapyr, DNOC, sulfluramid, binapacryl, dinobuton, dinocap;
(14) Nicotinic acetylcholine receptor (nAChR) channel blockers:
bensultap, cartap hydrochloride, nereistoxin, thiocyclam, thiosultap-sodium;
(15) Chitin biosynthesis inhibitors, type 0:

bistrifluron, chlorfluazuron, diflubenzuron, flucycloxuron, flufenoxuron, hexaflumuron, lufenuron, novaluron, noviflumuron, teflubenzuron, triflumuron, buprofezin, fluazuron;
(16) Chitin biosynthesis inhibitors, type 1:
buprofezin;
(17) Molting inhibitors:
cyromazine;
(18) Molting hormone (ecdysone) receptor agonists:
chromafenozide, halofenozide, methoxyfenozide, tebufenozide;
(19) Octopamine receptor agonists:
amitraz, demiditraz, chlordimeform;
(20) Mitochondrial electron transport system complex III inhibitors:
Hydramethylnon, acequinocyl, fluacrypyrim, bifenazate;
(21) Mitochondrial electron transport system complex I inhibitors (METI):
fenazaquin, fenpyroximate, pyridaben, pyrimidifen,tebufenpyrad, tolfenpyrad, rotenone;
(22) Voltage-dependent sodium channel blockers:
indoxacarb, metaflumizone;
(23) Acetyl CoA carboxylase inhibitors:
spirodiclofen, spiromesifen, spirotetramat, spiropidion.
(24) Mitochondrial electron transport system complex IV inhibitors:
aluminum phosphide, calcium phosphide, zinc phosphide, phosphine, cyanide, calcium cyanide, sodium cyanide, potassium cyanide;
(25) Mitochondrial electron transport system complex II inhibitors:
cyenopyrafen, cyflumetofen, pyflubumide.
(28) Ryanodine receptor modulators:
chlorantraniliprole, cyantraniliprole, cyclaniliprole, flubendiamide, cyhalodiamide, tetrachlorantraniliprole, tetraniliprole.
(29) Chordotonal organ modulator target site unspecified:
flonicamid;
(30) GABA-gated chloride ion channel allosteric modulators:
broflanilide, fluxamethamide, isocycloseram, afoxolaner, fluralaner, lotilaner, sarolaner;
(31) Other insecticides and acaricides:
azadirachtin, benzoximate, bifenazate, bromopropylate, quinomethionate, cryolite, dicofol, lime sulfur, mancozeb, pyridalyl, benclothiaz, sulfur, amidoflumet, 1,3-dichloropropene, DCIP, phenisobromolate, benzomate, metaldehyde, chlorobenzilate, clothiazoben, dicyclanil, fenoxacrim, fentrifanil, flubenzimine, fluphenazine, gossyplure, japonilure, metoxadiazone, oil, potassium oleate, tetrasul, triarathene, afidopyropen, flufiprole, fluensulfone, meperfluthrin, tetramethylfluthrin, tralopyril, dimefluthrin, methylneodecanamide, fluralaner, afoxolaner, fluxametamide, 5-[5-(3,5-dichlorophenyl)-5-trifluoromethyl-4,5-dihydroisoxazole-3-yl]-2-(1H-1,2,4-triazol-1-yl) benzonitrile (CAS: 943137-49-3), broflanilide, other metahnediamedes, steinernema carpocapsae, steinernema glaseri, pasteuria penetrans, paecilomyces tenuipes, paecilomyces fumosoroseus, beauveria bassiana, beauveria brongniartii, metarhizium anisopliae, verticillium lecanii, acynonapyr, benzpyrimoxan, flometoquin, fluhexafon, oxazosulfyl, tyclopyrazoflor.
(32) Anthelmintic agents:

(a) Benzimidazole-based: fenbendazole, albendazole, triclabendazole, oxibendazole, mebendazole, oxfendazole, parbendazole, flubendazole, febantel, netobimin, thiophanate, thiabendazole, cambendazole;
(b) Salicylanilide-based: closantel, oxyclozanide, rafoxanide, niclosamide;
(c) Substituted phenol-based: nitroxinil, nitroscanate;
(d) Pyrimidine-based: pyrantel, morantel;
(e) Imidazothiazole-based: levamisole, tetramisole;
(f) Tetrahydropyrimidine-based: praziquantel, epsiprantel;
(g) Other anthelmintic agents: cyclodiene, ryania, clorsulon, metronidazole, demiditraz, piperazine, diethylcarbamazine, dichlorophen, monepantel, tribendimidine, amidantel, thiacetarsamide, melarsomine, arsenamide.

Specific examples of plant regulatory agents which can be mixed or used with the agricultural and horticultural fungicide according to the present invention as the active ingredient (II) are shown below.

Abscisic acid, kinetin, benzylaminopurine, 1,3-diphenylurea, forchlorfenuron, thidiazuron, chlorfenuron, dihydrozeatin, gibberellin A, gibberellin A4, gibberellin A7, gibberellin A3, 1-methylcyclopropane, N-acetyl aminoethoxyvinyl glycine (another name: aviglycine), aminooxyacetic acid, silver nitrate, cobalt chloride, IAA, 4-CPA, cloprop, 2,4-D, MCPB,

indole-3-butyrate, dichlorprop, phenothiol, 1-naphthyl acetamide, ethychlozate, cloxyfonac, maleic acid hydrazide, 2,3,5-triiodobenzoic acid, salicylic acid, methyl salicylate, (-)-jasmonic acid, methyl jasmonate, (+)-strigol, (+)-deoxystrigol, (+)-orobanchol, (+)-sorgolactone, 4-oxo-4-(2-phenylethyl)aminobutyric acid, ethephon, chlormequat, mepiquat chloride, benzyl adenine, 5-aminolevulinic acid, daminozide.

(Preparation formulation)

The agricultural and horticultural fungicide according to the present invention is not particularly limited by the dosage form thereof. Examples of the dosage form include wettable powder, emulsion, powder, granule, water-soluble agent, suspension, granular wettable powder, and tablet. The method for preparing the formulation is not particularly limited, and any conventional preparation method may be adopted depending on the dosage form.
Some preparation examples are shown below. The following preparation formulations are shown as examples, and may be modified to the extent that the modification is consistent with the gist of the present invention, and the present invention is not intended to be limited to the following preparation examples. The term "part" means "part by mass" unless particularly mentioned.

(Preparation Example 1: wettable powder)

40 parts of a hydrazide compound according to the present invention, 53 parts of diatomaceous earth, 4 parts of higher alcohol sulfuric acid ester, and 3 parts of alkyl naphthalene sulfonate were mixed uniformly, and then finely pulverized to obtain wettable powders containing 40% by mass of the active ingredient.

(Preparation Example 2: emulsion)

30 parts of a hydrazide compound according to the present invention, 33 parts of xylene, 30 parts of dimethylformamide, and 7 parts of polyoxyethylene alkyl allyl ether were mixed and dissolved to obtain an emulsion containing 30% by mass of the active ingredient.

(Preparation Example 3: granule)

5 parts of a hydrazide compound according to the present invention, 40 parts of talc, 38 parts of clay, 10 parts of bentonite, and 7 parts of sodium alkyl sulfate were mixed uniformly, and then finely pulverized, followed by granulating to obtain a particle diameter of 0.5 to 1.0 mm, and thus granules containing 5% by mass of the active ingredient were obtained.

(Preparation Example 4: granule)

5 parts of a hydrazide compound according to the present invention, 73 parts of clay, 20 parts of bentonite, 1 part of sodium dioctyl sulfosuccinate, and 1 part of potassium phosphate were uniformly mixed and then pulverized, followed by adding water thereto, and then kneading the mixture. Then, the resultant was granulated and dried to obtain granules containing 5% by mass of the active ingredient.

(Preparation Example 5: suspension)

10 parts of a hydrazide compound according to the present invention, 4 parts of polyoxyethylene alkyl allyl ether, 2 parts of sodium polycarboxylate, 10 parts of glycerin, 0.2 parts of xanthan gum, and 73.8 parts of water were mixed, and then wet-pulverized until the particle size became 3 μm or less to obtain a suspension containing 10% by mass of the active ingredient.

(Preparation Example 6: granular wettable powder)

40 parts of a hydrazide compound according to the present invention, 36 parts of clay, 10 parts of potassium chloride, 1 part of sodium alkylbenzene sulfonate, 8 parts of sodium lignin sulfonate, and 5 parts of formaldehyde condensate of sodium alkylbenzene sulfonate were mixed uniformly and then pulverized finely. Next, an appropriate amount of water was added thereto and the mixure was kneaded until the resultant became clayey. The clayey resultant was granulated and then dried to obtain granular wettable powders containing 40% by mass of the active ingredient.
Next, compound production examples are shown to explain the present invention further specifically. However, the present invention is not intended to be limited by the following examples.

(Production Example 1)

N'-acetyl-3-(3-cyclopropyl-2-fluorophenoxy)-N'-(2,4-dimethylbenzyl)-6-methylpyridazine-4-carbohydrazide (English name: N'-acetyl-3-(3-cyclopropyl-2-fluorophenoxy)-N'-(2,4-dimethylbenzyl)-6-methylpyridazine-4-carbohydrazide (Compound b-5)) was produced by the following steps.

Step 1: Conversion to 3-(3-cyclopropyl-2-fluorophenoxy)-6-methylpyridazine-4-carbohydrazide (English name: 3-(3-cyclopropyl-2-fluorophenoxy)-6-methylpyridazine-4-carbohydrazide)

[Chemical Formula 37]

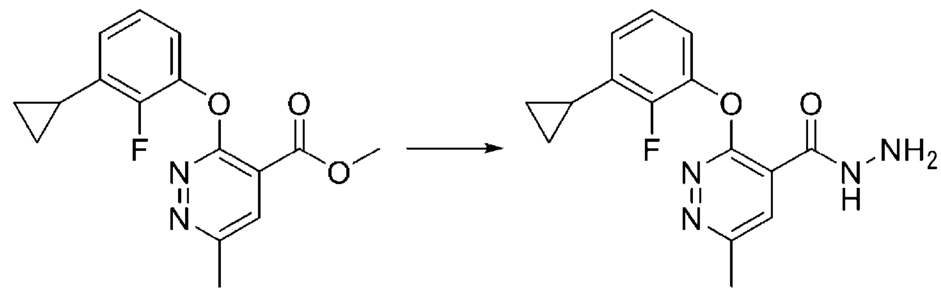

1 2

Compound 1 (1.1 g) was dissolved in tetrahydrofuran (12 mL). Hydrazine monohydrate (0.6 g) and diazabicycloundecene (0.3 mL) were added thereto at room temperature, and then stirred at room temperature for five hours. The obtained liquid was poured into ice water. The resultant was extracted with ethyl acetate and dried over anhydrous magnesium sulfate. The solvent was distilled off under reduced pressure, and the obtained residue was purified by silica gel column chromatography (developing solvent: n-hexane/ethyl acetate), thereby obtaining Compound 2 (1.2 g, at a yield of 92%).

Step 2: Conversion to (E)-3-(3-cyclopropyl-2-fluorophenoxy)-N'-(2,4-dimethylbenzylidene)-6-methylpyridazine-4-carbohydrazide (English name: (E)-3-(3-cyclopropyl-2-fluorophenoxy)-N'-(2,4-dimethylbenzylidene)-6-methylpyridazine-4-carbohydrazide)

[Chemical Formula 38]

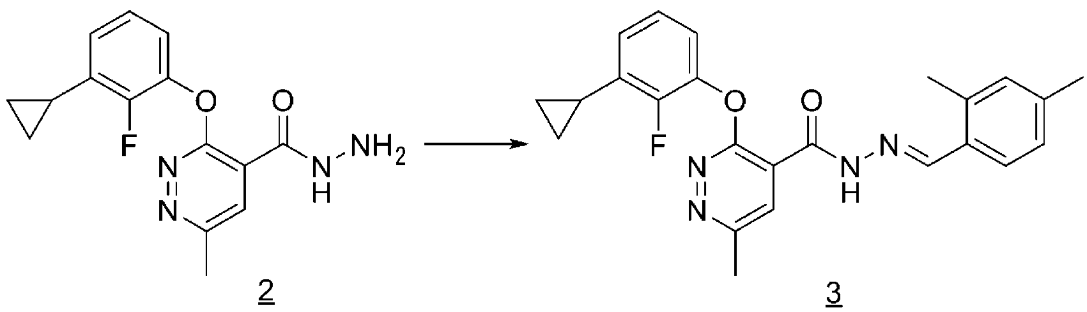

2 3

Compound 2 (1.2 g) was dissolved in ethanol (12 mL). 2,4-Dimethylbenzaldehyde (0.78 g) was added thereto at room temperature, and then stirred under heating to allow reflux to proceed for two hours. Then, the solvent was distilled off under reduced pressure. The obtained residue was purified by silica gel column chromatography (developing solvent: n-hexane/ethyl acetate), thereby obtaining Compound 3 (1.2 g, at a yield of 72%).

Step 3: Conversion to 3-(3-cyclopropyl-2-fluorophenoxy)-N'-(2,4-dimethylbenzyl)-6-methylpyridazine-4-carbohydrazide (English name: 3-(3-cyclopropyl-2-fluorophenoxy)-N'-(2,4-dimethylbenzyl)-6-methylpyridazine-4-carbohydrazide)

[Chemical Formula 39]

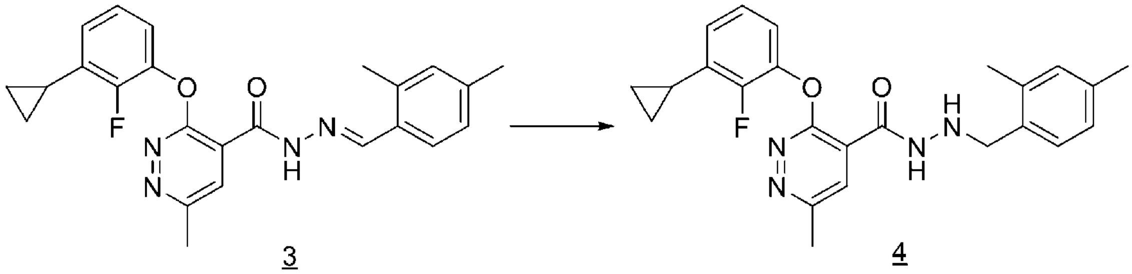

3 4

Compound 3 (0.36 g) was dissolved in tetrahydrofuran (4 mL) and acetic acid (1.1 mL). Sodium cyanoborohydride (1.1 g) was added thereto at 0°C, and then stirred at room temperature for two hours. The obtained liquid was poured into an aqueous solution of sodium carbonate. Then, the resultant was extracted with ethyl acetate and dried over anhydrous magnesium sulfate. The solvent was distilled off under reduced pressure, and the obtained residue was purified by silica gel column chromatography (developing solvent: n-hexane/ethyl acetate), thereby obtaining Compound 4 (0.24 g, at a yield of 67%).

Step 4: Conversion to N'-acetyl-3-(3-cyclopropyl-2-fluorophenoxy)-N'-(2,4-dimethylbenzyl)-6-methylpyridazine-4-carbohydrazide (English name: N'-acetyl-3-(3-cyclopropyl-2-fluorophenoxy)-N'-(2,4-dimethylbenzyl)-6-methylpyridazine-4-carbohydrazide)

[Chemical Formula 40]

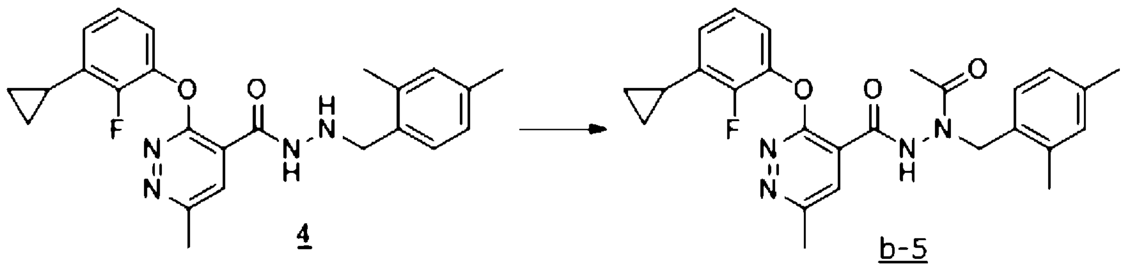

4 b-5

Compound 4 (0.05 g) was dissolved in chloroform (1 mL). Acetic acid (0.008 mL), 1-ethyl-3-(3-dimethylaminopropyl)carbodiimide hydrochloride (0.05 g), 1-hydroxybenzotriazole (0.01 g) and triethylamine (0.04 mL) were added thereto at 0°C, and then stirred at room temperature for 18 hours. The obtained liquid was poured into ice water, extracted with ethyl acetate, and then dried over anhydrous magnesium sulfate. The solvent was distilled off under reduced pressure, and the obtained residue was purified by silica gel column chromatography (developing solvent: n-hexane/ethyl acetate), thereby obtaining Compound b-5 (0.027 g, at a yield of 49%).

(Production Example 2)

3-(3-cyclopropyl-2-fluorophenoxy)-N'-(2,4-dimethylbenzyl)-N'-formyl-6-methylpyridazine-4-carbohydrazide (English name: 3-(3-cyclopropyl-2-fluorophenoxy)-N'-(2,4-dimethylbenzyl)-N'-formyl-6-methylpyridazine-4-carbohydrazide (Compound b-3)) was produced by the following steps.

Step 1: Conversion to 3-(3-cyclopropyl-2-fluorophenoxy)-N'-(2,4-dimethylbenzyl)-N'-formyl-6-methylpyridazine-4-carbohydrazide (English name: 3-(3-cyclopropyl-2-fluorophenoxy)-N'-(2,4-dimethylbenzyl)-N'-formyl-6-methylpyridazine-4-carbohydrazide)

[Chemical Formula 41]

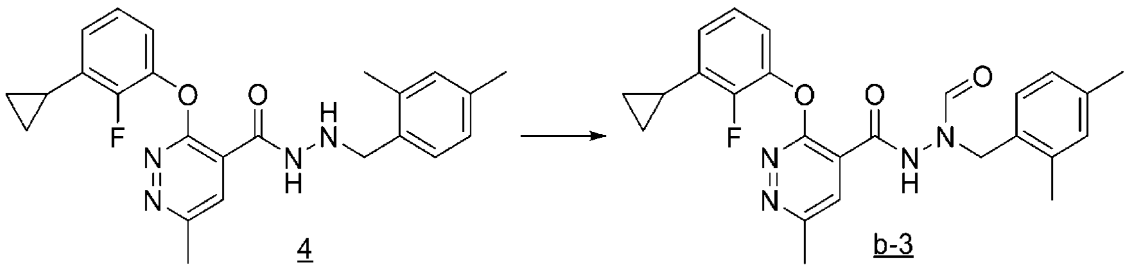

4 b-3

Compound 4 (0.07 g) was dissolved in chloroform (1 mL). Formic acid (0.01 mL) and carbonyldiimidazole (0.042 g) were added thereto at 0°C, and then stirred at room temperature for two hours. The obtained liquid was poured into ice water, extracted with ethyl acetate, and then dried over anhydrous magnesium sulfate. The solvent was distilled off under reduced pressure, and the obtained residue was purified by silica gel column chromatography (developing solvent: n-hexane/ethyl acetate), thereby obtaining Compound b-3 (0.045 g, at a yield of 60%).

(Production Example 3)

N'-acetyl-5-(3-cyclopropyl-2-fluorophenoxy)-N'-(2,4-dimethylbenzyl)-2-methylisonicotinohydrazide (English name: N'-acetyl-5-(3-cyclopropyl-2-fluorophenoxy)-N'-(2,4-dimethylbenzyl)-2-methylisonicotinohydrazide (Compound b-9)) was produced by the following steps.

Step 1: Conversion to N-(2,4-dimethylbenzyl)-N-(1,3-dioxoisoindolin-2-yl)acetamide (English name: N-(2,4-dimethylbenzyl)-N-(1,3-dioxoisoindolin-2-yl)acetamide)

[Chemical Formula 42]

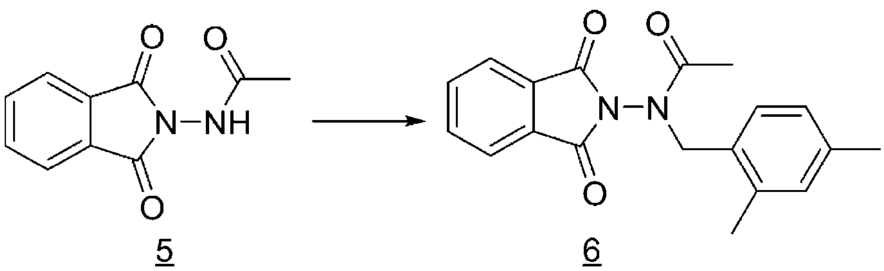

5 6

Compound 5 (0.6 g) was dissolved in acetonitrile (3 mL). Potassium carbonate (0.3 g) and benzyltriethylammonium chloride (0.07 g) were added thereto at room temperature, and then stirred at 80°C for two hours. The obtained liquid was poured into ice water. The resultant was extracted with ethyl acetate and dried over anhydrous magnesium sulfate. The solvent was distilled off under reduced pressure, and the obtained residue was purified by silica gel column chromatography (developing solvent: n-hexane/ethyl acetate), thereby obtaining Compound 6 (0.36 g, at a yield of 38%).

Step 2: Conversion to N-(2,4-dimethylbenzyl)acetohydrazide (English name: N-(2,4-dimethylbenzyl)acetohydrazide)

[Chemical Formula 43]

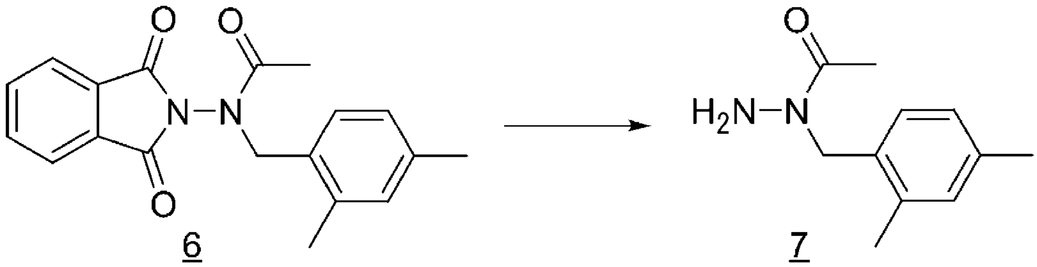

6

7

Compound 6 (0.3 g) was dissolved in ethanol (3 mL). Hydrazine monohydrate (0.22 mL) was added thereto at room temperature, and then stirred at 50°C for one hour. The obtained liquid was filtered, and the filtrate was poured into ice water, extracted with ethyl acetate, and then dried over anhydrous magnesium sulfate. The solvent was distilled off under reduced pressure, thereby obtaining Compound 7 (0.17 g, at a yield of 99%).

Step 3: Conversion to tert-butyl 2-bromo-5-(3-cyclopropyl-2-fluorophenoxy)isonicotinate (English name: tert-butyl 2-bromo-5-(3-cyclopropyl-2-fluorophenoxy)isonicotinate)

[Chemical Formula 44]

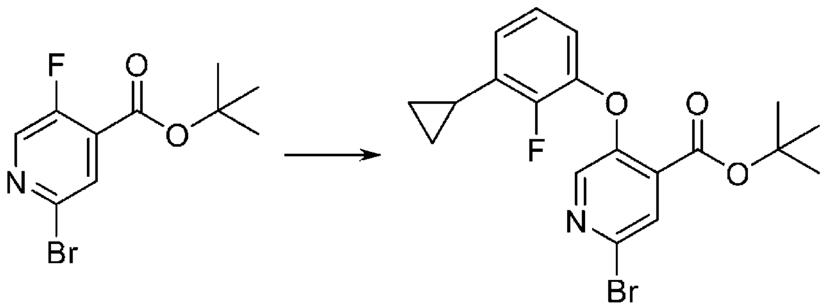

8

9

Compound 8 (5.7 g) was dissolved in N,N-dimethylformamide (120 mL). 3-Cyclopropyl-2-fluorophenol (5.5 g) and potassium carbonate (6.1 g) were added thereto at room temperature, and then stirred at 50°C for three hours. The obtained liquid was poured into ice water, extracted with ethyl acetate, and then dried over anhydrous magnesium sulfate. The solvent was distilled off under reduced pressure, and the obtained residue was purified by silica gel column chromatography (developing solvent: n-hexane/ethyl acetate), thereby obtaining Compound 9 (7.1 g, at a yield of 84%).

Step 4: Conversion to tert-butyl 5-(3-cyclopropyl-2-fluorophenoxy)-2-methylisonicotinate (English name: tert-butyl 5-(3-cyclopropyl-2-fluorophenoxy)-2-methylisonicotinate)

[Chemical Formula 45]

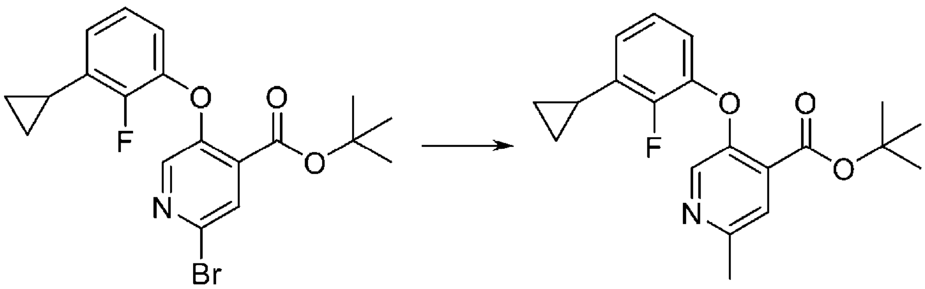

9 10

Compound 9 (7.1 g) was dissolved in 1,4-dioxane (100 mL). Trimethylboroxine (3.5 g), 1,1'-bis[(diphenylphosphino)ferrocene]dichloropalladium(II) (0.75 g) and potassium carbonate (5.6 g) were added thereto at room temperature, and then stirred under heating to allow reflux to proceed for 18 hours. The obtained liquid was poured into ice water, extracted with ethyl acetate, and then dried over anhydrous magnesium sulfate. The solvent was distilled off under reduced pressure, and the obtained residue was purified by silica gel column chromatography (developing solvent: n-hexane/ethyl acetate), thereby obtaining Compound 10 (4.1 g, at a yield of 69%).

Step 5: Conversion to 5-(3-cyclopropyl-2-fluorophenoxy)-2-methylisonicotinic acid (English name: 5-(3-cyclopropyl-2-fluorophenoxy)-2-methylisonicotinic acid)

[Chemical Formula 46]

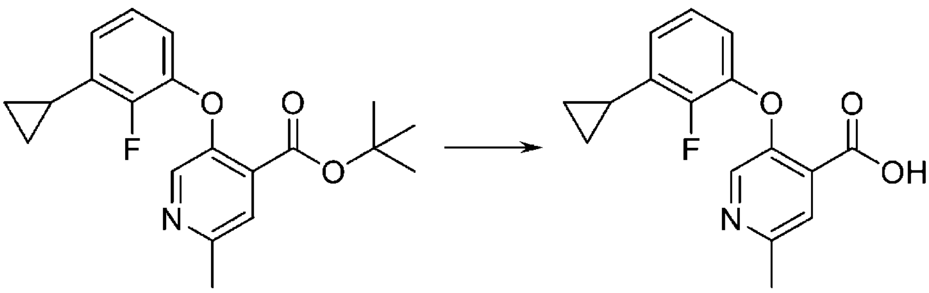

10 11

Compound 10 (4.1 g) was dissolved in dichloromethane (20 mL). Trifluoroacetic acid (20 mL) was added thereto at room temperature, and then stirred at room temperature for 18 hours. The solvent was distilled off under reduced pressure, thereby obtaining Compound 11 (3.0 g, at a yield of 89%).

Step 6: Conversion to N'-acetyl-5-(3-cyclopropyl-2-fluorophenoxy)-N'-(2,4-dimethylbenzyl)-2-methylisonicotinohydrazide (English name: N'-acetyl-5-(3-cyclopropyl-2-fluorophenoxy)-N'-(2,4-dimethylbenzyl)-2-methylisonicotinohydrazide)

[Chemical Formula 47]

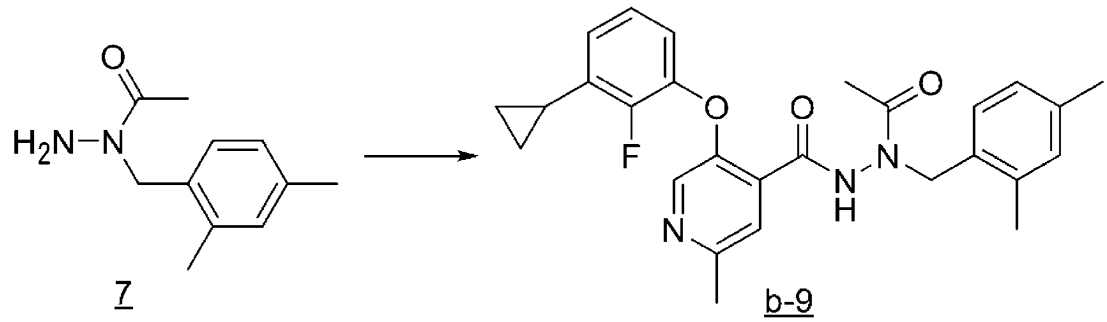

7

b-9

Compound 7 (0.03 g) was dissolved in dichloromethane (1 mL). Compound 11 (0.065 g), 1-ethyl-3-(3-dimethylaminopropyl)carbodiimide hydrochloride (0.05 g), 1-hydroxybenzotriazole (0.01 g) and triethylamine (0.04 mL) were added thereto, and then stirred at room temperature for 18 hours. The obtained liquid was poured into ice water, extracted with ethyl acetate, and then dried over anhydrous magnesium sulfate. The solvent was distilled off under reduced pressure, and the obtained residue was purified by silica gel column chromatography (developing solvent: n-hexane/ethyl acetate), thereby obtaining Compound b-9 (0.015 g, at a yield of 14%).

(Production Example 4)

Methyl 2-(3-(3-cyclopropyl-2-fluorophenoxy)-6-methylpyridazine-4-carbonyl)-1-(2,4-dimethylbenzyl)hydrazine-1-carboxylate (English name: methyl 2-(3-(3-cyclopropyl-2-fluorophenoxy)-6-methylpyridazine-4-carbonyl)-1-(2,4-dimethylbenzyl)hydrazine-1-carboxylate (Compound b-6)) was produced by the following steps.

Step 1: Conversion to methyl (2,4-dimethylbenzyl)(1,3-dioxoisoindolin-2-yl)carbamate (English name: methyl (2,4-dimethylbenzyl)(1,3-dioxoisoindolin-2-yl)carbamate)

[Chemical Formula 48]

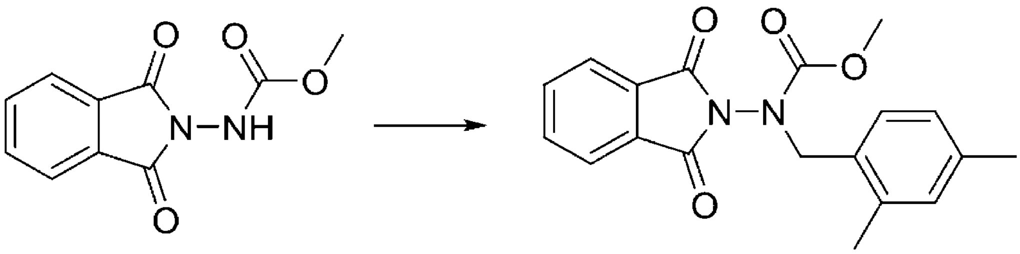

12

13

Compound 12 (0.5 g) was dissolved in acetonitrile (3 mL). Potassium carbonate (0.3 g) and benzyltriethylammonium chloride (0.07 g) were added thereto at room temperature, and then stirred at 80°C for two hours. The obtained liquid was poured into ice water. The resultant was extracted with ethyl acetate and dried over anhydrous magnesium sulfate. The solvent was distilled off under reduced pressure, and the obtained residue was purified by silica gel column chromatography (developing solvent: n-hexane/ethyl acetate), thereby obtaining Compound 13 (0.4 g, at a yield of 51%).

Step 2: Conversion to methyl 1-(2,4-dimethylbenzyl)hydrazine-1-carboxylate (English name: methyl 1-(2,4-dimethylbenzyl)hydrazine-1-carboxylate)

[Chemical Formula 49]

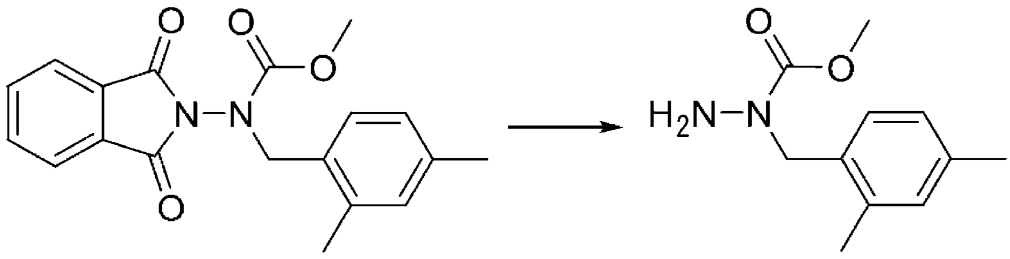

13 14

Compound 13 (0.3 g) was dissolved in ethanol (3 mL). Hydrazine monohydrate (0.22 mL) was added thereto at room temperature, and then stirred at 50°C for one hour. Solids were filtered out, and the obtained solution was poured into ice water, extracted with ethyl acetate, and then dried over anhydrous magnesium sulfate. The solvent was distilled off under reduced pressure, thereby obtaining Compound 14 (0.17 g, at a yield of 98%).

Step 3: Conversion to methyl 2-(3-(3-cyclopropyl-2-fluorophenoxy)-6-methylpyridazine-4-carbonyl)-1-(2,4-dimethylbenzyl)hydrazine-1-carboxylate (English name: methyl 2-(3-(3-cyclopropyl-2-fluorophenoxy)-6-methylpyridazine-4-carbonyl)-1-(2,4-dimethylbenzyl)hydrazine-1-carboxylate)

[Chemical Formula 50]

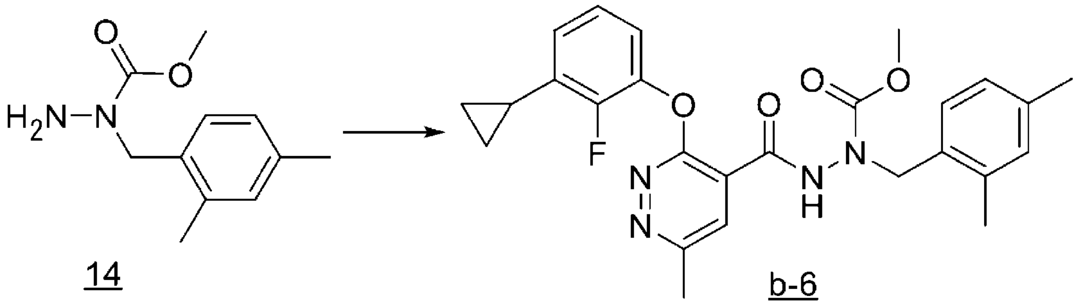

14 b-6

Compound 14 (0.035 g) was dissolved in dichloromethane (1 mL). 3-(3-Cyclopropyl-2-fluorophenoxy)-6-methylpyridazine -4-carboxylic acid (0.04 g), 1-ethyl-3-(3-dimethylaminopropyl)carbodiimide hydrochloride (0.04 g), 1-hydroxybenzotriazole (0.01 g) and triethylamine (0.04 mL) were added thereto at 0°C, and then stirred at room temperature for 18 hours. The obtained liquid was poured into ice water, extracted with ethyl acetate, and then dried over anhydrous magnesium sulfate. The solvent was distilled off under reduced pressure, and the obtained residue was purified by silica gel column chromatography (developing solvent: n-hexane/ethyl acetate), thereby obtaining Compound b-6 (0.05 g, at a yield of 75%).

(Production Example 5)

N'-(3-(3-cyclopropyl-2-fluorophenoxy)-6-methylpyridazine -4-carbonyl)-N-(2,4-dimethylbenzyl)methanesulfonohydrazide (Compound b-26) was produced by the following steps.

Step 1: Conversion to N-(1,3-dioxoisoindolin-2-yl)methanesulfonamide (English name: N-(1,3-dioxoisoindolin-2-yl) methanesulfonamide)

[Chemical Formula 51]

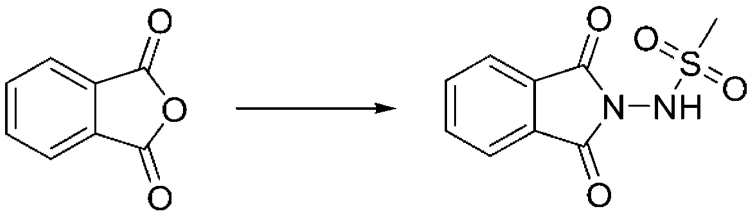

15 16

Compound 15 (0.53 g) was dissolved in tetrahydrofuran (18 mL). Methanesulfonylhydrazine (0.4 g) was added thereto at room temperature, and then stirred at room temperature for one hour. Thereafter, N,N'-dicyclohexylcarbodiimide (0.9 g) was added thereto, and then stirred at room temperature for two hours. Acetic acid (0.4 mL) and triethylamine (1 mL) were added thereto, and then stirred under heating to allow reflux to proceed for two hours. The obtained liquid was poured into ice water. The resultant was extracted with ethyl acetate and dried over anhydrous magnesium sulfate. The solvent was distilled off under reduced pressure, thereby obtaining Compound 16 (0.5 g, at a yield of 39%).

Step 2: Conversion to N-(2,4-dimethylbenzyl)-N-(1,3-dioxoindolin-2-yl)methanesulfonamide (English name: N-(2,4-dimethylbenzyl)-N-(1,3-dioxoisoindolin-2-yl)methanesulfonamide)

[Chemical Formula 52]

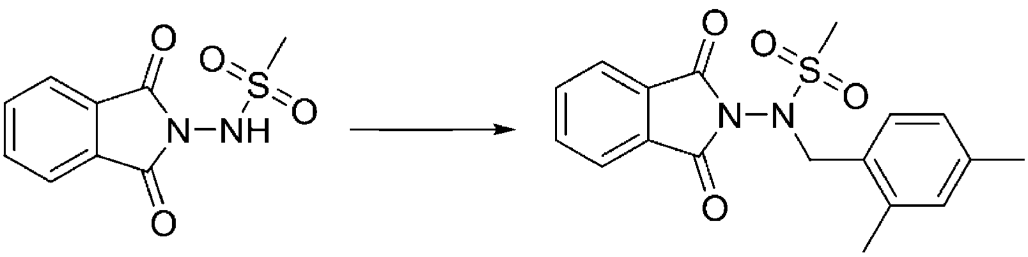

16 17

Compound 16 (0.45 g) was dissolved in N,N-dimethylformamide (3 mL). Potassium carbonate (1.1 g) and 2,4-dimethylbenzylbromide (0.6 g) were added thereto at room temperature, and then stirred at 50°C for five hours. The obtained liquid was poured into ice water. The resultant was extracted with ethyl acetate and dried over anhydrous magnesium sulfate. The solvent was distilled off under reduced pressure, and the obtained residue was purified by preparative high performance liquid chromatography (developing solvent: water/ acetonitrile), thereby obtaining Compound 17 (0.22 g, at a yield of 33%).

Step 3: Conversion to N-(2,4-dimethylbenzyl)methanesulfonohydrazide (English name: N-(2,4-dimethylbenzyl)methanesulfonohydrazide)

[Chemical Formula 53]

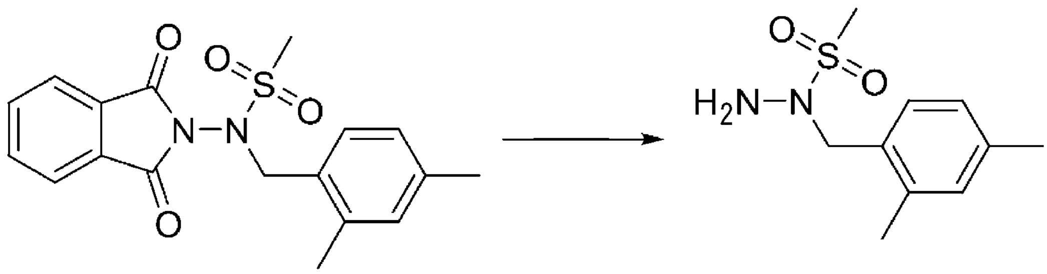

17

18

Compound 17 (0.22 g) was dissolved in ethanol (6 mL). Hydrazine monohydrate (0.2 mL) were added thereto at room temperature, and then stirred at 50°C for one hour. The obtained liquid was poured into ice water. The resultant was extracted with ethyl acetate and dried over anhydrous magnesium sulfate. The solvent was distilled off under reduced pressure, thereby obtaining Compound 18 (0.15 g, at a yield of 94%).

Step 4: Conversion to N'-(3-(3-cyclopropyl-2-fluorophenoxy)-6-methylpyridazine-4-carbonyl)-N-(2,4-dimethylbenzyl) methanesulfonohydrazide (English name: N'-(3-(3-cyclopropyl-2-fluorophenoxy)-6-methylpyridazine-4-carbonyl)-N-(2,4-dimethylbenzyl)methanesulfonohydrazide)

[Chemical Formula 54]

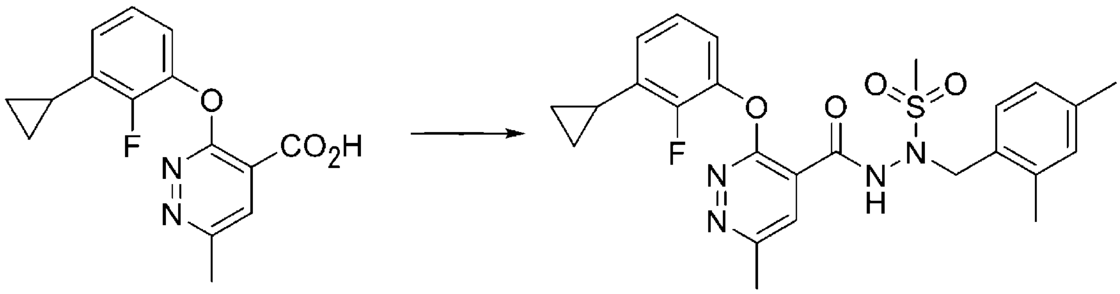

19

b-26

Compound 19 (0.38 g) was dissolved in dichloromethane (1 mL). Oxalyl chloride (0.36 mL) and N,N-dimethylformamide (0.1 mL) were added thereto, and then stirred at room temperature for two hours. The solvent was distilled off under reduced pressure, and the obtained residue was dissolved in dichloromethane (1 mL). Compound 18 (0.15 g) and cesium carbonate (0.5 g) were added thereto at room temperature, and then stirred at room temperature for two hours. The obtained liquid was poured into ice water. The resultant was extracted with ethyl acetate and dried over anhydrous magnesium sulfate. The solvent was distilled off under reduced pressure, and the obtained residue was purified by silica gel column chromatography (developing solvent: n-hexane/ethyl acetate), thereby obtaining Compound b-26 (0.03 g, at a yield of 9%).

(Production Example 6)

Methyl 2-(5-(3-cyclopropyl-2-fluorophenoxy)-2-methylpyrimidine-4-carbonyl)-1-(2,4-dimethylbenzyl)hydrazine-1-carboxylate (English name: methyl 2-(5-(3-cyclopropyl-2-fluorophenoxy)-2-methylpyrimidine-4-carbonyl)-1-(2,4-dimethylbenzyl)hydrazine-1-carboxylate (Compound b-42)) was produced by the following steps.

[Chemical Formula 55]

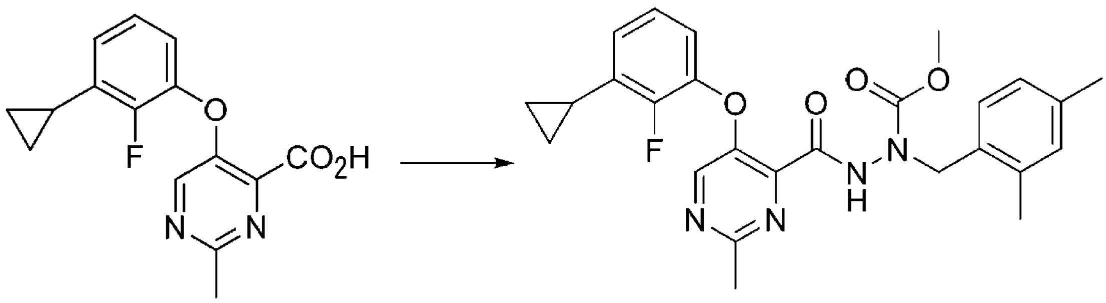

20 b-42

Compound 20 (0.08 g) was dissolved in N,N-dimethylformamide (1 mL). Compound 14 (0.092 g), 1-ethyl-3-(3-dimethylaminopropyl)carbodiimide hydrochloride (0.09 g), 1-hydroxybenzotriazole (0.01 g) and triethylamine (0.1 mL) were added thereto, and then stirrerd at room temperature for 18 hours. Methanol was added to the obtained liquid, and then the mixture was purified by preparative high performance liquid chromatography (developing solvent: water/-acetonitrile), thereby obtaining Compound b-42 (0.061 g, at a yield of 43%).

(Production Example 7)

3-(3-Cyclopropyl-2-fluorophenoxy)-N'-(2,4-dimethylbenzyl)-6-methyl-N'-(2-oxopropanoyl)pyridazine-4-carbohydrazide (English name: 3-(3-cyclopropyl-2-fluorophenoxy)-N'-(2,4-dimethylbenzyl)-6-methyl-N'-(2-oxopropanoyl)pyridazine-4-carbohydrazide (Compound b-58)) was produced by the following steps.

[Chemical Formula 56]

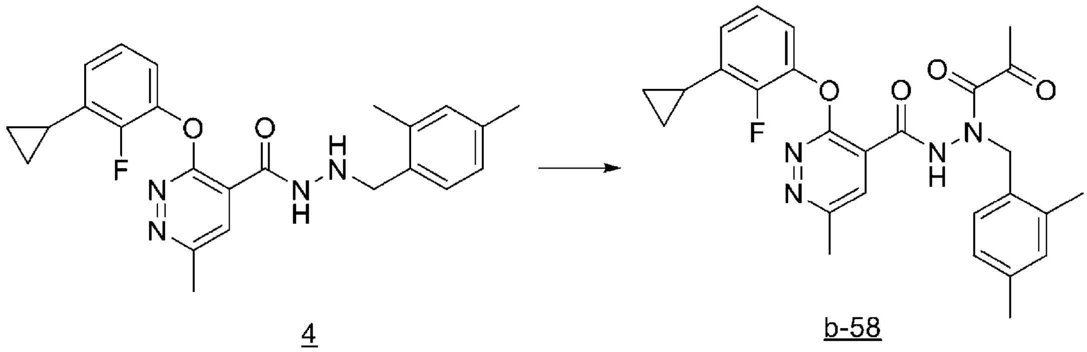

4 b-58

Compound 4 (0.08 g) was dissolved in N,N-dimethylformamide (1 mL). Pyruvic acid (0.04 g), 1-[bis(dimethylamino)methylene]-1H-1,2,3-triazolo[4,5-b]pyridinium 3-oxidehexafluorophosphate (0.17 g),1-hydroxybenzotriazole (0.01 g) and triethylamine (0.1 mL) were added thereto, and then stirred at room temperature for 18 hours. Methanol was added to the obtained liquid, and the mixture was purified by preparative high performance liquid chromatography (developing solvent: water/acetonitrile), thereby obtaining Compound b-58 (0.014 g, at a yield of 10%).

(Production Example 8)

Methyl 2-(3-(3-cyclopropyl-2-fluorophenoxy)-6-methylpyridazine-4-carbonothioyl)-1-(2,4-dimethylbenzyl)hydrazine-1-carboxylate (English name: methyl 2-(3-(3-cyclopropyl-2-fluorophenoxy)-6-methylpyridazine-4-carbonothioyl)-1-(2,4-dimethylbenzyl)hydrazine-1-carboxylate (Compound b-59)) was produced by the following steps.

[Chemical Formula 57]

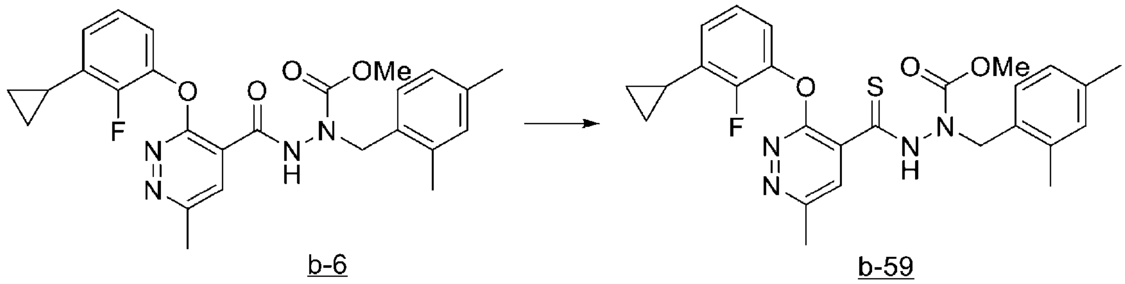

b-6 b-59

Compound b-6 (0.02 g) was dissolved in 1,4-dioxane (1 mL). Lawesson's reagent (0.02 g) was added thereto, and then stirred at 80°C for 18 hours. The solvent was distilled off under reduced pressure. The obtained residue was purified by silica gel column chromatography (developing solvent: n-hexane/ethyl acetate), thereby obtaining Compound b-59 (0.005 g, at a yield of 23%).

(Production Example 9)

Methyl 2-(3-(3-cyclopropyl-2-fluorophenoxy)-6-methylpyridazine-4-carbonyl)-1-((2,6-dichloropyridin-3-yl)methyl)hydrazine-1-carboxylate (Compound b-93) was produced by the following steps.

Step 1: Conversion to methyl ((2,6-dichloropyridin-3-yl)methyl)(1,3-dioxoindolin-2-yl)carbamate (English name: methyl ((2,6-dichloropyridin-3-yl)methyl)(1,3-dioxoisoindolin-2-yl)carbamate)

[Chemical Formula 58]

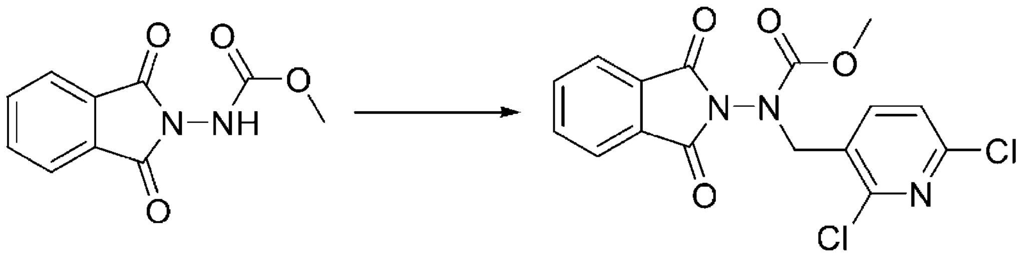

22 23

Compound 22 (0.55 g) was dissolved in N,N-dimethylformamide (7 ml). Cesium carbonate (1.6 g) and 2,6-dichloro-3-(chloromethyl)pyridine (0.5 g) were added thereto at room temperature, and then stirred at room temperature for five hours. The obtained liquid was poured into ice water. The resultant was extracted with ethyl acetate and dried over anhydrous magnesium sulfate. The solvent was distilled off under reduced pressure. The obtained residue was purified by preparative high performance liquid chromatography (developing solvent: water/acetonitrile), thereby obtaining Compound 23 (0.4 g, at a yield of 43%).

Step 2: Conversion to methyl 1-((2,6-dichloropyridin-3-yl)methyl)hydrazine-1-carboxylate (English name: methyl 1-((2,6-dichloropyridin-3-yl)methyl)hydrazine-1-carboxylate)

[Chemical Formula 59]

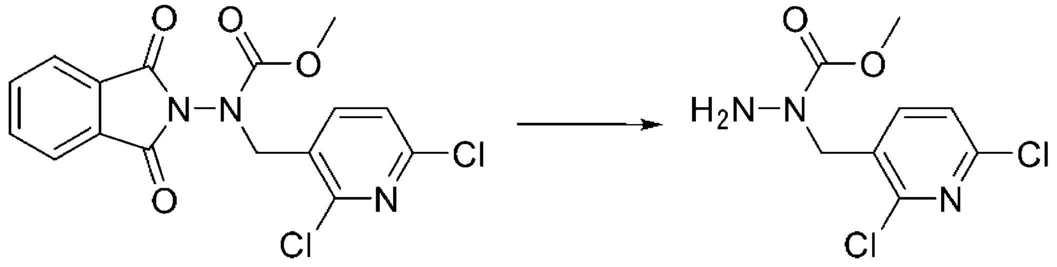

23 24

Compound 23 (0.4 g) was dissolved in ethanol (3 mL). Hydrazine monohydrate (0.25 mL) was added thereto at room temperature, and then stirred at room temperature for 18 hours. The obtained liquid was poured into ice water. The resultant was extracted with ethyl acetate and dried over anhydrous magnesium sulfate. The solvent was distilled off under reduced pressure, thereby obtaining Compound 24 (0.19 g, at a yield of 72%).

Step 3: Conversion to methyl 2-(3-(3-cyclopropyl-2-fluorophenoxy)-6-methylpyridazine-4-carbonyl)-1-((2,6-dichloropyridin-3-yl)methyl)hydrazine-1-carboxylate (English name: methyl 2-(3-(3-cyclopropyl-2-fluorophenoxy)-6-methylpyridazine-4-carbonyl)-1-((2,6-dichloropyridin-3-yl)methyl)hydrazine-1-carboxylate)

[Chemical Formula 60]

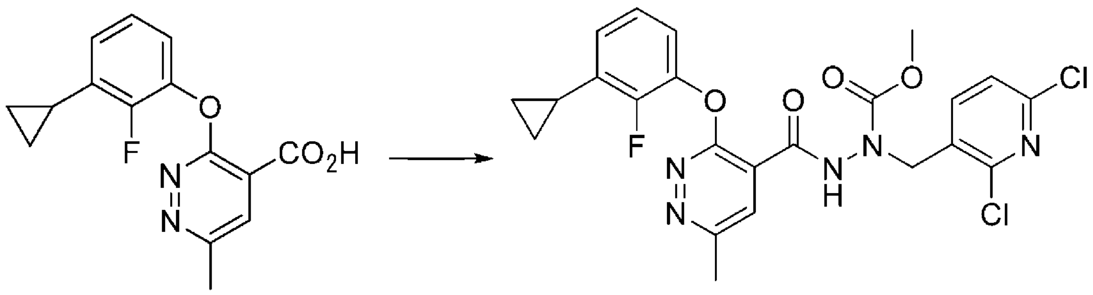

19 b-93

Compound 19 (0.05 g) was dissolved in N,N-dimethylformamide (1 mL). Compound 24 (0.092 g), 1-ethyl-3-(3-dimethylaminopropyl)carbodiimide hydrochloride (0.09 g), 1-hydroxybenzotriazole (0.01 g) and triethylamine (0.1 mL) were added thereto, and then stirred at room temperature for 18 hours. Methanol was added to the obtained liquid, and the mixture was purified by preparative high performance liquid chromatography (developing solvent: water/acetonitrile), thereby obtaining Compound b-93 (0.043 g, at a yield of 48%).

(Production Example 10)

3-(3-Cyclopropyl-2-fluorophenoxy)-N'-(2,4-dimethylbenzyl)-N'-ethanethioyl-6-methylpyridazine-4-carbohydrazide (English name: 3-(3-cyclopropyl-2-fluorophenoxy)-N'-(2,4-dimethylbenzyl)-N'-ethanethioyl-6-methylpyridazine-4-carbohydrazide (Compound b-66)) was produced by the following steps.

Step 1: Conversion to N-(2,4-dimethylbenzyl)ethanethiohydrazide (English name: N-(2,4-dimethylbenzyl)ethanethiohydrazide)

[Chemical Formula 61]

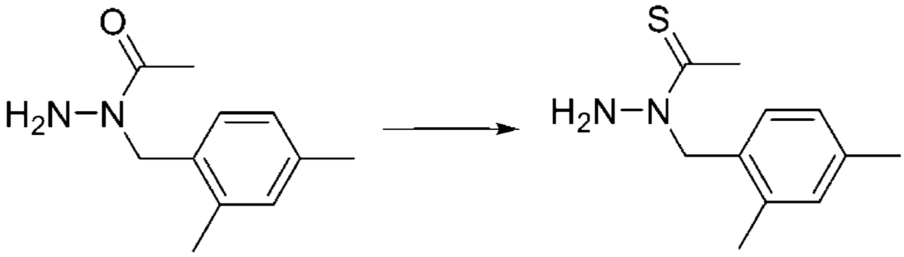

7 25

Compound 7 (0.14 g) was dissolved in dioxane (5 mL). Lawesson's reagent (0.15 g) was added thereto at room temperature, and then stirred at 50°C for one hour. Thereafter, the solvent was distilled off under reduced pressure. The obtained residue was purified by silica gel column chromatography (developing solvent: n-hexane/ethyl acetate), thereby obtaining Compound 25 (0.04 g, at a yield of 59%).

Step 2: Conversion to 3-(3-cyclopropyl-2-fluorophenoxy)-N'-(2,4-dimethylbenzyl)-N'-ethanethioyl-6-methylpyridazine-4-carbohydrazide (English name: 3-(3-cyclopropyl-2-fluorophenoxy)-N'-(2,4-dimethylbenzyl)-N'-ethanethioyl-6-methylpyridazine-4-carbohydrazide)

[Chemical Formula 62]

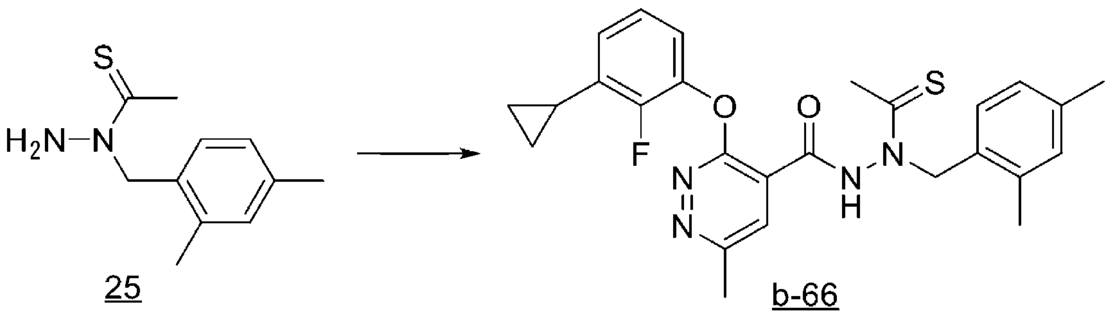

25 b-66

Compound 25 (0.035 g) was dissolved in N,N-dimethylformamide (1 mL). Compound 19 (0.08 g), 1-ethyl-3-(3-dimethylaminopropyl)carbodiimide hydrochloride (0.08 g), 1-hydroxybenzotriazole (0.01 g) and triethylamine (0.09 mL) were added thereto at 0°C, and then stirred at room temperature for 18 hours. The obtained liquid was poured into ice water. The resultant was extracted with ethyl acetate and dried over anhydrous magnesium sulfate. The solvent was distilled off under reduced pressure, and the obtained residue was purified by silica gel column chromatography (developing solvent: n-hexane/ethyl acetate), thereby obtaining Compound b-66 (0.026 g, at a yield of 20%).

(Production Example 11)

3-(3-Cyclopropyl-2-fluorophenoxy)-N'-(2,4-dimethylbenzyl)-6-methyl-N'-(2,2,2-trifluoroethyl)pyridazine-4-carbohydrazide (English name: 3-(3-cyclopropyl-2-fluorophenoxy)-N'-(2,4-dimethylbenzyl)-6-methyl-N'-(2,2,2-trifluoroethyl)pyridazine-4-carbohydrazide (Compound b-84)) was produced by the following steps.

[Chemical Formula 63]

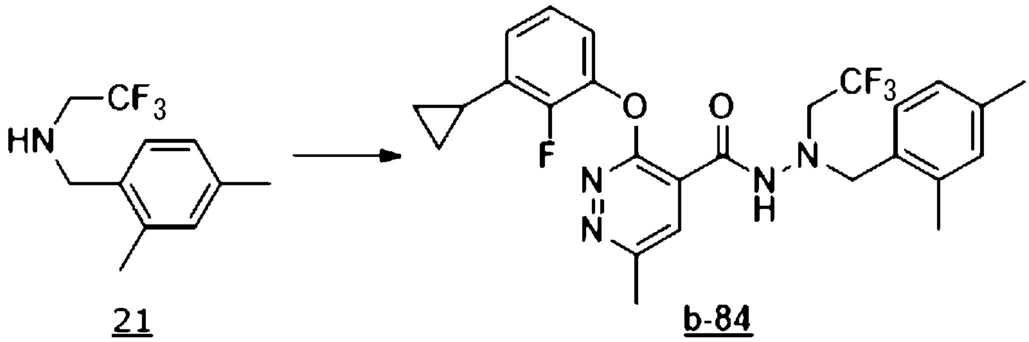

21 b-84

Compound 21(0.05 g) was dissolved in dichloromethane (1 mL). A dichloromethane solution of o-mesitylenesulfonylhydroxylamine (0.23 mL) was added thereto at room temperature, and then stirred at room temperature for 18 hours. The solvent was distilled off under reduced pressure, and the residue was dissolved in N,N-dimethylformamide (1 mL). Compound 19 (0.05 g), 1-ethyl-3-(3-dimethylaminopropyl) carbodiimide hydrochloride (0.05 g), 1-hydroxybenzotriazole (0.01 g) and triethylamine (0.06 mL) were added thereto at 0°C, and then stirred at room temperature for 18 hours. Methanol was added to the obtained liquid, and the mixture was purified by preparative high performance liquid chromatography (developing solvent: water/ acetonitrile), thereby obtaining Compound b-84 (0.01 g, at a yield of 9%).

(Production Example 12)

Methyl 2-(3-(3-chloro-2-fluorophenoxy)-6-methylpyridazine-4-carbonyl)-1-(2,4-dimethylbenzyl)hydrazine-1-carboxylate (English name: methyl 2-(3-(3-chloro-2-fluorophenoxy)-6-methylpyridazine-4-carbonyl)-1-(2,4-dimethylbenzyl)hydrazine-1-carboxylate (Compound b-23)) was produced by the following steps.

Step 1: Conversion to methyl 2-(3-chloro-6-methylpyridazine-4-carbonyl)-1-(2,4-dimethylbenzyl)hydrazine-1-carboxylate (English name: methyl 2-(3-chloro-6-methylpyridazine-4-carbonyl)-1-(2,4-dimethylbenzyl)hydrazine-1-carboxylate)

[Chemical Formula 64]

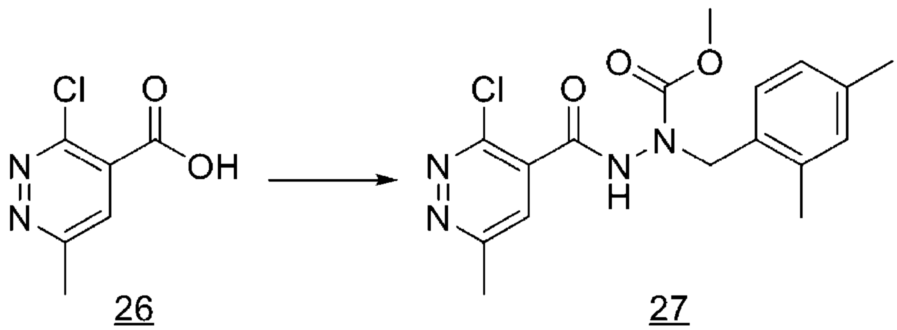

26 27

Compound 26 (0.13 g) and Compound 14 (0.17 g) were dissolved in tetrahydrofuran (2.2 ml), and propylphosphonic acid anhydride (50% ethyl acetate solution) (0.62 ml) and N,N-diisopropylethylamine (0.62 ml) were added thereto, followed by stirring the mixture at room temperature for three hours. An aqueous solution of ammonium chloride was added to the obtained liquid, and then the mixture was extracted with ethyl acetate. Then, the resultant was dried over anhydrous sodium sulfate. The solvent was distilled off under reduced pressure. The obtained residue was purified by silica gel column chromatography (developing solvent: n-hexane/ethyl acetate), thereby obtaining Compound 27 (0.18 g, at a yield of 63%).

Step 2: Conversion to methyl 2-(3-(3-chloro-2-fluorophenoxy)-6-methylpyridazine-4-carbonyl)-1-(2,4-dimethylbenzyl) hydrazine-1-carboxylate (English name: methyl 2-(3-(3-chloro-2-fluorophenoxy)-6-methylpyridazine-4-carbonyl)-1-(2,4-dimethylbenzyl) hydrazine-1-carboxylate)

[Chemical Formula 65]

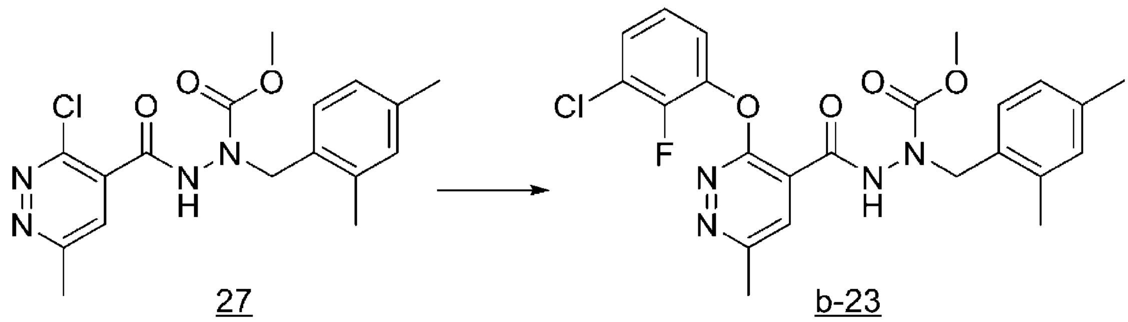

27 b-23

Compound 27 (0.120 g) was dissolved in N,N-dimethylformamide (1.10 ml), and 3-chloro-2-fluorophenol (0.13 ml) was added thereto, followed by stirring the mixture at 100°C for 22 hours. Water was added to the obtained liquid, and the mixture was extracted with ethyl acetate, and then dried over anhydrous sodium sulfate. The solvent was distilled off under reduced pressure. The obtained residue was purified by silica gel column chromatography (developing solvent: n-hexane/ethyl acetate), thereby obtaining Compound b-23 (0.060 g, at a yield of 38%).

(Production Example 13)

Methyl 1-(2,4-dimethylbenzyl)-2-(6-methyl-3-((3-(trifluoromethyl)phenyl)thio) pyridazine-4-carbonyl)hydrazine-1-carboxylate (English name: methyl 1-(2,4-dimethylbenzyl)-2-(6-methyl-3-((3-(trifluoromethyl)phenyl)thio)pyridazine-4-carbonyl)hydrazine-1-carboxylate (Compound b-78)) was produced by the following steps.

[Chemical Formula 66]

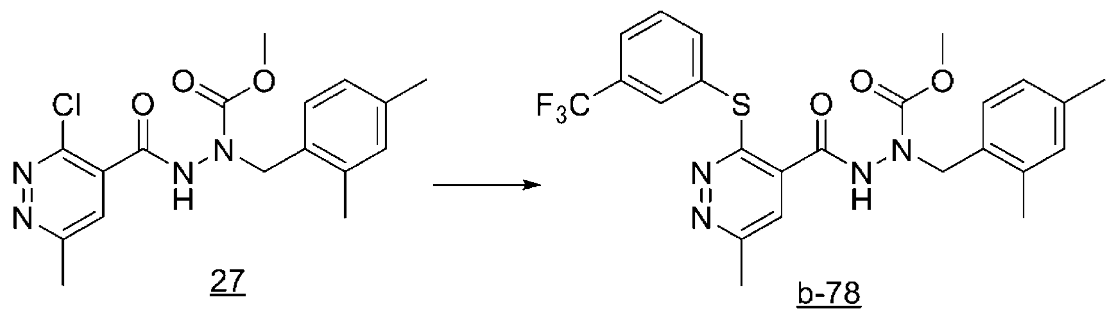

27 b-78

Compound 27 (0.150 g) was dissolved in N,N-dimethylformamide (1.40 ml), and 3-(trifluoromethyl)thiophenol (0.147 g) was added thereto, followed by stirring the mixture at 120°C for 17 hours. Water was added to the obtained liquid, and the mixture was extracted with ethyl acetate, and then dried over anhydrous sodium sulfate. The solvent was distilled off under reduced pressure. The obtained residue was purified by silica gel column chromatography (developing solvent: n-hexane/ethyl acetate), thereby obtaining Compound b-78 (0.167 g, at a yield of 80%).

(Production Example 14)

Methyl 2-(3-(3-bromo-2-fluorophenoxy)-6,7-dihydro-5H-cyclopenta[c]pyridazine-4-carbonyl)-1-(2,4-dimethylbenzyl)hydrazine-1-carboxylate (English name: methyl 2-(3-(3-bromo-2-fluorophenoxy)-6,7-dihydro-5H-cyclopenta[c] pyridazine-4-carbonyl)-1-(2,4-dimethylbenzyl)hydrazine-1-carboxylate (Compound b-92)) was produced by the following steps.

Step 1: Conversion to methyl 2-(3-chloro-6,7-dihydro-5H-cyclopenta[c]pyridazine-4-carbonyl)-1-(2,4-dimethylbenzyl) hydrazine-1-carboxylate (English name: methyl 2-(3-chloro-6,7-dihydro-5H-cyclopenta[c]pyridazine-4-carbonyl)-1-(2,4-dimethylbenzyl) hydrazine-1-carboxylate)

[Chemical Formula 67]

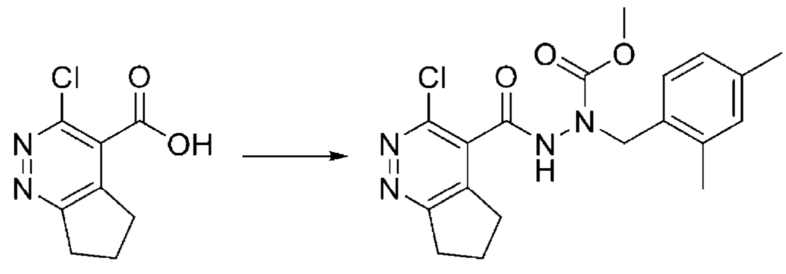

28 29

Compound 28 (0.100 g) and Compound 14 (0.110 g) were dissolved in tetrahydrofuran (1.70 ml), and then propylphosphonic acid anhydride (50% ethyl acetate solution) (0.416 ml) and N,N-diisopropylethylamine (0.897 ml) were added thereto, followed by stirring the mixture at room temperature for 16 hours. An aqueous solution of ammonium chloride was added to the obtained liquid, and the mixture was extracted with ethyl acetate. Then, the resultant was dried over anhydrous sodium sulfate. The solvent was distilled off under reduced pressure. The obtained residue was purified by silica gel column chromatography (developing solvent: n-hexane/ethyl acetate), thereby obtaining Compound 29 (0.085 g, at a yield of 43%).

Step 2: Conversion to methyl 2-(3-(3-bromo-2-fluorophenoxy)-6,7-dihydro-5H-cyclopenta[c]pyridazine-4-carbonyl)-1-(2,4-dimethylbenzyl)hydrazine-1-carboxylate (English name: methyl 2-(3-(3-bromo-2-fluorophenoxy)-6,7-dihydro-5H-cyclopenta[c]pyridazine-4-carbonyl)-1-(2,4-dimethylbenzyl)hydrazine-1-carboxylate)

[Chemical Formula 68]

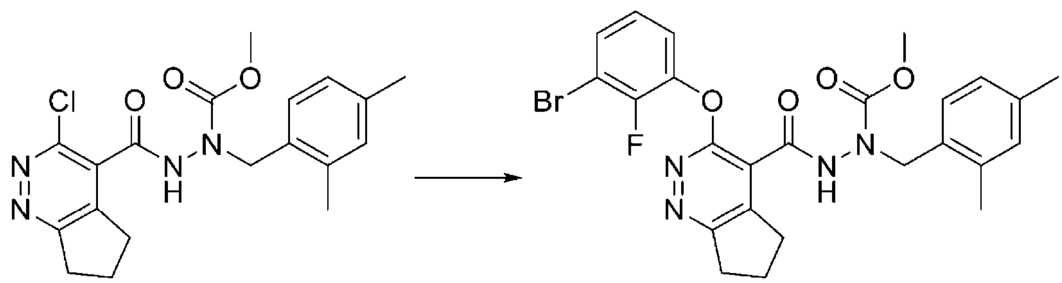

29 b-92

Compound 29 (0.085 g) was dissolved in N,N-dimethylformamide (0.730 ml), and then 3-bromo-2-fluorophenol (0.084 g) was added thereto, followed by stirring the mixture at 120°C for 21 hours. Water was added to the obtained liquid, and the mixture was extracted with ethyl acetate, and then dried over anhydrous sodium sulfate. The solvent was distilled off under reduced pressure. The obtained residue was purified by silica gel column chromatography (developing solvent: n-hexane/ethyl acetate), thereby obtaining Compound b-92 (0.031 g, at a yield of 26%).

(Production Example 15)

Methyl 2-(6-chloro-3-((3-(trifluoromethyl)phenyl)amino)pyridazine-4-carbonyl) -1-(2,4-dimethylbenzyl)hydrazine-1-carboxylate (English name: methyl 2-(6-chloro-3-((3-(trifluoromethyl)phenyl)amino)pyridazine-4-carbonyl)-1-(2,4-dimethylbenzyl)hydrazine-1-carboxylate (Compound b-91)) was produced by the following steps.

Step 1: Conversion to 6-chloro-3-((3-(trifluoromethyl)phenyl)amino)pyridazine--4-carboxylic acid (English name: 6-chloro-3-((3-(trifluoromethyl)phenyl)amino)pyridazine-4-carboxylic acid)

[Chemical Formula 69]

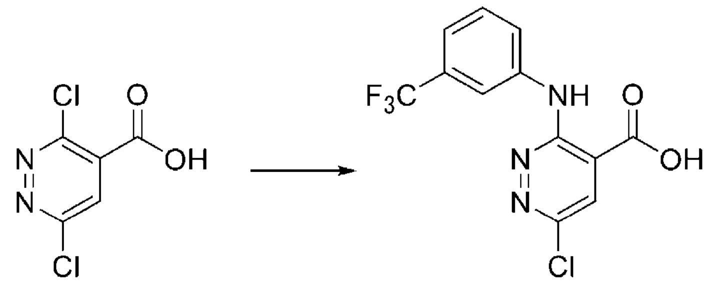

30

31

Compound 30 (1.00 g) was dissolved in ethanol (17.0 ml), and then 3-(trifluoromethyl)aniline (0.645 ml) was added thereto, followed by stirring the mixture at room temperature for 21 hours. Dilute hydrochloric acid was added to the obtained liquid, and the mixture was extracted with ethyl acetate. Then, the resultant was dried over anhydrous sodium sulfate. The solvent was distilled off under reduced pressure. The obtained residue was washed with chloroform, thereby obtaining Compound 31 (1.08 g, at a yield of 65%).

Step 2: Conversion to methyl 2-(6-chloro-3-((3-(trifluoromethyl)phenyl)amino)pyridazine -4-carbonyl)-1-(2,4-dimethylbenzyl)hydrazine-1-carboxylate (English name: methyl 2-(6-chloro-3-((3-(trifluoromethyl)phenyl)amino)pyridazine-4-carbonyl)-1-(2,4-dimethylbenzyl)hydrazine-1-carboxylate)

[Chemical Formula 70]

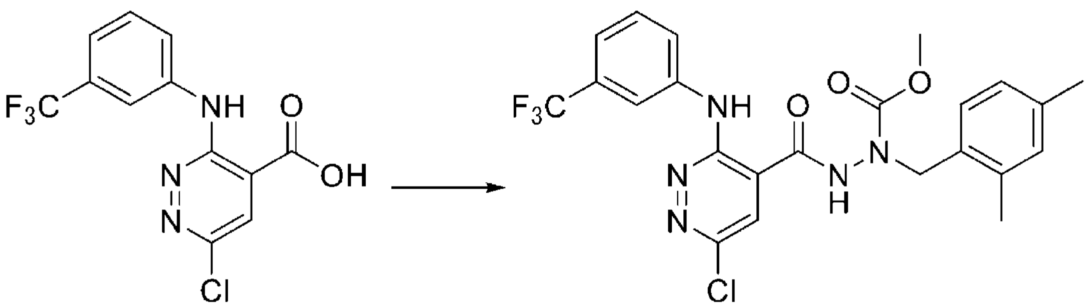

31

b-91

Compound 31 (0.041 g) and Compound 14 (0.06 g) were dissolved in tetrahydrofuran (0.630 ml), and then propylphosphonic acid anhydride (50% ethyl acetate solution) (0.156 ml) and N,N-diisopropylethylamine (0.336 ml) were added thereto, followed by stirring the mixture at room temperature for 24 hours. An aqueous solution of ammonium chloride was added to the obtained liquid, and the mixture was extracted with ethyl acetate. Then, the resultant was dried over anhydrous sodium sulfate. The solvent was distilled off under reduced pressure. The obtained residue was purified by silica gel column chromatography (developing solvent: n-hexane/ethyl acetate), thereby obtaining Compound b-91 (0.060 g, at a yield of 61%).

(Production Example 16)

Methyl 2-(3-(3-cyclopropyl-2-fluorophenoxy)cinnoline-4-carbonyl)-1-(2,4-dimethylbenzyl)hydrazine-1-carboxylate (English name: methyl 2-(3-(3-cyclopropyl-2-fluorophenoxy)cinnoline-4-carbonyl)-1-(2,4-dimethylbenzyl)hydrazine-1-carboxylate)) (Compound b-90) was produced by the following steps.

[Chemical Formula 71]

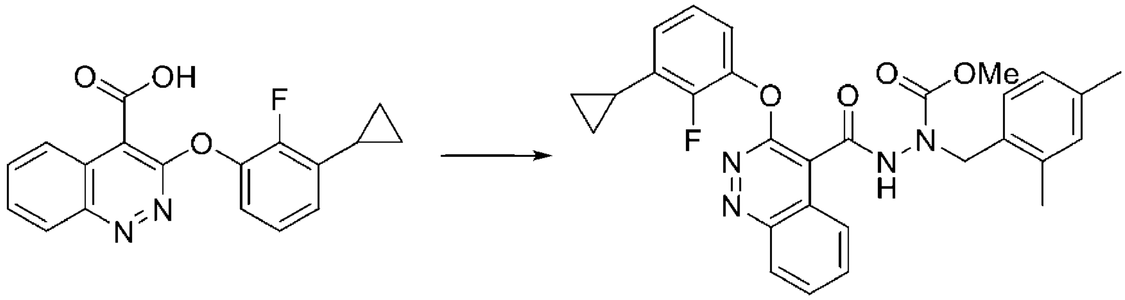

32

b-90

Compound 32 (0.08 g) was dissolved in N,N-dimethylformamide (1 mL). Compound 14 (0.08 g), 1-ethyl-3-(3-dimethylaminopropyl)carbodiimide hydrochloride (0.07 g), 1-hydroxybenzotriazole (0.004 g) and triethylamine (0.07 mL) were added thereto at room temperature, followed by stirring the mixture at room temperature for 18 hours. The solvent was distilled off from the obtained liquid under reduced pressure. The obtained residue was purified by silica gel column chromatography (developing solvent: n-hexane/ethyl acetate), thereby obtaining Compound b-90 (0.05 g, at a yield of 62%).

Compounds shown in Table 1 were produced through steps similar to those in the above-mentioned Production Examples. The melting point is recorded in the property column. The LCMS retention times of compounds, the external appearance of which was AMORPHOUS or VISCOUS OIL, are recorded.

The LCMS retention time (SQD2) was determined by the following method.

The measurement was carried out with a system using an ultra-high-performance liquid chromatography (ACQUITY UPLC H-Class: manufactured by Waters Corporation), a HPLC/UHPLC detector (ACQUITY UPLC photodiode array (PDA) eλ detector (manufactured by Waters Corporation)) and a single quadrupole MS detector (LC/MS) (SQ Detector 2 detector (positive and negative ion electrospray modes and an atmospheric pressure ionization mode), a CORTECS UPLC C18 column (manufactured by Waters Corporation, 2.1×50 mm, 1.6 μm) being attached to the system, at a temperature of 40°C, a flow rate of 0.6 mL/minute, and an injection amount of 2 μL. Water containing 0.1% formic acid was used as the mobile phase (A), and acetonitrile was used as the mobile phase (B). The concentration of the mobile phase (B) was maintained at 30% by mass for 0.15 minutes, and then increased from 30% by mass to 95% by mass at a constant velocity over 1.35 minutes, followed by maintaining the concentration at 95% by mass for 0.5 minutes, immediately decreasing the concentration of the mobile phase (B) to 30% by mass, and then finally maintaining the concentration of the mobile phase (B) at 30% by mass for 0.50 minutes.

The LCMS (QDa) retention time was determined by the following method.

The measurement was carried out with a system using an ultra-high-performance liquid chromatography (ACQUITY UPLC H-Class: manufactured by Waters Corporation), a HPLC/UHPLC detector (ACQUITY UPLC photodiode array (PDA) eλ detector (manufactured by Waters Corporation)) and a single quadrupole MS detector (LC/MS) (ACQUITY QDa detector (positive and negative ion electrospray modes), a CORTECS UPLC C18 column (manufactured by Waters Corporation, 2.1×50 mm, 1.6 μm) being attached to the system, at a temperature of 40°C, a flow rate of 0.6 mL/minute, and an injection amount of 2 μL. Water containing 0.1% formic acid was used as the mobile phase (A), and acetonitrile was used as the mobile phase (B). The concentration of the mobile phase (B) was maintained at 30% by mass for 0.15 minutes, and then increased from 30% by mass to 95% by mass at a constant velocity over 1.35 minutes, followed by maintaining the concentration at 95% by mass for 0.5 minutes, immediately decreasing the concentration of the mobile phase (B) to 30% by mass, and then finally maintaining the concentration of the mobile phase (B) at 30% by mass for 0.50 minutes.

[Table 1]

Table 1

| Compound No. | Chemical formula | Property | LCMS (SQD2) Retention time | LCMS (QDa) Retention time |
|---|---|---|---|---|
| b-1 | 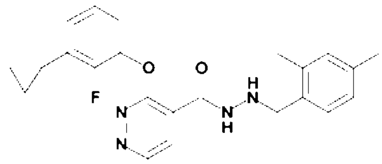 | m.p.:60-62°C | | |
| b-2 | 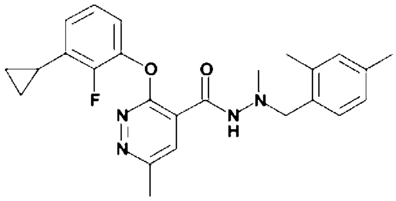 | AMORPHOUS | 1.66min | |
| b-3 | 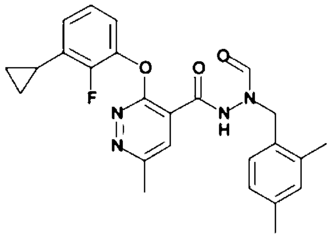 | m.p.:71-73°C | | |
| b-4 | 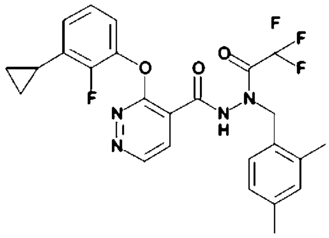 | m.p.:62-64°C | | |
| b-5 | 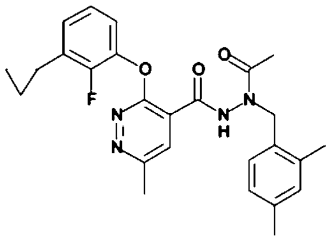 | AMORPHOUS | 1.61min | |

[Table 2]

Table 1 (continued)

| Compound No. | Chemical formula | Property | LCMS (SQD2) Retention time | LCMS (QDa) Retention time |
|---|---|---|---|---|
| b-6 | 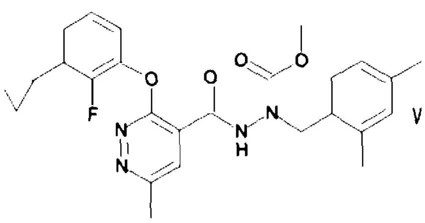 | VISCOUS OIL | 1.67min | |

(continued)

| Table 1 (continued) | | | | |
|---|---|---|---|---|
| Compound No. | Chemical formula | Property | LCMS (SQD2) Retention time | LCMS (QDa) Retention time |
| b-7 | 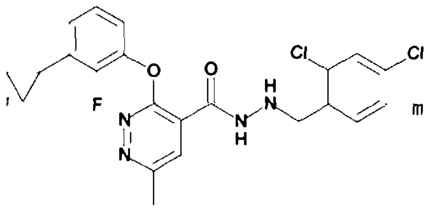 | m.p. :65-67°C | | |
| b-8 | 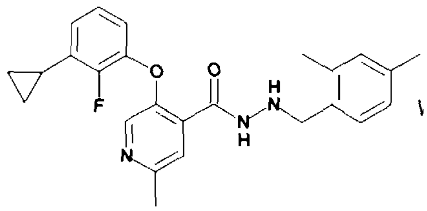 | VISCOUS OIL | 1.78min | |
| b-9 | 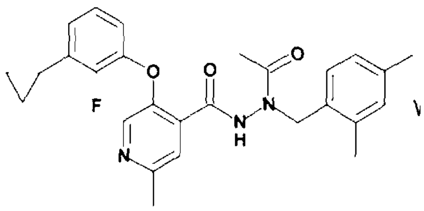 | VISCOUS OIL | 1.69min | |
| b-10 | 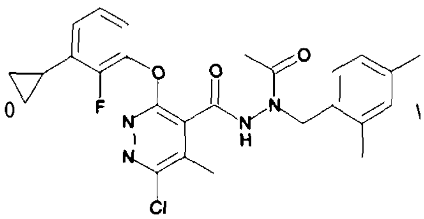 | VISCOUS OIL | 1.73min | |

[Table 3]

| Table 1 (continued) | | | | |
|---|---|---|---|---|
| Compound No. | Chemical formula | Property | LCMS (SQD2) Retention time | LCMS (QDa) Retention time |
| b-11 | 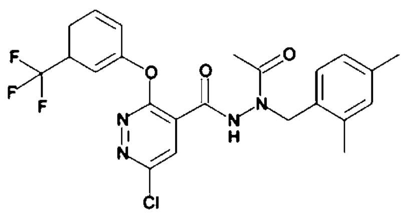 | VISCOUS OIL | 1.71min | |
| b-12 | 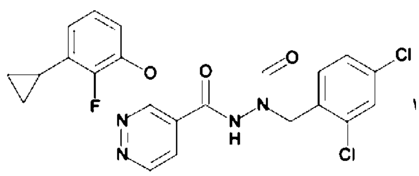 | VISCOUS OIL | 1.88min | |

(continued)

| Table 1 (continued) | | | | |
|---|---|---|---|---|
| Compound No. | Chemical formula | Property | LCMS (SQD2) Retention time | LCMS (QDa) Retention time |
| b-13 | 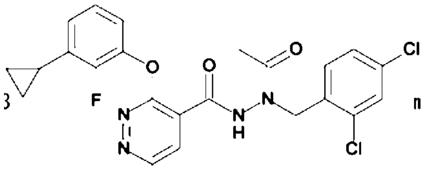 | m.p. :81-83°C | | |
| b-14 | 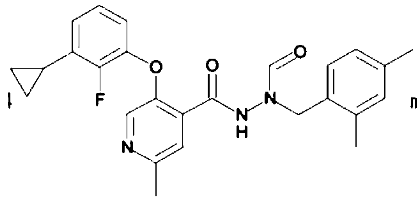 | m.p. :54-56°C | | |
| b-15 | 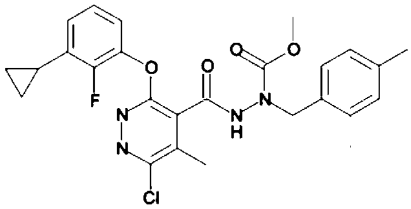 | AMORPHOUS | 1.81min | |

[Table 4]

| Table 1 (continued) | | | | |
|---|---|---|---|---|
| Compound No. | Chemical formula | Property | LCMS (SQD2) Retention time | LCMS (QDa) Retention time |
| b-16 | 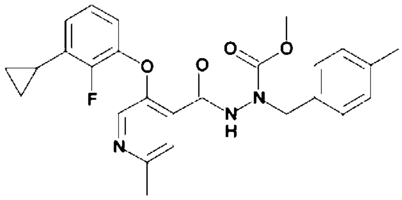 | VISCOUS OIL | 1.78min | |
| b-17 | 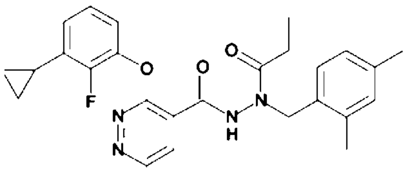 | VISCOUS OIL | 1.74min | |
| b-18 | 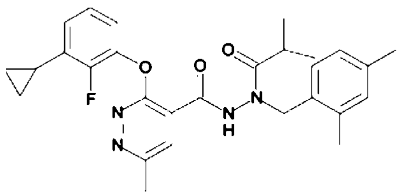 | VISCOUS OIL | 1.80min | |

(continued)

| Table 1 (continued) | | | | |
|---|---|---|---|---|
| Compound No. | Chemical formula | Property | LCMS (SQD2) Retention time | LCMS (QDa) Retention time |
| b-19 | 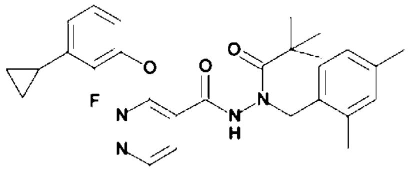 | VISCOUS OIL | 1.89min | |
| b-20 | 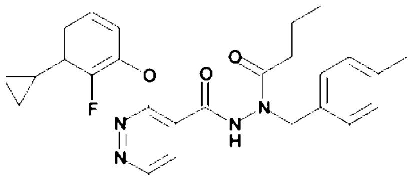 | VISCOUS OIL | 1.80min | |

[Table 5]

| Table 1 (continued) | | | | |
|---|---|---|---|---|
| Compound No. | Chemical formula | Property | LCMS (SQD2) Retention time | LCMS (QDa) Retentior time |
| b-21 | 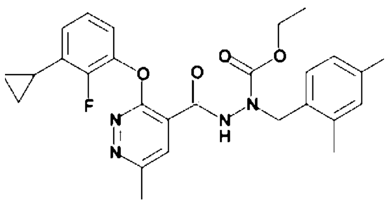 | m.p. :70-72°C | | |
| b-22 | 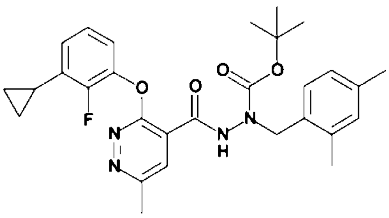 | m.p. :65-67°C | | |
| b-23 | 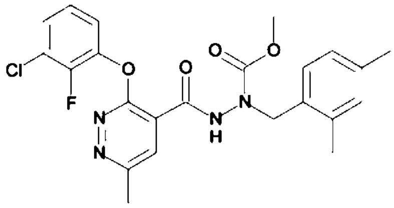 | AMORPHOUS | | 1.67min |
| b-24 | 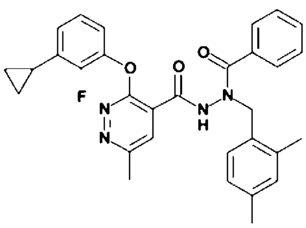 | m.p. :85-87°C | | |

(continued)

| Table 1 (continued) | | | | |
|---|---|---|---|---|
| Compound No. | Chemical formula | Property | LCMS (SQD2) Retention time | LCMS (QDa) Retentior time |
| b-25 | 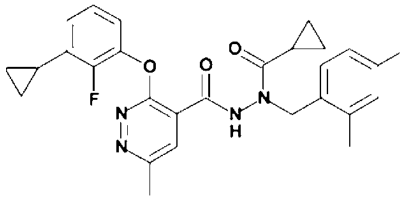 | m.p. :75-77°C | | |

[Table 6]

| Table 1 (continued) | | | | |
|---|---|---|---|---|
| Compound No. | Chemical formula | Property | LCMS (SQD2) Retention time | LCMS (QDa) Retention time |
| b-26 | 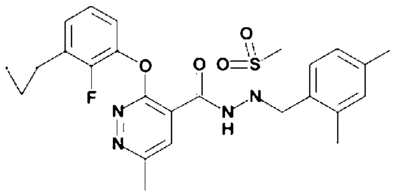 | m.p. :82-84°C | | |
| b-27 | 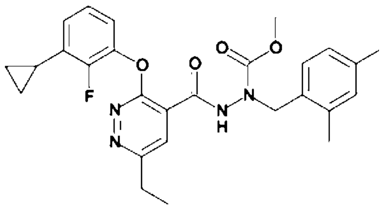 | VISCOUS OIL | 1.85min | |
| b-28 | 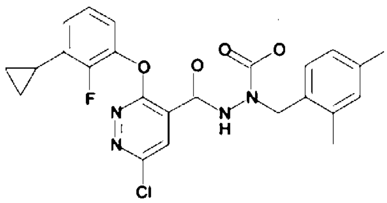 | m.p. :72-74°C | | |
| b-29 | 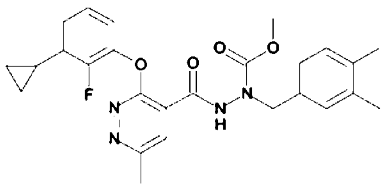 | m.p. :65-67°C | | |
| b-30 | 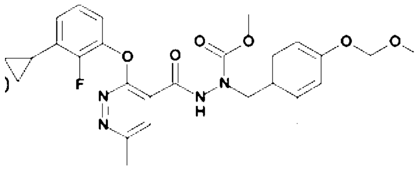 | m.p. :68-70°C | | |

[Table 7]

| Table 1 (continued) | | | | |
|---|---|---|---|---|
| Compound No. | Chemical formula | Property | LCMS (SQD2) Retention time | LCMS (QDa) Retention time |
| b-31 | 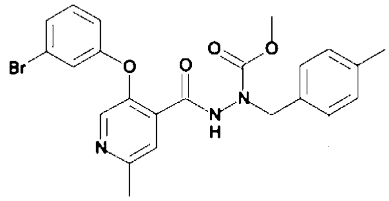 | m.p. :150-152°C | | |
| b-32 | 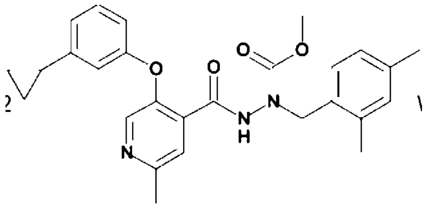 | VISCOUS OIL | 1.83min | |
| b-33 | 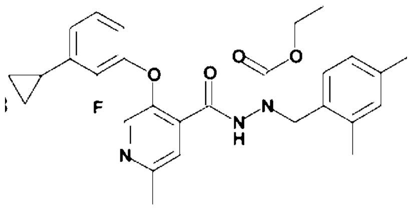 | m.p. :51-53°C | | |
| b-34 | 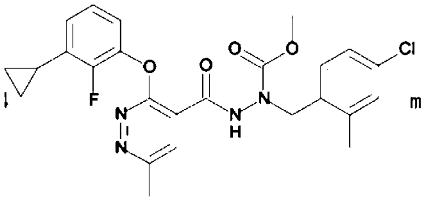 | m.p. :70-72°C | | |
| b-35 | 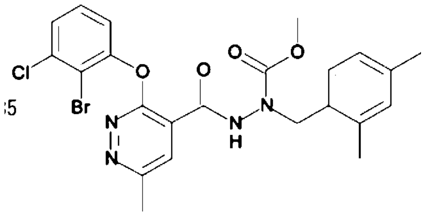 | AMORPHOUS | | 1.71min |

[Table 8]

| Table 1 (continued) | | | | |
|---|---|---|---|---|
| Compound No. | Chemical formula | Property | LCMS (SQD2) Retention time | LCMS (QDa) Retention time |
| b-36 | 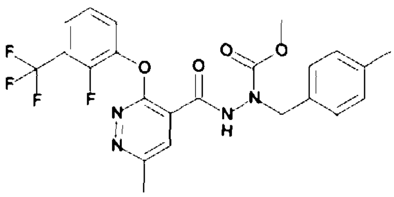 | m.p. : 125-126°C | | |

(continued)

| Table 1 (continued) | | | | |
|---|---|---|---|---|
| Compound No. | Chemical formula | Property | LCMS (SQD2) Retention time | LCMS (QDa) Retention time |
| b-37 | 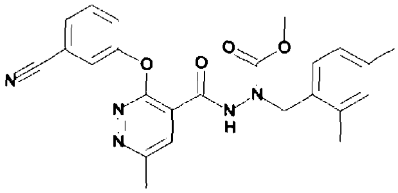 | AMORPHOUS | | 1.44min |
| b-38 | 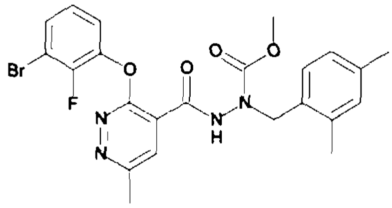 | AMORPHOUS | | 1.67min |
| b-39 | 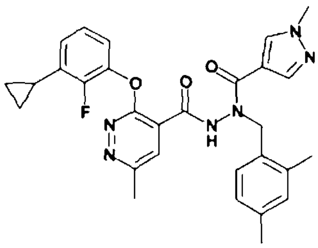 | m.p. : 70-72°C | | |
| b-40 | 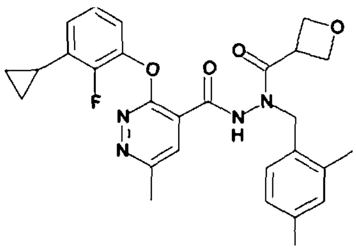 | m.p. : 93-95°C | | |

[Table 9]

| Table 1 (continued) | | | | |
|---|---|---|---|---|
| Compound No. | Chemical formula | Property | LCMS (SQD2) Retention time | LCMS (QDa) Retention time |
| b-41 | 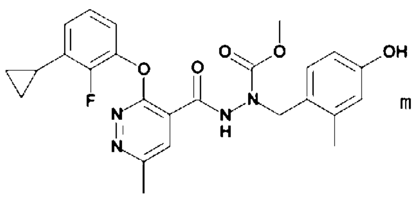 | m.p. :112-114°C | | |
| b-42 | 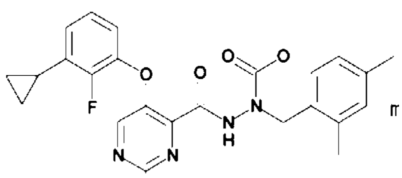 | m.p. :120-122°C | | |

(continued)

| Table 1 (continued) | | | | |
|---|---|---|---|---|
| Compound No. | Chemical formula | Property | LCMS (SQD2) Retention time | LCMS (QDa) Retention time |
| b-43 | 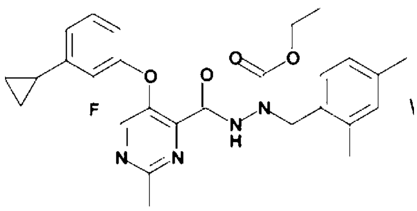 | VISCOUS OIL | 1.85min | |
| b-44 | 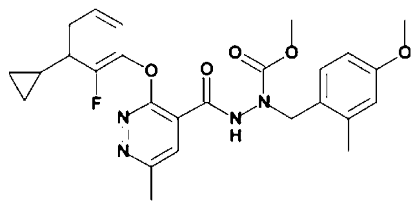 | m.p. :81-83°C | | |
| b-45 | 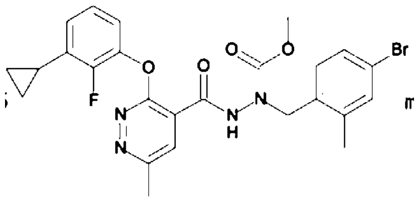 | m.p. :80-82°C | | |

[Table 10]

| Table 1 (continued) | | | | |
|---|---|---|---|---|
| Compound No. | Chemical formula | Property | LCMS (SQD2) Retention time | LCMS (QDa) Retention time |
| b-46 | 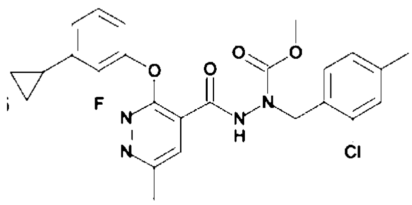 | m.p.:66-68°C | | |
| b-47 | 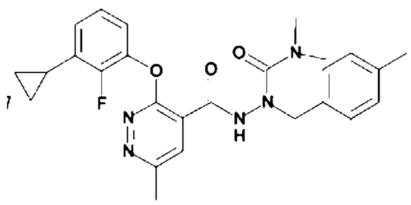 | m.p.:68-70°C | | |
| b-48 | 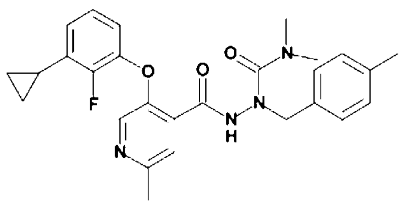 | VISCOUS OIL | | 1.72min |

(continued)

Table 1 (continued)

| Compound No. | Chemical formula | Property | LCMS (SQD2) Retention time | LCMS (QDa) Retention time |
|---|---|---|---|---|
| b-49 | 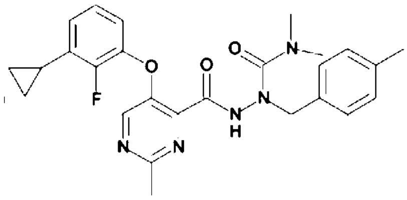 | m.p.:70-72°C | | |
| b-50 | 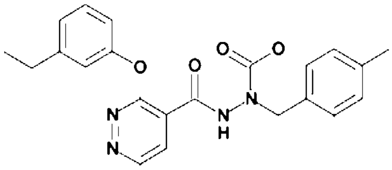 | AMORPHOUS | | 1.70min |

[Table 11]

Table 1 (continued)

| Compound No. | Chemical formula | Property | LCMS (SQD2) Retention time | LCMS (QDa) Retention time |
|---|---|---|---|---|
| b-51 | 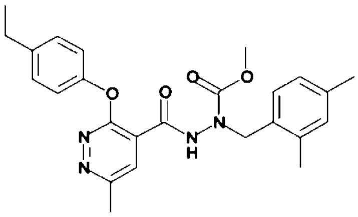 | AMORPHOUS | 1.70min | |
| b-52 | 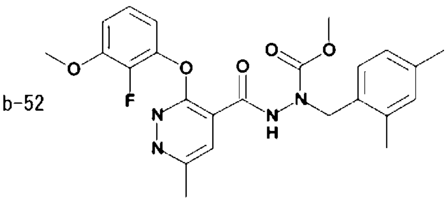 | AMORPHOUS | | 1.58min |
| b-53 | 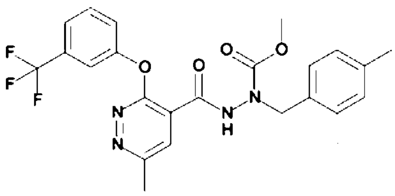 | AMORPHOUS | | 1.76min |
| b-54 | 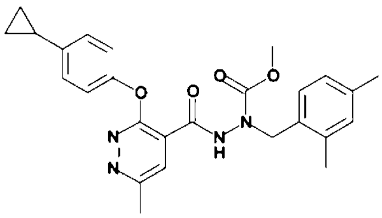 | m.p.:60-62°C | | |

(continued)

| Table 1 (continued) | | | | |
|---|---|---|---|---|
| Compound No. | Chemical formula | Property | LCMS (SQD2) Retention time | LCMS (QDa) Retention time |
| b-55 | 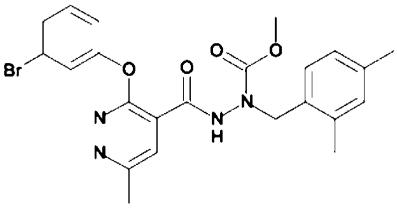 | AMORPHOUS | | 1.71min |

[Table 12]

| Table 1 (continued) | | | | |
|---|---|---|---|---|
| Compound No. | Chemical formula | Property | LCMS (SQD2) Retention time | LCMS (QDa) Retention time |
| b-56 | 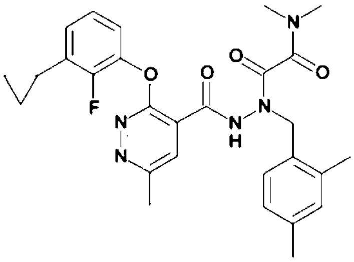 | VISCOUS OIL | | 1. 61min |
| b-57 | 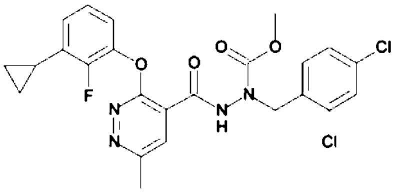 | m.p.:78-80°C | | |
| b-58 | 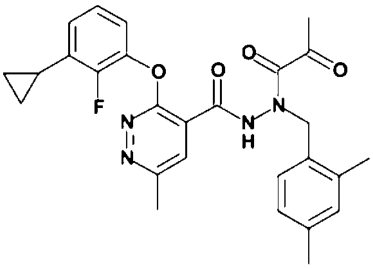 | VISCOUS OIL | 1.72min | |
| b-59 | 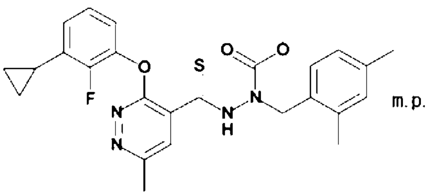 | m.p.:171-173°C | | |

(continued)

| Table 1 (continued) | | | | |
|---|---|---|---|---|
| Compound No. | Chemical formula | Property | LCMS (SQD2) Retention time | LCMS (QDa) Retention time |
| | b-60 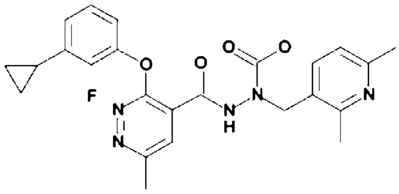 | VISCOUS OIL | 0.35min | |

[Table 13]

| Table 1 (continued) | | | | |
|---|---|---|---|---|
| Compound No. | Chemical formula | Property | LCMS (SQD2) Retention time | LCMS (QDa) Retention time |
| | b-61 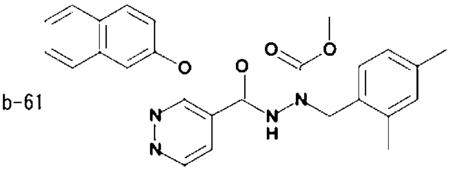 | AMORPHOUS | | 1.67min |
| | b-62 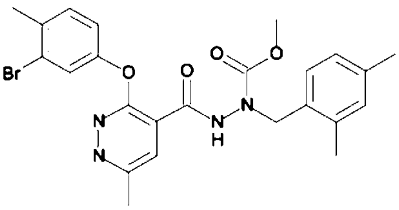 | AMORPHOUS | | 1.75min |
| | b-63 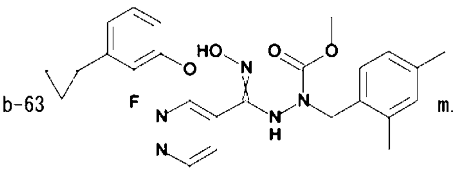 | m.p.:107-109°C | | |
| | b-64 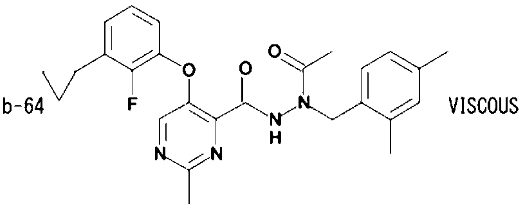 | VISCOUS OIL | 1.64min | |
| | b-65 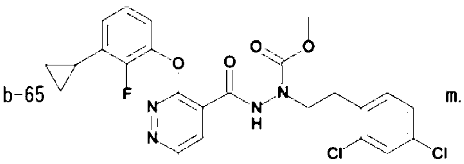 | m.p.:62-64°C | | |

[Table 14]

| Table 1 (continued) | | | | |
|---|---|---|---|---|
| Compound No. | Chemical formula | Property | LCMS (SQD2) Retention time | LCMS (QDa) Retention time |
| b-66 | 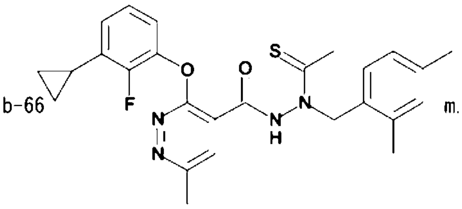 | m.p.:80-82°C | | |
| b-67 | 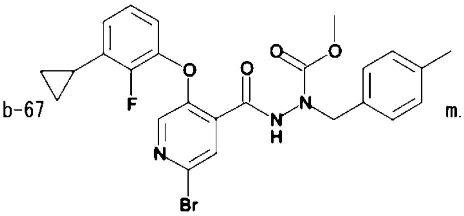 | m.p.:52-54°C | | |
| b-68 | 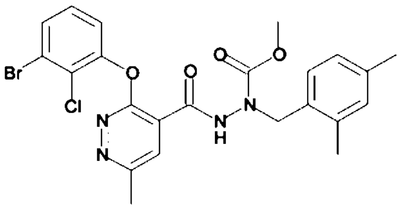 | AMORPHOUS | | 1.70min |
| b-69 | 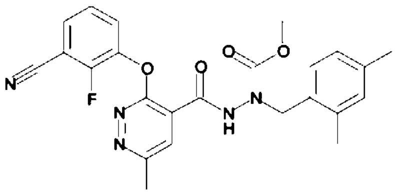 | AMORPHOUS | | 1.45min |
| b-70 | 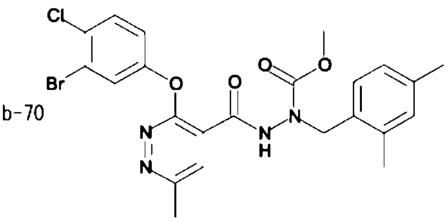 | AMORPHOUS | | 1.74min |

[Table 15]

| Table 1 (continued) | | | | |
|---|---|---|---|---|
| Compound No. | Chemical formula | Property | LCMS (SQD2) Retention time | LCMS (QDa) Retention time |
| b-71 | 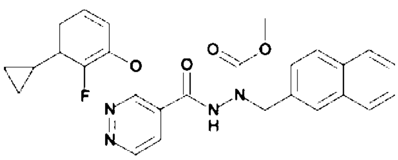 | VISCOUS OIL | 1.69min | |

(continued)

| Table 1 (continued) | | | | |
|---|---|---|---|---|
| Compound No. | Chemical formula | Property | LCMS (SQD2) Retention time | LCMS (QDa) Retention time |
| b-72 | 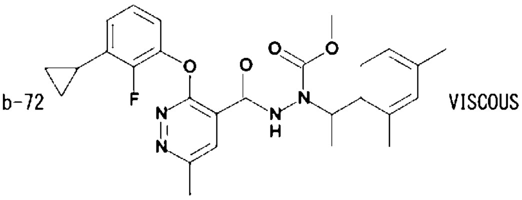 | VISCOUS OIL | 1.75min | |
| b-73 | 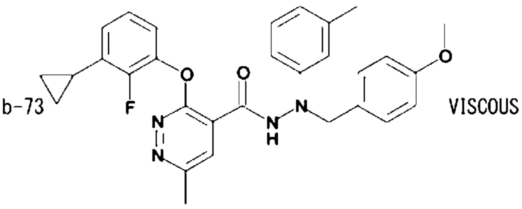 | VISCOUS OIL | 1.82min | |
| b-74 | 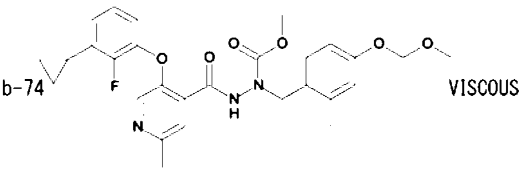 | VISCOUS OIL | 1.63min | |
| b-75 | 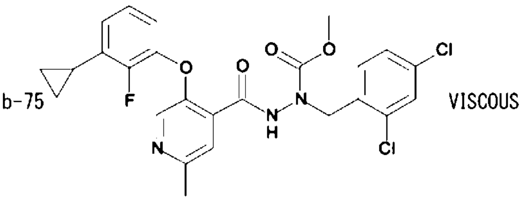 | VISCOUS OIL | 1.83min | |

[Table 16]

| Table 1 (continued) | | | | |
|---|---|---|---|---|
| Compound No. | Chemical formula | Property | LCMS (SQD2) Retention time | LCMS (QDa) Retention time |
| b-76 | 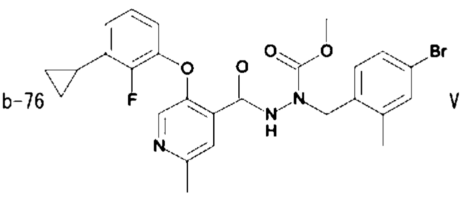 | VISCOUS OIL | 1. 82m in | |
| b-77 | 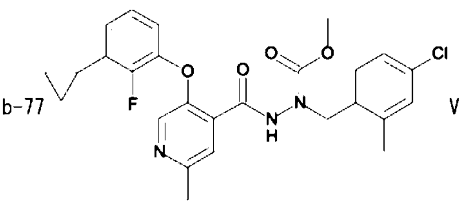 | VISCOUS OIL | 1. 79min | |

(continued)

| Table 1 (continued) | | | | |
|---|---|---|---|---|
| Compound No. | Chemical formula | Property | LCMS (SQD2) Retention time | LCMS (QDa) Retention time |
| | b-78 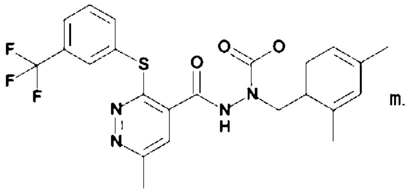 | m.p.:156-157°C | | |
| | b-79 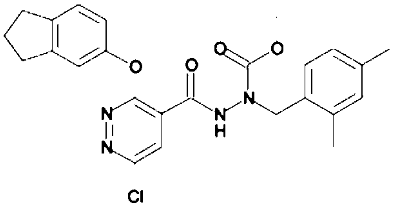 | AMORPHOUS | | 1. 85min |
| | b-80 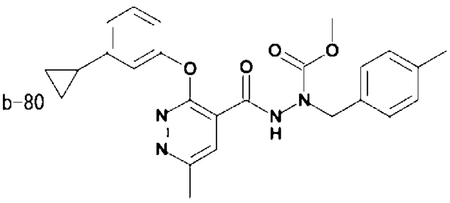 | AMORPHOUS | 1. 70min | |

[Table 17]

| Table 1 (continued) | | | | |
|---|---|---|---|---|
| Compound No. | Chemical formula | Property | LCMS (SQD2) Retention time | LCMS (QDa) Retention time |
| | b-81 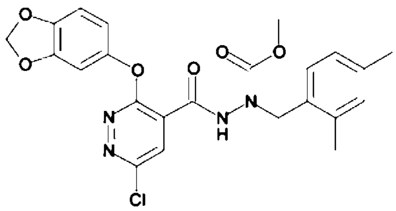 | AMORPHOUS | 1. 61min | |
| | b-82 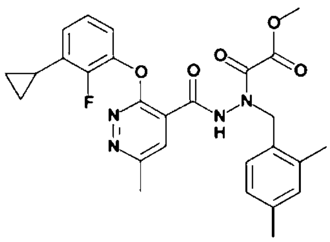 | VISCOUS OIL | 1. 71min | |

(continued)

| Table 1 (continued) | | | | |
|---|---|---|---|---|
| Compound No. | Chemical formula | Property | LCMS (SQD2) Retention time | LCMS (QDa) Retention time |
| b-83 | 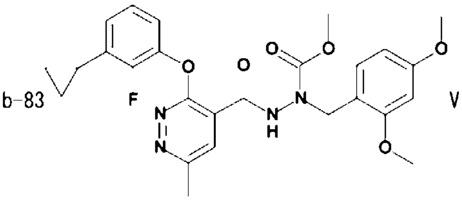 | VISCOUS OIL | 1. 55m in | |
| b-84 | 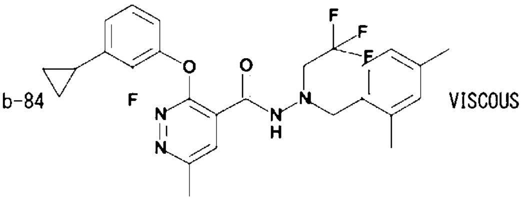 | VISCOUS OIL | 1.91min | |
| b-85 | 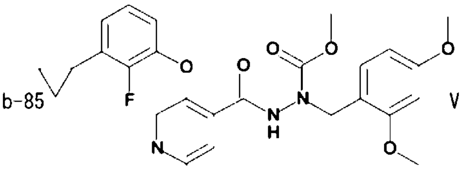 | VISCOUS OIL | 1. 61min | |

[Table 18]

| Table 1 (continued) | | | | |
|---|---|---|---|---|
| Compound No. | Chemical formula | Property | LCMS (SQD2) Retention time | LCMS (QDa) Retention time |
| b-86 | 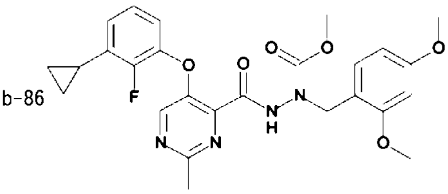 | VISCOUS OIL | 1.60min | |
| b-87 | 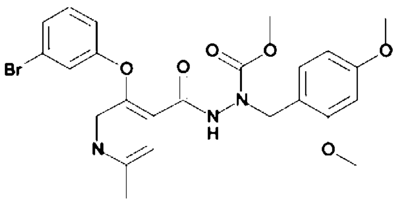 | VISCOUS OIL | 1.54min | |
| b-88 | 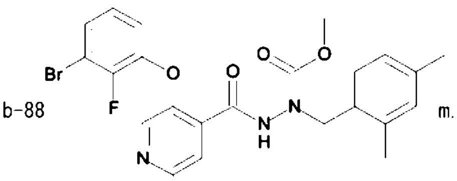 | m.p.:169-171°C | | |

(continued)

| Table 1 (continued) | | | | |
|---|---|---|---|---|
| Compound No. | Chemical formula | Property | LCMS (SQD2) Retention time | LCMS (QDa) Retention time |
| b-89 | 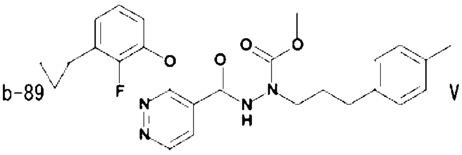 | VISCOUS OIL | 1. 76min | |
| b-90 | 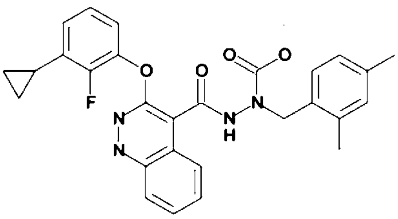 | AMORPHOUS | 1.93min | |

[Table 19]

| Table 1 (continued) | | | | |
|---|---|---|---|---|
| Compound No. | Chemical formula | Property | LCMS (SQD2) Retention time | LCMS (QDa) Retention time |
| | 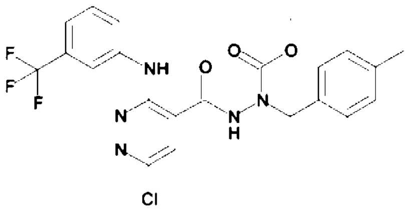 b-91 | AMORPHOUS | 1. 90min | |
| | 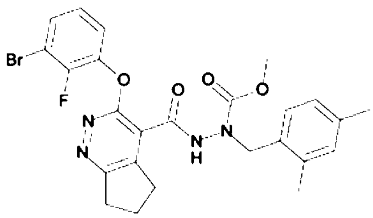 b-92 | AMORPHOUS | 1.64min | |
| | 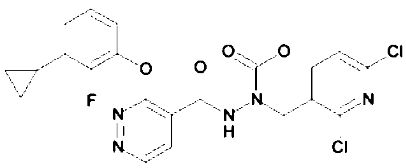 b-93 | VISCOUS OIL | 1.59min | |

(continued)

| Table 1 (continued) | | | | |
|---|---|---|---|---|
| Compound No. | Chemical formula | Property | LCMS (SQD2) Retention time | LCMS (QDa) Retention time |
| | 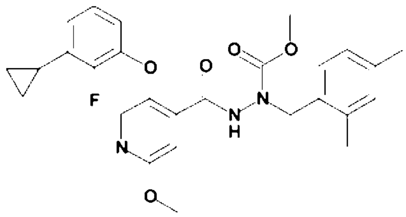 b-94 | VISCOUS OIL | 1.91min | |
| | 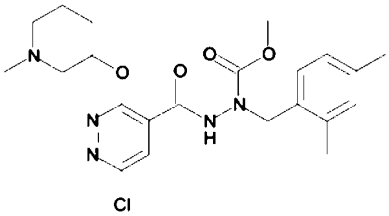 b-95 | AMORPHOUS | 0.67min | |

[Table 20]

| Table 1 (continued) | | | | |
|---|---|---|---|---|
| Compound No. | Chemical formula | Property | LCMS (SQD2) Retention time | LCMS (QDa) Retention time |
| | b-96 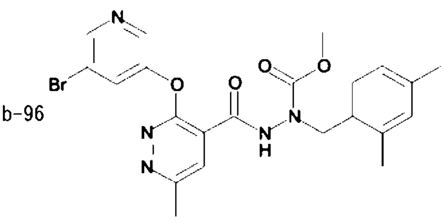 | AMORPHOUS | 1.46min | |
| | b-97 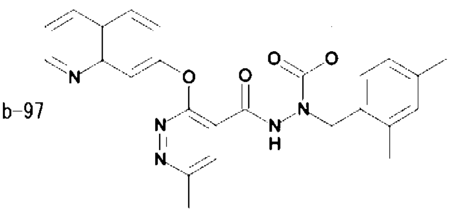 | AMORPHOUS | 1. 12min | |
| | b-98 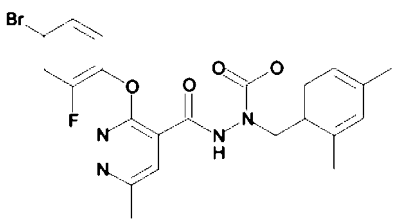 | m.p.:80-81°C | | |

(continued)

Table 1 (continued)

| Compound No. | Chemical formula | Property | LCMS (SQD2) Retention time | LCMS (QDa) Retention time |
|---|---|---|---|---|
| b-99 | 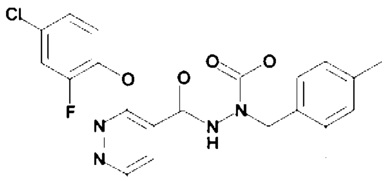 | m.p.:75-76°C | | |
| b-100 | 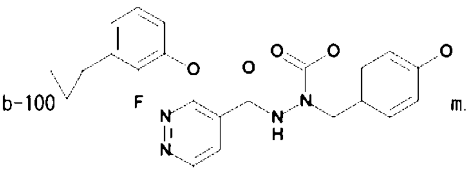 | m.p.:60-62°C | | |

(Production Example 17)

Methyl 2-(5-(3-cyclopropyl-2-fluorophenoxy)pyridazine-4-carbonyl)-1-(2,4-dimethylbenzyl)hydrazine-1-carboxylate (English name: methyl 2-(5-(3-cyclopropyl-2-fluorophenoxy)pyridazine-4-carbonyl)-1-(2,4-dimethylbenzyl)hydrazine-1-carboxylate (Compound b-142)) was produced by the following steps.

Step 1: Conversion to methyl 5-methoxypyridazine-4-carboxylate (English name: methyl 5-methoxypyridazine-4-carboxylate)

[Chemical Formula 72]

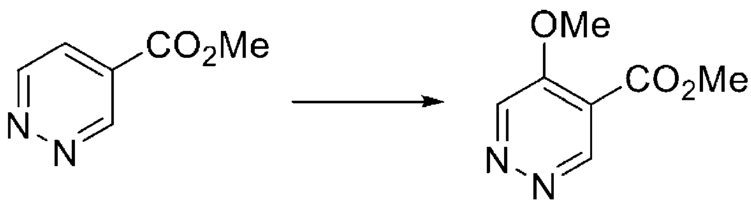

33 34

Compound 33 (0.5 g) was dissolved in N,N-dimethylformamide (1 mL). 2-Iodothiophene (1.2 g), tertiary butanol (0.5 mL) and methanol (0.2 mL) were added thereto at room temperature, and then stirred for 18 hours. The obtained liquid was poured into ice water. The resultant was extracted with ethyl acetate and dried over anhydrous magnesium sulfate. The solvent was distilled off under reduced pressure, and the obtained residue was purified by silica gel column chromatography (developing solvent: n-hexane/ethyl acetate), thereby obtaining Compound 34 (0.2 g, at a yield of 38%).

Step 2: Conversion to methyl 5-chloropyridazine-4-carboxylate (English name: methyl 5-chloropyridazine-4-carboxylate)

[Chemical Formula 73]

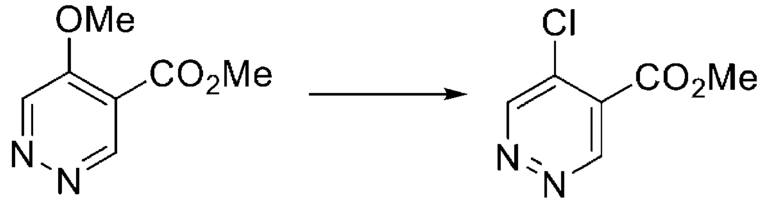

34 35

Compound 34 (0.1 g) was dissolved in concentrated hydrochloric acid (1 mL), and then stirred under heating to allow reflux to proceed for one hour. Then, the solvent was distilled off under reduced pressure, and the obtained residue was dissolved in methanol (1 mL). Thionyl chloride (0.05 mL) was added thereto at room temperature, and then stirred at room temperature for two hours. The solvent was distilled off under reduced pressure. The obtained residue was dissolved in acetonitrile. Phosphorus oxychloride (0.05 mL) and N,N-dimethylformamide (0.01 mL) were added thereto, and then stirred at 50°C for one hour. The obtained liquid was poured into an aqueous solution of sodium carbonate. The resultant was extracted with ethyl acetate and dried over anhydrous magnesium sulfate. The solvent was distilled off under reduced pressure, and the obtained residue was purified by silica gel column chromatography (developing solvent: n-hexane/ethyl acetate), thereby obtaining Compound 35 (0.04 g, at a yield of 49%).

Step 3: Conversion to 5-(3-cyclopropyl-2-fluorophenoxy)pyridazine-4-carboxylic acid (English name: 5-(3-cyclopropyl-2-fluorophenoxy)pyridazine-4-carboxylic acid)

[Chemical Formula 74]

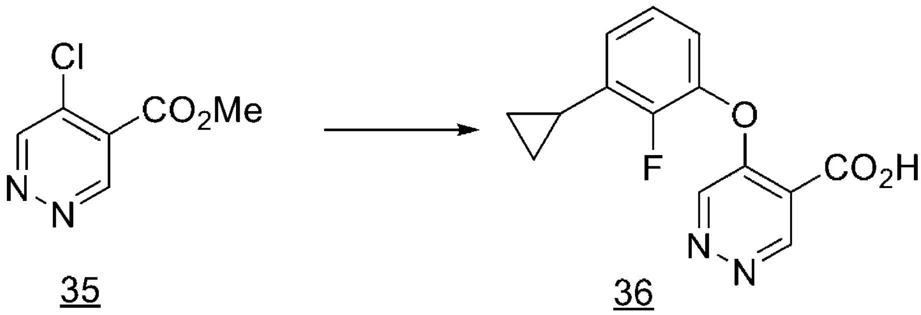

35 36

Compound 35 (0.04 g) was dissolved in N,N-dimethylformamide (1 mL). 3-Cyclopropyl-2-fluorophenol (0.05 g) and cesium carbonate (0.1 g) were added thereto at room temperature, and then stirred at room temperature for three hours. The obtained liquid was poured into a dilute hydrochloric acid solution. The resultant was extracted with ethyl acetate and dried over anhydrous magnesium sulfate. The solvent was distilled off under reduced pressure, thereby obtaining Compound 36 (0.02 g, at a yield of 26%).

Step 4: Conversion to methyl 2-(5-(3-cyclopropyl-2-fluorophenoxy)pyridazine-4-carbonyl)-1-(2,4-dimethylbenzyl)hydrazine-1-carboxylate (English name: methyl 2-(5-(3-cyclopropyl-2-fluorophenoxy)pyridazine-4-carbonyl)-1-(2,4-dimethylbenzyl)hydrazine-1-carboxylate)

[Chemical Formula 75]

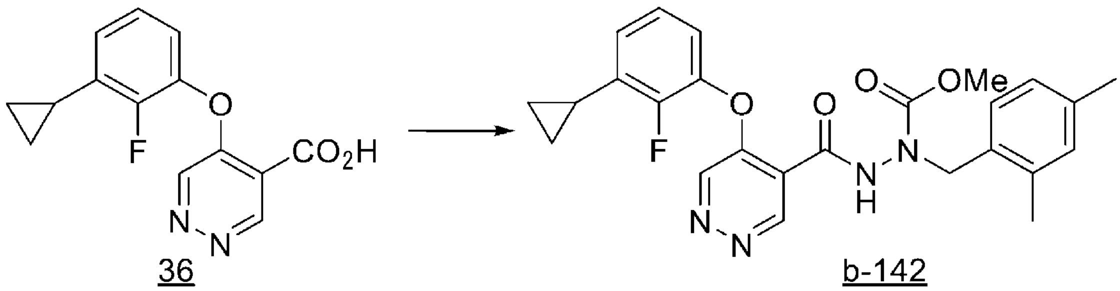

36 b-142

Compound 36 (0.02 g) was dissolved in N,N-dimethylformamide (1 mL). Compound 14 (0.032 g), 1-ethyl-3-(3-dimethylaminopropyl)carbodiimide hydrochloride (0.04 g), 1-hydroxybenzotriazole (0.01 g) and triethylamine (0.04 mL) were added thereto, and then stirred at room temperature for 18 hours. Methanol was added to the obtained liquid, and the mixture was purified by preparative high performance liquid chromatography (developing solvent: water/acetonitrile), thereby obtaining Compound b-142 (0.002 g, at a yield of 4%).

(Production Example 18)

S-methyl 2-(3-(3-cyclopropyl-2-fluorophenoxy)-6-methylpyridazine-4-carbonyl)-1-(2,4-dimethylbenzyl)hydrazine-1-carbothioate (English name: S-methyl 2-(3-(3-cyclopropyl-2-fluorophenoxy)-6-methylpyridazine-4-carbonyl)-1-(2,4-dimethylbenzyl)hydrazine-1-carbothioate (Compound b-166)) was produced by the following steps.

Step 1: Conversion to 2-(tert-butyl) 1-(4-nitrophenyl) 1-(2,4-dimethylbenzyl)hydrazine-1,2-dicarboxylate (English name: 2-(tert-butyl) 1-(4-nitrophenyl) 1-(2,4-dimethylbenzyl)hydrazine-1,2-dicarboxylate)

[Chemical Formula 76]

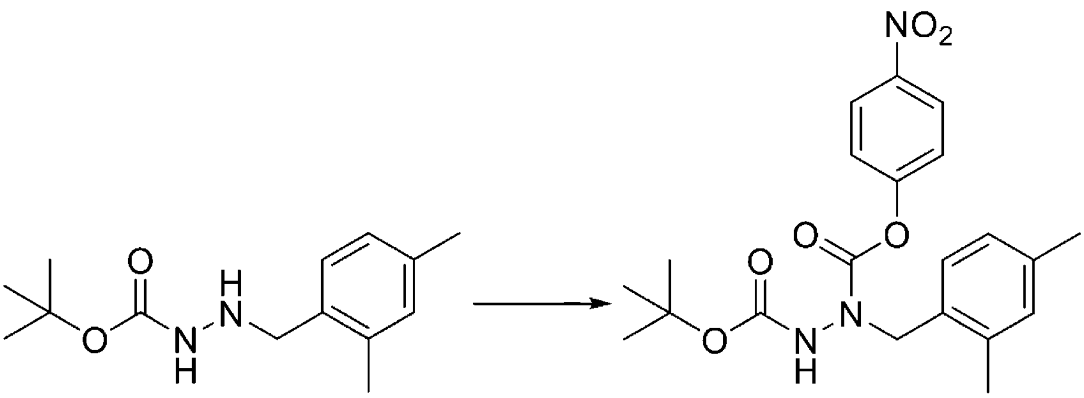

37 38

Compound 37 (0.1 g) was dissolved in tetrahydrofuran (3 mL). 4-Nitrophenyl chloroformate (0.2 g), and cesium carbonate (0.5 g) were added thereto at 0°C, and then stirred for three hours. The obtained liquid was poured into ice water. The resultant was extracted with ethyl acetate and dried over anhydrous magnesium sulfate. The solvent was distilled off under reduced pressure, and the obtained residue was purified by silica gel column chromatography (developing solvent: n-hexane/ethyl acetate), thereby obtaining Compound 38 (0.11 g, at a yield of 66%).

Step 2: Conversion to tert-butyl 2-(2,4-dimethylbenzyl)-2-((methylthio)carbonyl)hydrazine-1-carboxylate (English name: tert-butyl 2-(2,4-dimethylbenzyl)-2-((methylthio)carbonyl)hydrazine-1-carboxylate)

[Chemical Formula 77]

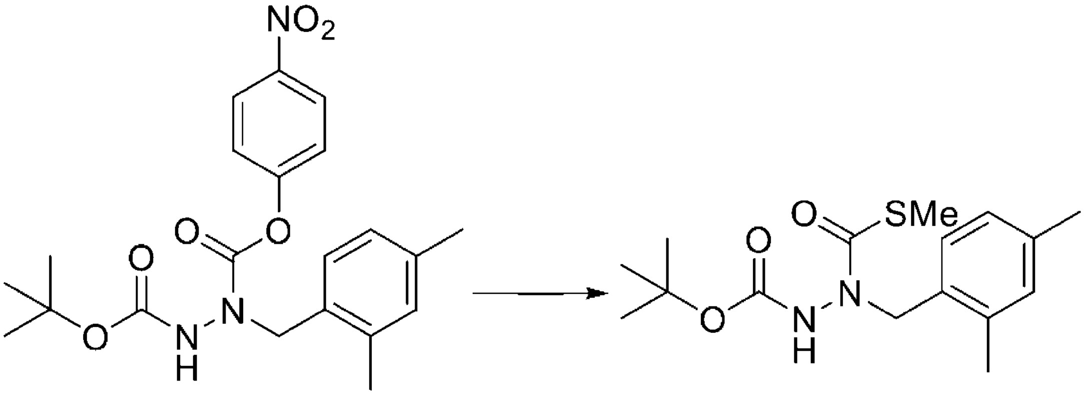

38 39

Compound 38 (0.03 g) was dissolved in tetrahydrofuran (1 mL). Sodium methylmercaptan (0.02 g) was added thereto at -78°C, and then stirred at -10°C for one hour. The obtained liquid was poured into ice water. The resultant was extracted with ethyl acetate and dried over anhydrous magnesium sulfate. The solvent was distilled off under reduced pressure, and the obtained residue was purified by silica gel column chromatography (developing solvent: n-hexane/ethyl acetate), thereby obtaining Compound 39 (0.01 g, at a yield of 41%).

Step 3: Conversion to S-methyl 2-(3-(3-cyclopropyl-2-fluorophenoxy)-6-methylpyridazine-4-carbonyl)-1-(2,4-dimethylbenzyl)hydrazine-1-carbothioate (English name: S-methyl 2-(3-(3-cyclopropyl-2-fluorophenoxy)-6-methylpyridazine-4-carbonyl)-1-(2,4-dimethylbenzyl)hydrazine-1-carbothioate)

[Chemical Formula 78]

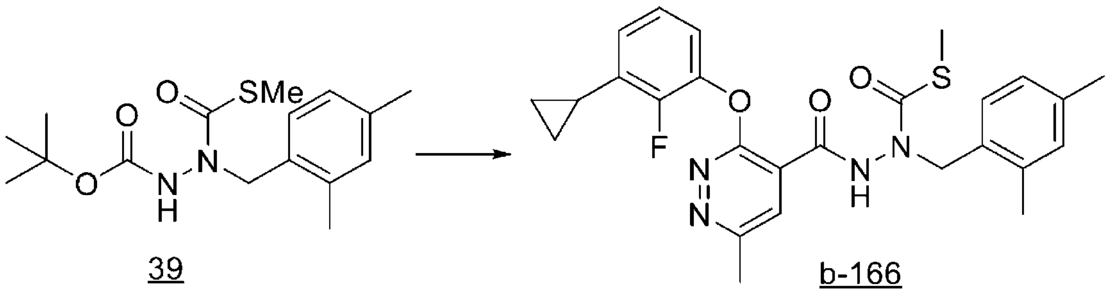

39 b-166

Compound 39 (0.01 g) was dissolved in dichloromethane (0.5 mL). Trifluoroacetic acid (0.1 mL) was added thereto at room temperature, and then stirred at room temperature for two hours. The solvent was distilled off under reduced pressure, the obtained residue was dissolved in dichloromethane (1 mL). Compound 24 (0.015 g), 1-ethyl-3-(3-dimethylaminopropyl)carbodiimide hydrochloride (0.02 g), 1-hydroxybenzotriazole (0.01g) and triethylamine (0.02 mL) were added thereto, and then stirred at room temperature for 18 hours. The obtained liquid was poured into ice water, extracted with ethyl acetate, and then dried over anhydrous magnesium sulfate. The solvent was distilled off under reduced pressure, and the obtained residue was purified by silica gel column chromatography (developing solvent: n-hexane/ethyl acetate), thereby obtaining Compound b-166 (0.002 g, at a yield of 8%).

(Production Example 19)

Methyl 1-(2,4-dimethylbenzyl)-2-(6-methyl-3-((5-(trifluoromethyl)thiophen-3-yl)oxy)pyridazine-4-carbonyl)hydrazine-1-carboxylate (English name: methyl 1-(2,4-dimethylbenzyl)-2-(6-methyl-3-((5-(trifluoromethyl)thiophen-3-yl)oxy)pyridazine-4-carbonyl)hydrazine-1-carboxylate) (Compound b-124) was produced by the following steps.

[Chemical Formula 79]

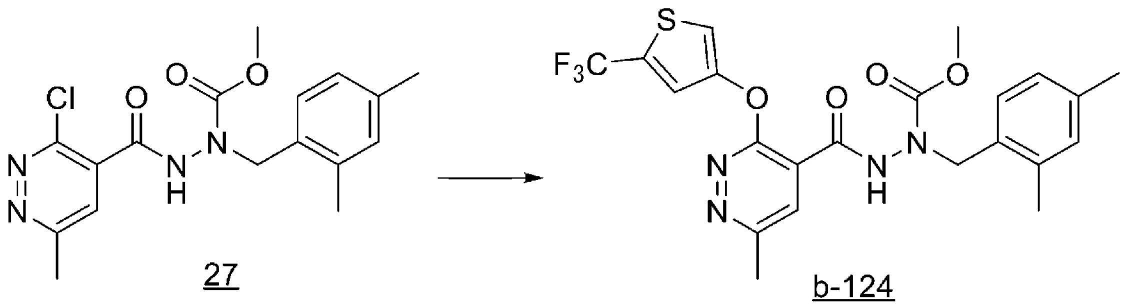

27 b-124

Compound 27 (0.150 g) was dissolved in N,N-dimethylformamide (1.40 ml). 4-Hydroxy-2-(trifluoromethyl) thiophene (0.1 g) was added thereto, and stirred at 120°C for 17 hours. Water was added to the obtained liquid, and the mixture was extracted with ethyl acetate, and then dried over anhydrous sodium sulfate. The solvent was distilled off under reduced pressure. The obtained residue was purified by silica gel column chromatography (developing solvent: n-hexane/ethyl acetate), thereby obtaining Compound b-124 (0.12 g, at a yield of 59%).

(Production Example 20)

Methyl 2-(3-(3-cyclopropyl-2-fluorophenoxy)-6-methylpyridazine-4-carbonyl)-1-((3,5-dimethylthiophen-2-yl) methyl)hydrazine-1-carboxylate (English name: methyl 2-(3-(3-cyclopropyl-2-fluorophenoxy)-6-methylpyridazine-4-carbonyl)-1-((3,5-dimethylthiophen-2-yl)methyl)hydrazine-1-carboxylate (Compound No. b-122)) was produced by the following steps.

Step 1: Conversion to methyl ((3,5-dimethylthiophen-2-yl)methyl)(1,3-dioxoisoindolin-2-yl)carbamate (English name: methyl ((3,5-dimethylthiophen-2-yl)methyl)(1,3-dioxoisoindolin-2-yl)carbamate)

[Chemical Formula 80]

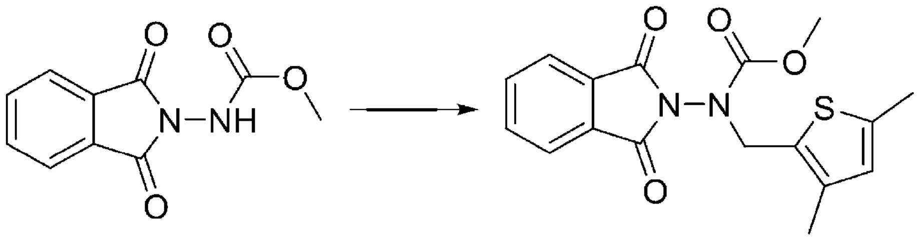

22 40

Compound 22 (0.25 g) was dissolved in tetrahydrofuran (7 mL). Bis(2-methoxyethyl) azodicarboxylate (0.84 g) and triphenylphosphine (1.0 g) were added thereto at room temperature, and then stirred at room temperature for 18 hours. The solvent was distilled off under reduced pressure, and the obtained residue was purified by preparative high performance liquid chromatography (developing solvent: water/acetonitrile), thereby obtaining Compound 40 (0.3 g, at a yield of 63%).

Step 2: Conversion to methyl 1-((3,5-dimethylthiophen-2-yl)methyl)hydrazine-1-carboxylate (English name: methyl 1-((3,5-dimethylthiophen-2-yl)methyl)hydrazine-1-carboxylate)

[Chemical Formula 81]

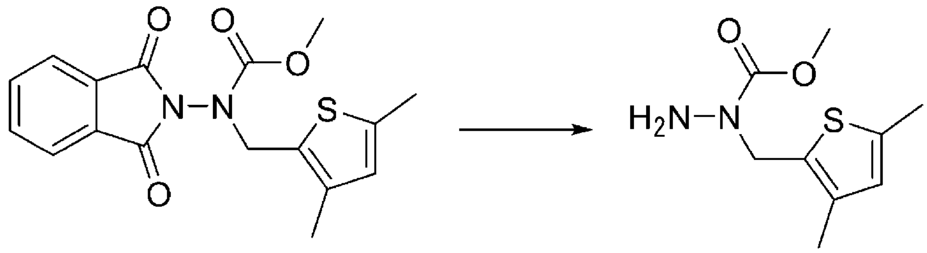

40 41

Compound 40 (0.3 g) was dissolved in ethanol (7 mL). Hydrazine monohydrate (0.5 mL) was added thereto at room temperature, and then stirred at room temperature for 18 hours. The obtained liquid was poured into ice water, extracted with ethyl acetate, and then dried over anhydrous magnesium sulfate. The solvent was distilled off under reduced pressure, thereby obtaining Compound 41 (0.19 g, at a yield of 72%).

Step 3: Conversion to methyl 2-(3-(3-cyclopropyl-2-fluorophenoxy)-6-methylpyridazine-4-carbonyl)-1-((3,5-dimethylthiophen-2-yl)methyl)hydrazine-1-carboxylate (English name: methyl 2-(3-(3-cyclopropyl-2-fluorophenoxy)-6-methylpyridazine-4-carbonyl)-1-((3,5-dimethylthiophen-2-yl)methyl)hydrazine-1-carboxylate)

[Chemical Formula 82]

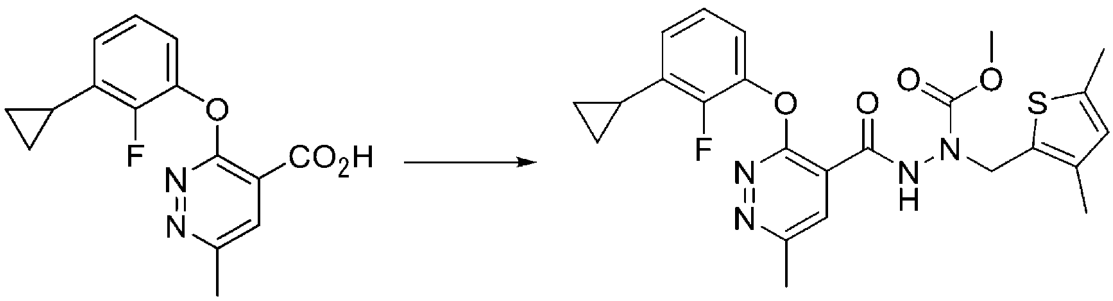

19 b-122

Compound 19 (0.042 g) was dissolved in N,N-dimethylformamide (1 mL). Compound 41 (0.04 g), 1-ethyl-3-(3-dimethylaminopropyl)carbodiimide hydrochloride (0.04 g), 1-hydroxybenzotriazole (0.002 g) and triethylamine (0.04 mL) were added thereto, and then stirred at room temperature for 18 hours. Methanol was added to the obtained liquid, and the mixture was purified by preparative high performance liquid chromatography (developing solvent: water/acetonitrile), thereby obtaining Compound b-122 (0.01 g, at a yield of 18%).

(Production Example 21)

Methyl 2-(2-(3-cyclopropyl-2-fluorophenoxy)imidazo[1,2-a]pyrimidine-3-carbonyl)-1-(2,4-dimethylbenzyl)hydrazine-1-carboxylate (English name: Methyl 2-(2-(3-cyclopropyl-2-fluorophenoxy)imidazo[1,2-a]pyrimidine-3-carbonyl)-1-(2,4-dimethylbenzyl)hydrazine-1-carboxylate (Compound b-226)) was produced by the following steps.

Step 1: Conversion to ethyl 2-(3-cyclopropyl-2-fluorophenoxy)imidazo[1,2-a]pyrimidine-3-carboxylate (English name: Ethyl 2-(3-cyclopropyl-2-fluorophenoxy)imidazo[1,2-a]pyrimidine-3-carboxylate

[Chemical Formula 83]

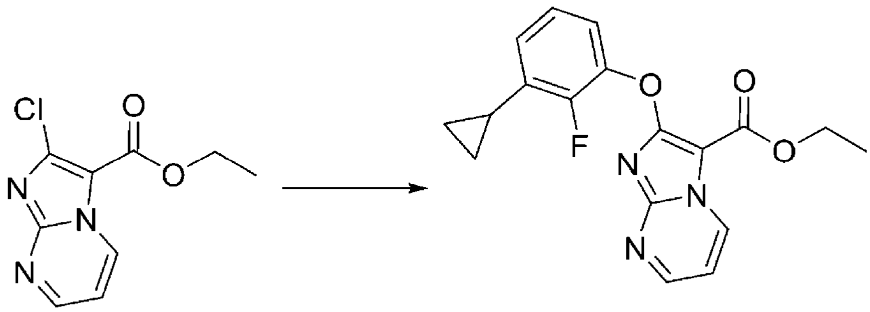

62

63

Compound 62 (0.135 g) was dissolved in N,N-dimethylacetamide (3 mL). 3-Cyclopropyl-2-fluorophenol (0.46 g) was added thereto, and then stirred at 140°C for seven hours. Water was added to the obtained liquid, and the mixture was extracted with ethyl acetate. The extracted phase was washed with a salt solution. Then, the resultant was dried over anhydrous magnesium sulfate, the solvent was distilled off under reduced pressure, and the resultant was purified by silica gel column chromatography (developing solvent: n-hexane/ethyl acetate), thereby obtaining Compound 63 (0.135 g, at a yield of 20%).

Step 2: Conversion to 2-(3-cyclopropyl-2-fluorophenoxy)imidazo[1,2-a]pyrimidine-3-carboxylic acid (English name: 2-(3-cyclopropyl-2-fluorophenoxy)imidazo[1,2-a]pyrimidine-3-carboxylic acid)

[Chemical Formula 84]

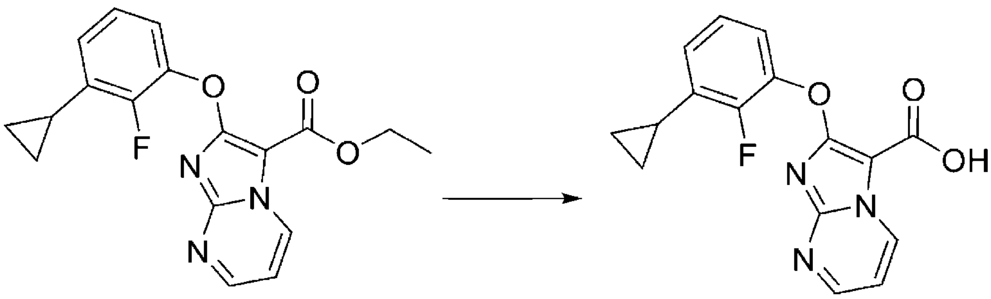

63

64

Compound 63 (0.135 g) was dissolved in a mixture solvent composed of tetrahydrofuran (3 mL), methanol (1 mL) and water (1 ml). Lithium hydroxide monohydrate (0.08 g) was added thereto, and then stirred at room temperature overnight. Dilute hydrochloric acid was added to the obtained liquid, and the mixture was extracted with ethyl acetate. The extracted phase was washed with a saturated salt solution. Then, the resultant was dried over anhydrous magnesium sulfate, and the solvent was distilled off under reduced pressure, thereby obtaining Compound 64 (0.101 g, at a yield of 80%).

Step 3: Conversion to methyl 2-(2-(3-cyclopropyl-2-fluorophenoxy)imidazo[1,2-a]pyrimidine-3-carbonyl)-1-(2,4-dimethylbenzyl)hydrazine-1-carboxylate (English name: methyl 2-(2-(3-cyclopropyl-2-fluorophenoxy)imidazo[1,2-a]pyrimidine-3-carbonyl)-1-(2,4-dimethylbenzyl)hydrazine-1-carboxylate

[Chemical Formula 85]

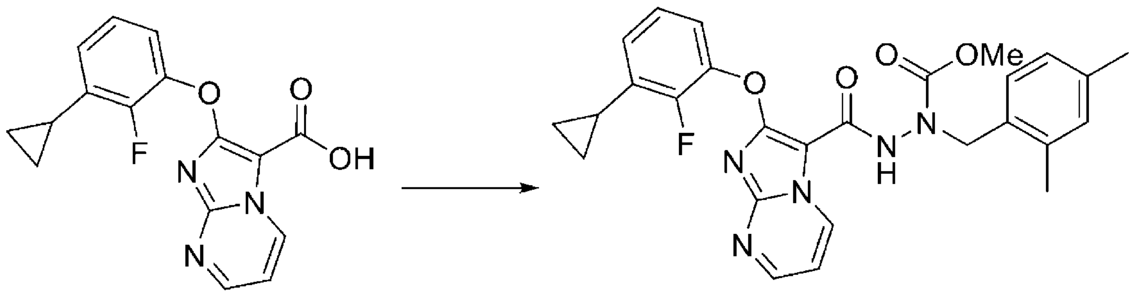

64 b-226

Compound 64 (0.03 g) was dissolved in N,N-dimethylformamide (0.5 mL). Compound 14 (0.03 g), 1-[bis(dimethylamino)methylene]-1H-1,2,3-triazolo[4,5-b]pyridinium3-oxide hexafluorophosphate (0.07g) and N,N-diisopropylethylamine (0.05 g) were added thereto, and then stirred at room temperature for two hours. The obtained liquid was purified by preparative HPLC (developing solvent: water/acetonitrile), thereby obtaining Compound b-226 (0.012g, at a yield of 25%).

(Production Example 22)

Methyl 2-(2-(3-cyclopropylphenoxy)imidazo[1,5-b]pyridazine-3-carbonyl)-1-(2,4-dimethylbenzyl)hydrazine-1-carboxylate (English name: methyl 2-(2-(3-cyclopropylphenoxy)imidazo[1,5-b]pyridazine-3-carbonyl)-1-(2,4-dimethylbenzyl)hydrazine-1-carboxylate (Compound b-210)) was produced by the following steps.

[Chemical Formula 86]

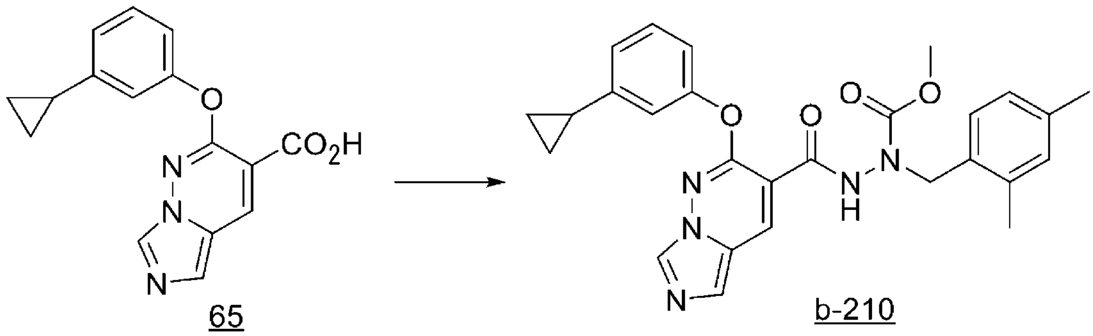

65 b-210

Compound 65 (0.003 g) was dissolved in tetrahydrofuran (0.5 mL). Compound 14 (0.002 g), propylphosphonic acid anhydride (50% ethyl acetate solution, 0.01 ml) and N,N-diisopropylethylamine (0.009 mL) were added thereto at room temperature, and then stirred for 18 hours.

The obtained liquid was poured into ice water. The resultant was extracted with ethyl acetate and dried over anhydrous magnesium sulfate. The solvent was distilled off under reduced pressure, the obtained residue was purified by preparative high performance liquid chromatography (developing solvent: water/acetonitrile), thereby obtaining Compound b-210 (0.002 g, at a yield of 40%).

(Production Example 23)

Methyl 2-(7-(3-cyclopropyl-2-fluorophenoxy)-2,2-dimethyl-3,4-dihydro-2H-pyrano [3,2-b]pyridine-8-carbonyl)-1-(2,4-dimethylbenzyl)hydrazine-1-carboxylate (English name: methyl 2-(7-(3-cyclopropyl-2-fluorophenoxy)-2,2-dimethyl-3,4-dihydro-2H-pyrano[3,2-b]pyridine-8-carbonyl)-1-(2,4-dimethylbenzyl)hydrazine-1-carboxylate (Compound b-209)) was produced by the following steps.

[Chemical Formula 87]

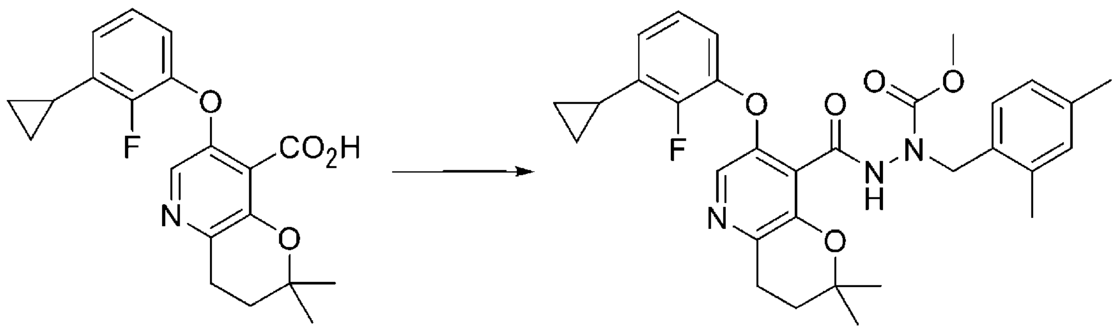

66

b-209

Compound 66 (0.025 g) was dissolved in N,N-dimethylformamide (0.5 mL). Compound 14 (0.022 g), 1-[bis(dimethylamino)methylene]-1H-1,2,3-triazolo[4,5-b]pyridinium 3-oxide hexafluorophosphate (0.04 g) and triethylamine (0.029 mL) were added thereto at room temperature, and then stirred for 18 hours.

The obtained liquid was poured into ice water. The resultant was extracted with ethyl acetate and dried over anhydrous magnesium sulfate. The solvent was distilled off under reduced pressure, and the obtained residue was purified by preparative high performance liquid chromatography (developing solvent: water/acetonitrile), thereby obtaining Compound b-209 (0.012 g, at a yield of 31%).

(Production Example 24)

Methyl 2-(7-(3-cyclopropyl-2-fluorophenoxy)-2,2-dimethyl-2H-pyrano[3,2-b]pyridine-8-carbonyl)-1-(2,4-dimethylbenzyl)hydrazine-1-carboxylate (English name: methyl 2-(7-(3-cyclopropyl-2-fluorophenoxy)-2,2-dimethyl-2H-pyrano[3,2-b]pyridine-8-carbonyl)-1-(2,4-dimethylbenzyl)hydrazine-1-carboxylate (Compound b-213)) was produced by the following steps.

[Chemical Formula 88]

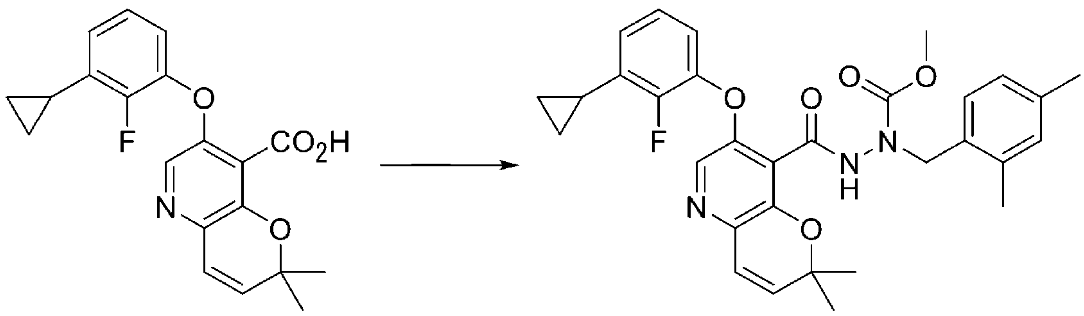

67

b-213

Compound 67 (0.025 g) was dissolved in tetrahydrofuran (0.5 mL). Compound 14 (0.022 g), 1-[bis(dimethylamino)methylene]-1H-1,2,3-triazolo[4,5-b]pyridinium 3-oxide hexafluorophosphate (0.04 g) and triethylamine (0.029 mL) were added thereto at room temperature, and then stirred for 18 hours.

The obtained liquid was poured into ice water. The resultant was extracted with ethyl acetate and dried over anhydrous magnesium sulfate. The solvent was distilled off under reduced pressure, and the obtained residue was purified by preparative high performance liquid chromatography (developing solvent: water/acetonitrile), thereby obtaining Compound b-213 (0.025 g, at a yield of 65%).

(Production Example 25)

Cyclopropyl 2-(3-(3-cyclopropyl-2-fluorophenoxy)-6-methylpyridazine-4-carbonyl)-1-(2,4-dimethylbenzyl)hydrazine-1-carboxylate (English name: cyclopropyl 2-(3-(3-cyclopropyl-2-fluorophenoxy)-6-methylpyridazine-4-carbonyl)-1-(2,4-dimethylbenzyl)hydrazine-1-carboxylate (Compound b-184)) was produced by the following steps.

Step 1: Conversion to cyclopropylhydrazinecarboxylate (English name: cyclopropylhydrazinecarboxylate)

[Chemical Formula 89]

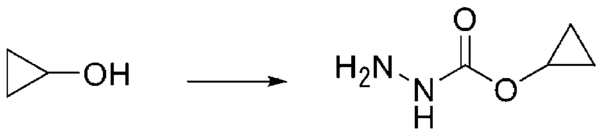

68 69

Compound 68 (1.0 g) was dissolved in dichloromethane (34 mL). Pyridine (2.1 mL) and phenyl chloroformate (2.1 mL) were added thereto at 0°C, and then stirred at room temperature for 18 hours. The obtained liquid was poured into an aqueous solution of 1N hydrochloric acid at 0°C.

The resultant was extracted with dichloromethane, and dried over anhydrous magnesium sulfate. The solvent was distilled off under reduced pressure, and the obtained residue was dissolved in ethanol (34 mL). Hydrazine monohydrate was added thereto at room temperature, and then stirred at 80°C for one hour. The obtained liquid was poured into an aqueous solution of 1N sodium hydroxide at 0°C. The resultant was extracted with dichloromethane, and dried over anhydrous magnesium sulfate. The solvent was distilled off under reduced pressure, and the obtained residue was purified by silica gel column chromatography (developing solvent: n-hexane/ethyl acetate), thereby obtaining Compound 69 (0.85 g, at a yield of 43%).

Step 2: Conversion to cyclopropyl(1,3-dioxoindolin-2-yl)carbamate (English name: cyclopropyl (1,3-dioxoisoindolin-2-yl)carbamate)

[Chemical Formula 90]

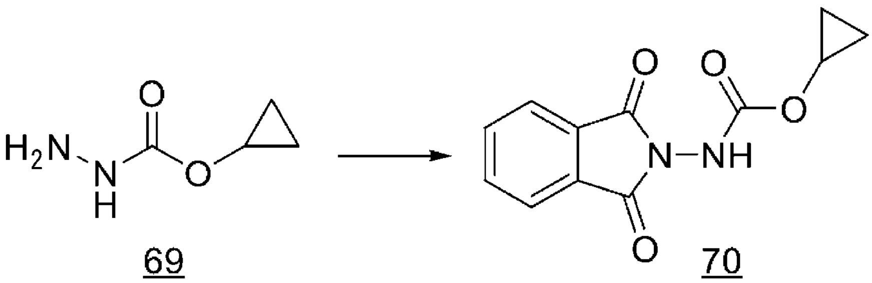

69 70

Phthalic anhydride (1.1 g) was dissolved in tetrahydrofuran (24 mL). Compound 69 (0.85 g) was added thereto at room temperature, and then stirred at room temperature for one hour. Then, N,N'-dicyclohexylcarbodiimide (1.8 g) was added thereto at room temperature and then stirred at room temperature for 18 hours. The formed solid was filtered, and acetic acid (0.84 mL) and triethylamine (2.0 mL) were added to the obtained liquid, followed by stirring the mixture under heating to allow reflux to proceed for one hour.

The obtained liquid was poured into ice water. The resultant was extracted with ethyl acetate and dried over anhydrous magnesium sulfate. The solvent was distilled off under reduced pressure, thereby obtaining Compound 70 (1.0 g, at a yield of 60%).

Step 3: Conversion to cyclopropyl(2,4-dimethylbenzyl)(1,3-dioxoindolin-2-yl)carbamate (English name: cyclopropyl(2,4-dimethylbenzyl)(1,3-dioxoisoindolin-2-yl)carbamate)

[Chemical Formula 91]

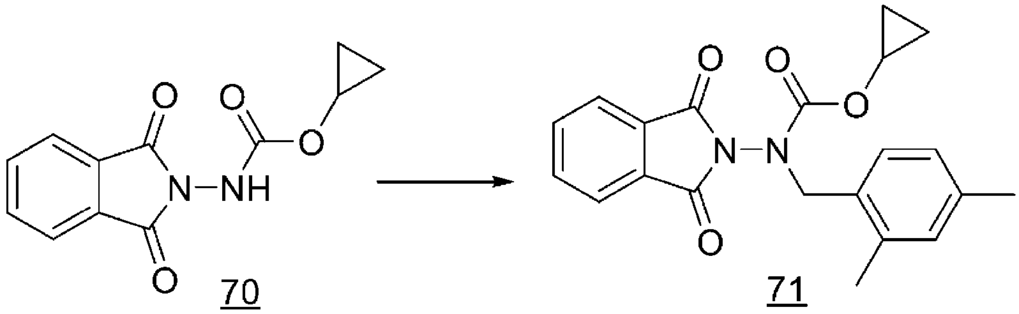

70 71

Compound 70 (1.0 g) was dissolved in N,N-dimethylformamide (3 mL). 1-(Bromomethyl)-2,4-dimethylbenzene (1.0 g) and cesium carbonate (2.0 g) were added thereto at room temperature, and then stirred at room temperature for 18 hours.

The obtained liquid was poured into ice water. The resultant was extracted with ethyl acetate and dried over anhydrous magnesium sulfate. The solvent was distilled off under reduced pressure, and the obtained residue was purified by silica gel column chromatography (developing solvent: n-hexane/ethyl acetate), thereby obtaining Compound 71 (1.3 g, at a yield of 86%).

Step 4: Conversion to cyclopropyl 1-(2,4-dimethylbenzyl)hydrazine-1-carboxylate (English name: cyclopropyl 1-(2,4-dimethylbenzyl)hydrazine-1-carboxylate)

[Chemical Formula 92]

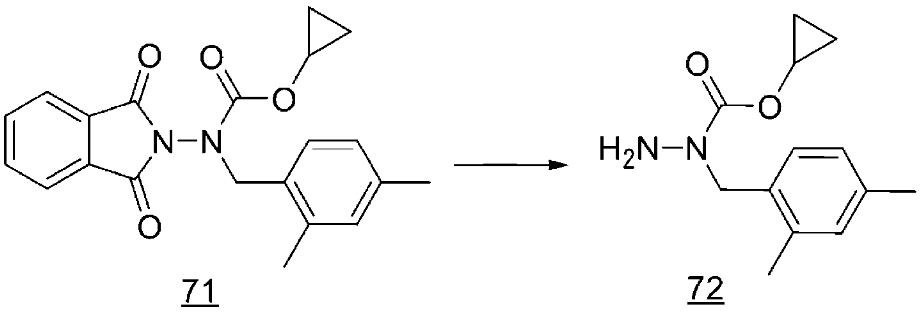

71 72

Compound 71 (1.3 g) was dissolved in ethanol (5 mL). Hydrazine monohydrate (1.3 mL) was added thereto at room temperature, and then stirred at 50°C for one hour. Solids were filtered out and the obtained solution was poured into ice water, extracted with ethyl acetate, and then dried over anhydrous magnesium sulfate. The solvent was distilled off under reduced pressure, thereby obtaining Compound 72 (0.9 g, at a yield of 98%).

Step 5: Conversion to cyclopropyl 2-(3-(3-cyclopropyl-2-fluorophenoxy)-6-methylpyridazine-4-carbonyl)-1-(2,4-dimethylbenzyl)hydrazine-1-carboxylate (English name: cyclopropyl 2-(3-(3-cyclopropyl-2-fluorophenoxy)-6-methylpyridazine-4-carbonyl)-1-(2,4-dimethylbenzyl)hydrazine-1-carboxylate) (Compound b-184)

[Chemical Formula 93]

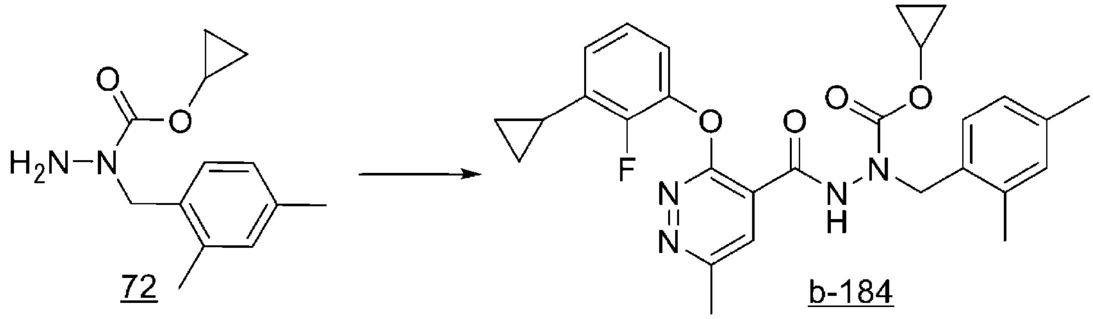

72 b-184

Compound 72 (0.18 g) was dissolved in N,N-dimethylformamide (1 mL). 3-(3-Cyclopropyl-2-fluorophenoxy)-6-methylpyridazine-4-carboxylic acid (0.15 g), 1-ethyl-3-(3-dimethylaminopropyl)carbodiimide hydrochloride (0.2 g), 1-hydroxybenzotriazole (0.01 g) and triethylamine (0.29 mL) were added thereto at 0°C, and then stirred at room temperature for 18 hours.

Methanol was added to the obtained liquid, and then the mixture was purified by preparative high performance liquid chromatography (developing solvent: water/acetonitrile), thereby obtaining Compound b-184 (0.1 g, at a yield of 40%).

(Production Example 26)

Methyl (Z)-2-((3-(3-chloro-2-fluorophenoxy)-6-methylpyridazin-4-yl)(methylthio)methylene)-1-(2,4-dimethylbenzyl)hydrazine-1-carboxylate (English name: methyl (Z)-2-((3-(3-chloro-2-fluorophenoxy)-6-methylpyridazin-4-yl)(methylthio) methylene)-1-(2,4-dimethylbenzyl)hydrazine-1-carboxylate (Compound No. b-219)) was produced by the following steps.

Step 1: Conversion to methyl 2-(3-(3-chloro-2-fluorophenoxy)-6-methylpyridazine-4-carbonothioyl)-1-(2,4-dimethylbenzyl)hydrazine-1-carboxylate (English name: methyl 2-(3-(3-chloro-2-fluorophenoxy)-6-methylpyridazine-4-carbonothioyl)-1-(2,4-dimethylbenzyl)hydrazine-1-carboxylate)

[Chemical Formula 94]

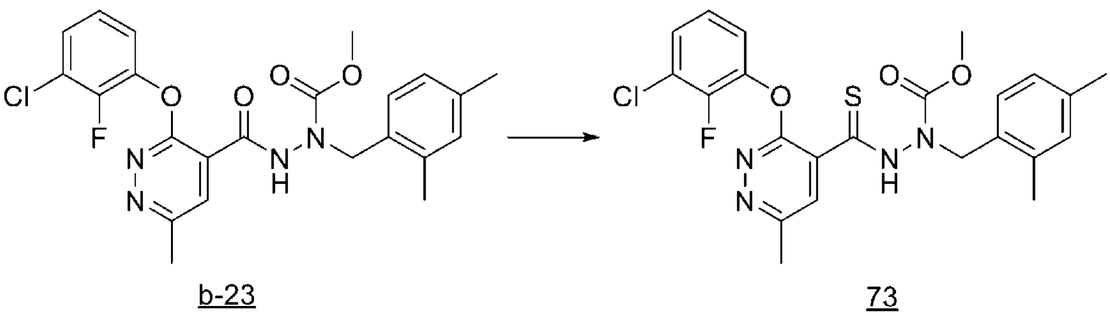

b-23 73

Methyl 2-(3-(3-chloro-2-fluorophenoxy)-6-methylpyridazine-4-carbonyl)-1-(2,4-dimethylbenzyl)hydrazine-1-carboxylate (0.50 g) was dissolved in 1.4-dioxane (3.5 mL). Lawesson's reagent (0.43 g) was added thereto, and then stirred at 80°C for two hours.

The solvent was distilled off under reduced pressure, and the obtained residue was purified by silica gel column chromatography (developing solvent: n-hexane/ethyl acetate), thereby obtaining 0.045 g of methyl 2-(3-(3-chloro-2-fluorophenoxy)-6-methylpyridazine-4-carbonothioyl)-1-(2,4-dimethylbenzyl)hydrazine-1-carboxylate (at a yield of 9%).

Step 2: Conversion to methyl (Z)-2-((3-(3-chloro-2-fluorophenoxy)-6-methylpyridazin-4-yl)(methylthio)methylene)-1-(2,4-dimethylbenzyl)hydrazine-1-carboxylate (English name: methyl (Z)-2-((3-(3-chloro-2-fluorophenoxy)-6-methylpyridazin-4-yl)(methylthio) methylene)-1-(2,4-dimethylbenzyl)hydrazine-1-carboxylate (Compound No. b-219))

[Chemical Formula 95]

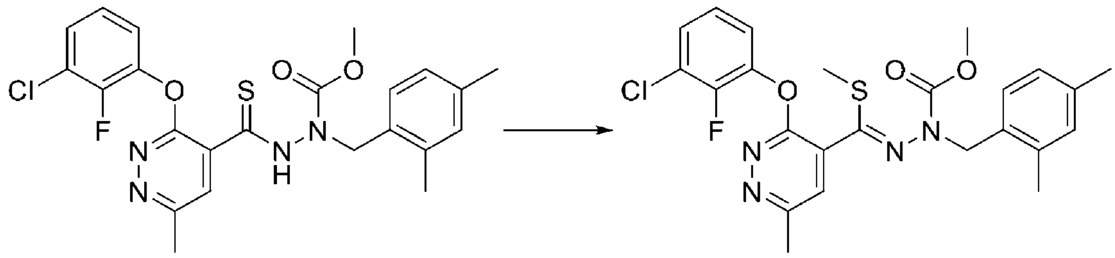

73

b-219

Methyl 2-(3-(3-chloro-2-fluorophenoxy)-6-methylpyridazine-4-carbonothioyl)-1-(2,4-dimethylbenzyl)hydrazine-1-carboxylate (0.05 g) was dissolved in acetonitrile (0.34 mL). Iodomethane (0.044 g) and potassium carbonate (0.028 g) were added thereto, and then stirred at room temperature for two hours.

Water was added to the reaction solution, and the mixture was extracted with ethyl acetate and dried over anhydrous magnesium sulfate, thereby obtaining 0.054 g of methyl (Z)-2-((3-(3-chloro-2-fluorophenoxy)-6-methylpyridazin-4-yl)(methylthio)methylene)-1-(2,4-dimethylbenzyl)hydrazine-1-carboxylate (Compound No. b-219) (at a yield of 99%).

(Production Example 27)

Methyl (Z)-2-((3-(3-chloro-2-fluorophenoxy)-6-methylpyridazin-4-yl)(methoxy) methylene)-1-(2,4-dimethylbenzyl)hydrazine-1-carboxylate (English name: methyl (Z)-2-((3-(3-chloro-2-fluorophenoxy)-6-methylpyridazin-4-yl)(methoxy)methylene)-1-(2,4-dimethylbenzyl)hydrazine-1-carboxylate (Compound No. b-220) was produced by the following steps.

Step 1: Conversion to methyl (Z)-2-((3-(3-chloro-2-fluorophenoxy)-6-methylpyridazin-4-yl)(methoxy)methylene)-1-(2,4-dimethylbenzyl)hydrazine-1-carboxylate (English name: methyl (Z)-2-((3-(3-chloro-2-fluorophenoxy)-6-methylpyridazin-4-yl)(methoxy) methylene)-1-(2,4-dimethylbenzyl)hydrazine-1-carboxylate (Compound No. b-220))

[Chemical Formula 96]

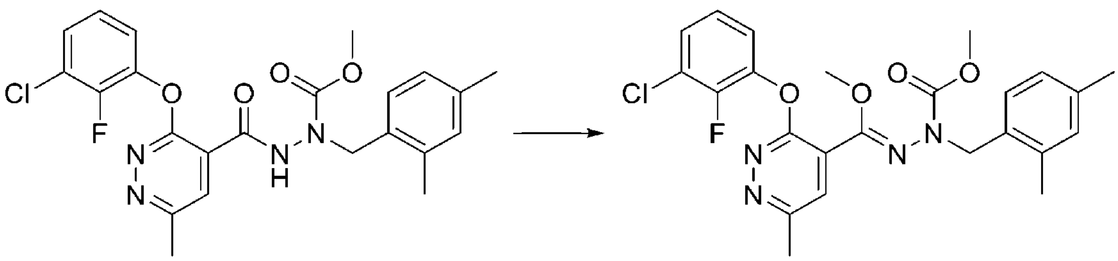

b-23

b-220

Methyl 2-(3-(3-chloro-2-fluorophenoxy)-6-methylpyridazine-4-carbonyl)-1-(2,4-dimethylbenzyl)hydrazine-1-carboxylate (0.10 g) was dissolved in dichloromethane (0.70 mL). Trimethyloxonium tetrafluoroborate (0.063 g) was added thereto at 0°C, and stirred at room temperature for three hours.

Ice water was added to the reaction solution, and the mixture was extracted with ethyl acetate and dried over anhydrous magnesium sulfate, thereby obtaining 0.1 g of methyl (Z)-2-((3-(3-chloro-2-fluorophenoxy)-6-methylpyridazin-4-yl)(methoxy) methylene-1-(2,4-dimethylbenzyl)hydrazine-1-carboxylate (Compound No. b-220) (at a yield of 100%).

Compounds shown in Table 2 were produced through steps similar to those in the above-mentioned Production Examples. The melting point or external appearance (AMORPHOUS or VISCOUS OIL) is recorded in the property column. The LCMS retention times of compounds, the external appearance of which was AMORPHOUS or VISCOUS OIL, are recorded.

[Table 21]

Table 2

| Compound No. | Chemical formula | Property | LCMS (SQD2) Retention time |
|---|---|---|---|
| b-101 | 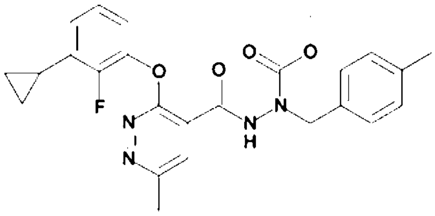 | m.p.:54-56°C | |
| b-102 | 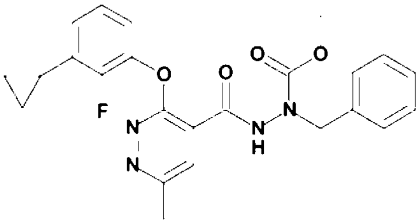 | m.p.:55-57°C | |
| b-103 | 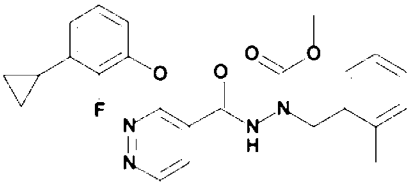 | m. p.:54-56°C | |
| b-104 | 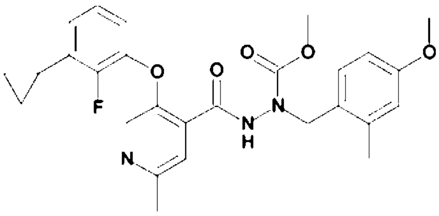 | VISCOUS OIL | 1. 65min |

[Table 22]

Table 2 (continued)

| Compound No. | Chemical formula | Property | LCMS (SQD2) Retention time |
|---|---|---|---|
| b-105 | 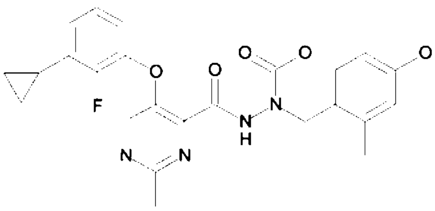 | VISCOUS OIL | 1.63min |

(continued)

| Table 2 (continued) | | | |
|---|---|---|---|
| Compound No. | Chemical formula | Property | LCMS (SQD2) Retention time |
| b-106 | 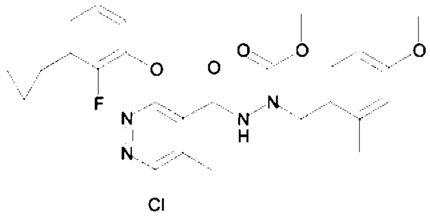 | VISCOUS OIL | 1.70min |
| b-107 | 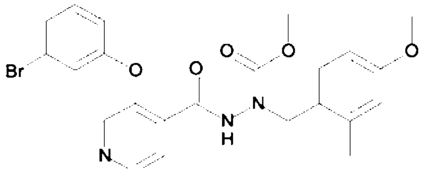 | VISCOUS OIL | 1.60min |
| b-108 | 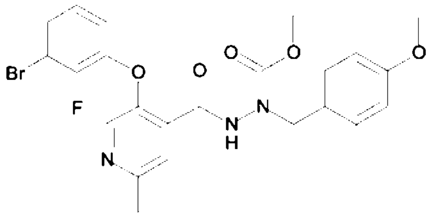 | VISCOUS OIL | 1.59min |

[Table 23]

| Table 2 (continued) | | | |
|---|---|---|---|
| Compound No. | Chemical formula | Property | LCMS (SQD2) Retention time |
| b-109 | 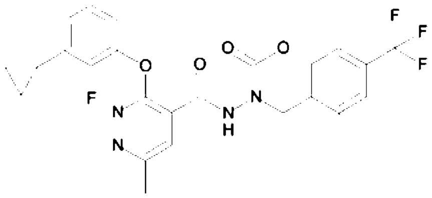 | VISCOUS OIL | 1. 75min |
| b-110 | 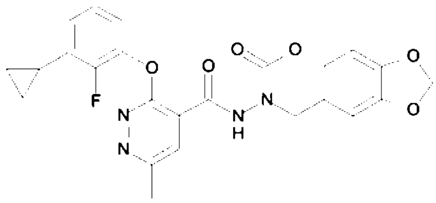 | VISCOUS OIL | 1.49min |
| b-111 | 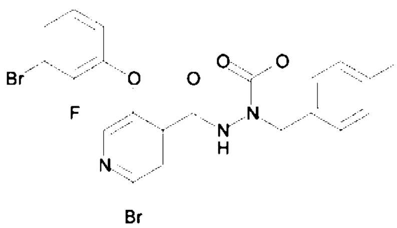 | AMORPHOUS | 1.87min |

(continued)

| Table 2 (continued) | | | |
|---|---|---|---|
| Compound No. | Chemical formula | Property | LCMS (SQD2) Retention time |
| b-112 | 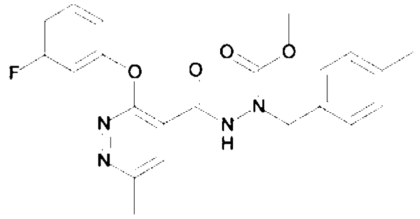 | m.p.:117-119°C | |
| b-113 | 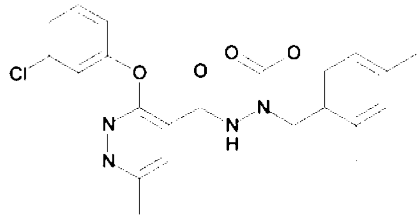 | VISCOUS OIL | 1.63min |

[Table 24]

| Table 2 (continued) | | | |
|---|---|---|---|
| Compound No. | Chemical formula | Property | LCMS (SQD2) Retention time |
| b-114 | 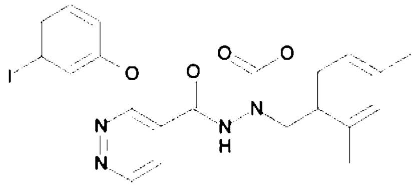 | VISCOUS OIL | 1. 70min |
| b-115 | 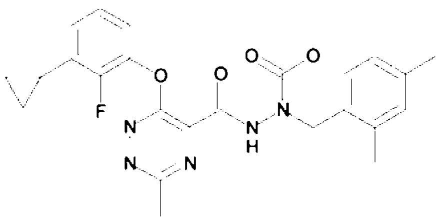 | m.p.:70-72°C | |
| b-116 | 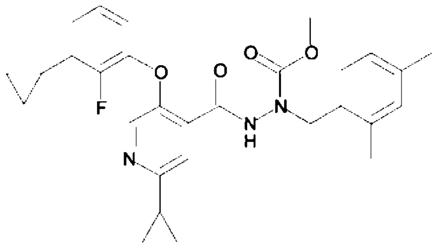 | VISCOUS OIL | 1. 95min |
| b-117 | 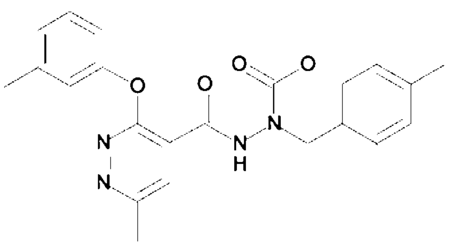 | AMORPHOUS | 1.6min |

[Table 25]

| Table 2 (continued) | | | |
|---|---|---|---|
| Compound No. | Chemical formula | Property | LCMS (SQD2) Retention time |
| b-118 | 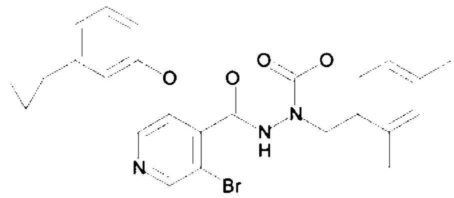 | VISCOUS OIL | 1. 79min |
| b-119 | 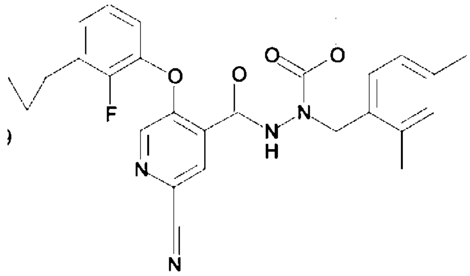 | AMORPHOUS | 1.83min |
| b-120 | 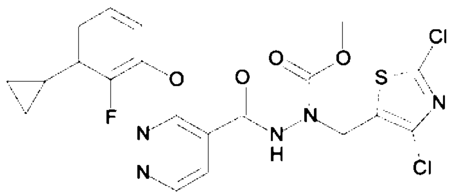 | VISCOUS OIL | 1. 63min |
| b-121 | 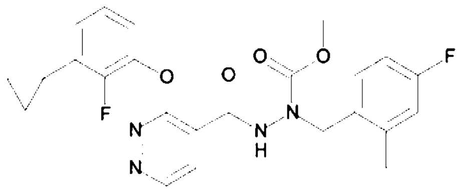 | VISCOUS OIL | 1. 63min |

[Table 26]

| Table 2 (continued) | | | |
|---|---|---|---|
| Compound No. | Chemical formula | Property | LCMS (SQD2) Retention time |
| b-122 | 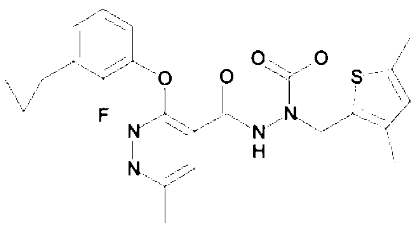 | VISCOUS OIL | 1.69min |
| b-123 | 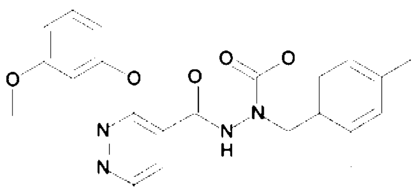 | VISCOUS OIL | 1.51min |

(continued)

| Table 2 (continued) | | | |
|---|---|---|---|
| Compound No. | Chemical formula | Property | LCMS (SQD2) Retention time |
| b-124 | 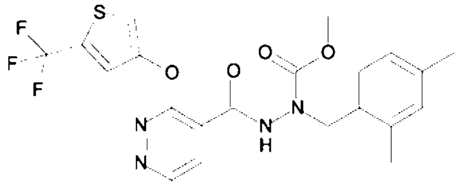 | VISCOUS OIL | 1.72min |
| b-125 | 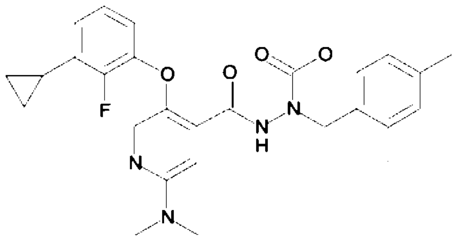 | m.p.:151-153°C | |

[Table 27]

| Table 2 (continued) | | | |
|---|---|---|---|
| Compound No. | Chemical formula | Property | LCMS (SQD2) Retention time |
| b-126 | 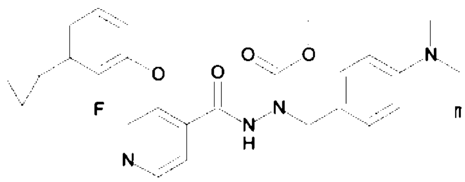 | m.p. :160-162°C | |
| b-127 | 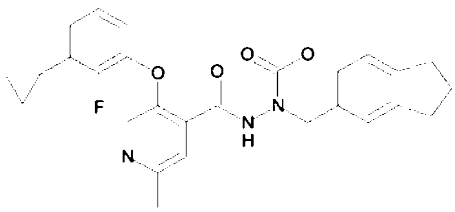 | m.p. :53-55°C | |
| b-128 | 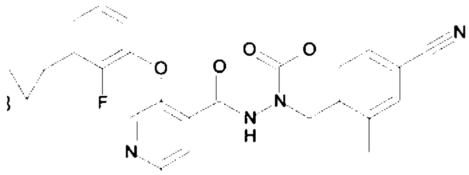 | VISCOUS OIL | 1. 59min |
| b-129 | 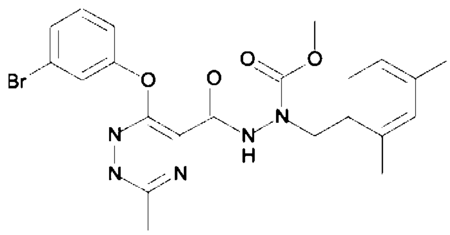 | m.p. :160-162°C | |

[Table 28]

| Table 2 (continued) | | | |
|---|---|---|---|
| Compound No. | Chemical formula | Property | LCMS (SQD2) Retention time |
| b-130 | 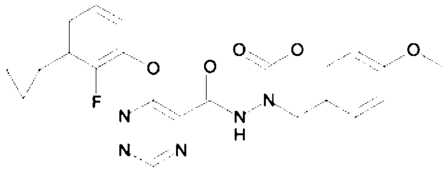 | m.p.:68-70°C | |
| b-131 | 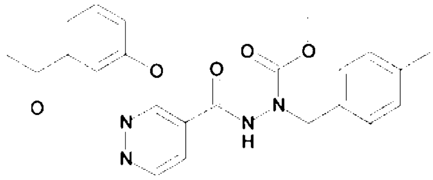 | m.p.:85-87°C | |
| b-132 | 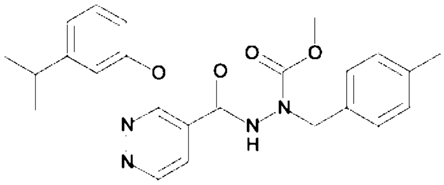 | VISCOUS OIL | 1. 59min |
| b-133 | 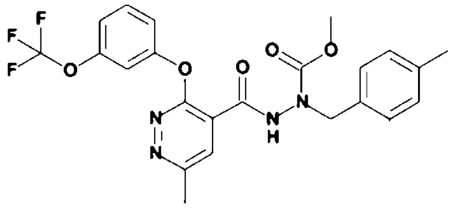 | m.p.:55-57°C | |

[Table 29]

| Table 2 (continued) | | | |
|---|---|---|---|
| Compound No. | Chemical formula | Property | LCMS (SQD2) Retention time |
| b-134 | 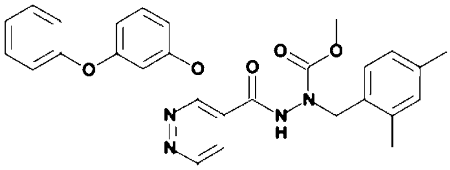 | m.p. :60-62°C | |
| b-135 | 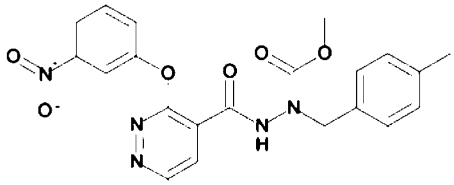 | m.p. :58-80°C | |
| b-136 | 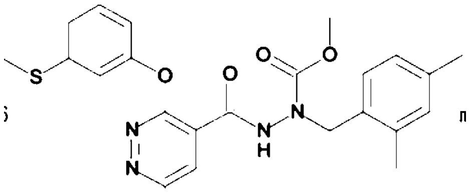 | m.p. :58-60°C | |

(continued)

| Table 2 (continued) | | | |
|---|---|---|---|
| Compound No. | Chemical formula | Property | LCMS (SQD2) Retention time |
| b-137 | 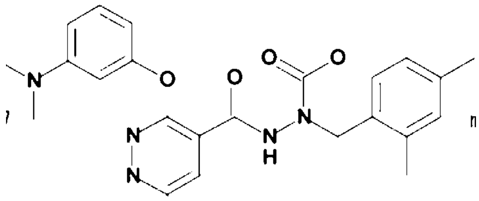 | m.p. :72-74°C | |

[Table 30]

| Table 2 (continued) | | | |
|---|---|---|---|
| Compound No. | Chemical formula | Property | LCMS (SQD2) Retention time |
| b-138 | 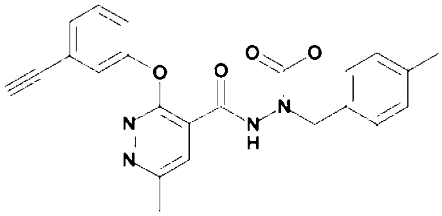 | m.p.:71-73°C | |
| b-139 | 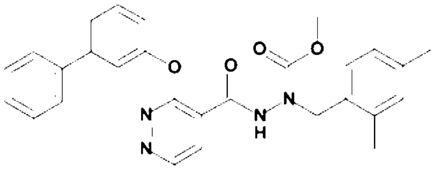 | m.p.:68-70°C | |
| b-140 | 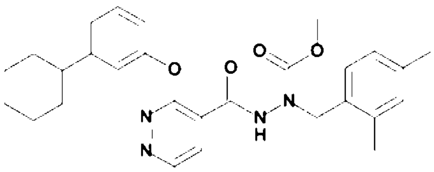 | m.p.:70-72°C | |
| b-141 | 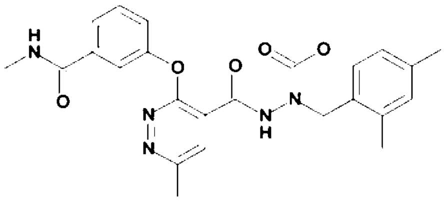 | m.p.:99-101°C | |

[Table 31]

| Table 2 (continued) | | | |
|---|---|---|---|
| Compound No. | Chemical formula | Property | LCMS (SQD2) Retention time |
| b-142 | 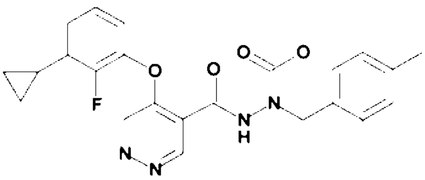 | VISCOUS OIL | 1. 61min |

(continued)

| Table 2 (continued) | | | |
|---|---|---|---|
| Compound No. | Chemical formula | Property | LCMS (SQD2) Retention time |
| b-143 | 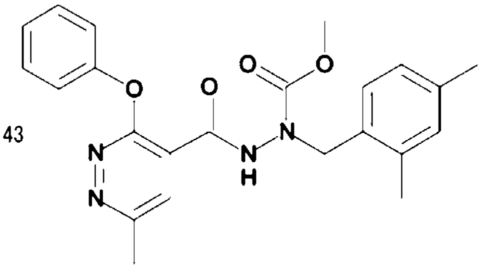 | VISCOUS OIL | 1. 56min |
| b-144 | 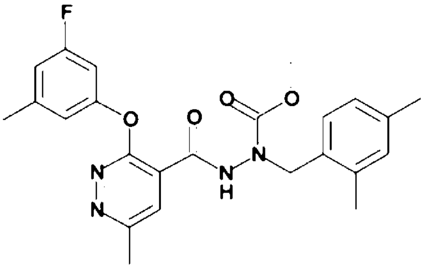 | VISCOUS OIL | 1. 65min |
| b-145 | 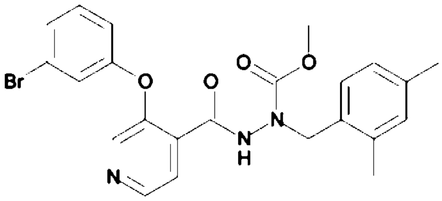 | VISCOUS OIL | 1. 71min |

[Table 32]

| Table 2 (continued) | | | |
|---|---|---|---|
| Compound No. | Chemical formula | Property | LCMS (SQD2) Retention time |
| b-146 | 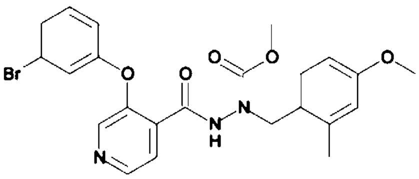 | VISCOUS OIL | 1.57min |
| b-147 | 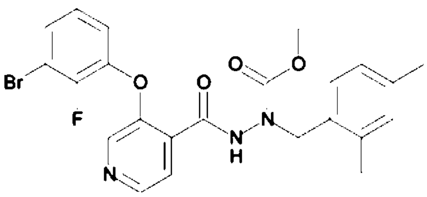 | VISCOUS OIL | 1.69min |
| b-148 | 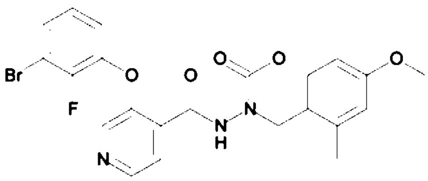 | VISCOUS OIL | 1.56min |

(continued)

| Table 2 (continued) | | | |
|---|---|---|---|
| Compound No. | Chemical formula | Property | LCMS (SQD2) Retention time |
| b-149 | 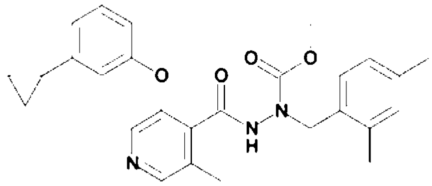 | VISCOUS OIL | 1.70min |

[Table 33]

| Table 2 (continued) | | | |
|---|---|---|---|
| Compound No. | Chemical formula | Property | LCMS (SQD2) Retention time |
| b-150 | 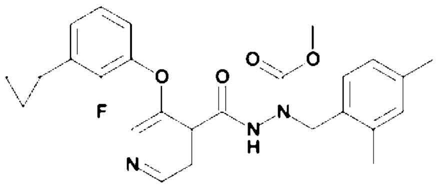 | VISCOUS OIL | 1. 74min |
| b-151 | 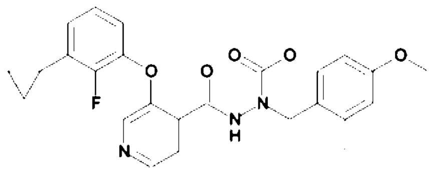 | VISCOUS OIL | 1.61min |
| b-152 | 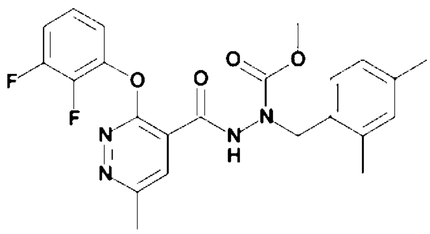 | m.p.:70-72°C | |
| b-153 | 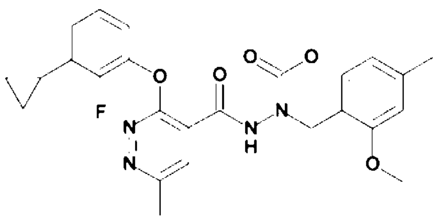 | m. p. :68-73°C | |

[Table 34]

| Table 2 (continued) | | | |
|---|---|---|---|
| Compound No. | Chemical formula | Property | LCMS (SQD2) Retention time |
| b-154 | 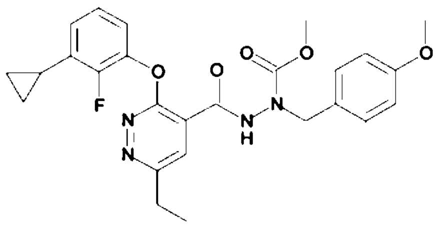 | m.p.:75-77°C | |

(continued)

| Table 2 (continued) | | | |
|---|---|---|---|
| Compound No. | Chemical formula | Property | LCMS (SQD2) Retention time |
| b-155 | 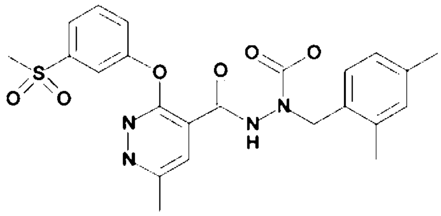 | m.p.:95-97°C | |
| b-156 | 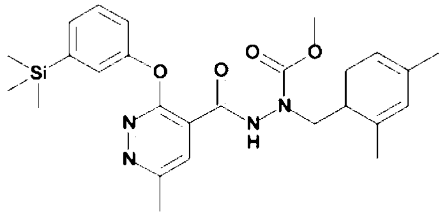 | m.p.:55-57°C | |
| b-157 | 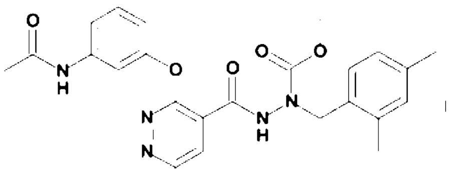 | m.p.:110-112°C | |

[Table 35]

| Table 2 (continued) | | | |
|---|---|---|---|
| Compound No. | Chemical formula | Property | LCMS (SQD2) Retention time |
| b-158 | 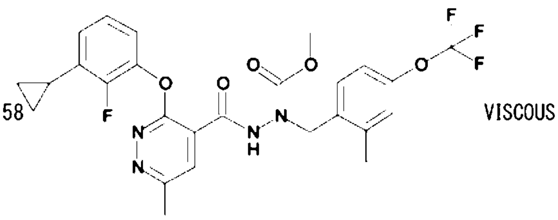 | VISCOUS OIL | 1.82min |
| b-159 | 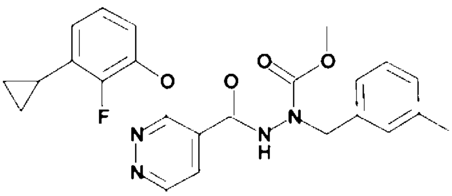 | VISCOUS OIL | 1.66min |
| b-160 | 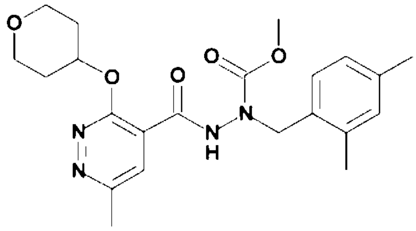 | VISCOUS OIL | 1. 36min |

(continued)

| Table 2 (continued) | | | |
|---|---|---|---|
| Compound No. | Chemical formula | Property | LCMS (SQD2) Retention time |
| | b-161 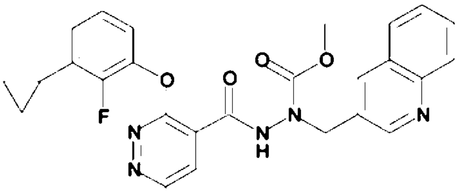 | m.p.:72-78°C | |

[Table 36]

| Table 2 (continued) | | | |
|---|---|---|---|
| Compound No. | Chemical formula | Property | LCMS (SQD2) Retention time |
| b-162 | 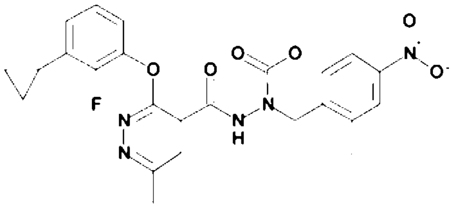 | m.p.:77-82°C | |
| b-163 | 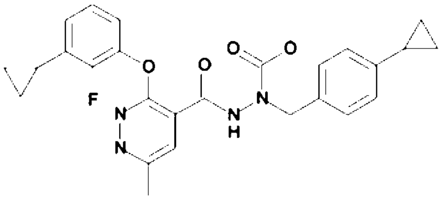 | m.p.:65-71°C | |
| b-164 | 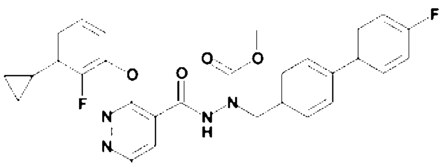 | m.p.:69-74°C | |
| b-165 | 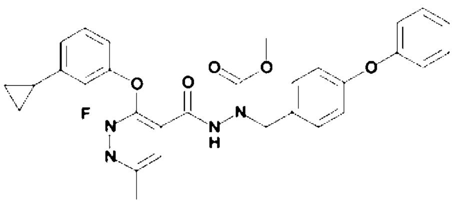 | m.p.:62-67°C | |

[Table 37]

| Table 2 (continued) | | | |
|---|---|---|---|
| Compound No. | Chemical formula | Property | LCMS (SQD2) Retention time |
| b-166 | 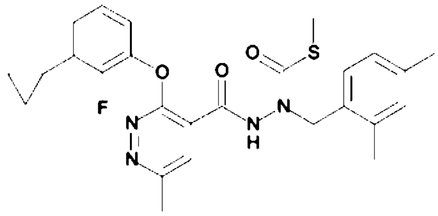 | VISCOUS OIL | 1.84min |
| b-167 | 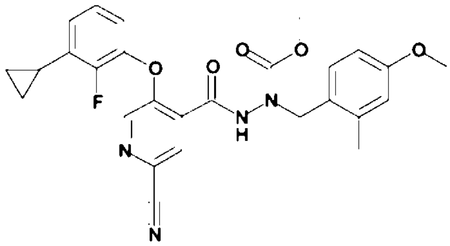 | m.p.:68-76°C | |
| b-168 | 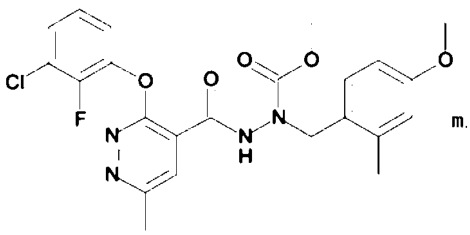 | m.p.:107-109°C | |
| b-169 | 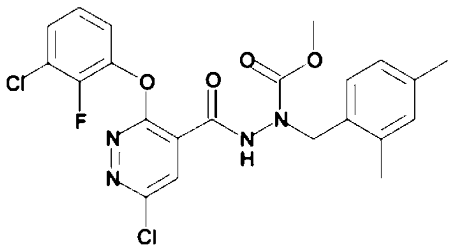 | m.p.:74-76°C | |

[Table 38]

| Table 2 (continued) | | | |
|---|---|---|---|
| Compound No. | Chemical formula | Property | LCMS (SQD2) Retention time |
| b-170 | 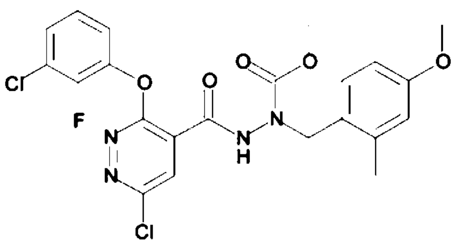 | m.p.:74-76°C | |

(continued)

| Table 2 (continued) | | | |
|---|---|---|---|
| Compound No. | Chemical formula | Property | LCMS (SQD2) Retention time |
| b-171 | 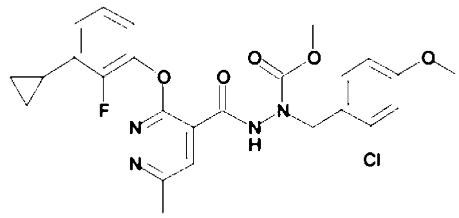 | m.p.:57-62°C | |
| b-172 | 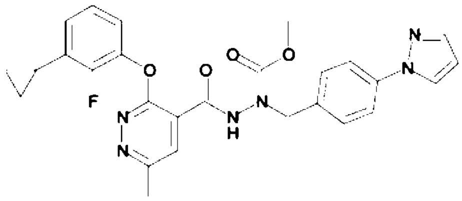 | m.p.:74-80°C | |
| b-173 | 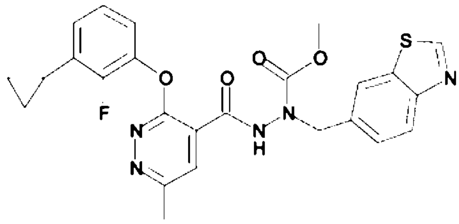 | m.p.:76-80°C | |

[Table 39]

| Table 2 (continued) | | | |
|---|---|---|---|
| Compound No. | Chemical formula | Property | LCMS (SQD2) Retention time |
| b-174 | 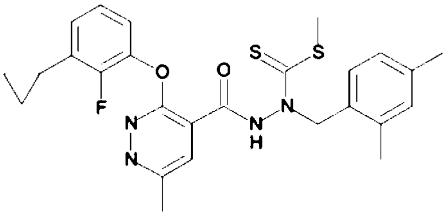 | m.p.:84-86°C | |
| b-175 | 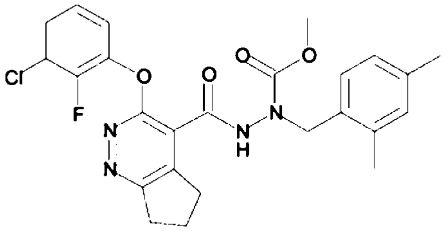 | VISCOUS OIL | 1.65min |
| b-176 | 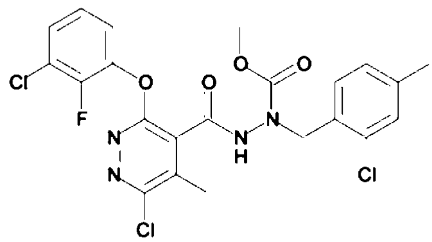 | m.p.:134-136°C | |

(continued)

| Table 2 (continued) | | | |
|---|---|---|---|
| Compound No. | Chemical formula | Property | LCMS (SQD2) Retention time |
| b-177 | 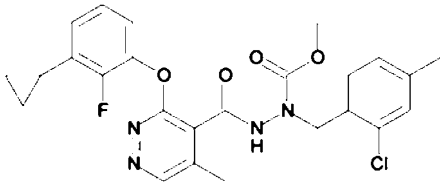 | m.p.:94-96°C | |

[Table 40]

| Table 2 (continued) | | | |
|---|---|---|---|
| Compound No. | Chemical formula | Property | LCMS (SQD2) Retention time |
| b-178 | 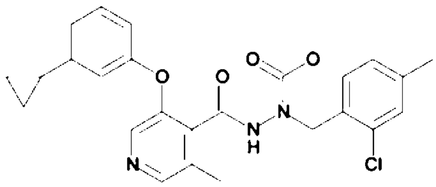 | AMORPHOUS | 1. 73min |
| b-179 | 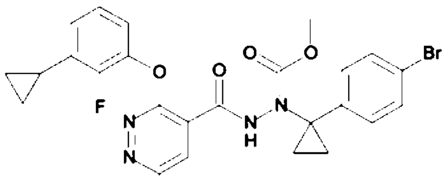 | VISCOUS OIL | 1. 82min |
| b-180 | 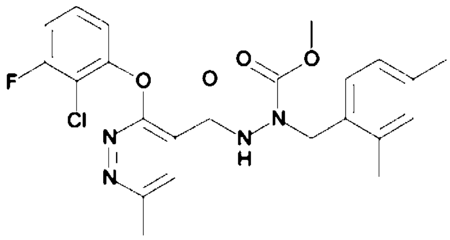 | m.p.:75-77°C | |
| b-181 | 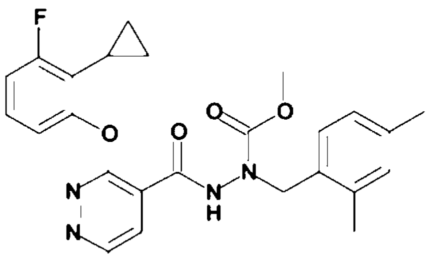 | VISCOUS OIL | 1. 73min |

[Table 41]

| Table 2 (continued) | | | |
|---|---|---|---|
| Compound No. | Chemical formula | Property | LCMS (SQD2) Retention time |
| b-182 | 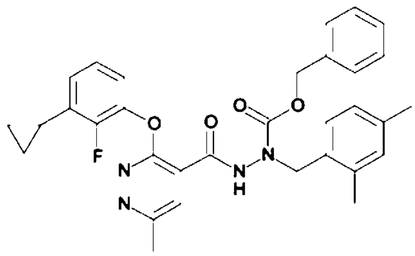 | m.p.:57-59°C | |
| b-183 | 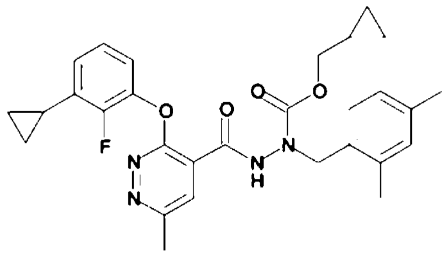 | m.p.:48-50°C | |
| b-184 | 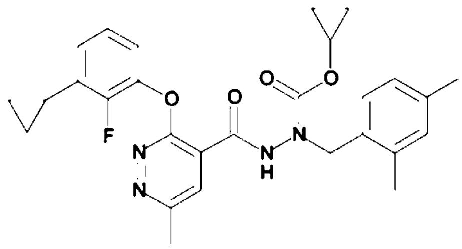 | m.p.:45-47°C | |
| b-185 | 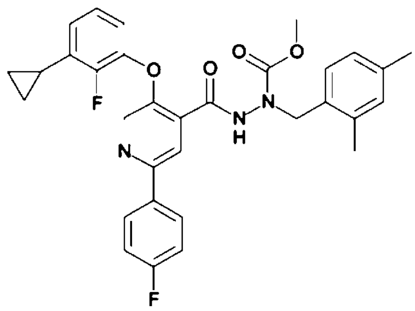 | VISCOUS OIL | 2.09min |

[Table 42]

| Table 2 (continued) | | | |
|---|---|---|---|
| Compound No. | Chemical formula | Property | LCMS (SQD2) Retention time |
| b-186 | 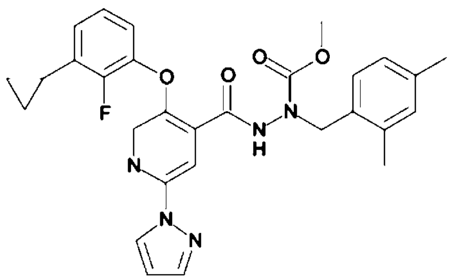 | VISCOUS OIL | 1.97min |

(continued)

| Table 2 (continued) | | | |
|---|---|---|---|
| Compound No. | Chemical formula | Property | LCMS (SQD2) Retention time |
| b-187 | 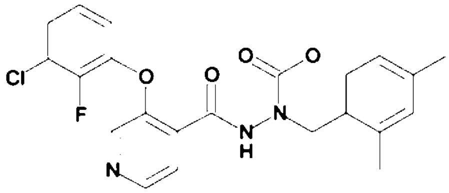 | AMORPHOUS | 1.67min |
| b-188 | 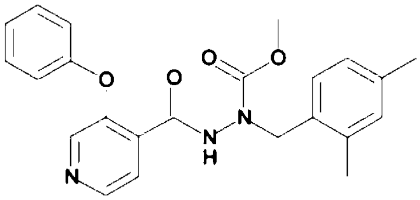 | AMORPHOUS | 1.58min |
| b-189 | 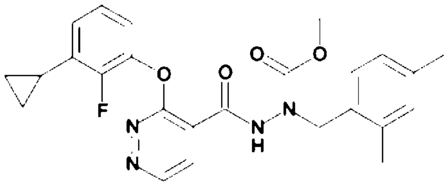 | AMORPHOUS | 1.70min |

[Table 43]

| Table 2 (continued) | | | |
|---|---|---|---|
| Compound No. | Chemical formula | Property | LCMS (SQD2) Retention time |
| b-190 | 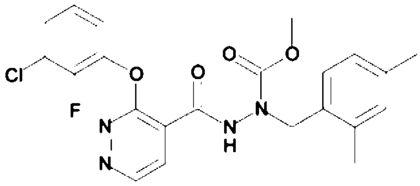 | AMORPHOUS | 1.63min |
| b-191 | 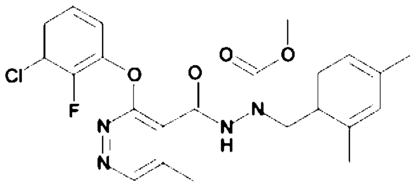 | AMORPHOUS | 1. 62m in |
| b-192 | 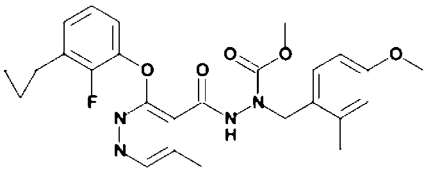 | m.p.:155-157°C | |

(continued)

| Table 2 (continued) | | | |
|---|---|---|---|
| Compound No. | Chemical formula | Property | LCMS (SQD2) Retention time |
| b-193 | 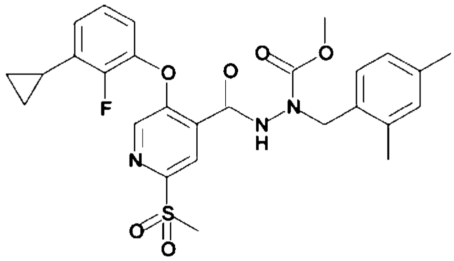 | m.p.:130-132°C | |

[Table 44]

| Table 2 (continued) | | | |
|---|---|---|---|
| Compound No. | Chemical formula | Property | LCMS (SQD2) Retention time |
| b-194 | 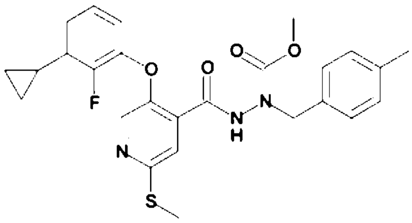 | VISCOUS OIL | 2.01min |
| b-195 | 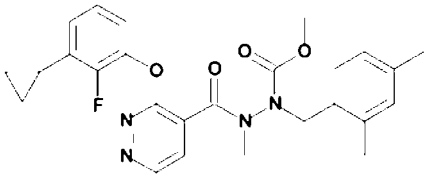 | m.p.:68-70°C | |
| b-196 | 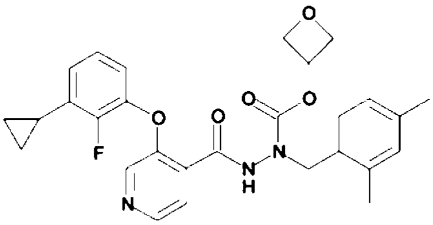 | m.p.:82-84°C | |
| b-197 | 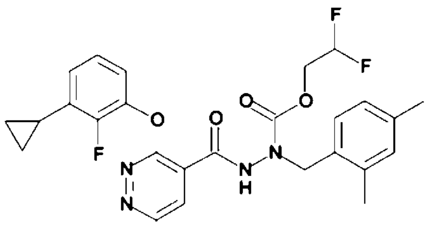 | AMORPHOUS | 1.81min |

[Table 45]

| Table 2 (continued) | | | |
|---|---|---|---|
| Compound No. | Chemical formula | Property | LCMS (SQD2) Retention time |
| b-198 | 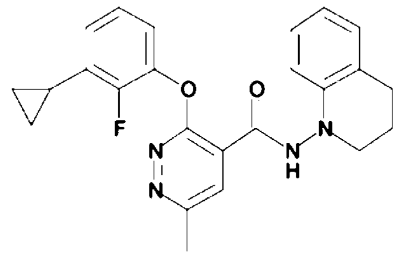 | AMORPHOUS | 1.71min |
| b-199 | 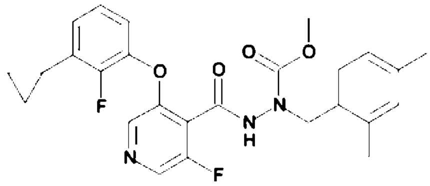 | m.p.:58-60°C | |
| b-200 | 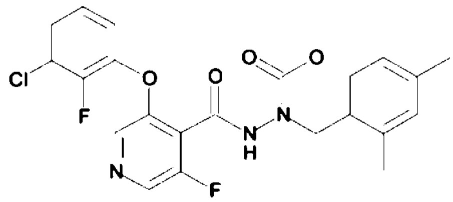 | AMORPHOUS | 1.72min |
| b-201 | 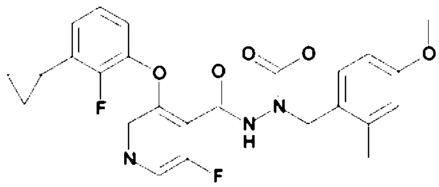 | AMORPHOUS | 1.67min |

[Table 46]

| Table 2 (continued) | | | |
|---|---|---|---|
| Compound No. | Chemical formula | Property | LCMS (SQD2) Retention time |
| b-202 | 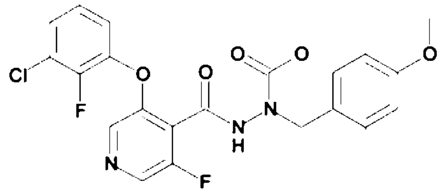 | AMORPHOUS | 1.59min |
| b-203 | 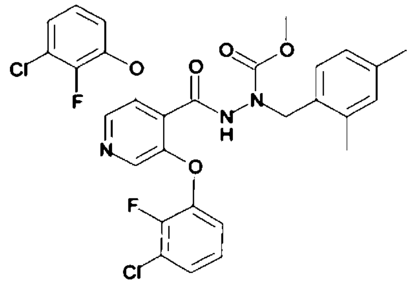 | m.p.:108-110°C | |

(continued)

| Table 2 (continued) | | | |
|---|---|---|---|
| Compound No. | Chemical formula | Property | LCMS (SQD2) Retention time |
| b-204 | 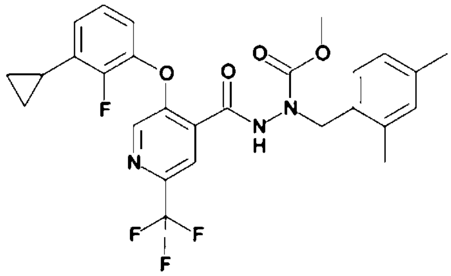 | AMORPHOUS | 1.97min |
| b-205 | 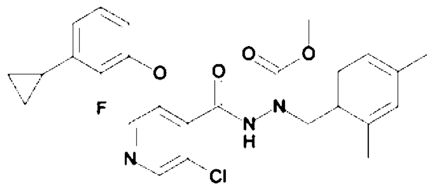 | m.p.:161-163°C | |

[Table 47]

| Table 2 (continued) | | | |
|---|---|---|---|
| Compound No. | Chemical formula | Property | LCMS (SQD2) Retention time |
| b-206 | 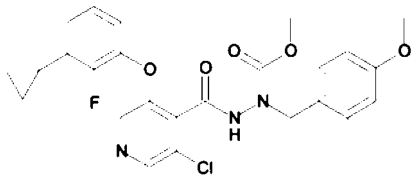 | AMORPHOUS | 1.70min |
| b-207 | 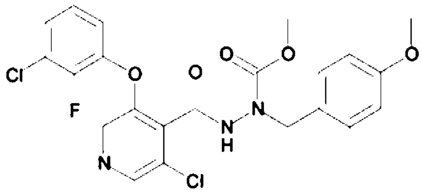 | m.p.:67-69°C | |
| b-208 | 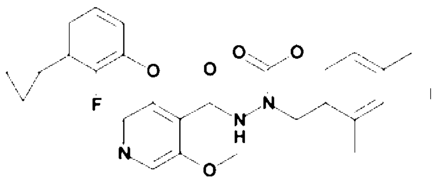 | m.p.:142-144°C | |
| b-209 | 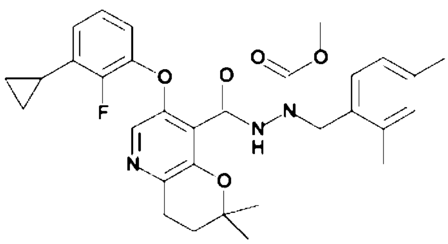 | m.p.:87-89°C | |

[Table 48]

| Table 2 (continued) | | | |
|---|---|---|---|
| Compound No. | Chemical formula | Property | LCMS (SQD2) Retention time |
| b-210 | 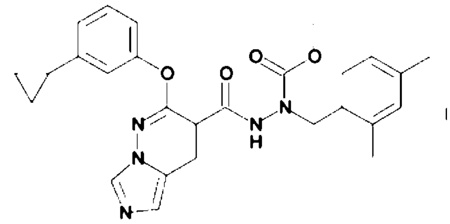 | m.p.:130-132°C | |
| b-211 | 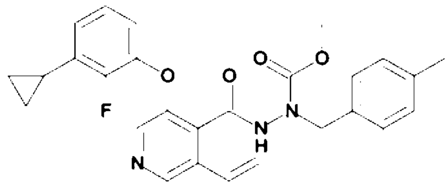 | m.p.:65-67°C | |
| b-212 | 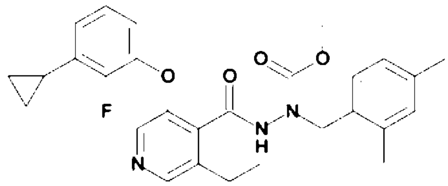 | VISCOUS OIL | 1.76min |
| b-213 | 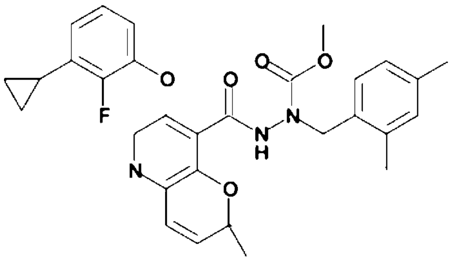 | m.p.:80-82°C | |

[Table 49]

| Table 2 (continued) | | | |
|---|---|---|---|
| Compound No. | Chemical formula | Property | LCMS (SQD2) Retention time |
| b-214 | 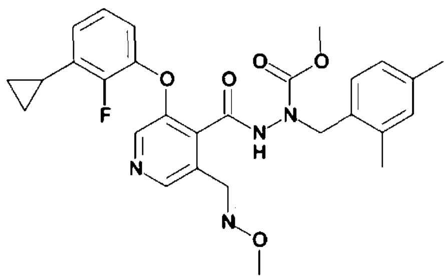 | m.p.:80-82°C | |
| b-215 | 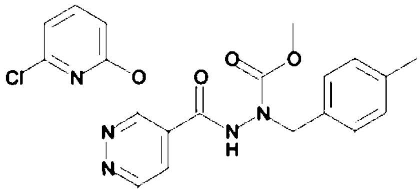 | m.p.:88-90°C | |

(continued)

| Table 2 (continued) | | | |
|---|---|---|---|
| Compound No. | Chemical formula | Property | LCMS (SQD2) Retention time |
| b-216 | 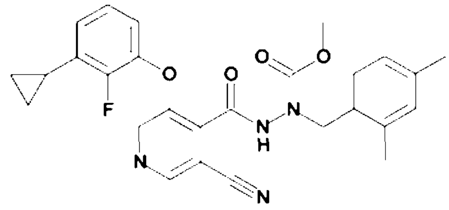 | VISCOUS OIL | 1.80min |
| b-217 | 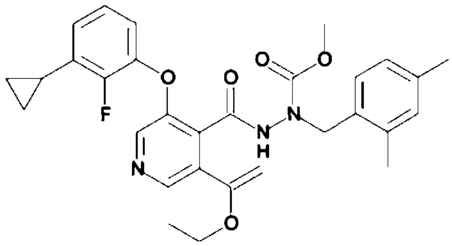 | VISCOUS OIL | 1.82min |

[Table 50]

| Table 2 (continued) | | | |
|---|---|---|---|
| Compound No. | Chemical formula | Property | LCMS (SQD2) Retention time |
| b-218 | 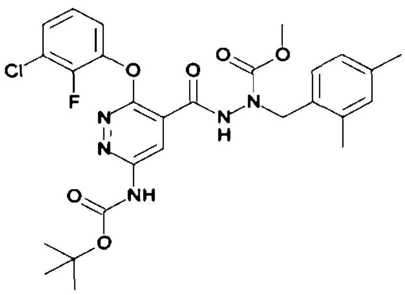 | VISCOUS OIL | 1. 86mi n |
| b-219 | 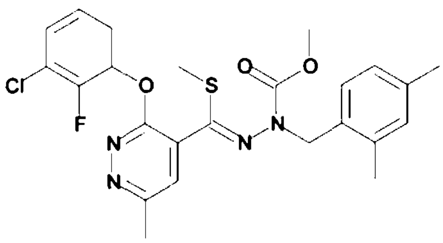 | m.p.:58-60°C | |
| b-220 | 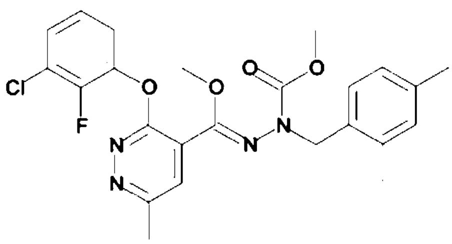 | m.p.:102-104°C | |

(continued)

| Table 2 (continued) | | | |
|---|---|---|---|
| Compound No. | Chemical formula | Property | LCMS (SQD2) Retention time |
| b-221 | 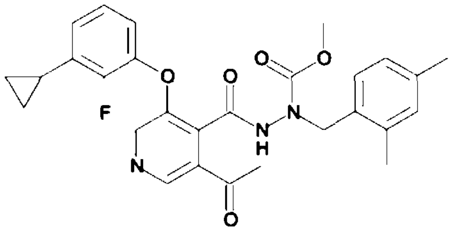 | VISCOUS OIL | 1. 69mi n |

[Table 51]

| Table 2 (continued) | | | |
|---|---|---|---|
| Compound No. | Chemical formula | Property | LCMS (SQD2) Retention time |
| b-222 | 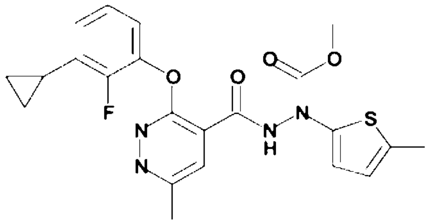 | VISCOUS OIL | 1. 61min |
| b-223 | 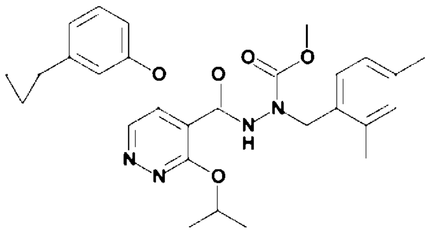 | AMORPHOUS | 1.74min |
| b-224 | 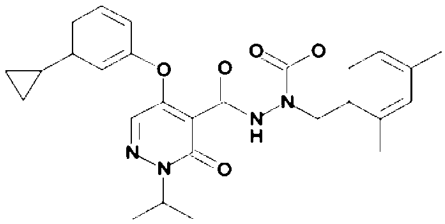 | VISCOUS OIL | 1.86min |
| b-225 | 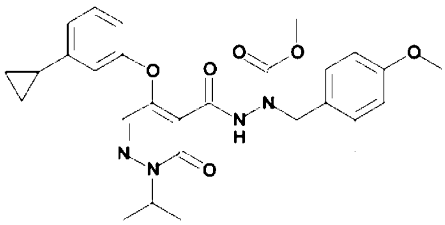 | VISCOUS OIL | 1. 75mi n |

[Table 52]

| Table 2 (continued) | | | |
|---|---|---|---|
| Compound No. | Chemical formula | Property | LCMS (SQD2) Retention time |
| b-226 | 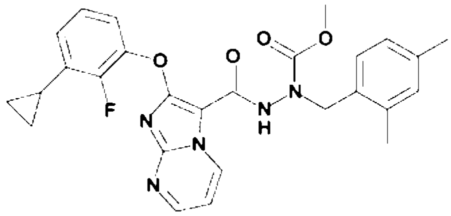 | VISCOUS OIL | 1.75min |
| b-227 | 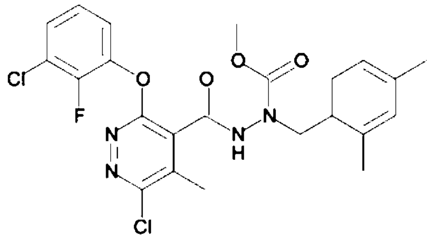 | m.p.:117-119°C | |
| b-228 | 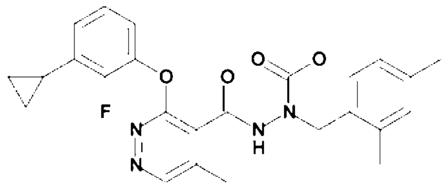 | m.p.:85-87°C | |
| b-229 | 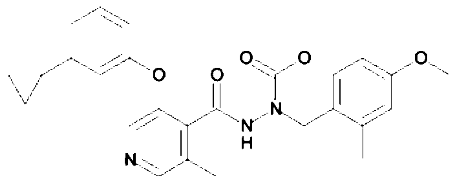 | VISCOUS OIL | 1. 59min |

[Table 53]

| Table 2 (continued) | | | |
|---|---|---|---|
| Compound No. | Chemical formula | Property | LCMS (SQD2) Retention time |
| b-230 | 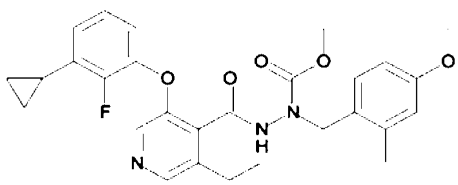 | VISCOUS OIL | 1.64min |
| b-231 | 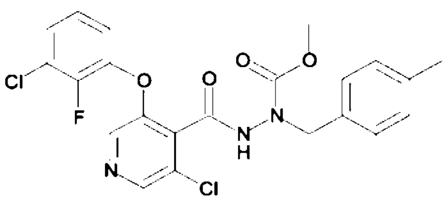 | m.p.:66-68°C | |

(continued)

| Table 2 (continued) | | | |
|---|---|---|---|
| Compound No. | Chemical formula | Property | LCMS (SQD2) Retention time |
| b-232 | 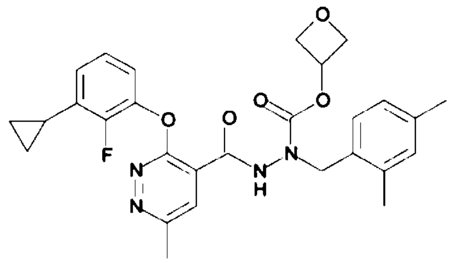 | VISCOUS OIL | 1.70min |

Effects of the present invention are exemplified by the following fungicidal effect test and disease control test.

(Fungicidal effect test against fungi causing gray mold)

A spore suspension of fungi causing gray mold disease (Botrytis cinerea, Vogel medium) was prepared. A hydrazide compound according to the present invention was added to dimethyl sulfoxide to obtain a drug liquid. This drug liquid and the spore suspension were mixed in equal amounts (based on volume) to obtain a test liquid in which the concentration of the hydrazide compound according to the present invention was 25 ppm by volume. The test liquid was added dropwise into a 96-well microplate (area treated with drug).

Dimethyl sulfoxide and the spore suspension were mixed in equal amounts (based on volume) to obtain a control liquid. The control liquid was added dropwise into a 96-well microplate (untreated area).

The microplates were left still in a dark place at 20°C for five days to carry out cultivation. The turbidity OD at a wavelength of 405 nm was measured using a microplate reader. The growth inhibition ratio was calculated by the following equation.

$$\text{Growth inhibition ratio (\%)} = 100\text{-}100\times \{[(\text{OD of area treated with drug after cultivation})\text{-}(\text{OD of area treated with drug before cultivation})] / [(\text{OD of untreated area after cultivation})\text{-}(\text{OD of untreated area before cultivation})]\}$$

Compound Nos. b-1 to b-18, b-20 to b-40, b-42 to b-46, b-48, b-50 to b-55, b-57 to b-59, b-61 to b-64, b-66 to b-69, b-71, b-72, b-74 to b-90, b-92 to b-94, b-96, b-98 to b-130, b-133, b-135 to b-136, b-138, b-142 to b-154, b-158 to b-159, b-162 to b-172, b-175 to b-180, b-182 to b-197, b-199 to b-202, b-204 to b-209, b-211 to b-215, b-217, b-218, b-220, b-221, b-223 to b-225, and b-227 to b-232 were subjected to the fungicidal effect test against fungi causing gray mold. The growth inhibition ratios of all compounds were at least 75%.

(Fungicidal effect test against fungi causing apple anthracnose)

The turbidity was measured to calculate the growth inhibition ratio by the same method as the fungicidal effect test against fungi causing gray mold, except that the spore suspension of fungi causing gray mold disease (Botrytis cinerea, Vogel medium) was replaced with a spore suspension of fungi causing apple anthracnose (Colletotrichum acutatum, Czapek medium), and the temperature and the time period when left still were changed to 25°C and four days.

Compound Nos. b-1 to b-34, b-36 to b-69, b-71, b-72, b-74 to b-84, b-87 to b-90, b-92 to b-94, b-96, b-100 to b-133, b-135 to b-138, b-142 to b-154, b-158 to b-159, b-161 to b-179, b-182 to b-197, b-199 to b-217, b-220 to b-221, b-223 to b-225, and b-227 to b-232 were subjected to a fungicidal effect test against fungi causing apple anthracnose. The

growth inhibition ratios of all compounds were at least 75%.

(Fungicidal effect test against fungi causing grape ripe rot disease)

The turbidity was measured to calculate the growth inhibition ratio by the same method as the fungicidal effect test against fungi causing gray mold, except that the spore suspension of fungi causing gray mold disease (Botrytis cinerea, Vogel medium) was replaced with a spore suspension of fungi causing grape ripe rot disease (Glomerella cingulata, Vogel medium), and the temperature and the time period when left still were changed to 25°C and three days.
Compound Nos. b-1 to b-18, b-20 to b-34, b-36 to b-69, b-71 to b-90, b-92 to b-94, b-96 to b-97, b-100 to b-103, b-105 to b-133, b-135 to b-138, b-142 to b-154, b-158 to b-159, b-161 to b-179, b-182 to b-217, b-220 to b-225, and b-227 to b-232 were subjected to the fungicidal effect test against fungi causing grape ripe rot disease. The growth inhibition ratios of all compounds were at least 75%.

(Fungicidal effect test against fungi causing wheat Fusarium head blight)

The turbidity was measured to calculate the growth inhibition ratio by the same method as the fungicidal effect test against fungi causing gray mold, except that the spore suspension of fungi causing gray mold disease (Botrytis cinerea, Vogel medium) was replaced with a spore suspension of fungi causing wheat Fusarium head blight disease (Fusarium graminearum, SD medium), and the temperature and the time period when left still were changed to 25°C and four days.
Compound Nos. b-1, b-3, b-6 to b-8, b-14 to b-16, b-22, b-23, b-27, b-28, b-31 to b-34, b-36 to b-38, b-42, b-43, b-45, b-46, b-53, b-55, b-67, b-74 to b-77, b-80, b-87, b-88, b-90, b-94, b-106 to b-108, b-111 to b-113, b-116 to b-119, b-124, b-126 to b-128, b-135, b-138, b-143, b-145 to b-150, b-152 to b-154, b-167 to b-171, b-175 to b-178, b-184, b-187, b-188, b-191, b-194, b-196, b-199, b-200, b-202, b-204, b-207 to b-209, b-211 to b-215, b-217, b-223, and b-227 to b-232 were subjected to the fungicidal effect test against fungi causing wheat Fusarium head blight. The growth inhibition ratios of all compounds were at least 75%.

(Control test of cucumber gray mold)

5 parts by mass of a hydrazide compound according to the present invention, 93.5 parts by mass of N,N-dimethylformamide, and 1.5 parts by mass of polyoxyethylene sorbitan monolaurate were mixed together to obtain an emulsion (1) having 5% by mass of the active ingredient.
Water was added to the emulsion (1) to obtain a drug liquid in which the concentration of the hydrazide compound according to the present invention was 125 ppm by mass or 25 ppm by mass. Then, the drug liquid was sprayed onto young cucumber seedlings cultivated in a nursery pot (variety "Jihai"-based, cotyledon stage), and then air-dried. Then, a conidiospore suspension of fungi causing cucumber gray mold disease (Botrytis cinerea) was inoculated dropwise thereon (treated area). As a control, a conidiospore suspension of fungi causing cucumber gray mold disease (Botrytis cinerea) was inoculated dropwise onto young cucumber seedlings unsprayed with the drug liquid (untreated area). They were left still in a moist room at 20°C. Four days after inoculation, leaves of the young cucumber seedlings were visually observed, and the control value was calculated from the lesion area ratio in accordance with the following equation.

$$\text{Control value (\%)} = 100 - \{\text{the lesion area ratio at the treated area / the lesion area ratio at the untreated area}\} \times 100$$

Compound Nos. b-3, b-5 to b-6, b-9 to b-10, b-13 to b-18, b-20 to b-23, b-25, b-27 to b-31, b-33 to b-34, b-36 to b-38, b-42 to b-46, b-50, b-52 to b-53, b-55, b-57 to b-59, b-64, b-66 to b-68, b-74 to b-77, b-79 to b-81, b-83, b-85 to b-86, b-88, b-90, b-92, b-94, b-96, b-101, b-103 to b-104, b-106 to b-109, b-111 to b-114, b-117 to b-118, b-121 to b-122, b-124, b-126 to b-127, b-130, b-138, b-145 to b-154, b-158 to b-159, b-163, b-168 to b-171, b-176 to b-178, b-182 to b-184, b-186 to b-192, b-196 to b-197, b-199 to b-202, b-204 to b-209, b-211 to b-214, b-220, b-223 to b-224, and b-227 to b-232 were subjected to the control test of cucumber gray mold at the concentration of 125 ppm by mass. The control values of all compounds were at least 75%.
Compound Nos. b-3, b-5, b-6, b-9, b-14 to b-17, b-21 to b-23, b-25, b-27 to b-32, b-34, b-36, b-38, b-42 to b-46, b-52, b-53, b-55, b-66, b-67, b-72, b-74 to b-77, b-80, b-85, b-86, b-88, b-90, b-92, b-94, b-101, b-103 to b-106, b-108 to b-109, b-113, b-115 to b-119, b-121, b-127, b-130, b-135, b-138, b-145, b-147 to b-154, b-158 to b-159, b-163, b-171,

b-176 to b-178, b-182 to b-184, b-186 to b-192, b-196 to b-197, b-199 to b-202, b-204, b-206 to b-209, b-211 to b-213, b-223, and b-227 to b-232 were subjected to the control test of cucumber gray mold at the concentration of 25 ppm by mass. The control values of all compounds were at least 75%.

(Control test of apple scab disease)

Water was added to the emulsion (1) such that the concentration of the hydrazide compound according to the present invention was 125 ppm by mass, followed by dissolving the hydrazide compound therein to obtain a drug liquid. Then, the drug liquid was sprayed onto young apple seedlings cultivated in a nursery pot (variety "Orin", three-to four-leaf stage), and then air-dried. Thereafter, conidiospores of fungi causing apple scab disease (Venturia inaequalis) were inoculated thereon (treated area). As a control, the conidiospores were inoculated in a similar manner to the above on young apple seedlings unsprayed with the drug liquid (untreated area). They were left still at 20°C in a moist room with light and dark cycles repeated every 12 hours.

12 Days to 14 days after inoculation, leaves of young apple seedlings were visually observed, and the control value was calculated from the lesion area ratio in accordance with the equation used in the control test of cucumber gray mold.

Compound Nos. b-5, b-6, b-9, b-15 to b-18, b-20 to b-23, b-25 to b-26, b-28 to b-31, b-33 to b-36, b-38, b-42 to b-46, b-52 to b-53, b-55, b-57, b-65 to b-68, b-71, b-74 to b-77, b-79 to b-81, b-83, b-85 to b-86, b-88, b-90, b-92, b-94, b-96, b-100 to b-104, b-106 to b-114, b-117 to b-118, b-121, b-123 to b-124, b-127, b-138, b-144 to b-154, b-158 to b-159, b-163 to b-165, b-168 to b-174, b-176 to b-178, b-180, b-182 to b-184, b-186 to b-187, b-189 to b-192, b-196, b-199, b-201 to b-202, b-204 to b-206, b-208, b-212, b-220, b-223, and b-227 to b-232 were subjected to the control test of apple scab disease. The control values of all compounds were at least 75%.

(Control test of wheat powdery mildew disease)

Water was added to the emulsion (1) such that the concentration of the hydrazide compound according to the present invention was 125 ppm by mass, and the hydrazide compound was dissolved therein to obtain a drug liquid. Then, the drug liquid was sprayed onto young wheat seedlings cultivated in a nursery pot (variety "Chihoku", one-to two-leaf stage), and then air-dried. Thereafter, conidiospores of fungi causing wheat powdery mildew disease (Erysiphe graminis f.sp.tritici) were sprinkled on the young wheat seedlings to inoculate them (treated area). As a control, the conidiospores of fungi causing wheat powdery mildew disease (Erysiphe graminis f.sp.tritici) were sprinkled on young wheat seedlings unsprayed with the drug liquid (untreated area). They were left still in a greenhouse at 20°C. Six days after inoculation, leaves of young wheat seedlings were visually observed, and the lesion area ratio was determined to calculate the control value in a similar manner to the previous test.

Compound Nos. b-3, b-5, b-6, b-9, b-13 to b-18, b-20 to b-23, b-25, b-28 to b-31, b-33, b-34, b-36 to b-38, b-40, b-42 to b-46, b-50 to b-53, b-55, b-57, b-58, b-60, b-62, b-65 to b-69, b-74 to b-77, b-80, b-81, b-83, b-85, b-86, b-88, b-90, b-92 to b-94, b-96, b-100 to b-104, b-106 to b-114, b-117, b-118, b-121, b-123 to b-125, b-127, b-145 to b-154, b-158, b-159, b-162, b-163, b-168 to b-171, b-176 to b-178, b-180, b-182 to b-184, b-187 to b-192, b-196, b-199 to b-202, b-204 to b-208, b-211 to b-213, b-220, b-223, and b-227 to b-232 were subjected to the control test of wheat powdery mildew disease. The control values of all compounds were at least 75%.

(Control test of wheat red rust disease)

Water was added to the emulsion (1) such that the concentration of the hydrazide compound according to the present invention was 125 ppm by mass, followed by dissolving the hydrazide compound therein to obtain a drug liquid. Then, the drug liquid was sprayed onto young wheat seedlings cultivated in a nursery pot (variety "Norin 61", one- to two-leaf stage), and then air-dried. Thereafter, urediniospores of fungi causing wheat red rust disease (Puccinia recondita) were sprinkled on the young wheat seedlings to inoculate them (treated area). As a control, urediniospores of fungi causing wheat red rust disease (Puccinia recondita) were sprinkled on young wheat seedlings unsprayed with the drug liquid (untreated area). They were left still in a high humidity room at 20°C for one day. Thereafter, they were left still in a low humidity room at 20°C for 12 days. Then, leaves of young wheat seedlings were visually observed, and the lesion area ratio was determined to calculate the control value in a similar manner to the previous test.

Compound Nos. b-51 and b-62 were subjected to the control test of wheat red rust disease. The control values of all compounds were at least 75%.

Since all compounds selected from the hydrazide compounds according to the present invention at random exhibited the above-mentioned effects, it can be understood that the hydrazide compounds according to the present invention, encompassing compounds not listed as examples, have fungicidal effects, do not cause phytotoxicity to plants, and exhibit low toxicity to humans, livestock, and fish, and have low impact on the environment.

INDUSTRIAL APPLICABILITY

The hydrazide compound according to the present invention exhibits excellent fungicidal activity against pathogens that affect agricultural and horticultural plants, has excellent safety, and can be synthesized in an industrially advantageous manner. The agricultural and horticultural fungicide according to the present invention can effectively control diseases of agricultural and horticultural plants.

## Claims

**1.** A compound of formula (Z-1) or a salt thereof,

[Chemical Formula 1]

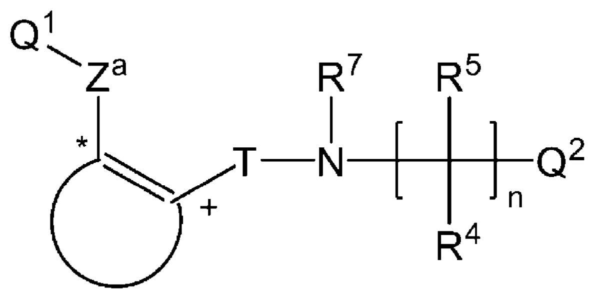

(Z − I)

(in the formula (Z-1),

$Q^1$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group,
$Q^2$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group,
a carbon atom that constitutes a ring in $Q^2$ may bond, instead of $R^7$, to a nitrogen atom having $R^7$,
a partial structure Het:

[Chemical Formula 2]

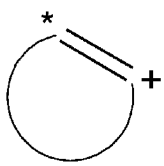

(IIet)

is a substituted or unsubstituted six-membered heteroaryl ring, a substituted or unsubstituted six-membered nitrogen-containing hetero ring having an oxo group, or a substituted or unsubstituted nine- or ten-membered heteroaryl ring,
carbon atoms marked with * and + in the partial structure correspond to carbon atoms marked with * and + in the formula (Z-1),
$Z^a$ is an oxygen atom, a sulfur atom, or a group of formula: $NR^a$,
$R^a$ is a hydrogen atom, or a substituted or unsubstituted C1-6 alkyl group,
T is a partial structure (T-1) or a partial structure (T-2),

[Chemical Formula 3]

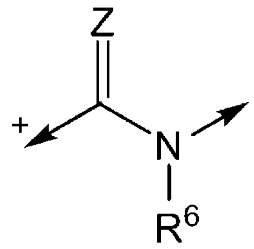

(T-1)

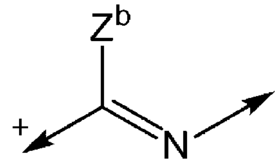

(T-2)

in the partial structure (T-1), Z is an oxygen atom, a sulfur atom, or a group of formula: $NR^b$, an arrow marked with + is bonded to a carbon atom marked with + in the partial structure, the other arrow bonded to a nitrogen atom having $R^7$,
$R^b$ is a hydrogen atom, a substituted or unsubstituted C1-6 alkyl group, a hydroxy group, or a substituted or unsubstituted C1-6 alkoxy group,
$R^6$ is a hydrogen atom, a substituted or unsubstituted C1-6 alkyl group, a substituted or unsubstituted C2-6 alkenyl group, a substituted or unsubstituted C2-6 alkynyl group, a formyl group, a substituted or unsubstituted C1-6 alkylcarbonyl group, a substituted or unsubstituted C1-6 alkoxycarbonyl group, a substituted or unsubstituted C3-6 cycloalkyl group, a substituted or unsubstituted phenyl group, a substituted or unsubstituted naphthyl group, or a substituted or unsubstituted five- or six-membered heterocyclyl group,
in the partial structure (T-2), $Z^b$ is a C1-6 alkoxy group, or a C1-6 alkylthio group,
$R^4$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a halogeno group,
$R^5$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a halogeno group,
$R^4$ and $R^5$ on an identical carbon atom may form together a divalent organic group,
n is an integer of 1 to 3,
$R^7$ is a hydrogen atom, a substituted or unsubstituted C1-6 alkyl group, a substituted or unsubstituted C2-6 alkenyl group, a substituted or unsubstituted C2-6 alkynyl group, a formyl group, a group of formula: $R^c$-CO-, a group of formula: $R^c$-CS-, a group of formula: $R^c$-CO-CO-, a group of formula: $R^c$-$SO_2$-, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a cyano group, and
$R^c$ is each independently a substituted or unsubstituted C1-6 alkyl group, a substituted or unsubstituted C1-6 alkoxy group, a substituted or unsubstituted C3-6 cycloalkoxy group, a substituted or unsubstituted C1-6 alkylthio group, a substituted or unsubstituted amino group, a substituted or unsubstituted C3-6 cycloalkyl group, a substituted or unsubstituted phenyl group, a substituted or unsubstituted naphthyl group, a substituted or unsubstituted three- to six-membered heterocyclyl group, or a substituted or unsubstituted three- to six-membered heterocyclyloxy group.)

**2.** The compound or the salt thereof according to claim 1, wherein the formula (Z-1) is formula (I),

[Chemical Formula 4]

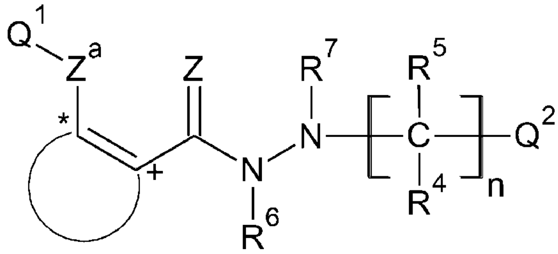

(I)

(in the formula (I),

$Q^1$, $Q^2$, $Z^a$, $R^4$, $R^5$, $R^7$ and n are identical to $Q^1$, $Q^2$, $Z^a$, $R^4$, $R^5$, $R^7$ and n in the formula (Z-1), and Z and $R^6$ are identical to Z and $R^6$ in the partial structure (T-1).)

**3.** The compound or the salt thereof according to claim 1, wherein the partial structure Het:

[Chemical Formula 5]

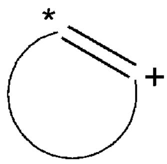

(Het)

is a six-membered heteroaryl ring of formula (II),

[Chemical Formula 6]

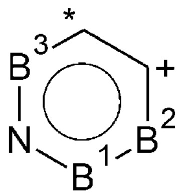

(II)

(in the formula (II),

$B^1$ is a nitrogen atom, or a group of formula: $CR^1$,
$R^1$ is a hydrogen atom, a substituted or unsubstituted C1-6 alkyl group, a substituted or unsubstituted C2-6 alkenyl group, a substituted or unsubstituted C2-6 alkynyl group, a hydroxy group, a substituted or unsubstituted C1-6 alkoxy group, a substituted or unsubstituted C1-6 alkylcarbonyl group, a substituted or unsubstituted C1-6 alkoxycarbonyl group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, a substituted or unsubstituted phenyloxy group, a substituted or unsubstituted naphthyloxy group, a substituted or unsubstituted five- or six-membered heterocyclyloxy group, a substituted or unsubstituted amino group, a substituted or unsubstituted aminocarbonyl group, a N-(C1-6 alkoxy)imino group, a nitro group, a cyano group, or a halogeno group,
$B^2$ is a nitrogen atom, or a group of formula: $CR^2$,
$R^2$ is a hydrogen atom, a substituted or unsubstituted C1-6 alkyl group, a substituted or unsubstituted C2-6 alkenyl group, a substituted or unsubstituted C2-6 alkynyl group, a hydroxy group, a substituted or unsubstituted

C1-6 alkoxy group, a substituted or unsubstituted C1-6 alkylcarbonyl group, a substituted or unsubstituted C1-6 alkoxycarbonyl group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, a substituted or unsubstituted phenyloxy group, a substituted or unsubstituted naphthyloxy group, a substituted or unsubstituted five- or six-membered heterocyclyloxy group, a substituted or unsubstituted amino group, a substituted or unsubstituted aminocarbonyl group, an N-(C1-6 alkoxy)imino group, a nitro group, a cyano group, or a halogeno group,
$R^1$ and $R^2$ may form together a divalent organic group,
$B^3$ is a nitrogen atom, or a group of formula: $CR^3$,
$R^3$ is a hydrogen atom, a substituted or unsubstituted C1-6 alkyl group, a substituted or unsubstituted C2-6 alkenyl group, a substituted or unsubstituted C2-6 alkynyl group, a hydroxy group, a substituted or unsubstituted C1-6 alkoxy group, a substituted or unsubstituted C1-6 alkylcarbonyl group, a substituted or unsubstituted C1-6 alkoxycarbonyl group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, a substituted or unsubstituted phenyloxy group, a substituted or unsubstituted naphthyloxy group, a substituted or unsubstituted five- or six-membered heterocyclyloxy group, a substituted or unsubstituted amino group, a substituted or unsubstituted aminocarbonyl group, a nitro group, a cyano group, or a halogeno group, and
carbon atoms marked with * and + in the partial structure correspond to carbon atoms marked with * and + in the formula (Z-1).)

**4.** The compound or the salt thereof according to claim 3, wherein $Z^a$ is an oxygen atom,

Z is an oxygen atom,
$R^6$ is a hydrogen atom, and
$R^7$ is a formyl group, a group of formula: $R^c$-CO-, a group of formula: $R^c$-CS-, a group of formula: $R^c$-CO-CO-, or a group of formula: $R^c$-$SO_2$-.

**5.** An agricultural and horticultural fungicide comprising at least one selected from the group consisting of a compound and a salt thereof of any one of claims 1 to 4, as an active ingredient.

**6.** A method for producing a compound of formula (I-1), the method comprising a step in which a compound of formula (im-1) and a compound of formula (im-2) are reacted in the presence of an acid scavenger,

[Chemical Formula 7]

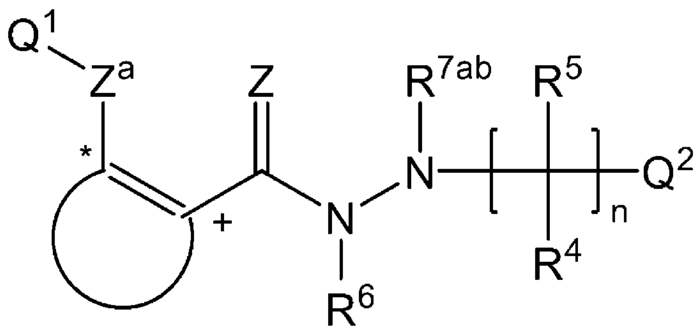

(I − 1)

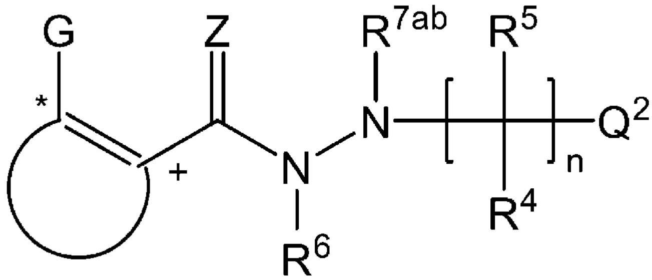

( i m− 1 )

$Q^1-Z^a-H$ ( i m− 2 )

(in each formula shown above,

$Q^1$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group,
$Q^2$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group,
a carbon atom that constitutes a ring in $Q^2$ may bond, instead of $R^{7ab}$, to a nitrogen atom having $R^{7ab}$,
the partial structure Het:

[Chemical Formula 8]

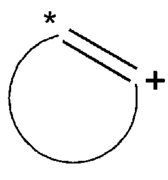

(IIct)

is a substituted or unsubstituted six-membered heteroaryl ring, a substituted or unsubstituted six-membered nitrogen-containing hetero ring having an oxo group, or a substituted or unsubstituted nine- or ten-membered heteroaryl ring,
carbon atoms marked with * and + in the partial structure correspond to carbon atoms marked with * and + in the formula (Z-1),
$Z^a$ is an oxygen atom, a sulfur atom, or a group of formula: $NR^a$,
$R^a$ is a hydrogen atom, or a substituted or unsubstituted C1-6 alkyl group,
Z is an oxygen atom, a sulfur atom, or a group of formula: $NR^b$,
$R^b$ is a hydrogen atom, a substituted or unsubstituted C1-6 alkyl group, a hydroxy group, or a substituted or unsubstituted C1-6 alkoxy group,

$R^4$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a halogeno group,
$R^5$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a halogeno group,
$R^4$ and $R^5$ on an identical carbon atom may form together a divalent organic group,
n is an integer of 1 to 3,
$R^6$ is a hydrogen atom, a substituted or unsubstituted C1-6 alkyl group, a substituted or unsubstituted C2-6 alkenyl group, a substituted or unsubstituted C2-6 alkynyl group, a formyl group, a substituted or unsubstituted C1-6 alkylcarbonyl group, a substituted or unsubstituted C1-6 alkoxycarbonyl group, a substituted or unsubstituted C3-6 cycloalkyl group, a substituted or unsubstituted phenyl group, a substituted or unsubstituted naphthyl group, or a substituted or unsubstituted five- or six-membered heterocyclyl group,
$R^{7ab}$ is a substituted or unsubstituted C1-6 alkyl group, a substituted or unsubstituted C2-6 alkenyl group, a substituted or unsubstituted C2-6 alkynyl group, a formyl group, a group of formula: $R^c$-CO-, a group of formula: $R^c$-CS-, a group of formula: $R^c$-CO-CO-, a group of formula: $R^c$-$SO_2$-, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a cyano group,
$R^c$ is each independently a substituted or unsubstituted C1-6 alkyl group, a substituted or unsubstituted C1-6 alkoxy group, a substituted or unsubstituted C3-6 cycloalkoxy group, a substituted or unsubstituted C1-6 alkylthio group, a substituted or unsubstituted amino group, a substituted or unsubstituted C3-6 cycloalkyl group, a substituted or unsubstituted phenyl group, a substituted or unsubstituted naphthyl group, a substituted or unsubstituted three- to six-membered heterocyclyl group, or a substituted or unsubstituted three- to six-membered heterocyclyloxy group, and
G is a halogeno group.)

**7.** A method for producing a compound of formula (I-3), the method comprising a step in which a compound of formula (im-5) and a compound of formula (im-6) are reacted in the presence of a condensing agent when $U^1$ in the formula (im-5) is a hydroxy group, or in the presence of an acid scavenger when $U^1$ is a halogeno group,

[Chemical Formula 9]

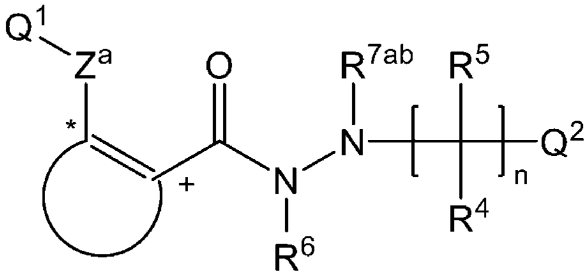

(I - 3)

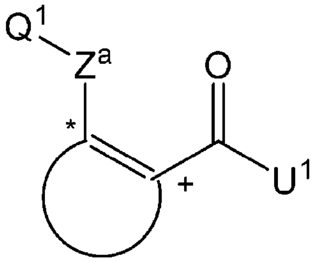

(im - 5)

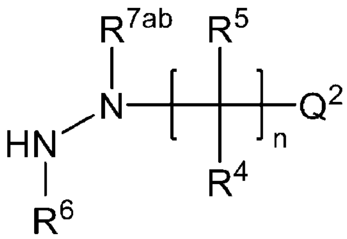

(im - 6)

(in each formula shown above,

$Q^1$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group,

$Q^2$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group,

a carbon atom that constitutes a ring in $Q^2$ may bond, instead of $R^{7ab}$, to a nitrogen atom having $R^{7ab}$,

the partial structure Het:

[Chemical Formula 10]

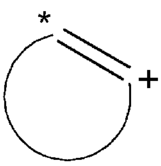

(Het)

is a substituted or unsubstituted six-membered heteroaryl ring, a substituted or unsubstituted six-membered nitrogen-containing hetero ring having an oxo group, or a substituted or unsubstituted nine- or ten-membered heteroaryl ring,

carbon atoms marked with * and + in the partial structure correspond to carbon atoms marked with * and + in the formula (Z-1),

$Z^a$ is an oxygen atom, a sulfur atom, or a group of formula: $NR^a$,

$R^a$ is a hydrogen atom, or a substituted or unsubstituted C1-6 alkyl group,

$R^b$ is a hydrogen atom, a substituted or unsubstituted C1-6 alkyl group, a hydroxy group, or a substituted or unsubstituted C1-6 alkoxy group,

$R^4$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a halogeno group,

$R^5$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a halogeno group,

$R^4$ and $R^5$ on an identical carbon atom may form together a divalent organic group,

n is an integer of 1 to 3,

$R^6$ is a hydrogen atom, a substituted or unsubstituted C1-6 alkyl group, a substituted or unsubstituted C2-6 alkenyl group, a substituted or unsubstituted C2-6 alkynyl group, a formyl group, a substituted or unsubstituted C1-6 alkylcarbonyl group, a substituted or unsubstituted C1-6 alkoxycarbonyl group, a substituted or unsubstituted C3-6 cycloalkyl group, a substituted or unsubstituted phenyl group, a substituted or unsubstituted naphthyl group, or a substituted or unsubstituted five- or six-membered heterocyclyl group,

$R^{7ab}$ is a substituted or unsubstituted C1-6 alkyl group, a substituted or unsubstituted C2-6 alkenyl group, a substituted or unsubstituted C2-6 alkynyl group, a formyl group, a group of formula: $R^c$-CO-, a group of formula: $R^c$-CS-, a group of formula: $R^c$-CO-CO-, a group of formula: $R^c$-$SO_2$-, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a cyano group,

$R^c$ is each independently a substituted or unsubstituted C1-6 alkyl group, a substituted or unsubstituted C1-6 alkoxy group, a substituted or unsubstituted C3-6 cycloalkoxy group, a substituted or unsubstituted C1-6 alkylthio group, a substituted or unsubstituted amino group, a substituted or unsubstituted C3-6 cycloalkyl group, a substituted or unsubstituted phenyl group, a substituted or unsubstituted naphthyl group, a substituted or unsubstituted three- to six-membered heterocyclyl group, or a substituted or unsubstituted three- to six-membered heterocyclyloxy group, and

$U^1$ is a hydroxy group or a halogeno group.)

**8.** A method for producing a compound of formula (I-A1), the method comprising a step in which a compound of formula (im-A1) and a compound of formula (im-A2) are reacted in the presence of an acid scavenger,

[Chemical Formula 11]

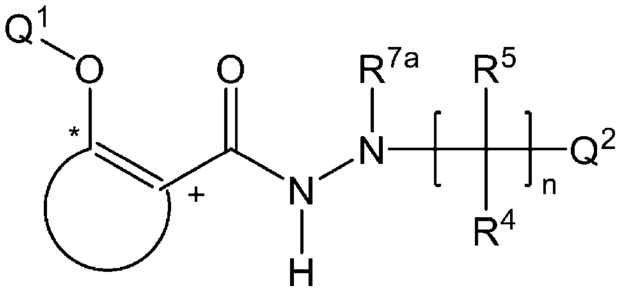

(I-A1)

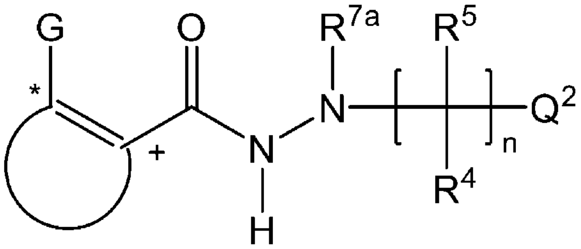

(im-A1)

$Q^1-O-H$ (im-A2)

(in each formula shown above,

$Q^1$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group,
$Q^2$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group,
a carbon atom that constitutes a ring in $Q^2$ may bond, instead of $R^{7a}$, to a nitrogen atom having $R^{7a}$,
the partial structure Het:

[Chemical Formula 12]

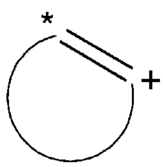

(Het)

is a substituted or unsubstituted six-membered heteroaryl ring, a substituted or unsubstituted six-membered nitrogen-containing hetero ring having an oxo group, or a substituted or unsubstituted nine- or ten-membered heteroaryl ring,
carbon atoms marked with * and + in the partial structure correspond to carbon atoms marked with * and + in the formula (Z-1),
$R^4$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a halogeno group,
$R^5$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a halogeno group, $R^4$ and $R^5$ on an identical carbon atom may form together a divalent organic group,
n is an integer of 1 to 3,
$R^{7a}$ is a formyl group, a group of formula: $R^c$-CO-, a group of formula: $R^c$-CS-, a group of formula: $R^c$-CO-CO-, or a group of formula: $R^c$-$SO_2$-,
$R^c$ is each independently a substituted or unsubstituted C1-6 alkyl group, a substituted or unsubstituted C1-6 alkoxy group, a substituted or unsubstituted C3-6 cycloalkoxy group, a substituted or unsubstituted C1-6 alkylthio group, a substituted or unsubstituted amino group, a substituted or unsubstituted C3-6 cycloalkyl group, a substituted or unsubstituted phenyl group, a substituted or unsubstituted naphthyl group, a substituted or unsubstituted three- to six-membered heterocyclyl group, or a substituted or unsubstituted three- to six-membered heterocyclyloxy group, and
G is a halogeno group.)

**9.** A method for producing a compound of formula (I-A1), the method comprising a step in which a compound of formula (im-A5) and a compound of formula (im-6a) are reacted in the presence of a condensing agent when $U^1$ in the formula (im-A5) is a hydroxy group, or in the presence of an acid scavenger when $U^1$ is a halogeno group,

[Chemical Formula 13]

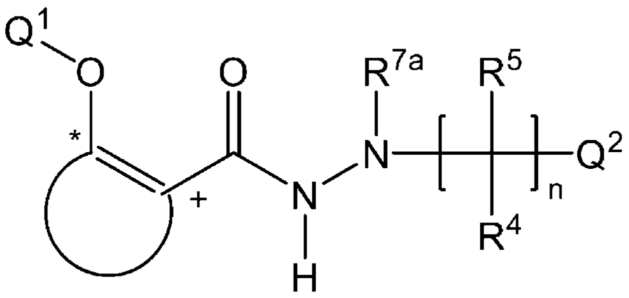

(I-A1)

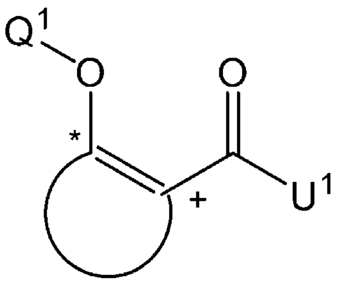

(im-A5)

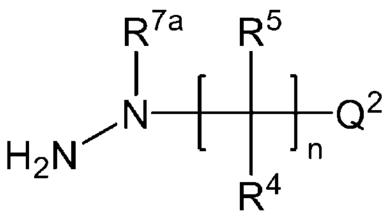

(im-6a)

(in each formula shown above,

$Q^1$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group,
$Q^2$ is a substituted or unsubstituted phenyl group, a substituted or unsubstituted naphthyl group, a substituted or

unsubstituted five- or six-membered heterocyclyl group, or a substituted or unsubstituted nine- or ten-membered heterocyclyl group,
a carbon atom that constitutes a ring in $Q^2$ may bond, instead of $R^{7a}$, to a nitrogen atom having $R^{7a}$,
the partial structure Het:

[Chemical Formula 14]

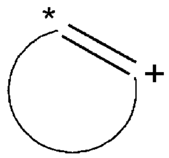

(Het)

is a substituted or unsubstituted six-membered heteroaryl ring, a substituted or unsubstituted six-membered nitrogen-containing hetero ring having an oxo group, or a substituted or unsubstituted nine- or ten-membered heteroaryl ring,
carbon atoms marked with * and + in the partial structure correspond to carbon atoms marked with * and + in the formula (Z-1),
$R^4$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a halogeno group,
$R^5$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, or a halogeno group,
$R^4$ and $R^5$ on an identical carbon atom may form together a divalent organic group,
n is an integer of 1 to 3,
$R^{7a}$ is a formyl group, a group of formula: $R^c$-CO-, a group of formula: $R^c$-CS-, a group of formula: $R^c$-CO-CO-, or a group of formula: $R^c$-$SO_2$-,
$R^c$ is each independently a substituted or unsubstituted C1-6 alkyl group, a substituted or unsubstituted C1-6 alkoxy group, a substituted or unsubstituted C3-6 cycloalkoxy group, a substituted or unsubstituted C1-6 alkylthio group, a substituted or unsubstituted amino group, a substituted or unsubstituted C3-6 cycloalkyl group, a substituted or unsubstituted phenyl group, a substituted or unsubstituted naphthyl group, a substituted or unsubstituted three- to six-membered heterocyclyl group, or a substituted or unsubstituted three- to six-membered heterocyclyloxy group, and
$U^1$ is a hydroxy group or a halogeno group.)

**INTERNATIONAL SEARCH REPORT**

International application No.
**PCT/JP2023/046585**

**A. CLASSIFICATION OF SUBJECT MATTER**

***C07D 213/65***(2006.01)i; ***A01N 43/40***(2006.01)i; ***A01N 43/54***(2006.01)i; ***A01N 43/58***(2006.01)i; ***A01N 47/24***(2006.01)i; ***A01P 3/00***(2006.01)i; ***C07D 213/81***(2006.01)i; ***C07D 213/84***(2006.01)i; ***C07D 213/86***(2006.01)i; ***C07D 231/18***(2006.01)i; ***C07D 231/20***(2006.01)i; ***C07D 237/24***(2006.01)i; ***C07D 237/26***(2006.01)i; ***C07D 237/28***(2006.01)i; ***C07D 239/34***(2006.01)i; ***C07D 249/04***(2006.01)i; ***C07D 253/07***(2006.01)i; ***C07D 401/12***(2006.01)i; ***C07D 403/12***(2006.01)i; ***C07D 405/12***(2006.01)i; ***C07D 409/12***(2006.01)i; ***C07D 487/04***(2006.01)i

FI: C07D213/65; A01N43/40 101D; A01N43/54 B; A01N43/58 A; A01N43/58 E; A01N47/24 D; A01P3/00; C07D213/81 CSP; C07D213/84 Z; C07D213/86; C07D231/18; C07D231/20 Z; C07D237/24 CSP; C07D237/26; C07D237/28; C07D239/34; C07D249/04 506; C07D253/07; C07D401/12; C07D403/12; C07D405/12; C07D409/12; C07D487/04 144

According to International Patent Classification (IPC) or to both national classification and IPC

**B. FIELDS SEARCHED**

Minimum documentation searched (classification system followed by classification symbols)

C07D213/65; A01N43/40; A01N43/54; A01N43/58; A01N47/24; A01P3/00; C07D213/81; C07D213/84; C07D213/86; C07D231/18; C07D231/20; C07D237/24; C07D237/26; C07D237/28; C07D239/34; C07D249/04; C07D253/07; C07D401/12; C07D403/12; C07D405/12; C07D409/12; C07D487/04

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| P, X | WO 2023/049199 A1 (ZENO MANAGEMENT, INC.) 30 March 2023 (2023-03-30) paragraph [0158], etc. | 1-2 |
| P, A | | 3-9 |
| P, X | CN 116332922 A (WUHAN RENFU INNOVATION DRUG RESEARCH AND DEVELOPMENT CENTER CO., LTD.) 27 June 2023 (2023-06-27) paragraph [0147], etc. | 1-2 |
| P, A | | 3-9 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 March 2024** | **26 March 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No.
PCT/JP2023/046585

C. DOCUMENTS CONSIDERED TO BE RELEVANT

| Category* | Citation of document, with indication, where appropriate, of the relevant passages | Relevant to claim No. |
|---|---|---|
| X<br>A | WO 2004/007481 A2 (AMGEN INC.) 22 January 2004 (2004-01-22)<br>pages 353, 357, etc. | 1-2<br>3-9 |
| X<br>A | WO 2006/058730 A1 (BASF AKTIENGESELLSCHAFT) 08 June 2006 (2006-06-08)<br>page 103, etc. | 1-2<br>3-9 |
| X<br>A | WO 2021/102204 A1 (VIVACE THERAPEUTICS, INC.) 27 May 2021 (2021-05-27)<br>example 8 and later, etc. | 1-2<br>3-9 |
| X<br>A | WO 2007/081630 A2 (JANSSEN PHARMACEUTICA, N.V.) 19 July 2007 (2007-07-19)<br>page 124, etc. | 1-2<br>3-9 |
| X<br>A | WO 2009/055299 A1 (JANSSEN PHARMACEUTICA N.V.) 30 April 2009 (2009-04-30)<br>claim 11, etc. | 1-2<br>3-9 |
| X<br>A | US 8153791 B2 (XU, Guozhang) 10 April 2012 (2012-04-10)<br>column 154, etc. | 1-2<br>3-9 |
| X<br>A | WO 2011/107394 A1 (GLAXO GROUP LIMITED) 09 September 2011 (2011-09-09)<br>page 4, etc. | 1-2<br>3-9 |
| X<br>A | WO 2013/083991 A1 (IMPERIAL INNOVATIONS LIMITED) 13 June 2013 (2013-06-13)<br>page 113 and later, etc. | 1-2<br>3-9 |
| X<br>A | CN 104193675 A (ZHEJIANG UNIVERSITY OF TECHNOLOGY) 10 December 2014 (2014-12-10)<br>examples, etc. | 1-2, 6, 8<br>3-5, 7, 9 |
| X<br>A | CN 104744358 A (ZHEJIANG UNIVERSITY OF TECHNOLOGY) 01 July 2015 (2015-07-01)<br>examples, etc. | 1-2, 6, 8<br>3-5, 7, 9 |
| X<br>A | WO 01/78648 A2 (DONG WHA PHARM. IND. CO., LTD.) 25 October 2001 (2001-10-25)<br>example 22, etc. | 1-2, 7<br>3-6, 8-9 |
| X<br>A | WO 2004/056823 A1 (GLAXO GROUP LIMITED) 08 July 2004 (2004-07-08)<br>examples, tables, etc. | 1-2, 7<br>3-6, 8-9 |
| X<br>A | WO 2009/013462 A1 (UCB PHARMA S.A.) 29 January 2009 (2009-01-29)<br>pages 31-32, etc. | 1-2, 7<br>3-6, 8-9 |
| X<br>A | WO 02/089745 A2 (CYTOVIA, INC.) 14 November 2002 (2002-11-14)<br>examples, etc. | 1-2, 7, 9<br>3-6, 8 |
| X<br>A | WO 2010/059838 A2 (DECODE GENETICS EHF) 27 May 2010 (2010-05-27)<br>paragraphs [00255], [00263], etc. | 1-2, 7, 9<br>3-6, 8 |

Form PCT/ISA/210 (second sheet) (July 2022)

INTERNATIONAL SEARCH REPORT

International application No. **PCT/JP2023/046585**

C. DOCUMENTS CONSIDERED TO BE RELEVANT

| Category* | Citation of document, with indication, where appropriate, of the relevant passages | Relevant to claim No. |
|---|---|---|
| X<br>A | WO 2006/045514 A1 (APPLIED RESEARCH SYSTEMS ARS HOLDING N.V.) 04 May 2006 (2006-05-04)<br>examples 28, 55, etc. | 1-3, 7<br>4-6, 8-9 |
| X<br>A | Pharmaceutical Chemistry Journal 2011, vol. 45, pages 153-155<br>page 153, etc. | 1-2<br>3-9 |
| X<br>A | Bioorg. Med. Chem. Lett. 2008, vol. 18, pages 4896-4899<br>scheme 4, etc. | 1-2<br>3-9 |
| X<br>A | Bioorg. Med. Chem. Lett. 2010, vol. 20, pages 5803-5806<br>scheme 1, etc. | 1-2<br>3-9 |
| X<br>A | Org. Proc. Res. Dev. 2010, vol. 14, pages 1153-1161<br>scheme 4, etc. | 1-2<br>3-9 |
| X<br>A | J. Med. Chem. 2014, vol. 57, pages 2773-2788<br>schemes 3-4, etc. | 1-2<br>3-9 |
| X<br>A | Khimiko-Farmatsevticheskii Zhurnal 1998, vol. 32, pages 15-16<br>page 15, left column, etc. | 1-2<br>3-9 |
| X<br>A | Khimiko-Farmatsevticheskii Zhurnal 1991, vol. 25, pages 20-21<br>page 20, left column, etc. | 1-2<br>3-9 |
| X<br>A | Chin. J. Org. Chem. 2016, vol. 36, pages 1051-1059<br>scheme 1, etc. | 1-2, 7, 9<br>3-6, 8 |

Form PCT/ISA/210 (second sheet) (July 2022)

**INTERNATIONAL SEARCH REPORT**
**Information on patent family members**

International application No.
**PCT/JP2023/046585**

| Patent document cited in search report | Publication date (day/month/year) | Patent family member(s) | Publication date (day/month/year) |
|---|---|---|---|
| WO 2023/049199 A1 | 30 March 2023 | (Family: none) | |
| CN 116332922 A | 27 June 2023 | WO 2023/116877 A1 | |
| WO 2004/007481 A2 | 22 January 2004 | JP 2006-502118 A | |
| | | JP 2004-527499 A | |
| | | US 2003/0203922 A1 | |
| | | US 2003/0195230 A1 | |
| | | US 2006/0194848 A1 | |
| | | EP 1562933 A2 | |
| | | EP 1467721 A2 | |
| | | CN 1538836 A | |
| | | KR 10-2003-0078068 A | |
| WO 2006/058730 A1 | 08 June 2006 | JP 2008-521856 A | |
| | | US 2007/0265231 A1 | |
| | | EP 1819224 A1 | |
| | | KR 10-2007-0090898 A | |
| | | CN 101068468 A | |
| WO 2021/102204 A1 | 27 May 2021 | US 2023/0106583 A1 | |
| | | EP 4061364 A4 | |
| | | CN 115279368 A | |
| WO 2007/081630 A2 | 19 July 2007 | US 2007/0270425 A1 | |
| | | US 2008/0249304 A1 | |
| | | US 2012/0157412 A1 | |
| | | WO 2008/073519 A1 | |
| WO 2009/055299 A1 | 30 April 2009 | US 2009/0111810 A1 | |
| US 8153791 B2 | 10 April 2012 | US 2007/0270425 A1 | |
| | | US 2008/0249304 A1 | |
| | | US 2012/0157412 A1 | |
| | | WO 2008/073519 A1 | |
| | | WO 2007/081630 A2 | |
| WO 2011/107394 A1 | 09 September 2011 | (Family: none) | |
| WO 2013/083991 A1 | 13 June 2013 | (Family: none) | |
| CN 104193675 A | 10 December 2014 | (Family: none) | |
| CN 104744358 A | 01 July 2015 | (Family: none) | |
| WO 01/78648 A2 | 25 October 2001 | US 2003/0092709 A1 | |
| | | KR 10-2001-0096176 A | |
| WO 2004/056823 A1 | 08 July 2004 | JP 2007-514704 A | |
| | | JP 2006-513258 A | |
| | | JP 2006-503108 A | |
| | | US 2007/0111995 A1 | |
| | | US 2008/0132536 A1 | |
| | | US 2006/0252790 A1 | |
| | | US 2006/0089375 A1 | |
| | | US 2008/0175914 A1 | |
| | | WO 2005/058892 A1 | |
| | | WO 2004/024728 A2 | |
| | | EP 1581532 A1 | |
| | | EP 1737857 A1 | |
| | | EP 1539753 A2 | |

Form PCT/ISA/210 (patent family annex) (July 2022)

**INTERNATIONAL SEARCH REPORT**
**Information on patent family members**

International application No.
**PCT/JP2023/046585**

| Patent document cited in search report | Publication date (day/month/year) | Patent family member(s) | Publication date (day/month/year) |
|---|---|---|---|
| | | CN 1751042 A | |
| | | CN 1914205 A | |
| | | KR 10-2005-0088214 A | |
| | | KR 10-2005-0052501 A | |
| WO 2009/013462 A1 | 29 January 2009 | US 2010/0179124 A1 | |
| | | EP 2183253 A1 | |
| WO 02/089745 A2 | 14 November 2002 | US 2003/0013743 A1 | |
| | | US 2004/0186078 A1 | |
| | | EP 1392219 A4 | |
| WO 2010/059838 A2 | 27 May 2010 | (Family: none) | |
| WO 2006/045514 A1 | 04 May 2006 | JP 2008-517024 A | |
| | | US 2009/0093462 A1 | |
| | | US 2011/0224192 A1 | |
| | | US 2012/0295889 A1 | |
| | | US 2014/0051686 A1 | |
| | | EP 1802579 A1 | |
| | | CN 101065358 A | |
| | | KR 10-2007-0067727 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2022212728 A
- JP 2023069619 A
- JP 2023131664 A
- JP 2022508277 W
- JP 2022514651 W

### Non-patent literature cited in the description

- *CHEMICAL ABSTRACTS*, 943137-49-3